# EUROPEAN PATENT SPECIFICATION

(11) **EP 2 225 211 B1**
(45) Date of publication and mention of the grant of the patent: **02.10.2013**
(21) Application number: 08867997.2
(22) Date of filing: 19.12.2008
(51) Int. Cl.: C07D 237/16, C07D 401/14, C07D 403/04, C07D 403/10, C07D 405/14, C07D 409/14, C07D 413/04, C07D 417/04, C07D 417/14, A61K 31/501, A01N 43/58

(54) **HERBICIDAL PYRIDAZINONE DERIVATIVES**
HERBIZIDE PYRIDAZINONDERIVATE
DÉRIVÉS HERBICIDES DE PYRIDAZINONE

(30) Priority: 21.12.2007 US 8861
(43) Date of publication of application: 08.09.2010
(73) Proprietor: E. I. du Pont de Nemours and Company, Wilmington, DE 19898 (US)
(72) Inventor: STEVENSON, Thomas, Martin, Newark DE 19702 (US); MARSHALL, Eric, Allen, Rising Sun MD 21911 (US); TAGGI, Andrew, E., Newark DE 19711 (US)
(74) Representative: Beacham, Annabel Rose
(86) International application number: PCT/US2008/087577
(87) International publication number: WO 2009/086041

(56) References cited:
- WO-A-2007/119434

## Description

### FIELD OF THE INVENTION

This invention relates to certain pyridazinone derivatives, their *N*-oxides, salts and compositions, and methods of their use for controlling undesirable vegetation.

### BACKGROUND OF THE INVENTION

The control of undesired vegetation is extremely important in achieving high crop efficiency. Achievement of selective control of the growth of weeds especially in such useful crops as rice, soybean, sugar beet, maize, potato, wheat, barley, tomato and plantation crops, among others, is very desirable. Unchecked weed growth in such useful crops can cause significant reduction in productivity and thereby result in increased costs to the consumer. The control of undesired vegetation in noncrop areas is also important. Many products are commercially available for these purposes, but the need continues for new compounds that are more effective, less costly, less toxic, environmentally safer or have different sites of action.

WO 2007/119434 discloses pyridazinone compounds useful as herbicides.

### SUMMARY OF THE INVENTION

This invention is directed to compounds of Formula 1 (including all geometric and stereoisomers), N-oxides, and salts thereof, agricultural compositions containing them and their use as herbicides: 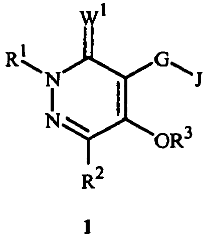 wherein
- R¹: is H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, tetrahydropyranyl, -C(=W⁶)R⁴, -C(=W²)W³R⁵, -S(=O)₂R⁶, -P(=W⁴)R⁷R⁸ or -C(=W⁵)NR⁹R¹⁰;
- R²: is H, halogen, cyano, -C(=O)OH, -C(=O)NH₂, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ haloalkoxy, C₁-C₆ alkylthio, C₁-C₆ haloalkylthio, nitro, C₃-C₆ cycloalkoxy or C₄-C₈ cycloalkylalkoxy;
- R³: is H, -C(=W⁶)R⁴, -C(=W²)W³R⁵, -S(=O)₂R⁶, -P(=W⁴)R⁷R⁸ or -C(=W⁵)NR⁹R¹⁰;
- G: is a phenyl ring or a 5- or 6-membered heteroaromatic ring, each ring optionally substituted with R^{x} on nitrogen ring members and optionally substituted with up to 4 substituents selected from R^{w} on carbon ring members;
- J: is a phenyl ring or a 5- or 6-membered heteroaromatic ring, each ring optionally substituted with up to 5 substituents independently selected from R^{u};
- each R⁴: is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₃-C₁₀ diallcylaminoalkyl, C₂-C₈ haloalkylaminoalkyl or C₄-C₁₀ cycloalkylaminoalkyl, naphthalenyl or -(CR¹¹R¹²)ₙG^{A};
- each R⁵: is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₅-C₁₂ alkylcycloalkylalkyl, C₃-C₁₀ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₃-C₁₀ dialkylaminoalkyl, C₂-C₈ haloalkylaminoalkyl, C₄-C₁₀ cycloalkylaminoalkyl, naphthalenyl or -(CR¹¹R¹²)ₙG^{A};
- each R⁶: is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₄-C₁₀ halocycloalkylalkyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₃-C₁₀ dialkylaminoalkyl, C₂-C₈ haloalkylaminoalkyl, C₄-C₁₀ cycloalkylaminoalkyl, C₁-C₆ alkylamino, C₂-C₈ dialkylamino, C₂-C₆ haloalkylamino, C₂-C₈ halodialkylamino, C₃-C₈ cycloalkylamino, C₂-C₈ alkylcarbonylamino, C₂-C₈ haloalkylcarbonylamino, C₁-C₆ alkylsulfonylamino, C₁-C₆ haloalkylsulfonylamino, naphthalenyl or-(CR¹¹R¹²)ₙG^{A};
- each R⁷ and R⁸: is independently C₁-C₆ alkyl, C₁-C₆ alkoxy, C₁-C₆ alkylamino, C₂-C₈ dialkylamino, C₂-C₆ haloalkylamino, C₂-C₈ halodialkylamino, C₃-C₈ cycloalkylamino, naphthalenyl or -(CR¹¹R¹²)ₙG^{A};
- each R⁹: is independently H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₄-C₁₀ dialkylaminoalkyl, naphthalenyl or -(CR¹¹R¹²)ₙG^{A};
- each R¹⁰: is independently H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl or C₄-C₁₀ cycloalkylalkyl; or
- R⁹ and R¹⁰: are taken together with the nitrogen to which they are attached to form a 3-to 7-membered heterocyclic ring containing, in addition to the linking nitrogen, ring members selected from carbon and optionally O, S and NR¹³, the carbon ring members optionally in the form of C(=O), and the ring optionally substituted on carbon ring members with up to 4 substituents independently selected from the group consisting of halogen, -CN, C₁-C₃ alkyl and C₁-C₃ alkoxy;
- each R¹¹ and R¹²: is independently H or C₁-C₃ alkyl;
- each R¹³: is independently H or C₁-C₃ alkyl;
- each G^{A}: is independently a phenyl ring or a 5- or 6-membered heteroaromatic ring, each ring optionally substituted with up to 5 substituents independently selected from R^{u}; or a naphthalenyl ring system optionally substituted with up to 5 substituents independently selected from R^{u};
- each R^{u}: is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₄-C₁₀ alkylcycloalkyl, C₄-C₁₀ cycloalkylalkyl, C₄-C₁₀ halocycloalkylalkyl, C₅-C₁₂ alkylcycloalkylalkyl, C₂-C₈ alkylcarbonyl, C₂-C₈ haloalkylcarbonyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₁-C₆ alkylaminosulfonyl, C₂-C₈ dialkylaminosulfonyl, C₃-C₁₀ trialkylsilyl, C₁-C₆ alkylamino, C₂-C₈ dialkylamino, C₂-C₈ alkylcarbonylamino, C₁-C₆ alkylsulfonylamino, phenyl, pyridinyl or thienyl;
- each R^{w}: is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₄-C₁₀ alkylcycloalkyl, C₄-C₁₀ cycloalkylalkyl, C₄-C₁₀ halocycloalkylalkyl, C₅-C₁₂ alkylcycloalkylalkyl, C₂-C₈ alkylcarbonyl, C₂-C₈ haloalkylcarbonyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₁-C₆ alkylaminosulfonyl, C₂-C₈ dialkylaminosulfonyl, C₃-C₁₀ trialkylsilyle, C₁-C₆ alkylamino, C₂-C₈ dialkylamino, C₂-C₈ alkylcarbonylamino, C₁-C₆ alkylsulfonylamino, naphthalenyl, -O(CR¹¹R¹²)ₙG^{A} or -(CR¹¹R¹²)ₙG^{A};
- each R^{x}: is independently H, C₁-C₃ alkyl or C₃-C₇ cycloalkyl;
- each W¹, W², W³, W⁴, W⁵ and W⁶: is independently O or S; and
- each n: is independently an integer selected from 0 through 3.

More particularly, this invention pertains to a compound of Formula **1** (including all geometric and stereoisomers), an *N*-oxide or a salt thereof. This invention also relates to a herbicidal composition comprising a herbicidally effective amount of a compound of Formula **1** and at least one component selected from the group consisting of surfactants, solid diluents and liquid diluents. This invention further relates to a method for controlling the growth of undesired vegetation comprising contacting the vegetation or its environment with a herbicidally effective amount of a compound of Formula **1** (e.g., as a composition described herein).

### DETAILS OF THE INVENTION

As used herein, the terms "comprises", "comprising", "includes", "including", "has", "having", "contains" or "containing" or any other variation thereof, are intended to cover a non-exclusive inclusion. For example, a composition, process, method, article, or apparatus that comprises a list of elements is not necessarily limited to only those elements but may include other elements not expressly listed or inherent to such composition, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As referred to herein, the term "broadleaf' used either alone or in words such as "broadleaf weed" means dicot or dicotyledon, a term used to describe a group of angiosperms characterized by embryos having two cotyledons.

In the above recitations, the term "alkyl", used either alone or in compound words such as "alkylthio" or "haloalkyl" includes straight-chain or branched alkyl, such as, methyl, ethyl, *n*-propyl, *i*-propyl, or the different butyl, pentyl or hexyl isomers. "Alkenyl" includes straight-chain or branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as ethynyl, 1-propynyl, 2-propynyl and the different butynyl, pentynyl and hexynyl isomers. "Alkynyl" can also include moieties comprised of multiple triple bonds such as 2,5-hexadiynyl.

"Alkoxy" includes, for example, methoxy, ethoxy, n-propyloxy, isopropyloxy and the different butoxy, pentoxy and hexyloxy isomers. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. "Alkoxyalkoxy" denotes alkoxy substitution on alkoxy. "Alkoxyalkoxyalkyl" denotes at least one straight-chain or branched alkoxy moieties bonded to a straight-chain or branched alkoxy moieties of alkoxyalkyl moiety. Examples of "alkoxyalkoxyalkyl" include CH₃OCH₂OCH₂-, CH₃CH₂O(CH₃)CHOCH₂- and (CH₃O)₂CHOCH₂-. "Alkylthio" includes branched or straight-chain alkylthio moieties such as methylthio, ethylthio, and the different propylthio, butylthio, pentylthio and hexylthio isomers. "Alkylsulfinyl" includes both enantiomers of an alkylsulfinyl group. Examples of "alkylsulfinyl" include CH₃S(O)-, CH₃CH₂S(O)-, CH₃CH₂CH₂S(O)-, (CH₃)₂CHS(O)- and the different butylsulfinyl, pentylsulfinyl and hexylsulfinyl isomers. Examples of "alkylsulfonyl" include CH₃S(O)₂-, CH₃CH₂S(O)₂-, CH₃CH₂CH₂S(O)₂-, (CH₃)₂CHS(O)₂-, and the different butylsulfonyl, pentylsulfonyl and hexylsulfonyl isomers. "Alkylthioalkyl" denotes alkylthio substitution on alkyl. Examples of "alkylthioalkyl" include CH₃SCH₂, CH₃SCH₂CH₂, CH₃CH₂SCH₂, CH₃CH₂CH₂CH₂SCH₂ and CH₃CH₂SCH₂CH₂. "Alkylamino", "dialkylamino", and the like, are defined analogously to the above examples.

"Cycloalkyl" includes, for example, cyclopropyl, cyclobutyl, cyclopentyl and cyclohexyl. The term "alkylcycloalkyl" denotes alkyl substitution on a cycloalkyl moiety and includes, for example, ethylcyclopropyl, i-propylcyclobutyl, 3-methylcyclopentyl and 4-methylcyclohexyl. The term "cycloalkylalkyl" denotes cycloalkyl substitution on an alkyl moiety. Examples of "cycloalkylalkyl" include cyclopropylmethyl, cyclopentylethyl, and other cycloalkyl moieties bonded to straight-chain or branched alkyl groups. The term "cycloalkylcycloalkyl" denotes a cycloalkyl group substituted with other cycloalkyl group. Examples of "cycloalkylcycloalkyl" include 2-cyclopropylcyclopropyl and 3-cyclopropylcyclopentyl. The term "halocycloalkylalkyl" denotes halogen substitution on the cycloalkyl moiety, alkyl moiety or both of the cycloalkylalkyl moieties. Examples of "halocycloalkylalkyl" include 2-chlorocyclopropylmethyl, cyclopentyl-1-chloroethyl, and 3-chlorocyclopentyl-1-chloroethyl. The term "cycloalkoxy" denotes cycloalkyl linked through an oxygen atom such as cyclopentyloxy and cyclohexyloxy. "Cycloalkylalkoxy" denotes cycloalkylalkyl linked through an oxygen atom attached to the alkyl chain. Examples of "cycloalkylalkoxy" include cyclopropylmethoxy, cyclopentylethoxy, and other cycloalkyl moieties bonded to straight-chain or branched alkoxy groups. "Cycloalkenyl" includes groups such as cyclopentenyl and cyclohexenyl as well as groups with more than one double bond such as 1,3- and 1,4-cyclohexadienyl.

The term "tetrahydropyran" denotes a six-membered ring with ring members consisting of 5 carbon atoms and one oxygen atom. The point of attachment of the tetrahydropyran ring to the remainder of the compound of Formula 1 is at any one of the 5 carbon atoms. The term "thien" refers to a 5-membered aromatic ring containing one sulfur atom and four carbon atoms.

The term "halogen", either alone or in compound words such as "haloalkyl", or when used in descriptions such as "alkyl substituted with halogen" includes fluorine, chlorine, bromine or iodine. Further, when used in compound words such as "haloalkyl", or when used in descriptions such as "alkyl substituted with halogen" said alkyl may be partially or fully substituted with halogen atoms which may be the same or different. Examples of "haloalkyl" or "alkyl substituted with halogen" include F₃C-, ClCH₂-, CF₃CH₂- and CF₃CCl₂-. The terms "halocycloalkyl", "haloalkoxy", "haloalkylthio", "haloalkenyl", "haloalkynyl", and the like, are defined analogously to the term "haloalkyl". Examples of "haloalkoxy" include CF₃O-, CCl₃CH₂O-, HCF₂CH₂CH₂O- and CF₃CH₂O-. Examples of "haloalkylthio" include CCl₃S-, CF₃S-, CCl₃CH₂S- and ClCH₂CH₂CH₂S-. Examples of "haloalkylsulfinyl" include CF₃S(O)-, CCl₃S(O)-, CF₃CH₂S(O)- and CF₃CF₂S(O)-. Examples of "haloalkylsulfonyl" include CF₃S(O)₂-, CCl₃S(O)₂-, CF₃CH₂S(O)₂- and CF₃CF₂S(O)₂-. Examples of "haloalkenyl" include (Cl)₂C=CHCH₂- and CF₃CH₂CH=CHCH₂-. Examples of "haloalkynyl" include HC≡CCHCl-, CF₃C≡C-, CCl₃C≡C- and FCH₂C≡CCH₂-. Examples of "haloalkoxyalkoxy" include CF₃OCH₂O-, ClCH₂CH₂OCH₂CH₂O-, Cl₃CCH₂OCH₂O- as well as branched alkyl derivatives. "Haloalkylaminoalkyl" denotes a halogen group substituted with an alkylaminoalkyle group. "Haloalkylaminoalkyl" includes a halogen group attached to any alkyl groups as well as nitrogen. Examples of "haloalkylaminoalkyl" include CH₃NHCH₂CHCl-, (CH₃)CHClCH(CH₃)NHCH₂- and CH₃NClCH(CH₃)-. The term "halodialkylamino" denotes at least one halogen group substituted on any alkyl moiety of the dialkylamino, group. Examples of "halodialkylamino" include CF₃(CH₃)N-, (CF₃)₂N- and CH₂Cl(CH₃)N-. "Cycloalkylamino" means the amino nitrogen atom is attached to a cycloalkyl radical and a hydrogen atom and includes groups such as cyclopropylamino, cyclobutylamino, cyclopentylamino and cyclohexylamino.

"Alkylcarbonyl" denotes a straight-chain or branched alkyl moieties bonded to a C(=O) moiety. Examples of "alkylcarbonyl" include CH₃C(=O)-, CH₃CH₂CH₂C(=O)- and (CH₃)₂CHC(=O)-. Examples of "alkoxycarbonyl" include CH₃OC(=O)-, CH₃CH₂OC(=O)-, CH₃CH₂CH₂OC(=O)-, (CH₃)₂CHOC(=O)- and the different butoxy- or pentoxycarbonyl isomers. The term "cycloalkylalkoxycarbonyl" denotes a cycloalkylalkyl moiety bonded to an oxygen atom of an oxycarbonyl moiety. Examples of "cycloalkylalkoxycarbonyl" include cyclopropyl-CH₂OC(=O)-, cyclopropyl-CH(CH₃)OC(=O)- and cyclopentyl-CH₂OC(=O)-.

The total number of carbon atoms in a substituent group is indicated by the "Cᵢ-Cⱼ" prefix where i and j are numbers from 1 to 14. For example, C₁-C₄ alkylsulfonyl designates methylsulfonyl through butylsulfonyl; C₂ alkoxyalkyl designates CH₃OCH₂-; C₃ alkoxyalkyl designates, for example, CH₃CH(OCH₃)-, CH₃OCH₂CH₂- or CH₃CH₂OCH₂-; and C₄ alkoxyalkyl designates the various isomers of an alkyl group substituted with an alkoxy group containing a total of four carbon atoms, examples including CH₃CH₂CH₂OCH₂- and CH₃CH₂OCH₂CH₂-.

The term "optionally substituted" means unsubstituted or substituted. Therefore an optionally substituted group (i.e. radical) is unsubstituted or has at least 1 non-hydrogen substituent. When an optionally substituted group is defined without specifying the optional substituents, the group is unsubstituted or has at least one non-hydrogen substituent that does not extinguish the biological activity possessed by the unsubstituted analog. When a list of possible substituents is specified, the optional substituents are independently selected from the list. Unless a particular limit is recited, a group can be substituted with as many optional substituents as can be accommodated by replacing a hydrogen atom with a non-hydrogen substituent on any available carbon or nitrogen atom in the group. Furthermore, each substitution is independent of the other. When the term "optionally substituted" is accompanied by a limit such as for the groups listed for J, the number of optional substituents cannot exceed the limit even if further positions for substitution are available. Therefore, for example, the phrase "optionally substituted with up to 5 substituents" means no substituent may be present, 1 substituent may be present, or up to 5 substituents may be present if accommodated by the number of positions available for substitution.

When the subscript indicates a range, e.g. (R)ᵢ₋ⱼ, then the number of substituents may be selected from the integers between i and j inclusive. When a group contains a substituent which can be hydrogen, for example R¹, R², R³, R⁹, R¹⁰, R¹¹, R¹² or R¹³, when this substituent is taken as hydrogen, it is recognized that this is equivalent to said group being unsubstituted. When a linking group is shown to be optionally present, for example (CR¹¹R¹²)ₙ wherein n may be 0, then the linking group may be a direct bond (i.e. when n is 0). When one or more positions on a group are said to be "not substituted" or "unsubstituted", then hydrogen atoms are attached to take up any free valency.

"Aromatic" indicates that each of the ring atoms is essentially in the same plane and has a p-orbital perpendicular to the ring plane, and that (4n + 2) π electrons, where n is a positive integer, are associated with the ring to comply with Hückel's rule. When a fully unsaturated carbocyclic ring satisfies Hückel's rule, then said ring is also called an "aromatic ring". When a fully unsaturated heterocyclic ring satisfies Hückel's rule, then said ring is also called a "heteroaromatic ring". Unless otherwise indicated, heterocyclic rings can be attached through any available carbon or nitrogen by replacement of a hydrogen on said carbon or nitrogen.

When J, G, or G^{A} is a 5- or 6-membered nitrogen-containing heteroaromatic ring, it can be attached to the remainder of Formula 1 through any available carbon or nitrogen ring atom, unless otherwise described. As noted above, each J and G^{A} can be (among others) independently a phenyl optionally substituted with one or more substituents selected from a group of substituents as defined in the Summary of Invention. An example of phenyl optionally substituted with one to five substituents is the ring illustrated as U-1 in Exhibit 1, wherein R^{v} is R^{u} as defined in the Summary of the Invention for J and G^{A} and r is an integer from 0 to 5.

As noted above, J and G^{A} can be (among others) a 5- or 6-membered heteroaromatic ring, substituted or optionally substituted with one or more substituents selected from a group of substituents as defined in the Summary of the Invention. Examples of a 5- or 6-membered heteroaromatic ring, substituted or optionally substituted with one or more substituents include the rings U-2 through U-61 illustrated in Exhibit 1 wherein R^{v} is any substituent as defined in the Summary of the Invention for J and G^{A} (i.e. R^{u}) and r is an integer from 0 to 4, limited by the number of available positions on each U group. In U-1 through U-61, the radical R^{v} corresponds to R^{u} in the Summary of the Invention and all Embodiments, Examples, Tables and Schemes. The subscript "r" represents the number of substituents that can be present on

U-1 through U-61. As U-29, U-30, U-36, U-37, U-38, U-39, U-40, U-41, U-42 and U-43 have only one available position, for these U groups r is limited to the integers 0 or 1, and r being 0 means that the U group is unsubstituted and a hydrogen is present at the position indicated by (R^{v})ᵣ.

### Exhibit 1

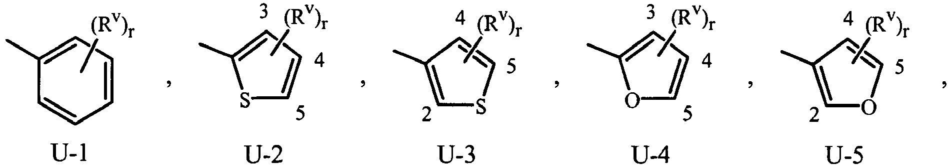

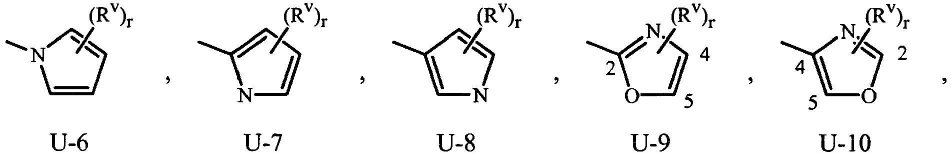

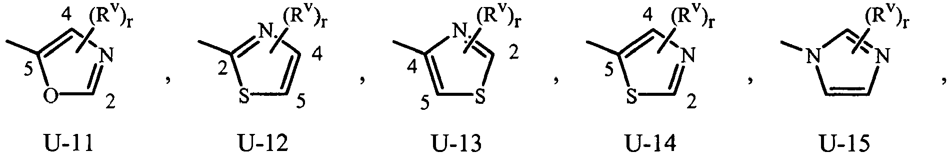

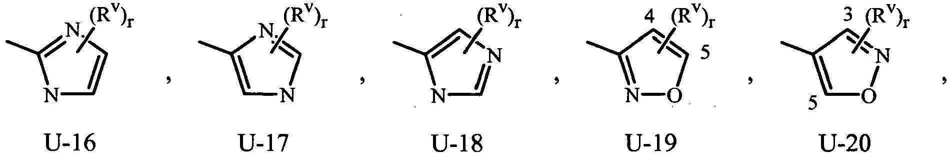

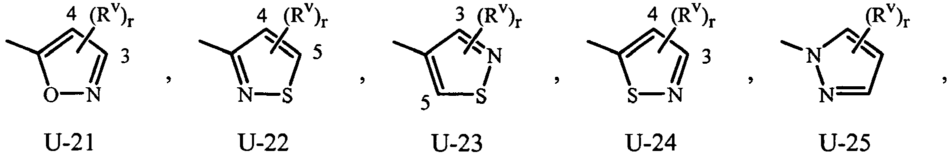

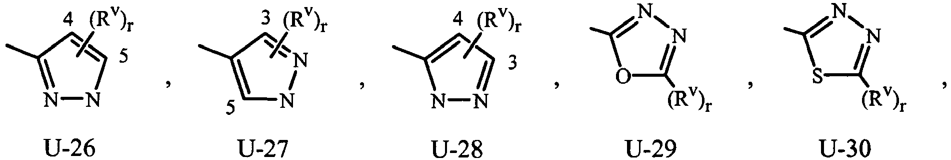

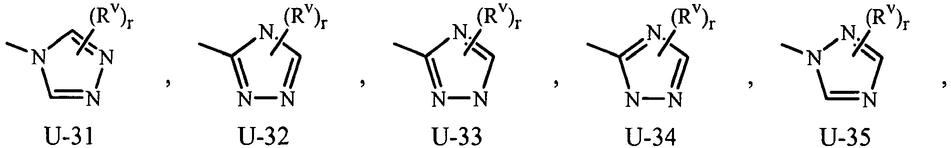

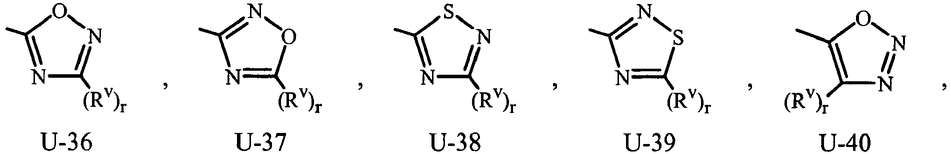

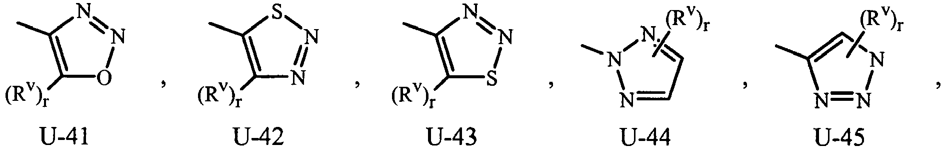

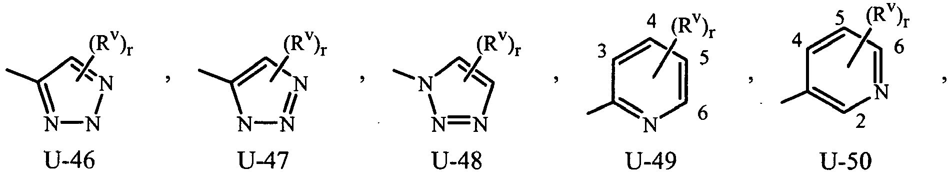

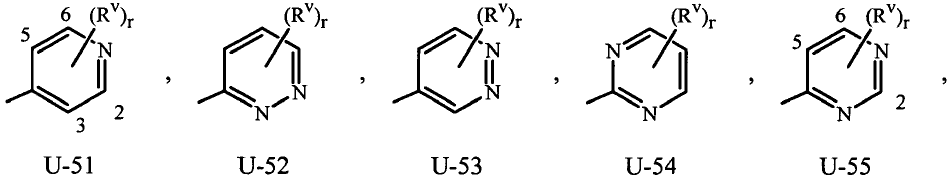

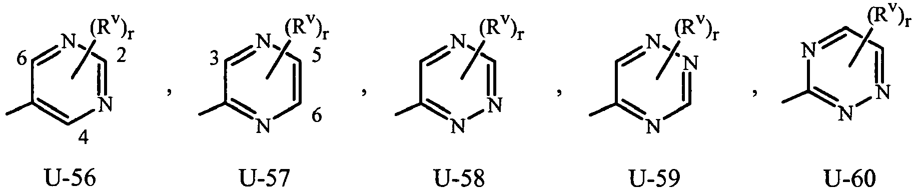

and

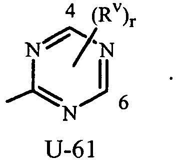

Although R^{v} (i.e R^{u}) groups are shown in the structures U-1 through U-61, it is noted that they do not need to be present since they are optional substituents. The nitrogen atoms that require substitution to fill their valence are substituted with H or R^{v} (i.e R^{u}). Note that when the attachment point between (R^{v})ᵣ (i.e (R^{u})ₚ) and the U group is illustrated as floating, (R^{v})ᵣ (i.e (R^{u})ₚ) can be attached to any available carbon atom or nitrogen atom of the U group. Note that when the attachment point on the U group is illustrated as floating, the U group can be attached to the remainder of Formula **1** through any available carbon or nitrogen of the U group by replacement of a hydrogen atom. Note that some U groups can only be substituted with less than 4 R^{v} groups (e.g., U-2 through U-5, U-7 through U-48, and U-52 through U-61).

A wide variety of synthetic methods are known in the art to enable preparation of aromatic and nonaromatic heterocyclic rings and ring systems; for extensive reviews see the eight volume set of Comprehensive Heterocyclic Chemistry, A. R. Katritzky and C. W. Rees editors-in-chief, Pergamon Press, Oxford, 1984 and the twelve volume set of Comprehensive Heterocyclic Chemistry II, A. R. Katritzky, C. W. Rees and E. F. V. Scriven editors-in-chief, Pergamon Press, Oxford, 1996.

Compounds of this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, individual stereoisomers or as an optically active form.

One skilled in the art will appreciate that not all nitrogen-containing heterocycles can form *N*-oxides since the nitrogen requires an available lone pair for oxidation to the oxide; one skilled in the art will recognize those nitrogen containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethyldioxirane. These methods for the preparation of *N*-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

One skilled in the art recognizes that because in the environment and under physiological conditions salts of chemical compounds are in equilibrium with their corresponding nonsalt forms, salts share the biological utility of the nonsalt forms. Thus a wide variety of salts of the compounds of Formula **1** are useful for control of undesired vegetation (i.e. are agriculturally suitable). The salts of the compounds of Formula **1** include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. When a compound of Formula **1** contains an acidic moiety such as a carboxylic acid or enol (e.g., when R³ is H), salts also include those formed with organic or inorganic bases such as pyridine, triethylamine or ammonia, or amides, hydrides, hydroxides or carbonates of sodium, potassium, lithium, calcium, magnesium or barium. Accordingly, the present invention comprises compounds selected from Formula **1**, *N*-oxides and agriculturally suitable salts thereof.

One skilled in the art appreciates compounds of Formula **1**, *N*-oxides and salts thereof typically exist in more than one form, and Formula **1**, *N*-oxides and salts thereof thus include all crystalline and non-crystalline forms of the compounds they represent. Non-crystalline forms include embodiments which are solids such as waxes and gums as well as embodiments which are liquids such as solutions and melts. Crystalline forms include embodiments which represent essentially a single crystal type and embodiments which represent a mixture of polymorphs (i.e. different crystalline types). The term "polymorph" refers to a particular crystalline form of a chemical compound that can crystallize in different crystalline forms, these forms having different arrangements and/or conformations of the molecules in the crystal lattice. Although polymorphs can have the same chemical composition, they can also differ in composition due the presence or absence of co-crystallized water or other molecules, which can be weakly or strongly bound in the lattice. Polymorphs can differ in such chemical, physical and biological properties as crystal shape, density, hardness, color, chemical stability, melting point, hygroscopicity, suspensibility, dissolution rate and biological availability. One skilled in the art will appreciate that a polymorph of a compound of Formula **1** or *N*-oxides or salts thereof can exhibit beneficial effects (e.g., suitability for preparation of useful formulations, improved biological performance) relative to another polymorph or a mixture of polymorphs of the same compound of Formula **1** or *N*-oxides or salts thereof. Preparation and isolation of a particular polymorph of a compound of Formula **1** or *N*-oxides or salts thereof can be achieved by methods known to those skilled in the art including, for example, crystallization using selected solvents and temperatures.

Embodiments of the present invention as described in the Summary of the Invention include those described below. In the following Embodiments, Formula **1** includes *N*-oxides and salts thereof, and reference to "a compound of Formula **1**" includes the definitions of substituents specified in the Summary of the Invention unless further defined in the Embodiments.
Embodiment 1. A compound of Formula **1** wherein R¹ is H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl or C₂-C₈ alkylsulfonylalkyl.
Embodiment 1a. A compound of Embodiment 1 wherein R¹ is H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₂-C₈ alkoxyalkyl, C₄-C₁₀ cycloalkoxyalkyl or C₃-C₁₀ alkoxyalkoxyalkyl.
Embodiment 2. A compound of Embodiment 1a wherein R¹ is H, C₁-C₆ alkyl, C₁-C₆ haloalkyl or C₃-C₈ cycloalkyl.
Embodiment 3. A compound of Embodiment 2 wherein R¹ is H or C₁-C₆ alkyl.
Embodiment 4. A compound of Embodiment 3 wherein R¹ is CH₃.
Embodiment 4a. A compound of Formula **1** wherein R¹ is C₄-C₉ cycloalkylcarbonyl.
Embodiment 4b. A compound of Embodiment 4a wherein R¹ is cyclopropylcarbonyl.
Embodiment 4c. A compound of Formula **1** wherein R¹ is tetrahydropyranyl.
Embodiment 5. A compound of Formula **1 or** any one of Embodiments 1 through 4c wherein R² is H, halogen, cyano, -C(=O)OH, -C(=O)NH₂, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ haloalkoxy, C₁-C₆ alkylthio or C₁-C₆ haloalkylthio.
Embodiment 5a. A compound of Embodiment 5 wherein R² is H, halogen, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₂-C₈ alkoxycarbonyl, C₁-C₆ alkoxy or C₁-C₆ haloalkoxy.
Embodiment 5b. A compound of Embodiment 5 wherein R² is H, halogen, C₁-C₆ alkyl, C₁-C₆ haloalkyl, C₃-C₈ cycloalkyl, C₂-C₈ alkoxyalkyl or C₁-C₄ alkoxy.
Embodiment 5c. A compound of Embodiment 5b wherein R² is H, halogen, C₁-C₆ alkyl or C₁-C₄ alkoxy.
Embodiment 5d. A compound of Embodiment 5c wherein R² is H, Cl, CH₃, Et or OMe.
Embodiment 6. A compound of Embodiment 5 wherein R² is H, halogen, C₁-C₆ alkyl, C₁-C₆ haloalkyl, C₃-C₈ cycloalkyl or C₂-C₈ alkoxyalkyl.
Embodiment 7. A compound of Embodiment 6 wherein R² is H, halogen or C₁-C₆ alkyl.
Embodiment 8. A compound of Embodiment 7 wherein R² is H, Cl, CH₃ or Et (i.e. CH₂CH₃).
Embodiment 9. A compound of Embodiment 5d or 8 wherein R² is H.
Embodiment 10. A compound of Embodiment 5d or 8 wherein R² is Cl.
Embodiment 11. A compound of Embodiment 5d or 8 wherein R² is CH₃.
Embodiment 12. A compound of Formula **1** or any one of Embodiments 1 through 11
   wherein R³ is H, -C(=W⁶)R⁴, -C(=W²)W³R⁵, -S(=O)₂R⁶ or -C(=W⁵)NR⁹R¹⁰.
Embodiment 12a. A compound of Embodiment 12 wherein R³ is H, -CO₂-*i*-Pr (i.e. -CO₂CH(CH₃)₂), -CO₂-*i*-Bu (i.e. -CO₂CH₂CH(CH₃)₂), CO-(2-chlorophenyl), or -CO-*c*-Pr (i.e. -C(O)-cyclopropyl).
Embodiment 12b. A compound of Embodiment 12 wherein R³ is H, CO₂-*i*-Pr or CO-*t*-Bu.
Embodiment 12c. A compound of Embodiment 12b wherein R³ is H or CO₂-*i*-Pr.
Embodiment 13. A compound of Embodiment 12 wherein R³ is H.
Embodiment 14. A compound of Embodiment 12 wherein R³ is -C(=W⁶)R⁴.
Embodiment 15. A compound of Embodiment 12 wherein R³ is -C(=W²)W³R⁵.
Embodiment 16. A compound of Embodiment 12 wherein R³ is -S(=O)₂R⁶.
Embodiment 17. A compound of Embodiment 12 wherein R³ is -C(=W⁵)NR⁹R¹⁰.
Embodiment 18. A compound of Formula **1** or any one of Embodiments 1 through 17
   wherein the compound is in the form of a salt.
Embodiment 18a. A compound of Formula **1** or any one of Embodiments 1 through 18 wherein each W⁶ is independently O.
Embodiment 18b. A compound of Formula **1** or any one of Embodiments 1 through 18 wherein each W⁶ is independently S.
Embodiment 19. A compound of Formula **1** or any one of Embodiments 1 through 18b wherein each R⁴ is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, naphthalenyl or -(CR¹¹R¹²)ₙG^{A}.
Embodiment 20. A compound of Embodiment 19 wherein each R⁴ is independently C₁-C₆ alkyl, C₁-C₆ haloalkyl, C₃-C₈ cycloalkyl, naphthalenyl or -(CR¹¹R¹²)ₙG^{A}.
Embodiment 21. A compound of Embodiment 20 wherein each R⁴ is independently C₁-C₆ alkyl.
Embodiment 22. A compound of Embodiment 21 wherein each R⁴ is *t*-Bu (i.e. C(CH₃)₃).
Embodiment 23. A compound of Formula **1** or any one of Embodiments 1 through 22 wherein each W² is O.
Embodiment 24. A compound of Formula **1** or any one of Embodiments 1 through 22 wherein each W² is S.
Embodiment 25. A compound of Formula **1** or any one of Embodiments 1 through 24 wherein each W³ is O.
Embodiment 26. A compound of Formula **1** or any one of Embodiments 1 through 24 wherein each W³ is S.
Embodiment 27. A compound of Formula **1** or any one of Embodiments 1 through 26 wherein each R⁵ is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloallcyl, C₄-C₁₀ alkylcycloalkyl, C₄-C₁₀ cycloalkylallcyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, naphthalenyl or -(CR¹¹R¹²)ₙG^{A}.
Embodiment 28. A compound of Embodiment 27 wherein each R⁵ is independently C₁-C₆ alkyl, C₁-C₆ haloalkyl, C₃-C₈ cycloalkyl, naphthalenyl or -(CR¹¹R¹²)ₙG^{A}.
Embodiment 29. A compound of Embodiment 28 wherein each R⁵ is independently C₁-C₆ alkyl.
Embodiment 30. A compound of Embodiment 29 wherein each R⁵ is independently CH₃ or *i-*Pr (i.e. CH(CH₃)₂.
Embodiment 31. A compound of Formula **1** or any one of Embodiments 1 through 30 wherein each R⁶ is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₂-C₈ alkoxyalkyl, C₄-C₁₀ cycloalkoxyalkyl, C₃-C₁₀ alkoxyalkoxyalkyl, C₂-C₈ alkylthioalkyl, C₁-C₆ alkylamino, C₂-C₈ dialkylamino, C₂-C₆ haloalkylamino, C₂-C₈ halodialkylamino, C₃-C₈ cycloalkylamino, C₂-C₈ alkylcarbonylamino, C₂-C₈ haloalkylcarbonylamino, naphthalenyl or -(CR¹¹R¹²)ₙG^{A}.
Embodiment 32. A compound of Embodiment 31 wherein each R⁶ is independently C₁-C₆ alkyl, C₁-C₆ haloalkyl, C₃-C₈ cycloalkyl, C₁-C₆ alkylamino, C₂-C₈ dialkylamino, naphthalenyl or -(CR¹¹R¹²)ₙG^{A}.
Embodiment 33. A compound of Embodiment 32 wherein each R⁶ is independently C₁-C₆ alkyl.
Embodiment 34. A compound of Embodiment 33 wherein each R⁶ is CH₃.
Embodiment 35. A compound of Formula **1** or any one of Embodiments 1 through 34 wherein each W⁵ is O.
Embodiment 36. A compound of Formula **1** or any one of Embodiments 1 through 34 wherein each W⁵ is S.
Embodiment 37. A compound of Formula **1** or any one of Embodiments 1 through 36 wherein each R⁹ is independently H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₂-C₈ alkoxyalkyl, naphthalenyl or -(CR¹¹R¹²)ₙG^{A}.
Embodiment 38. A compound of Embodiment 37 wherein each R⁹ is independently H, C₁-C₆ alkyl, C₁-C₆ haloalkyl, C₃-C₈ cycloalkyl, C₃-C₈ halocycloalkyl, C₂-C₈ alkoxyalkyl, naphthalenyl or -(CR¹¹R¹²)ₙG^{A}.
Embodiment 39. A compound of Embodiment 38 wherein each R⁹ is independently H or C₁-C₆ alkyl.
Embodiment 40. A compound of Embodiment 39 wherein each R⁹ is independently H or CH₃.
Embodiment 41. A compound of Embodiment 40 wherein each R⁹ is H.
Embodiment 42. A compound of Embodiment 40 wherein each R⁹ is CH₃.
Embodiment 43. A compound of Formula **1** or any one of Embodiments 1 through 42 wherein each R¹⁰ is independently H, C₁-C₆ alkyl, C₂-C₆ alkenyl, C₂-C₆ alkynyl, C₁-C₆ haloalkyl or C₃-C₈ cycloalkyl.
Embodiment 44. A compound of Embodiment 43 wherein each R¹⁰ is independently H, C₁-C₆ alkyl, C₁-C₆ haloalkyl or C₃-C₈ cycloalkyl.
Embodiment 45. A compound of Embodiment 44 wherein each R¹⁰ is independently H or C₁-C₆ alkyl.
Embodiment 46. A compound of Embodiment 45 wherein each R¹⁰ is independently H or CH₃.
Embodiment 47. A compound of Embodiment 46 wherein each R¹⁰ is H.
Embodiment 48. A compound of Formula **1** or any one of Embodiments 1 through 36 wherein when R⁹ and R¹⁰ are taken together with the nitrogen to which they are attached to form a heterocyclic ring, the ring is 5- to 6-membered and contains, in addition to the linking nitrogen, ring members selected from carbon and optionally O, S and NR¹³, the carbon ring members optionally in the form of C(=O), and the ring optionally substituted on carbon ring members with up to 4 substituents independently selected from the group consisting of halogen, -CN, C₁-C₃ alkyl and C₁-C₃ alkoxy.
Embodiment 49. A compound of Embodiment 48 wherein when R⁹ and R¹⁰ are taken together with the nitrogen to which they are attached to form a heterocyclic ring, the ring is 5- to 6-membered and contains, in addition to the linking nitrogen, ring members selected from carbon and optionally O and S, the carbon ring members optionally in the form of C(=O), and the ring optionally substituted on carbon ring members with up to 3 substituents independently selected from the group consisting of halogen, C₁-C₃ alkyl and C₁-C₃ alkoxy.
Embodiment 50. A compound of Embodiment 49 wherein when R⁹ and R¹⁰ are taken together with the nitrogen to which they are attached to form a heterocyclic ring, the ring is 5- to 6-membered and contains, in addition to the linking nitrogen, ring members selected from carbon and optionally O, and the ring optionally substituted on carbon ring members with up to 3 substituents independently selected from the group consisting of halogen and C₁-C₃ alkyl.
Embodiment 51. A compound of Formula **1** or any one of Embodiments 1 through 50 wherein the compound is in the form of a metal salt.
Embodiment 52. A compound of Formula **1** or any one of Embodiments 1 through 50 wherein the compound is in the form of an ammonium salt.
Embodiment 53. A compound of Embodiment 51 wherein the metal salt is an alkali metal salt.
Embodiment 54. A compound of Embodiment 53 wherein the metal salt is a sodium or potassium salt.
Embodiment 54a. A compound of Formula **1** or any one of Embodiments 1 through 54 wherein G is other than phenyl.
Embodiment 55. A compound of Formula **1** or any one of Embodiments 1 through 54 wherein G is a phenyl ring or a 5- or 6-membered heteroaromatic ring, each ring optionally substituted with R^{x} on nitrogen ring members and optionally substituted with up to 2 substituents selected from R^{w} on carbon ring members.
Embodiment 55a. A compound of Formula **1** or any one of Embodiments 1 through 55 wherein each R^{x} is independently H or C₁-C₃ alkyl.
Embodiment 56. A compound of Formula **1** or any one of Embodiments 1 through 55a wherein G is one of G-1 through G-72 depicted in Exhibit 2; 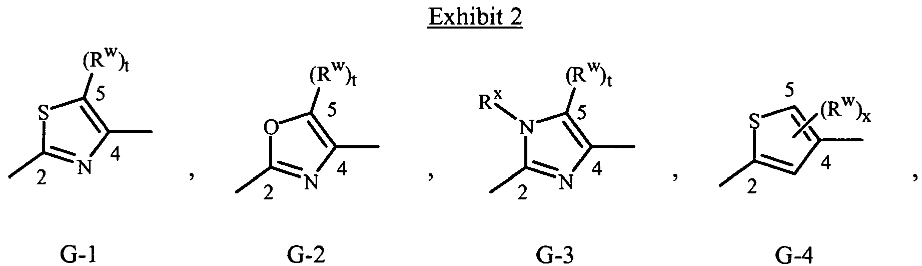 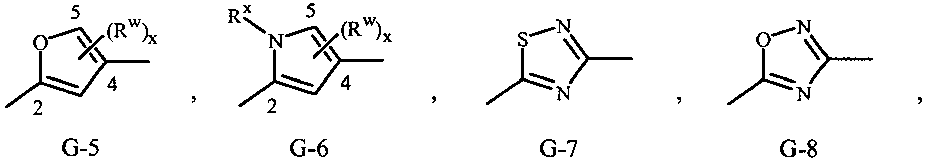 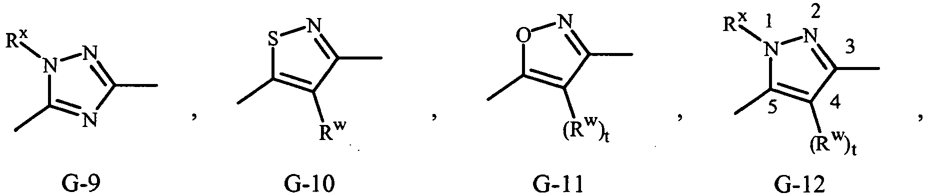 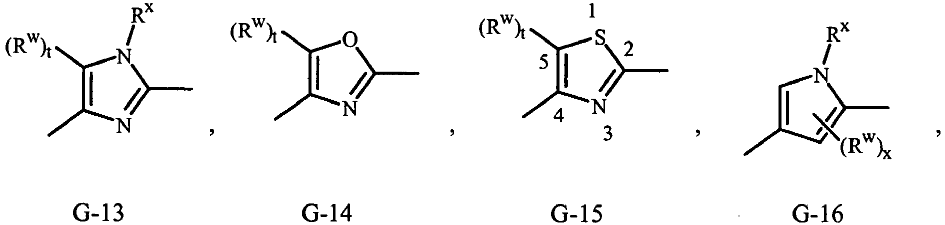 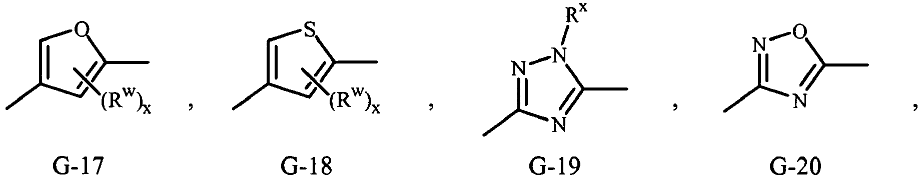 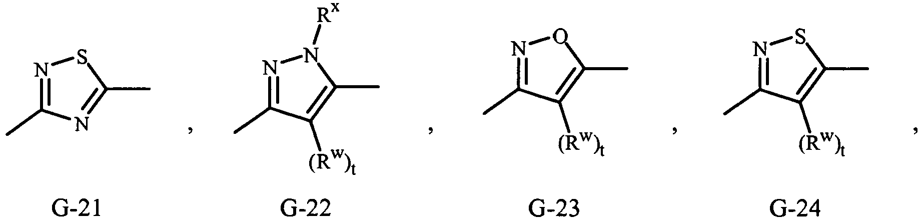 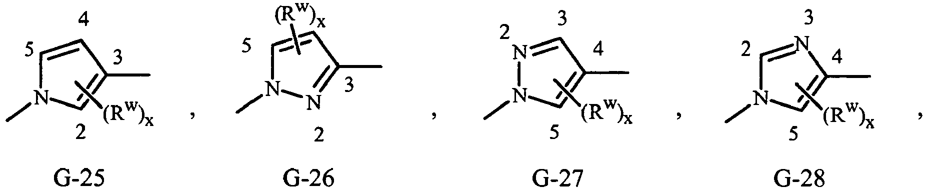 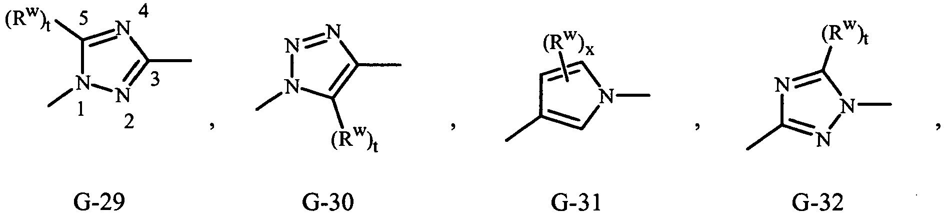 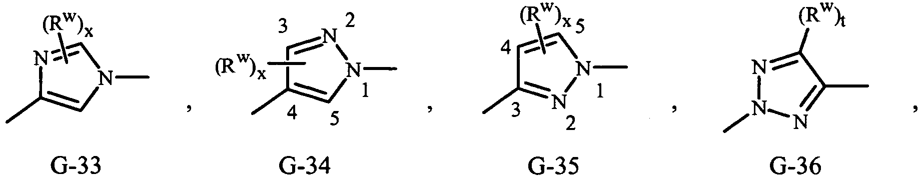 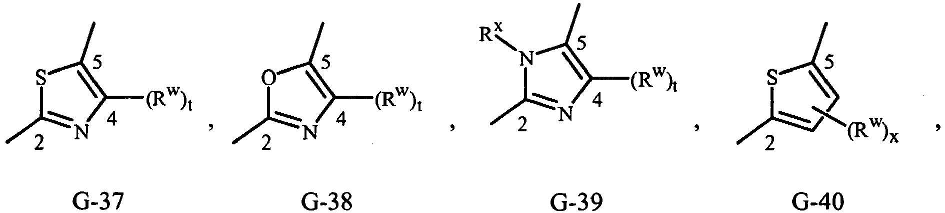 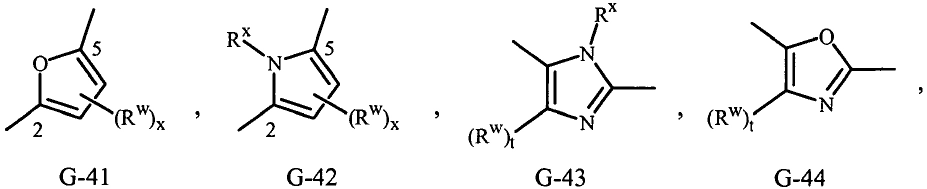 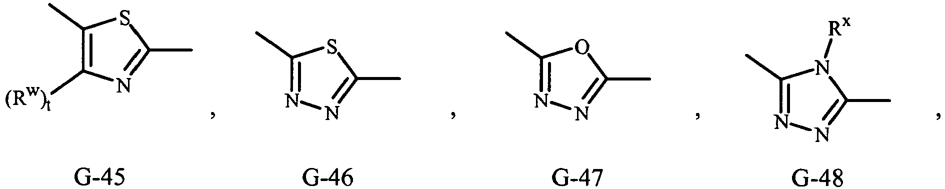 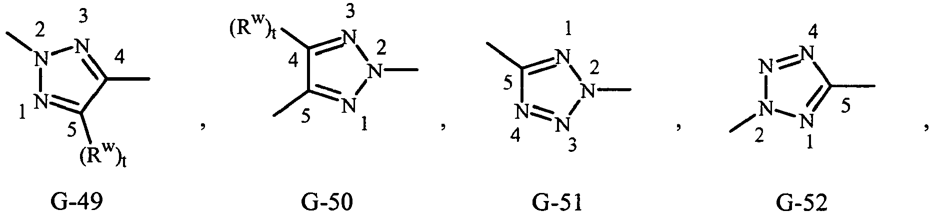 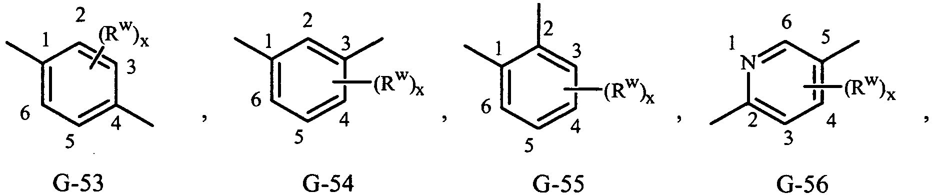 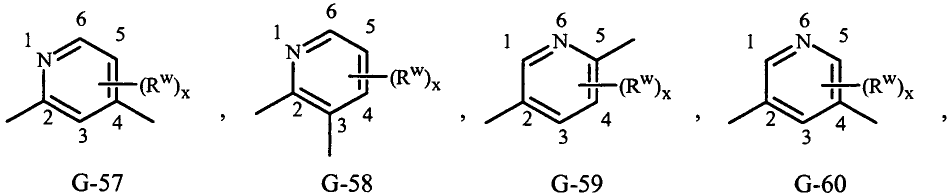 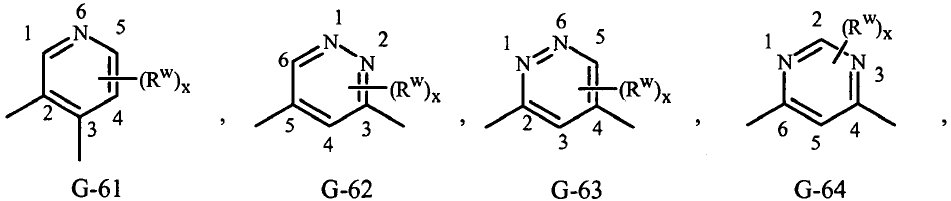 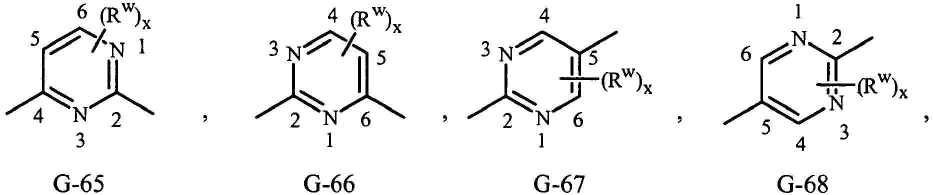 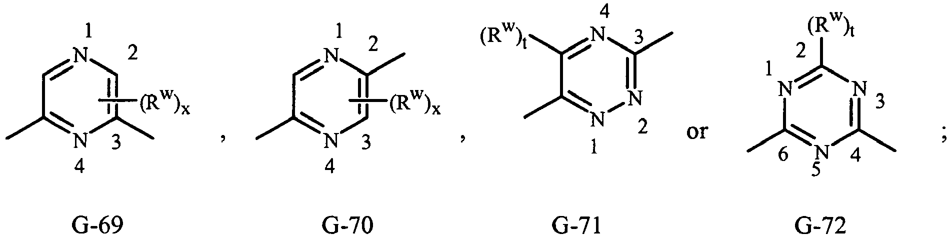 wherein the bond projecting to the left is bonded to the pyridazinone ring of Formula 1, and the bond projecting to the right is bonded to J; and x is an integer selected from 0 through 2; and t is an integer selected from 0 or 1.
Embodiment 57. A compound of Embodiment 56 wherein G is bonded to J other than at the 2-position of G.
Embodiment 57a. A compound of Embodiment 56 wherein G is bonded to J at the 3- or 4-position of G.
Embodiment 57b. A compound of any one of Embodiments 56 through 57a wherein G is selected from G-3 through G-6, G-9 through G-18, G-22 through G-31, G-33 through G-35, G-39 through G-45, G-48, G-50, G-53 through G-60, G-62 through G-65 and G-68 through G-71.
Embodiment 58. A compound of Embodiment 57b wherein G is selected from G-12 through G-15, G-26 through G-29, G-34, G-35, G-54 and G-65.
Embodiment 59. A compound of Embodiment 58 wherein G is selected from G-12, G-15, G-26, G-28, G-29, G-34, G-35, G-54 and G-65.
Embodiment 60. A compound of any one of Embodiments 56 through 59 wherein x is 1.
Embodiment 61. A compound of any one of Embodiments 56 through 59 wherein x is 2.
Embodiment 62. A compound of any one of Embodiments 56 through 59 wherein at least one R^{w} is positioned vicinal to the connection of the G group to the pyridazinone ring.
Embodiment 63. A compound of any one of Embodiments 56 through 60 wherein G is G-26.
Embodiment 64. A compound of any one of Embodiments 56 through 59 or 63 wherein x is 1 and R^{w} is positioned at the 5-position of G-26.
Embodiment 65. A compound of any one of Embodiments 56 through 59 or 63 wherein x is 2 and R^{w} is positioned at the 4- and 5-positions of G-26.
Embodiment 66. A compound of any one of Embodiments 56 through 59 wherein G is G-15.
Embodiment 67. A compound of any one of Embodiments 56 through 59 wherein G is G-29.
Embodiment 68. A compound of any one of Embodiments 56 through 59 wherein G is G-28, x is 1, and R^{w} is positioned at the 2-position of G-28.
Embodiment 69. A compound of any one of Embodiments 56 through 59 wherein G is G-28, x is 2, and R^{w} is positioned at the 2- and 5-positions of G-28.
Embodiment 69a. A compound of any one of Embodiments 56 through 57b wherein G is G-45.
Embodiment 70. A compound of any one of Embodiments 56 through 59 wherein G is G-54, x is 1, and R^{w} is positioned at the 6-position of G-54.
Embodiment 71. A compound of any one of Embodiments 56 through 59 wherein G is G-54, x is 2, and R^{w} is positioned at the 2- and 6-position of G-54.
Embodiment 72. A compound of any one of Embodiments 56 through 59 wherein G is G-65, x is 1, and R^{w} is positioned at the 5-position of G-65.
Embodiment 73. A compound of any one of Embodiments 56 through 59 wherein G is G-35, x is 1, and R^{w} is positioned at the 4-position of G-35.
Embodiment 74. A compound of any one of Embodiments 56 through 59 wherein G is G-34, x is 1, and R^{w} is positioned at the 3-position of G-34.
Embodiment 75. A compound of any one of Embodiments 56 through 59 wherein G is G-12.
Embodiment 76. A compound of Formula **1** or any one of Embodiments 1 through 75 wherein each R^{w} is independently halogen, cyano, hydroxy, amino, nitro, -CHO, -C(=O)OH, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₄-C₁₀ alkylcycloalkyl, C₄-C₁₀ cycloalkylalkyl, C₄-C₁₀ halocycloalkylalkyl, C₅-C₁₂ alkylcycloalkylalkyl, C₂-C₈ alkylcarbonyl, C₂-C₈ haloalkylcarbonyl, C₂-C₈ alkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ alkylsulfonyl, C₁-C₆ haloalkylsulfonyl, C₁-C₆ alkylaminosulfonyl, C₂-C₈ dialkylaminosulfonyl, C₁-C₆ alkylamino, C₂-C₈ dialkylamino, C₂-C₈ alkylcarbonylamino, C₁-C₆ alkylsulfonylamino, naphthalenyl, -O(CR¹¹R¹²)ₙG^{A} or -(CR¹¹R¹²)ₙG^{A}.
Embodiment 77. A compound of Embodiment 76 wherein each R^{w} is independently halogen, hydroxy, C₁-C₆ alkyl, C₁-C₆ haloalkyl, C₃-C₈ cycloalkyl, C₁-C₆ alkoxy, C₁-C₆ haloalkoxy, -O(CR¹¹R¹²)ₙG^{A} or -(CR¹¹R¹²)ₙG^{A}.
Embodiment 77a. A compound of Embodiment 77 wherein each R^{w} is independently halogen, C₁-C₆ alkyl, C₁-C₆ haloalkyl, C₃-C₈ cycloalkyl, C₁-C₆ alkoxy, C₁-C₆ haloalkoxy, -O(CR¹¹R¹²)ₙG^{A} or -(CR¹¹R¹²)ₙG^{A}.
Embodiment 78. A compound of Embodiment 77a wherein each R^{w} is independently halogen, C₁-C₆ alkyl or -O(CR¹¹R¹²)ₙG^{A}.
Embodiment 79. A compound of Embodiment 78 wherein each R^{w} is independently halogen or C₁-C₆ alkyl.
Embodiment 79a. A compound of Embodiment 79 wherein each R^{w} is independently halogen, CH₃, Et, or *n*-Pr (i.e. -(CH₂)₂CH₃).
Embodiment 80. A compound of Embodiment 79a wherein each R^{w} is independently halogen, CH₃ or Et (i.e. CH₂CH₃).
Embodiment 81. A compound of Embodiment 80 wherein each R^{w} is independently CH₃ or Et.
Embodiment 82. A compound of Formula **1** or any one of Embodiments 1 through 81 wherein each n is independently an integer selected from 0 through 1.
Embodiment 83. A compound of Embodiment 82 wherein each n is 0.
Embodiment 84. A compound of Formula **1** or any one of Embodiments 1 through 83 wherein J is a phenyl ring or a 5- or 6-membered heteroaromatic ring, each ring optionally substituted with up to 3 substituents independently selected from R^{u}.
Embodiment 85. A compound of Embodiment 84 wherein J is a phenyl ring or a 6-membered heteroaromatic ring, each ring optionally substituted with up to 3 substituents independently selected from R^{u}.
Embodiment 86. A compound of Embodiment 85 wherein J is a phenyl ring optionally substituted with up to 3 substituents independently selected from R^{u}.
Embodiment 87. A compound of Embodiment 86 wherein J is a phenyl ring substituted with a substituent selected from R^{u}.
Embodiment 88. A compound of Embodiment 87 wherein J is a phenyl ring substituted at the para position with a substituent selected from R^{u}.
Embodiment 88a. A compound of Formula **1** or any one of Embodiments 1 through 83 wherein J is phenyl substituted at the 3-, 4- or 5-position with halogen or C₁-C₆ haloalkyl.
Embodiment 89. A compound of Formula **1** wherein each G^{A} is independently a phenyl ring or a 5- or 6-membered heteroaromatic ring, each ring optionally substituted with up to 5 substituents independently selected from R^{u}.
Embodiment 89a. A compound of Embodiment 89 wherein each G^{A} is independently a phenyl ring or a 5- or 6-membered heteroaromatic ring, each ring optionally substituted with up to 3 substituents independently selected from R^{u}.
Embodiment 90. A compound of Embodiment 89 wherein each G^{A} is independently a phenyl ring or a 5- or 6-membered heteroaromatic ring, each ring optionally substituted with up to 1 substituent independently selected from R^{u}.
Embodiment 91. A compound of Formula **1** or any one of Embodiments 1 through 90 wherein each R^{u} is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₂-C₈ alkylcarbonyl, C₂-C₈ haloalkylcarbonyl, C₂-C₈ alkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfonyl, C₁-C₆ haloalkylsulfonyl, C₁-C₆ alkylaminosulfonyl, C₂-C₈ dialkylaminosulfonyl, C₁-C₆ alkylamino, C₂-C₈ dialkylamino, C₂-C₈ alkylcarbonylamino, C₁-C₆ alkylsulfonylamino, phenyl, pyridinyl or thienyl.
Embodiment 92. A compound of Embodiment 91 wherein each R^{u} is independently halogen, cyano, nitro, -CHO, -C(=O)OH, C₁-C₆ alkyl, C₁-C₆ haloalkyl, C₃-C₈ cycloalkyl, C₃-C₈ halocycloalkyl, C₁-C₆ alkoxy, C₁-C₆ haloalkoxy, C₁-C₆ alkylamino, C₂-C₈ dialkylamino or phenyl.
Embodiment 93. A compound of Embodiment 92 wherein each R^{u} is independently halogen, cyano, nitro, C₁-C₆ alkyl, C₁-C₆ haloalkyl, C₃-C₈ cycloalkyl, C₁-C₆ alkoxy or C₁-C₆ haloalkoxy.
Embodiment 94. A compound of Embodiment 93 wherein each R^{u} is independently halogen, C₁-C₆ alkyl or C₁-C₆ haloalkyl.
Embodiment 95. A compound of Embodiment 94 wherein each R^{u} is independently halogen, C₁-C₃ alkyl or C₁-C₃ haloalkyl.
Embodiment 96. A compound of Embodiment 95 wherein each R^{u} is independently Cl, Br or CF₃.
Embodiment 97. A compound of Formula **1** or any one of Embodiments 1 through 96 wherein W¹ is O.
Embodiment 98. A compound of Formula **1** or any one of Embodiments 1 through 96 wherein W¹ is S.

Embodiments of this invention, including Embodiments 1-98 above as well as any other embodiments described herein, can be combined in any manner, and the descriptions of variables in the embodiments pertain not only to the compounds of Formula 1 but also to the starting compounds and intermediate compounds useful for preparing the compounds of Formula 1. In addition, embodiments of this invention, including Embodiments 1-98 above as well as any other embodiments described herein, and any combination thereof, pertain to the compositions and methods of the present invention.

Combinations of Embodiments 1-98 are illustrated by:
Embodiment A1. A compound of Formula **1** wherein
   R¹ is H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₂-C₈ alkoxyalkyl, C₄-C₁₀ cycloalkoxyalkyl or C₃-C₁₀ alkoxyalkoxyalkyl;
   R² is H, halogen, C₁-C₆ alkyl, C₁-C₆ haloalkyl, C₃-C₈ cycloalkyl or C₂-C₈ alkoxyalkyl;
   R³ is H, -C(=W⁶)R⁴, -C(=W²)W³R⁵, -S(=O)₂R⁶ or -C(W⁵)NR⁹R¹⁰;
   each R^{x} is independently H or C₁-C₃ alkyl;
   J is a phenyl ring or a 5- or 6-membered heteroaromatic ring, each ring optionally substituted with up to 3 substituents independently selected from R^{u}; and
   each R^{u} is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₂-C₈ alkylcarbonyl, C₂-C₈ haloalkylcarbonyl, C₂-C₈ alkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfonyl, C₁-C₆ haloalkylsulfonyl, C₁-C₆ alkylaminosulfonyl, C₂-C₈ dialkylaminosulfonyl, C₁-C₆ alkylamino, C₂-C₈ dialkylamino, C₂-C₈ alkylcarbonylamino, C₁-C₆ alkylsulfonylamino, phenyl, pyridinyl or thienyl.
Embodiment A2. A compound of Embodiment A1 wherein
   R¹ is H, C₁-C₆ alkyl, C₁-C₆ haloalkyl or C₃-C₈ cycloalkyl;
   R² is H, halogen or C₁-C₆ alkyl;
   G is selected from G-3 through G-6, G-9 through G-18, G-22 through G-31, G-33 through G-35, G-39 through G-45, G-48, G-50, G-53 through G-60, G-62 through G-65 and G-68 through G-71 (wherein the bond projecting to the left is bonded to the pyridazinone ring of Formula **1**, and the bond projecting to the right is bonded to J); and x is an integer selected from 0 through 2;
   each R^{w} is independently halogen, hydroxy, C₁-C₆ alkyl, C₁-C₆ haloalkyl, C₃-C₈ cycloalkyl, C₁-C₆ alkoxy, C₁-C₆ haloalkoxy, -O(CR¹¹R¹²)ₙG^{A} or -(CR¹¹R¹²)ₙG^{A};
   J is a phenyl ring optionally substituted with up to 3 substituents independently selected from R^{u};
   each R^{u} is independently is halogen, cyano, nitro, -CHO, -C(=O)OH, C₁-C₆ alkyl, C₁-C₆ haloalkyl, C₃-C₈ cycloalkyl, C₃-C₈ halocycloalkyl, C₁-C₆ alkoxy, C₁-C₆ haloalkoxy, C₁-C₆ alkylamino, C₂-C₈ dialkylamino or phenyl; and
   W¹ is O.
Embodiment A3. A compound of Embodiment A2 wherein
   R¹ is H or C₁-C₆ alkyl;
   R² is H, Cl, CH₃ or Et;
   R³ is H or CO₂-*i*-Pr;
   G is selected from G-12 through G-15, G-26 through G-29, G-34, G-35, G-54 and G-65;
   each R^{w} is independently halogen, C₁-C₆ alkyl or -O(CR¹¹R¹²)ₙG^{A};
   J is a phenyl ring substituted with a substituent selected from R^{u}; and
   each R^{u} is independently halogen, C₁-C₆ alkyl or C₁-C₆ haloalkyl.
Embodiment A4. A compound of Formula **1** wherein
   R¹ is H, C₁-C₆ alkyl, C₂-C₆ alkenyl, C₂-C₆ alkynyl, C₁-C₆ haloalkyl, C₂- C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₂-C₈ alkoxyalkyl, C₄-C₁₀ cycloalkoxyalkyl or C₃-C₁₀ alkoxyalkoxyalkyl;
   R² is H, halogen, C₁-C₆ alkyl, C₁-C₆ haloalkyl, C₃-C₈ cycloalkyl, C₂-C₈ alkoxyalkyl or C₁-C₄ alkoxy;
   R³ is H, -C(=W⁶)R⁴, -C(=W²)W³R⁵, -S(=O)₂R⁶ or -C(W⁵)NR⁹R¹⁰;
   G is a phenyl ring or a 5- or 6-membered heteroaromatic ring, each ring optionally substituted with R^{x} on nitrogen ring members and optionally substituted with up to 2 substituents selected from R^{w} on carbon ring members;
   each R^{x} is independently H or C₁-C₃ alkyl;
   J is a phenyl ring or a 5- or 6-membered heteroaromatic ring, each ring optionally substituted with up to 3 substituents independently selected from R^{u}; and
   each R^{u} is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₂-C₈ alkylcarbonyl, C₂-C₈ haloalkylcarbonyl, C₂-C₈ alkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfonyl, C₁-C₆ haloalkylsulfonyl, C₁-C₆ alkylaminosulfonyl, C₂-C₈ dialkylaminosulfonyl, C₁-C₆ alkylamino, C₂-C₈ dialkylamino, C₂-C₈ alkylcarbonylamino, C₁-C₆ alkylsulfonylamino, phenyl, pyridinyl or thienyl.
Embodiment A5. A compound of Embodiment A4 wherein
   R¹ is H, C₁-C₆ alkyl, C₁-C₆ haloalkyl or C₃-C₈ cycloalkyl;
   R² is H, halogen, C₁-C₆ alkyl or C₁-C₄ alkoxy;
   G is selected from G-3 through G-6, G-9 through G-18, G-22 through G-31, G-33 through G-35, G-39 through G-45, G-48, G-50, G-53 through G-60, G-62 through G-65 and G-68 through G-71 (wherein the bond projecting to the left is bonded to the pyridazinone ring of Formula 1, and the bond projecting to the right is bonded to J); and x is an integer selected from 0 through 2;
   each R^{w} is independently halogen, hydroxy, C₁-C₆ alkyl, C₁-C₆ haloalkyl, C₃-C₈ cycloalkyl, C₁-C₆ alkoxy, C₁-C₆ haloalkoxy, -O(CR¹¹R¹²)ₙG^{A} or -(CR¹¹R¹²)ₙG^{A};
   J is a phenyl ring optionally substituted with up to 3 substituents independently selected from R^{u};
   each R^{u} is independently is halogen, cyano, nitro, -CHO, -C(=O)OH, C₁-C₆ alkyl, C₁-C₆ haloalkyl, C₃-C₈ cycloalkyl, C₃-C₈ halocycloalkyl, C₁-C₆ alkoxy, C₁-C₆ haloalkoxy, C₁-C₆ alkylamino, C₂-C₈ dialkylamino or phenyl; and
   W¹ is O.
Embodiment A6. A compound of Embodiment A5 wherein
   R¹ is H or C₁-C₆ alkyl;
   R² is H, Cl, CH₃, Et or OMe;
   R³ is H, CO₂-*i*-Pr or CO-*t*-Bu;
   G is selected from G-12 through G-15, G-26 through G-29, G-34, G-35, G-54 and G-65;
   each R^{w} is independently halogen, C₁-C₆ alkyl or -O(CR¹¹R¹²)ₙG^{A};
   J is a phenyl ring substituted with a substituent selected from R^{u}; and
   each R^{u} is independently halogen, C₁-C₆ alkyl or C₁-C₆ haloalkyl.
Embodiment A7. A compound of Embodiment A3 or A6 wherein
   R¹ is CH₃;
   R² is H;
   G is selected from G-12, G-15, G-26, G-28, G-29, G-34, G-35, G-54 and G-65;
   each R^{w} is independently CH₃ or Et;
   J is a phenyl ring substituted at the para position with a substituent selected from R^{u}; and
   each R^{u} is independently halogen, C₁-C₃ alkyl or C₁-C₃ haloalkyl.
Embodiment A8. A compound of Embodiment A7 wherein
   G is G-26;
   x is 1;
   R^{w} is positioned at the 5-position of G-26; and
   each R^{u} is independently Cl, Br or CF₃.

Specific embodiments include compounds of Formula **1** selected from the group consisting of:
5-hydroxy-2-methyl-4-[5-methyl-3-[4-(trifluoromethyl)phenyl]-1*H*-pyrazol-1-yl]-3(2*H*)-pyridazinone (Compound 1),
4-[3-(4-chlorophenyl)-5-methyl-1*H-*pyrazol-1-yl]-5-hydroxy-2-methyl-3(2*H*)-pyridazinone (Compound 3),
5-[3-(4-chlorophenyl)-5-methyl-1*H*-pyrazol-1-yl]-1,6-dihydro-1-methyl-6-oxo-4-pyridazinyl-1-methylethyl carbonate (Compound 7),
and also include compounds of Formula **1** selected from the group consisting of:
4-[3-(4-bromophenyl)-5-methyl-1*H*-pyrazol-1-yl]-5-hydroxy-3(2*H*)-pyridazinone (Compound 78),
4-[5-ethyl-3-[4-(trifluoromethyl)phenyl]-1*H*-pyrazol-1-yl]-5-hydroxy-2-methyl-3(2*H*)-pyridazinone (Compound 11) and
4-[3-(4-chlorophenyl)-5-ethyl-4-methyl-1*H*-pyrazol-1-yl]-5-hydroxy-2-methyl-3(2*H*)-pyridazinone (Compound 26).
Of note is a compound of Formula **1** wherein
R¹ is H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl or C₂-C₈ alkylsulfonylalkyl;
R² is H, halogen, cyano, -C(=O)OH, -C(=O)NH₂, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ haloalkoxy, C₁-C₆ alkylthio or C₁-C₆ haloalkylthio;
R³ is H, -C(=O)R⁴, -C(=W²)W³R⁵, -S(=O)₂R⁶, -P(=W⁴)R⁷R⁸ or -C(=W⁵)NR⁹R¹⁰; and
each G^{A} is independently a phenyl ring or a 5- or 6-membered heteroaromatic ring, each ring optionally substituted with up to 5 substituents independently selected from R^{u}.

Of also note are the above embodiments, including Embodiments 1 through 98 and A1 through A8, wherein Formula **1** does not include *N*-oxides thereof, does not include salts thereof, or does not include *N*-oxides and salts thereof.

This invention also relates to a method for controlling undesired vegetation comprising applying to the locus of the vegetation herbicidally effective amounts of the compounds of the invention (e.g., as a composition described herein). Of note as embodiments relating to methods of use are those involving the compounds of embodiments described above. Also noteworthy as embodiments are herbicidal compositions of the present invention comprising the compounds of embodiments described above.

One or more of the following methods and variations as described in Schemes 1-12 can be used to prepare the compounds of Formula **1**. The definitions of R¹, R², R³, W¹, G and J in the compounds of Formulae **1-17** below are as defined above in the Summary of the Invention unless otherwise noted. Formulae **1a-1b** and Formulae **3a-3d** are various subsets of Formulae **1** and **3** respectively.

As shown in Scheme 1, compounds of Formula **1** wherein R³ is other than H can be prepared by reaction of compounds of Formula **1a** (Formula **1** wherein R³ is H) with acylation, sulfonylation or phosphorylation agents of Formula **2** in the presence of acid acceptors. Suitable acid acceptors for the reaction include inorganic bases such as alkali or alkaline earth metal (e.g., lithium, sodium, potassium and cesium) hydrides, alkoxides, carbonates, phosphates and hydroxides, and organic bases such as triethylamine, *N,N-*diisopropylethylamine and 1,8-diazabicyclo[5.4.0]undec-7-ene. Preferred acid acceptors are trialkylamines and potassium hydroxide. A wide variety of solvents are suitable for the reaction including, for example but are not limited to, tetrahydrofuran, dichloromethane, *N,N*-dimethylformamide, *N,N-*dimethylacetamide, *N-*methylpyrrolidinone, acetonitrile and acetone as well as mixtures of these solvents. Typical examples of compounds of Formula 2 wherein LG is a leaving group such as halogen, sulfonate and carboxylate include carboxylic acid halides, carboxylic acid anhydrides, chloroformates, carbamoyl halides, sulfonyl halides, sulfonyl anhydrides, sulfamoyl halides, halophosphates and halophosphonates, which can be prepared by methods well known in the art. Many are commercially available. This reaction can be conducted between about -20 and 200 °C, and more typically between about 0 and 50 °C.

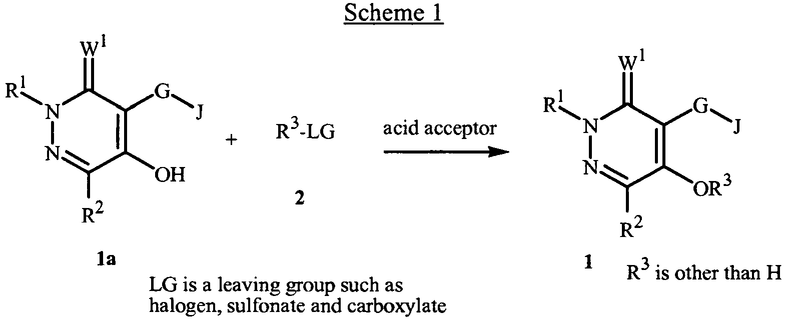

As shown in Scheme 2, compounds of Formula **1a** (Formula 1 wherein R³ is H) can be prepared by the reaction of compounds of Formula **3** with an alkali or alkaline earth metal (e.g., lithium, sodium, potassium and cesium) hydroxide. The reaction is typically carried out in an organic solvent or a mixture of water with an organic solvent such as ethanol or other lower alkyl alcohols, tetrahydrofuran, dioxane, 1,2-dimethoxyethane and *N,N-*dimethylformamide. The reaction can be carried out at temperatures ranging from 0 to 150 °C and preferably from 50 to 120 °C. The hydroxide is generally present in excess ranging from 1.5 to 40-fold with respect to the compound of Formula **3**.

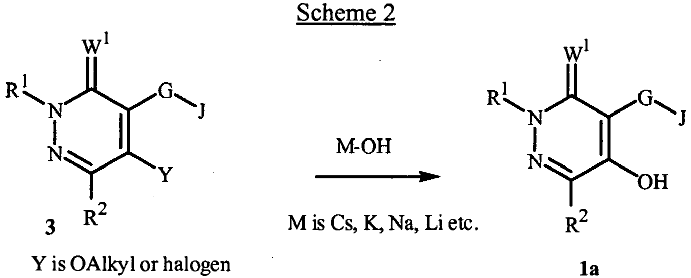

As shown in Scheme 3, compounds of Formula **1b** wherein A is an azole such as pyrazole, imidazole, pyrrole and triazole bonded through N to the pyridazinone ring can be made by the reaction of compounds of Formula **4** with an alkali or alkaline earth metal (e.g., lithium, sodium, potassium and cesium) hydroxide. The reaction is typically carried out in an organic solvent or a mixture of water with an organic solvent such as ethanol and other lower alkyl alcohols, tetrahydrofuran, dioxane, 1,2-dimethoxyethane and *N,N-*dimethylformamide. The reaction can be carried out at temperatures ranging from 0 to 150 °C and more typically from 50 to 120 °C. The hydroxide is generally present in excess ranging from 1.5 to 40-fold with respect to the compound of Formula **4**.

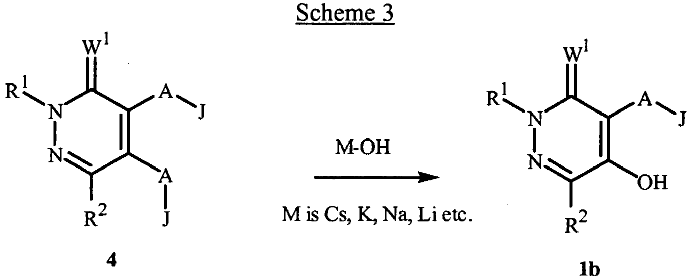

Scheme 4 shows the synthesis of compounds of Formula **1a** by the dealkylation of compounds of Formula **3a** (Formula **3** wherein Y is OAlkyl). Many dealkylation reagents which dealkylate ethers (such as compounds of Formula **3a**), including aluminum trichloride, hydrogen bromide, iodotrimethylsilane and boron tribromide, are known in the art. Boron tribromide is preferred for the dealkylation. The alkoxy compound of Formula **3a** is treated with boron tribromide in a solvent such as a chlorinated hydrocarbon (e.g., dichloromethane and chloroform). The reaction is typically carried out at 0 to 30 °C, but can also be carried out at more elevated temperatures such as between about 50 and 120 °C. Other conditions and reagents for dealkylation are described by Larock, "Comprehensive Organic Transformations", VCH Publishing, New York, 1989, pp 501-504.

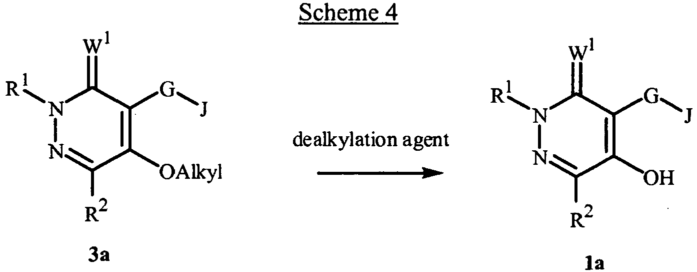

Scheme 5 shows the synthesis of compounds of Formula **1a** by the cyclization of hydrazono esters of Formula **5** in the presence of an acid acceptor. Suitable acid acceptors for this reaction include inorganic bases such as alkali or alkaline earth metal (e.g., lithium, sodium, potassium and cesium) hydrides and alkoxides or organic bases such as triethylamine, *N,N-*diisopropylethylamine and 1,8-diazabicyclo[5.4.0]undec-7-ene. A wide variety of solvents are suitable for the reaction including, for example but not limited to, aromatic hydrocarbons (e.g., toluene and xylenes), tetrahydrofuran, dioxane, 1,2-dimethoxyethane, *N,N-*dimethylformamide, *N,N*-dimethylacetamide, *N*-methylpyrrolidinone, and acetonitrile, as well as mixtures of these solvents. This reaction can be conducted between about -20 and 200 °C and more typically between about 20 and 120 °C.

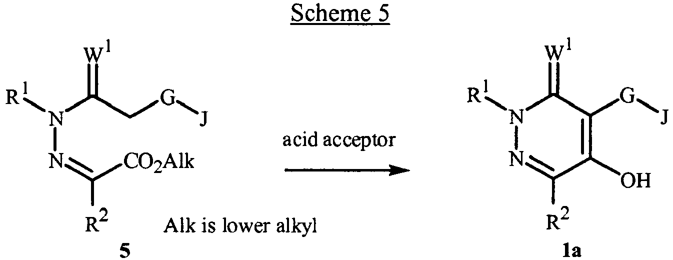

As shown in Scheme 6, compounds of Formula **3b** wherein A is an azole ring such as pyrazole, imidazole, pyrrole and triazole bonded through N to the pyridazinone ring can be prepared by reacting compounds of Formula **4** with alcohols in the presence of an acid acceptor. The reaction is typically carried out in the alcohol as solvent or co-solvent in the presence of the acid acceptor. Suitable acid acceptors for the reaction include inorganic bases such as alkali or alkaline earth metal (e.g., lithium, sodium, potassium and cesium) hydrides and alkoxides or organic bases such as triethylamine, *N,N-*diisopropylethylamine and 1,8-diazabicyclo[5.4.0]undec-7-ene. This reaction can be conducted between about -20 and 200 °C, and more typically between about 20 and 120 °C. A particularly useful solvent and alcohol combination is sodium methoxide in methanol solvent, for which reaction temperatures ranging from 0 to 70 °C are suitable.

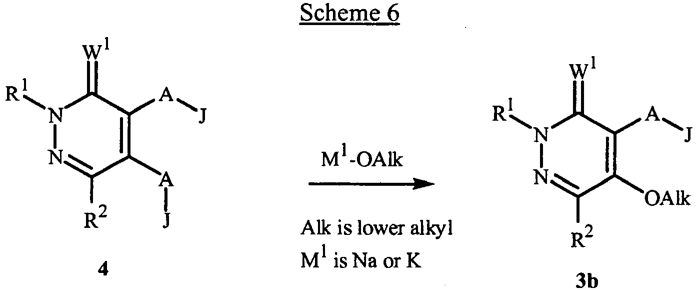

The synthesis of compounds of Formula **3c,** in which G^{a} is a phenyl group or a heterocycle bonded through C to the pyridazinone ring, by means of a transition metal-catalyzed reaction of a halopyridazinone of Formula **6** with an organometallic species represented by Formula **7** is shown in Scheme 7. A variety of metals, catalysts and ligands can be used in this process. For synthesis of organometallic heterocycles of Formula **7** suitable for use in this reaction see Li and Gribble, "Palladium in Heterocyclic Chemistry", first edition, Pergamon Press, Amsterdam, 2000 and 2nd edition, Elsevier, Amsterdam, 2007. This book also describes a wide variety of catalysts and reaction conditions suitable for carrying out the cross-coupling reactions described in Scheme 7.

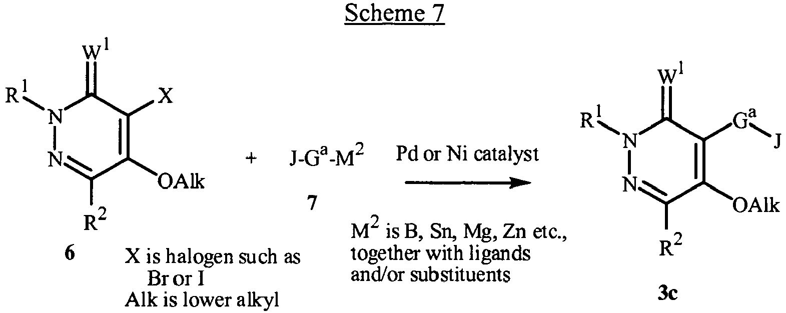

The synthesis of compounds of Formula **3d,** in which G^{a} is a phenyl group or a heterocycle bonded through C to the pyridazinone ring, by means of a transition metal-catalyzed reaction of a differentially substituted (X and Y of Formula **7** are different halogens) halopyridazinone of Formula **7** with an organometallic species represented by Formula **8** is shown in Scheme 8. A variety of metals, catalysts and ligands may be used in this process. Synthesis of pyridazinones of Formula 7 with differentially substituted halogens on the ring can be found in T. M. Stevenson et al. J. Heterocyclic Chem., 2005, 42, 427-435 and Zhang et al. Tetrahedron Lett., 2006, 47, 8733-8735. An alternative approach to position-selective palladium-catalyzed cross-coupling reactions on pyridazinones is found in U.S. Patent 6,307,047.

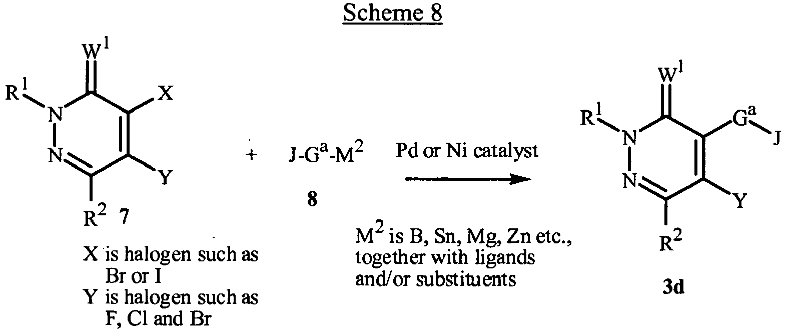

Alternatively compounds of Formula **3c** in which G^{a} is a phenyl group or a heterocycle bonded through C to the pyridazinone ring can be made from organometallic derivatives of pyridazinones of Formula **9** as shown in Scheme 9. Most preferably the organometallic reagent of Formula **9** is made by the reaction of a bimetallic reagent such as hexamethylditin with halopyridazinone compounds of Formula **6** under palladium catalysis. The resulting tin compound of Formula **9** can be transformed to compounds of Formula **3c** by palladium-catalyzed coupling reaction with haloheterocycles of Formula **10**. Conditions and catalysts for reactions of tin pyridazinones with aromatic and heteroaromatic halides as well as conditions for the synthesis of compounds of Formula **9** are described in T. M. Stevenson et al., J. Heterocyclic Chem., 2005, 42, 427-435. Other reagents such as bis-(pinacolato)diboron can also be used for this synthesis.

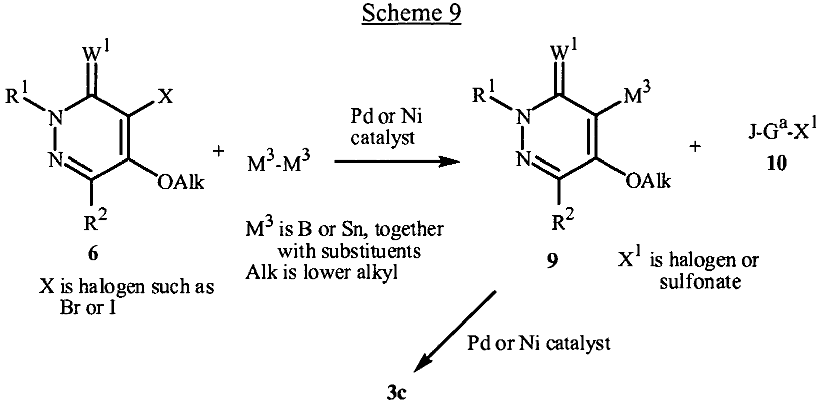

Compounds of Formula **5** can be synthesized by the reaction of an activated acid derivative of Formula **12** with a hydrazone of Formula **11** in the presence of an acid acceptor as shown in Scheme 10. Suitable acid acceptors for the reaction include inorganic bases such as alkali or alkaline earth metal (e.g., lithium, sodium, potassium and cesium) hydrides, alkoxides, carbonates, phosphates and hydroxides, and organic bases such as triethylamine, *N,N*-diisopropylethylamine and 1,8-diazabicyclo[5.4.0]undec-7-ene. Particularly useful acid acceptors are trialkylamines and potassium hydroxide. A wide variety of solvents are suitable for this reaction including, for example but not limited to, tetrahydrofuran, dichloromethane, *N,N*-dimethylformamide, *N,N-*dimethylacetamide, *N-*methylpyrrolidinone, acetonitrile and acetone, as well as mixtures of these solvents. This reaction can be conducted between about -20 and 100 °C, and more typically between about 0 and 50 °C. Activated acid derivatives include, for example but not limited to, acid chlorides, acid bromides, acylimidazoles, mixed anhydrides and acylcyanides.

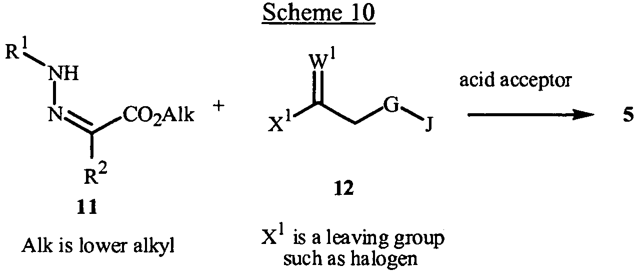

Hydrazones of Formula **11** can be synthesized as described in Li et al., Synthesis, 2007, 3301-3308 and references cited therein. Procedures for the synthesis of hydrazone intermediates and their cyclization are found in US Patent Application 2008/0090814, Bioorg. Med. Chem. Lett., 2008, 1413, Bioorg. Med. Chem Lett., 2008, 1419, Bioorg. Med. Chem. Lett., 2008, 3421, Bioorg. Med. Chem. Lett., 2008, 4628, Bioorg. Med. Chem. Lett., 2008, 3446, Synthesis, 2008, 610-616**,** and Tet. Lett., 2008, 49, 811.

Compounds of Formula **4** wherein A is an azole ring such as pyrazole, imidazole, pyrrole and triazole bonded through N to the pyridazinone ring can be made by the reaction of corresponding azoles of Formula **14** with dihalopyridazinones of Formula **13** in the presence of an acid acceptor as shown in Scheme 11. Suitable acid acceptors for the reaction include inorganic bases such as alkali or alkaline earth metal (e.g., lithium, sodium, potassium and cesium) hydrides, alkoxides, carbonates, phosphates and hydroxides. A variety of solvents are suitable for the reaction including, for example but not limited to, *N,N*-dimethylformamide, *N,N-*dimethylacetamide, *N*-methylpyrrolidinone, acetonitrile and acetone. Particularly useful reaction conditions include using potassium carbonate as acid acceptor and *N,N-*dimethylacetamide as solvent at temperatures between 80 and 180 °C. To enable complete conversion, the acid acceptor and azole of Formula **14** are charged in a molar ratio of at least 2 compared to the compound of Formula **13**.

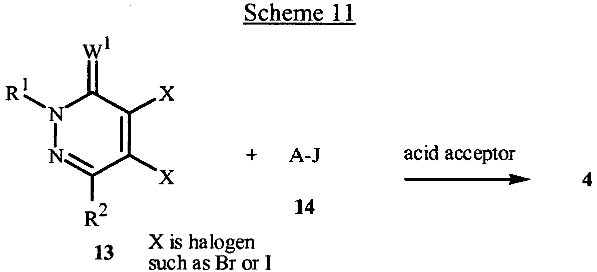

Pyridazinones of Formulae **6** and **13** can be prepared as described in T. M. Stevenson et al., J. Heterocyclic Chem., 2005, 42, 427-435 and references cited therein. Other pyridazinones are found in U.S. Patent 2,782,195 and Maes and Lemiere in Katritsky editor, Comprehensive Heterocyclic Chemistry III, Volume 8, 1-117; Elsevier, Oxford. Azoles of Formula **14** can be prepared using chemistry disclosed in U.S. Patent 7,230,116 and references cited therein.

As shown in Scheme 12 compounds of Formula **5** can be prepared by a two-step route. In the first step compounds of Formula **16** can be prepared by condensing activated acids of Formula **12** with a substituted hydrazine of Formula **15** in the presence of an acid acceptor to give hydrazides of Formula **16**. Suitable acid acceptors for the reaction include inorganic bases such as alkali or alkaline earth metal (e.g., lithium, sodium, potassium and cesium) carbonates, phosphates and hydroxides, and organic bases such as triethylamine, *N,N-*diisopropylethylamine and 1,8-diazabicyclo[5.4.0]undec-7-ene. Particularly useful acid acceptors are trialkylamines and potassium hydroxide. A wide variety of solvents are suitable for this reaction including, for example but not limited to, tetrahydrofuran, dichloromethane, *N,N*-dimethylformamide, *N,N*-dimethylacetamide, *N*-methylpyrrolidinone, acetonitrile and acetone, as well as mixtures of these solvents. This reaction can be conducted between about -20 and 100 °C, and more typically between about 0 and 50 °C. Compounds of Formula 12 include, for example but not limited to, acid chlorides, acid bromides, acylimidazoles, mixed anhydrides and acylcyanides. Further treatment of compounds of Formula 16 with ketoesters of Formula 17 gives compounds of Formula 5. A wide variety of solvents are suitable for this reaction including, for example but not limited to, lower aliphatic alcohols, tetrahydrofuran, dichloromethane, dioxane, chloroform, *N,N-*dimethylformamide, *N,N*-dimethylacetamide, *N*-methylpyrrolidinone, acetonitrile and acetone, as well as mixtures of these solvents. The reaction can also be carried out without the presence of a solvent. This reaction can be conducted between about -20 and 200 °C, and more typically between about 0 and 100 °C.

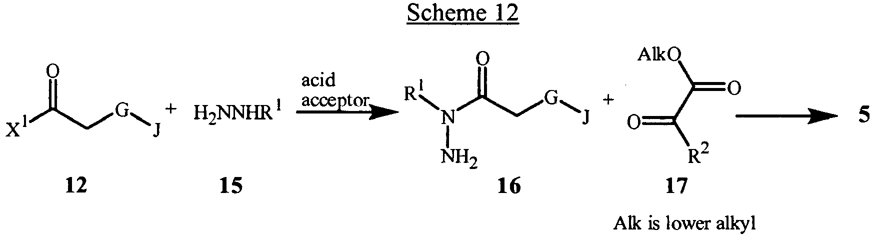

Acid precursors to compounds of Formula **12** can be prepared using methods described in U.S. Patents 6,767,864 and 7,230,116 and PCT Patent Publications WO 2007/014290, WO 2005/005428 and W02007/119434.

It is recognized that some reagents and reaction conditions described above for preparing compounds of Formula **1** may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. Protective Groups in Organic Synthesis, 2nd ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula **1**. One skilled in the art will also recognize that it may be necessary to perform a combination of the steps illustrated in the above schemes in an order other than those implied by the particular sequence presented to prepare the compounds of Formula **1**.

One skilled in the art will also recognize that compounds of Formula **1** and the intermediates described herein can be subjected to various electrophilic, nucleophilic, radical, organometallic, oxidation, and reduction reactions to add substituents or modify existing substituents.
Without further elaboration, it is believed that one skilled in the art using the preceding description can utilize the present invention to its fullest extent. The following Examples are, therefore, to be construed as merely illustrative, and not limiting of the disclosure in any way whatsoever. Steps in the following Examples illustrate a procedure for each step in an overall synthetic transformation, and the starting material for each step may not have necessarily been prepared by a particular preparative run whose procedure is described in other Examples or Steps. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane at 400 MHz ; "s" means singlet, "m" means multiplet, d means doublet and bs means broad singlet. For melting points "dec." means "decomposition". Mass spectra are reported as the molecular weight of the highest isotopic abundance parent ion (M+1) formed by addition of H⁺ (molecular weight of 1) to the molecule, observed by mass spectrometry using atmospheric pressure chemical ionization (AP⁺).

### EXAMPLE 1

### Preparation of 5-hydroxy-2-methyl-4-[5-methyl-3-[4-(trifluoromethyl)phenyl]-1H-pyrazol-1-yl]-3(2H)-pyridazinone (Compound 1)

### Step A: Preparation of 2-methyl-4,5-bis[5-methyl-3-[4-(trifluoromethyl)phenyl]-1H-pyrazol-1-yl]-3(2H)-pyridazinone

A solution of 3-methyl-5-(4-trifluoromethylphenyl)pyrazole (2 g, 8.9 mmol; prepared as described in U.S. Patent 7,230,116) and 4,5-dichloro-2-methyl-(2*H*)-pyridazinone (Aldrich, 0.85 g, 4.7 mmol) in *N,N*-dimethylformamide (15 mL) was treated with potassium carbonate (powdered, 1.9 g, 13.8 mmol). The reaction mixture was heated at 100 °C for 11 h and then poured onto ice water. The resulting solid was filtered and washed with water. The solid was dissolved in dichloromethane (50 mL) and dried over magnesium sulfate. The residue after filtration and evaporation was subjected to chromatography on a 20 g silica gel column using a gradient of 6:1 to 4:1 hexanes/ethyl acetate as eluent to give 0.75 g of the title compound as a yellow oil.
¹H NMR (CDCl₃) δ 8.17 (s, 1H), 7.85 (m, 2H), 7.70 (m, 2H), 7.65 (m, 2H), 6.45 (s, 1H), 6.40 (s, 1H), 3.96 (s, 3H), 2.26 (s, 3H), 2.05 (s, 3H).

### Step B: Preparation of 5-methoxy-2-methyl-4-[5-methyl-3-[4-(trifluoromethyl)phenyl]-1H-pyrazol-1-yl]-3(2H)-pyridazinone

A solution of 2-methyl-4,5-bis[5-methyl-3-[4-(trifluoromethyl)phenyl]-1*H-*pyrazol-1-yl]-3(2*H*)-pyridazinone (i.e. the product of Step A) (0.74 g, 1.3 mmol) in methanol (20 mL) was treated with sodium methoxide (4.5 N solution in methanol, 0.8 mL, 3.6 mmol). The reaction mixture was heated to reflux to dissolve suspended solids and then stirred at 25 °C for 6 h. The resulting mixture was quenched with saturated aqueous ammonium chloride solution (20 mL) and diluted with ethyl acetate (30 mL). The ethyl acetate layer was dried with magnesium sulfate, filtered and evaporated. The residue was subjected to chromatography on a 10 g silica gel cartridge using a gradient of 3:1 to 1:1 hexanes/ethyl acetate as eluent to give 0.4 g of the title compound as an oil which solidified on addition of diethyl ether.
¹H NMR (CDCl₃) δ 7.90 (m, 2H), 7.85 (s, 1H), 7.63 (m, 2H), 6.57 (s, 1H), 3.83 (m, 6H), 2.25 (s, 3H).

### Step C: Preparation of 5-hydroxy-2-methyl-4-[5-methyl-3-[4-(trifluoromethyl)phenyl]-1H-pyrazol-1-yl]-3(2H)-pyridazinone

To a solution of 5-methoxy-2-methyl-4-[5-methyl-3-[4-(trifluoromethyl)phenyl]-1*H-*pyrazol-1-yl]-3(2*H*)-pyridazinone (i.e. the product of Step B) (0.25 g, 0.69 mmol) in dichloromethane (5 mL) was added a solution of boron tribromide (1.0 M solution in dichloromethane, 0.82 mL, 0.82 mmol). The reaction mixture was stirred at room temperature for 2 h and then treated with 1 N aqueous sodium hydroxide solution (4 mL). After stirring for 15 min, the reaction mixture was adjusted to pH 4 with 1 N aqueous hydrochloric acid (4 mL). The reaction mixture was then dried with magnesium sulfate, and purified by pouring onto a Varian Chem Elut^{®} tube and eluting with dichloromethane. The eluted solution was concentrated in vacuo to give 210 mg of the title product; a compound of the present invention.
¹H NMR (CDCl₃) δ 7.89 (s, 3H), 7.86 (m, 2H), 7.68 (m, 2H), 6.60 (s, 1H), 3.81 (s, 3H), 2.35 (s, 3H).

### EXAMPLE 2

### Preparation of 4-[3-(4-chlorophenyl)-5-methyl-1H-pyrazol-1-yl]-5-hydroxy-2-methyl-3(2H)-pyridazinone (Compound 3)

### Step A: Preparation of 4,5-bis[3-(4-chlorophenyl)-5-methyl-1H-pyrazol-1-yl]-2-methyl-3(2H)-pyridazinone

A solution of 3-methyl-5-(4-chlorophenyl)pyrazole (2.6 g, 13.5 mmol; prepared as described in U.S. Patent 7,230,116) and *N*-methyl-4,5-dichloropyridazinone (Aldrich, 1.07 g, 5.9 mmol) in *N,N*-dimethylacetamide (10 mL) was treated with potassium carbonate (powdered, 3.2 g, 23.2 mmol). The reaction mixture was heated at 120-130 °C for 4 h and then poured onto ice water. The solid that separated was filtered and washed with water. The solid was dissolved in dichloromethane (50 mL) and dried over magnesium sulfate. The solution was filtered and concentrated in vacuo. The resulting residue was subjected to chromatography on a 20 g silica gel cartridge using dichloromethane as eluent to give 1.6 g of the title compound as a yellow solid.
¹H NMR (CDCl₃) δ 8.16 (s, 1H), 7.65 (m, 2H), 7.50 (m, 2H), 7.35 (m, 2H), 7.20 (m, 2H), 6.40 (2 x s, 2H), 3.94 (s, 3H), 2.21(s, 3H), 1.98 (s, 3H).

### Step B: Preparation of 4-[3-(4-chlorophenyl)-5-methyl-1H-pyrazol-1-yl]-5-hydroxy-2-methyl-3(2H)-pyridazinone

4,5-Bis[3-(4-chlorophenyl)-5-methyl-1*H*-pyrazol-1-yl]-2-methyl-3(2*H*)-pyridazinone (i.e. the product of Step A) (1.35 g, 2.7 mmol) and aqueous sodium hydroxide (50 %, 3 mL) were dissolved in a mixture of methanol (40 mL) and water (10 mL). The heterogeneous reaction mixture was heated at reflux for 2 h during which time it became homogeneous. The reaction mixture was evaporated under reduced pressure, and the residue was acidified with 6 N aqueous hydrochloric acid. The resulting gum was separated, washed with water and then dissolved in hot ethyl acetate (150 mL). The reaction solution was dried over magnesium sulfate, filtered and evaporated. The resulting residue was subjected to chromatography on a 20 g silica gel cartridge using dichloromethane followed by a gradient mixture of dichloromethane/ethyl acetate from 90:10 to 0:100 as eluent to give 460 mg of the title product, a compound of the present invention, as an off-white solid.
¹H NMR (CDCl₃) δ 7.77 (s, 1H), 7.66 (m, 2H), 7.40 (m, 2H), 6.46 (s, 1H), 3.80 (s, 3H), 2.27 (s, 3H).

### EXAMPLE 3

### Preparation of 5-[3-(4-chlorophenyl)-5-methyl-1H-pyrazol-1-yl]-1,6-dihydro-1-methyl-6-oxo-4-pyridazinyl-1-methylethyl carbonate (Compound 7)

A solution of 4-[3-(4-chlorophenyl)-5-methyl-1*H*-pyrazol-3-yl]-5-hydroxy-2-methyl-3(2*H*)-pyridazinone (i.e. the product of Example 2, Step B) (0.175 g, 0.54 mmol) in dichloromethane (15 mL) was treated with triethylamine (0.6 mL, 4.2 mmol) followed by isopropyl chloroformate (1 M solution in toluene, 1.0 mL, 1 mmol). The reaction mixture was stirred at 25 °C for 2 h. It was then quenched with 1 N aqueous hydrochloric acid (10 mL) and diluted with dichloromethane (10 mL). The organic layer was washed with saturated aqueous sodium bicarbonate solution (20 mL). The organic layer was separated, dried over magnesium sulfate, and filtered. The resulting filtrate was evaporated under reduced pressure, and the resulting residue was subjected to chromatography on a 10 g silica gel cartridge using a gradient of 1-chlorobutane (100 %) to 1-chlorobutane/ethyl acetate (90:10) as eluent to give 200 mg of the title product, a compound of the present invention, as an off-white solid.
¹H NMR (CDCl₃) δ 7.89 (s, 1H), 7.69 (m, 2H), 7.35 (m, 2H), 6.45 (s, 1H), 4.75 (m, 1H), 3.88 (s, 3H), 2.33 (s, 3H), 1.12 (m, 6H).

### EXAMPLE 4

### Preparation of 4-[2-(4-chlorophenyl)-5-methyl-4-thiazolyl]-5-hydroxy-2,6-dimethyl-3(2H)-pyridazinone (Compound 79)

### Step A Preparation of 2-(4-chlorophenyl)-5-methyl-4-thiazoleacetic acid 2-(2-ethoxy-1-methyl-2-oxoethylidine)-1-methylhydrazide

A suspension of 2-(4-chlorophenyl)-5-methylthiazole-4-acetic acid (1.2 g, 4.2 mmol) in dichloromethane (10 mL) at 23°C was treated with methanesulfonyl chloride (0.35 mL). The purple-tinged mixture was stirred for 10 min and then treated with methyl hydrazine (0.35 g). After stirring for 30 min the mixture was treated with ethyl acetate (40 mL) and saturated aqueous sodium bicarbonate solution (30 mL). The organic layer was dried over magnesium sulfate and concentrated to a foam. The residue was suspended in ethanol (15 mL), treated with ethyl pyruvate (0.35 g, 3.0 mmol) and heated to reflux. After 30 min the solvent was evaporated, and the residue was subjected to silica gel chromatography (20 g silica) using a gradient of ethyl acetate/hexanes (6:1 to 3:1). Appropriate fractions were combined and evaporated to give 0.53 g of a thick oil containing the title compound with with a small amount of ethyl acetate.

### Step B Preparation of 4-[2-(4-chlorophenyl)-5-methyl-4-thiazolyl]-5-hydroxy-2,6-dimethyl-3(2H)-pyridazinone

A solution of 2-(4-chlorophenyl)-5-methyl-4-thiazoleacetic acid 2-(2-ethoxy-1-methyl-2-oxoethylidine)-1-methylhydrazide (i.e. the product of Step A) (0.52 g) in tetrahydrofuran (15 mL) at 23°C was treated dropwise with potassium *tert*-butoxide (1 M in THF, 4 mL). The red-colored mixture was stirred for 30 min and then quenched with hydrochloric acid (1 N, 20 mL). The mixture was extracted with ethyl acetate (2 x 50 mL), dried over magnesium sulfate and evaporated. The residue was subjected to silica gel chromatography (20 g silica) using a gradient of dichloromethane/ethyl acetate (100: 1 to 20:1 to 10:1), and appropriate fractions were combined and evaporated to yield 70 mg of the title product, a compound of the present invention.
¹H NMR (CDCl₃) δ 7.79 (m, 2H), 7.43 (m, 2H), 3.78 (s, 3H), 2.66 (s, 3H), 2.35 (s, 3H).

### EXAMPLE 5

### Preparation of 5-hydroxy-2,6-dimethyl-4-[5-methyl-3-[4-(trifluoromethyl)phenyl]-1H pyrazol-1-yl]-3(2H)-pyridazinone (Compound 75)

### Step A Preparation of 6-bromo-4,5-dichloro-3(2H)-pyridazinone

A mixture of 4,5-dichloropyridazinone (5.0 g, 30 mmol) and water (30 mL) was heated with bromine (1.9 mL, 36 mmol) at 180 °C for 30 min in a microwave oven. Upon cooling the mixture was diluted with water (50 mL) and the solid was collected by filtration and washed with water (10 mL) to give the title compound as an off-white solid (6.75 g) after drying.

### Step B Prepartion of 6-bromo-4,5-dichloro-2-methyl-3(2H)-pyridazinone

6-Bromo-4,5-dichloro-3(2*H*)-pyridazinone (i.e. the product of step A) (6.6 g, 27 mmol) was dissolved in *N,N*-dimethylformamide (30 mL) and treated with potassium carbonate (11 g, 81 mmol) and iodomethane (1.7 mL, 27 mmol). The mixture was stirred for 18 h at 23 °C, diluted with water (50 mL) and extracted with ethyl acetate (3 x 20 mL). The combined extract was washed with brine (50 mL) and dried over magnesium sulfate. After concentration the residue was subjected to silica gel chromatography (40 g silica) using a gradient of ethyl acetate/hexanes (0:100 to 100:0), and appropriate fractions were combined and evaporated to give 4.88 g of the title compound.
¹H NMR (CDCl₃) δ.3.82 (s, 3H).

### Step C Preparation of 4,5-dichloro-2,6-dimethyl-3(2H)-pyridazinone

6-Bromo-4,5-dichloro-2-methyl-3(2*H*)-pyridazinone (i.e. the product of Step B) (3.3 g, 13 mmol), dichloro[1,1'-bis(diphenylphosphino)ferrocene]palladium(II) dichloromethane adduct (1:1) (also known as PdCl₂(dppf)) (0.94 g, 1.3 mmol), cesium carbonate (6.9 g, 21 mmol) and trimethylboroxine (1.3 mL, 9.3 mmol) were combined with dioxane (30 mL) and heated at reflux for 18 h. Additional trimethylboroxine (1.0 mL) was added, and reflux was continued for an additional 4 h. The mixture was diluted with water (20 mL), saturated aqueous ethylenediaminetetraacetic acid sodium salt solution (40 mL) and dichloromethane (40 mL). The aqueous layer was extracted with dichloromethane (2 x 20 mL), and the combined organic layers were dried over magnesium sulfate. The residue after evaporation was subjected to silica gel chromatography (40 g silica) using a gradient of ethyl acetate/hexanes (0:100 to 100:0), and appropriate fractions were combined and evaporated to give 1.7 g of the title compound as a white solid.
¹H NMR (CDCl₃) δ 3.82 (s, 3H), 2.43 (s, 3H).

### Step D Preparation of 5-methoxy-2,6-dimethyl-4-[5-methyl-3-[4-(trifluoromethyl)phenyl]-1H-pyrazol-1-yl]-3(2H)-pyridazinone

4,5-Dichloro-2,6-dimethyl-3(2*H*)-pyridazinone (i.e. the product of Step C) (0.62 g, 3.2 mmol), potassium carbonate (2.9 g, 21 mmol) and 3-(4-trifluoromethylphenyl)-5-methylpyrazole (1.46 g, 6.4 mmol) were mixed with *N,N-*dimethylacetamide (10 mL) and heated at 140°C for 3 h. The mixture was then poured into ice-water (100 mL) extracted with dichloromethane (3 x 20 mL) and concentrated to a brown oil (1.33 g), which was dissolved in methanol (20 mL) and treated with sodium methoxide solution (25% in methanol, 2.5 mL, 11 mmol). The dark mixture was heated at reflux for 3.5 h. The mixture was then diluted with aqueous hydrochloric acid (30 mL) and extracted with ethyl acetate (3 x 30 mL). The combined extract was washed with brine (20 mL) and dried over magnesium sulfate. The residue after evaporation was subjected to silica gel chromatography (40 g silica) using a gradient of ethyl acetate/hexanes (0:100 to 100:0), and appropriate fractions were combined and evaporated to give 0.35 g of the title compound.
¹H NMR (CDCl₃) δ 7.91 (m, 2H), 7.63 (m, 2H), 6.58 (s, 1H), 3.74 (s, 3H), 3.52 (s, 3H), 2.30 (s, 6H).

### Step E Preparation of 5-hydroxy-2,6-dimethyl-4-[5-methyl-3-[4-(trifluoromethyl)phenyl]-1H pyrazol-1-yl]-3(2H)-pyridazinone

5-Methoxy-2,6-dimethyl-4-[5-methyl-3-[4-(trifluoromethyl)phenyl]-1*H*-pyrazol-1-yl]-3(2*H*)-pyridazinone (i.e. the product of Step D) (0.35 g, 0.9 mmol) was dissolved in dioxane (20 mL) and treated with aqueous sodium hydroxide solution (50%, 10 mL, excess). The mixture was heated at reflux for 18 h. The mixture was then diluted with water and extracted with ethyl acetate (30 mL). The aqueous layer was acidified with aqueous 6 N hydrochloric acid and extracted with ethyl acetate (2 x 30 mL). The combined organic extracts from the extraction after acidification were dried with magnesium sulfate and evaporated to provide 0.12 g of the title compound as a solid, a compound of the present invention, melting at 191-194 °C.
¹H NMR (CDCl₃) δ 7.87 (m, 2H), 7.67 (m, 2H), 6.58 (s, 1H), 3.79 (s, 3H), 2.40 (s, 3H), 2.36 (s, 3H).

### EXAMPLE 6

### Preparation of 4-[4-bromo-1-[4-(trifluoromethyl)phenyl]-1H-pyrazol-3-yl]-5-hydroxy-2-methyl-3(2H)-pyridazinone (Compound 87)

### Step A Preparation of 3-iodo-1-[4-(trifluoromethyl)phenyl]-1H-pyrazole

A mixture of 3-iodopyrazole (prepared as described in WO2007/035309) (1.0 g, 5.1 mmol), 4-fluorobenzotrifluoride (0.72 mL, 5.7 mmol) and potassium carbonate (0.93g, 6.7 mmol) was stirred in *N,N*-dimethylformamide (10 mL) at 100 °C for 2 h. The mixture was diluted with water (60 mL) and extracted with ethyl acetate (2 x 30 mL). The organic layers were combined, dried over magnesium sulfate and concentrated. The residue after evaporation was subjected to silica gel chromatography (40 g silica) using a gradient of ethyl acetate/chlorobutane (0:100 to 50:50), and appropriate fractions were combined and evaporated to give the title compound (1.27 g).
¹H NMR (CDCl₃) δ 7.79 (m, 3H), 7.71 (m, 2H), 6.67 (d, 1H).

### Step B Preparation of 5-methoxy-2-methyl-4-[1-[4-(trifluoromethyl)phenyl]-1H pyrazol-3-yl]-3(2H)-pyridazinone

3-Iodo-1-[4-(trifluoromethyl)phenyl]-1*H*-pyrazole (i.e. the product of Step A) (1.3 g, 2.6 mmol), 5-methoxy-2-methyl-4-trimethylstannyl-2*H*-pyridazin-3-one (prepared as described in J. Heterocyclic Chem., 2005, 42, 427) (0.95 g, 3.1 mmol), bis(triphenylphosphine)palladium(II) dichloride (0.36 g, 0.52 mmol) and copper(I) iodide (50 mg, 0.26 mmol) were mixed in dioxane (15 mL) and heated at reflux for 2 h. The reaction mixture was filtered through Celite^{®} diatomaceous filter aid, and the Celite^{®} was washed with ethyl acetate (20 mL). The organics were evaporated and the residue was subjected to silica gel chromatography (40 g silica) using a gradient of ethyl acetate/hexanes (0:100 to 80:20). The appropriate fractions were combined and evaporated to give the title compound (0.51 g) as a solid.
¹H NMR (CDCl₃) δ 8.04 (m, 1H), 7.90 (s, 1H), 7.88 (m, 2H), 7.71 (m, 2H), 7.13 (m, 1H), 4.04 (s, 3H), 3.84 (s, 3H).

### Step C Preparation of 4-[4-bromo-1-[4-(trifluoromethyl)phenyl]-1H-pyrazol-3-yl]-5-methoxy-2-methyl-3(2H)-pyridazinone

5-Methoxy-2-methyl-4-[1-[4-(trifluoromethyl)phenyl]-1*H*-pyrazol-3-yl]-3(2H)-pyridazinone (i.e. the product of Step B) (0.15 g, 0.43 mmol) and *N*-bromosuccinimide (0.076 g, 0.43 mmol) were dissolved in *N,N*-dimethylformamide (5 m L) and stirred at 80 °C for 4 h. *N*-bromosuccinimide (30 mg) was added, and heating was continued for an additional 16 h. The reaction mixture was then diluted with water (40 mL) and extracted with ethyl acetate (3 x 20 mL). The organic extracts were dried over magnesium sulfate and evaporated. The residue was subjected to silica gel chromatography (12 g silica) using a gradient of ethyl acetate/hexanes (0:100 to 80:20). The appropriate fractions were combined and evaporated to give the title compound as a white solid (0.13 g). ¹H NMR (CDCl₃) δ 8.10 (s, 1H), 7.87 (s, 1H), 7.83-7.68 (m, 4H), 3.94 (s, 3H), 3.84 (s, 3H).

### Step D Preparation of 4-[4-bromo-1-[4-(trifluoromethyl)phenyl]-1H-pyrazol-3-yl]-5-hydroxy-2-methyl-3(2H)-pyridazinone

4-[4-Bromo-1-[4-(trifluoromethyl)phenyl]-1*H*-pyrazol-3-yl]-5-methoxy-2-methyl-3(2*H*)-pyridazinone (i.e. the product of Step C) (0.13 g, 0.31 mmol) was mixed with dioxane (10 mL), aqueous sodium hydroxide (50 %, 5 mL) and methanol (1 mL). The reaction mixture was heated at 90 °C for 16 h. The reaction mixture was then diluted with water (20 mL) and extracted with ethyl acetate (15 mL). The aqueous layer was then acidified with 6 M hydrochloric acid and extracted with ethyl acetate (3 x 20 mL). The combined organic extracts from the extraction after acidification were dried over magnesium sulfate and evaporated. The residue was subjected to silica gel chromatography (12 g silica) using a gradient of ethyl acetate/hexanes (20:80 to 100:0), and appropriate fractions were combined and evaporated to give the title product, a compound of the present invention, as a solid (0.07 g) melting at 237-241 °C.
¹HNMR(CDCl₃) δ 8.19 (s, 1H), 7.80 -7.73 (m, 5H), 3.83 (s, 3H).

### EXAMPLE 7

### Preparation of 5-hydroxy-2-methyl-4-[4-methyl-1-[4-(trifluoromethyl)phenyl]-1H-pyrazol-3-yl]-3(2H)-pyridazinone (Compound 85)

### Step A Preparation of 3-iodo-4-methyl-1H-pyrazole

A solution of 4-methylpyrazole (10.0 g, 122 mmol) in *N,N*-dimethylformamide (100 mL) at 23 °C was treated with *N*-iodosuccinimide (27.4 g, 122 mmol) for 18 h. The reaction mixture was diluted with water (100 mL) and then filtered. The filtrate was extracted with ethyl acetate (3 x 50 mL). The combined organic extracts were washed with brine (100 mL) and dried over magnesium sulfate. The residue after evaporation was subjected to silica gel chromatography (80 g silica) using a gradient of ethyl acetate/hexanes (0:100 to 80:20) and appropriate fractions were combined and evaporated to give 5.39 g of the title compound as a solid.
¹H NMR (CDCl₃) δ 7.35 (s, 1H), 2.04 (s, 3H).

### Step B Preparation of 3-iodo-4-methyl-1-[4-(trifluoromethyl)phenyl]-1H-pyrazole

3-Iodo-4-methyl-1*H*-pyrazole (i.e. the product of Step A) (0.50 g, 2.4 mmol), 4-fluorobenzotrifluoride (0.34 mL, 2.6 mmol) and potassium carbonate (0.43 g, 3.1 mmol) were combined with *N,N*-dimethylformamide (20 mL) and heated at 100 °C for 2 h. The reaction mixture was diluted with water (50 mL) and extracted with ethyl acetate (2 x 30 mL). The organic layers were combined, dried over magnesium sulfate and concentrated. The residue after evaporation was subjected to silica gel chromatography (12 g silica) using a gradient of ethyl acetate/hexanes (0:100 to 80:20), and appropriate fractions were combined and evaporated to give the title compound (0.32 g).
¹H NMR (CDCl₃) δ 7.75 (m, 2H), 7.68 (m, 2H), 7.61 (s, 1H), 2.07 (s, 3H).

### Step C Preparation of 5-methoxy-2-methyl-4-[4-methyl-1-[4-(trifluoromethyl)phenyl]-1H-pyrazol-3-yl]-3(2H)-pyridazinone

3-Iodo-4-methyl-1-[4-(trifluoromethyl)phenyl]-1*H*-pyrazole (i.e. the product of Step B) (0.32 g, 0.76 mmol), 5-methoxy-2-methyl-4-trimethylstannyl-2*H*-pyridazin-3-one (prepared as described in J. Heterocyclic Chem., 2005, 42, 427) (0.23 g, 0.9 mmol), bis(triphenylphosphine)palladium(II) dichloride (0.10 g, 0.15 mmol) and copper iodide (10 mg, 0.076 mmol) were mixed in dioxane (10 mL) and heated at reflux for 2 h. The reaction mixture was filtered through Celite^{®}, and the Celite^{®} was washed with ethyl acetate (20 mL). The filtrate was evaporated, and the residue was subjected to silica gel chromatography (12 g silica) using a gradient of ethyl acetate/chlorobutane (0:100 to 100:0), and appropriate fractions combined and evaporated to give the title compound (0.11 g) as a solid.
¹H NMR (CDCl₃) δ 7.85 (m, 2H), 7.80 (m, 2H), 7.68 (m, 2H), 3.90 (s, 3H), 3.82 (s, 3H), 2.07 (s, 3H).

### Step D Preparation of 5-hydroxy-2-methyl-4-[4-methyl-1-[4-(trifluoromethyl)phenyl]-1H-pyrazol-3-yl]-3(2H)-pyridazinone

5-Methoxy-2-methyl-4-[4-methyl-1-[4-(trifluoromethyl)phenyl]-1*H*-pyrazol-3-yl]-3(2*H*)-pyridazinone (i.e. the product of Step C) (0.10 g, 0.24 mmol) was mixed with dioxane (10 mL), aqueous sodium hydroxide (50%, 4 mL) and methanol (1 mL). The mixture was heated at 90 °C for 31 h. The reaction was diluted with water (20 mL) and extracted with ethyl acetate (15 mL). The aqueous layer was acidified with hydrochloric acid and extracted with ethyl acetate (3 x 20 mL). The combined organic extracts from the extraction after acidification were dried over magnesium sulfate and evaporated. The residue was subjected to silica gel chromatography (12 g silica) using a gradient of ethyl acetate/hexanes (20:80 to 100:0), and appropriate fractions were combined and evaporated to give the title product, a compound of the present invention, as a solid (0.09 g) melting at 150-154 °C.
¹H NMR (CDCl₃) δ 7.91 (s, 1H), 7.75 (m, 5H), 3.81 (s, 3H), 2.38 (s, 3H).

### EXAMPLE 8

### Preparation of 4-[1-(4-chlorophenyl)-4-methyl-1H-pyrazol-3-yl]-5-hydroxy-2-methyl-3(2H)-pyridazinone (Compound 30)

### Step A Preparation of 5-chloro-2-methyl-4-(1-propyn-1-yl)-3(2H)-pyridazinone

A mixture of 2-methyl-4-iodo-5-chloro-2*H*-pyridazin-3-one (prepared as described in J. Heterocyclic Chem., 2005, 42, 427) (2.5 g, 9.3 mmol), bis(triphenylphosphine)palladium(II) dichloride (0.13 g) and tributyl(1-propynyl)tin (3.3 g, 10 mmol) in dioxane (12 mL) was heated at reflux for 3 h. The residue after evaporation was subjected to silica gel chromatography (20 g silica) using gradient of ethyl acetate/chlorobutane (0:100 to 10:90), and appropriate fractions were combined and evaporated and then triturated with a mixture of hexanes/diethyl ether (10: 1) to give the title compound (1.1 g) as a solid.
¹H NMR (CDCl₃) δ 7.71 (s, 1H), 3.76 (s, 3H), 2.22 (s, 3H).

### Step B Preparation of 4-[1-(4-chlorophenyl)-4-methyl-1H-pyrazol-3-yl]-5-hydroxy-2-methyl-3(2H)-pyridazinone

5-Chloro-2-methyl-4-(1-propyn-1-yl)-3(2*H*)-pyridazinone (i.e. the product of Step A) (0.46 g, 2.5 mmol) and 4-chlorophenylsydnone (as prepared as described in J. Org. Chem., 1974, 39, 3676) (0.50 g, 2.5 mmol) were mixed in mesitylene (5 mL) and heated at 140 °C for 18 h. The mesitylene was distilled off, and the residue was subjected to silica gel chromatography (20 g silica) using a gradient of ethyl acetate/dichloromethane (5:95 to 30:70), and appropriate fractions were combined and evaporated to give the chloropyridazinone intermediate (0.10 g). The residue was dissolved in a mixture of dioxane (10 mL), aqueous sodium hydroxide (50%, 4 mL) and methanol (1 mL) and then heated at 90°C for 31 h. The reaction mixture was diluted with water (20 mL) and extracted with ethyl acetate (15 mL). The aqueous layer was then acidified with hydrochloric acid to give a solid, which was collected by filtration. The solid was dissolved in ethyl acetate (20 mL), dried over magnesium sulfate and evaporated to provide 0.061 g of the title product, a compound of the present invention, melting at 147-151 °C.
¹H NMR (CDCl₃) δ 7.81 (s, 1H), 7.75 (s, 1H), 7.56 (m, 2H), 7.45 (m, 2H), 3.81 (s, 3H), 2.37 (s, 3H).

### EXAMPLE 9

### Preparation of 4-[3-(4-bromophenyl)-5-methyl-1H-pyrazol-1-yl]-5-hydroxy-2-(tetrahydro-2H-pyran-2-yl)-3(2H)-pyridazinone (Compound 77)

### Step A Preparation of 4,5-bis[3-(4-bromophenyl)-5-methyl-1H-pyrazol-1-yl]-2-(tetrahydro-2H-pyran-2-yl)-3(2H)-pyridazinone

4,5-Dichloro-2-(tetrahydro-2H-pyran-2-yl)-3(2*H*)-pyridazinone, (0.525 g, 2.11 mmol; prepared as described in J. Heterocyclic Chem., 1995, 32, 1473-1476) was added to a mixture of 3-(4-bromophenyl)-5-methyl-1*H*-pyrazole (1.0 g, 4.2 mmol) and potassium carbonate (1.5 g, 11 mmol) in *N,N*-dimethylacetamide (7 mL) and heated to 140° C for 16 h. The reaction mixture was then diluted with water and extracted with ethyl acetate. The combined organic extracts were dried over magnesium sulfate, filtered and concentrated to yield 1.38 g of crude product, which was used without purification.

### Step B Preparation of 4-[3-(4-bromophenyl)-5-methyl-1H-pyrazol-1-yl]-5-methoxy-2-(tetrahydro-2H-pyran-2-yl)-3(2H)-pyridazinone

To a solution of the crude product from Step A (i.e, 4,5-bis[3-(4-bromophenyl)-5-methyl-1*H*-pyrazol-1-yl]-2-(tetrahydro-2*H*-pyran-2-yl)-3(2*H*)-pyridazinone) (1.38 g, 2.12 mmol) dissolved in methanol (9 mL) was added 1 mL of a 25 % w/w solution of sodium methoxide in methanol. The mixture was heated at reflux for 1 h and then quenched with 10 mL 1 N hydrochloric acid. The mixture was extracted with ethyl acetate, and the combined organic extracts were dried over magenesium sulfate, filtered and concentrated. The crude residue was purified by silica gel chromatography (0 to 100 % gradient of ethyl acetate in hexanes as eluant) to yield 0.31 g of the title compound as a solid.
¹H NMR (CDCl₃) δ 7.91 (s, 1H) 7.66 (d, 2H) 7.49 (m, 2H) 6.48 (s, 1H) 6.04 (d, 1H) 4.13 (m, 1H) 3.79 (s, 3H) 3.70 (m, 1H) 2.23 (s, 3H) 2.13 (m, 1H) 2.03 (bs., 1H) 1.67 (m, 4H).

### Step C 4-[3-(4-bromophenyl)-5-methyl-1H pyrazol-1-yl]-5-hydroxy-2-(tetrahydro-2H-pyran-2-yl)-3(2H)-pyridazinone

4-[3-(4-Bromophenyl)-5-methyl-1*H*-pyrazol-1-yl]-5-methoxy-2-(tetrahydro-2*H-*pyran-2-yl)-3(2*H*)-pyridazinone (i.e. the product of Step B) (0.29 g, 0.65 mmol) was added to a solution of potassium hydroxide (0.37 g, 6.5 mmol) in dioxane (8 mL) and water (8 mL), which was then heated at reflux for 2 h. The reaction mixture was neutralized with 10 mL 1 N hydrochloric acid and extracted with ethyl acetate yielding (0.28 g) of the title product, a compound of the present invention. AP⁺ = 432.

### EXAMPLE 10

### Preparation of 4-[3-(4-bromophenyl)-5-methyl-1H-pyrazol-1-yl]-5-hydroxy-3(2H)-pyridazinone (Compound 78)

To a solution of 4-[3-(4-bromophenyl)-5-methyl-1*H*-pyrazol-1-yl]-5-hydroxy-2-(tetrahydro-2*H*-pyran-2-yl)-3(2*H*)-pyridazinone (i.e. the product of Example 9, Step C) (0.22 g, 0.52 mmol) in methanol (4.0 mL) was added 0.42 mL of 6 M hydrochloric acid. The reaction mixture was heated at reflux for 2 h and then an additional 0.42 mL of 6 M hydrochloric acid was added, and heating was continued for an additional 3 h. The reaction mixture was cooled and then filtered through a sintered glass frit to provide the title product, a compound of the present invention, as a white solid (0.08 g) melting at 325-335° C (dec.). ¹H NMR (DMSO-*d₆*) δ 13.06 (s, 1H) 12.07 (bs, 1H) 7.87 (s, 1H) 7.72 (d, 2H) 7.59 (d, 2H) 6.68 (s, 1H) 2.10 (s, 3H).

### EXAMPLE 11

### Preparation of 5-[3-(4-bromophenyl)-5-methyl-1H-pyrazol-1-yl]-1-(cyclopropylcarbonyl)-1,6-dihydro-6-oxo-4-pyridazinyl cyclopropanecarboxylate (Compound 80)

To a solution of 4-[3-(4-bromophenyl)-5-methyl-1*H*-pyrazol-1-yl]-5-hydroxy-3(2*H*)-pyridazinone (i.e. the product of Example 10) (0.017 g, 0.049 mmol) in dichloromethane (2 mL) was added cyclopropylcarbonyl chloride (0.016 g, 0.16 mmol) and triethylamine (0.022 g, 0.22 mmol). The mixture was stirred for 18 h at room temperature, washed two times with saturated aqueous sodium carbonate solution, dried over magnesium sulfate and concentrated to yield the title compound (0.021 g), a compound of the present invention.
¹H NMR (CDCl₃) δ 7.96 (s, 1H) 7.66 (m, 2H) 7.51 (m, 2H) 6.48 (s, 1H) 2.88 (m, 1H) 2.71 (m, 1H) 2.32 (s, 3H) 1.69 (m, 2H) 1.19 (m, 4H) 1.03 (m, 2H).

By the procedures described herein together with methods known in the art, the following compounds of Tables 1A to 11 can be prepared. The following abbreviations are used in the Tables which follow: *t* means tertiary, *s* means secondary, *n* means normal, *i* means iso, *c* means cyclo, Me means methyl, Et means ethyl, Pr means propyl, *i*-Pr means isopropyl, Bu means butyl, Ph means phenyl, CS means thiocarbonyl (C(S)), CO means carbonyl (C(O)), CO₂ means carbonyloxy (C(O)O), SO means sulfinyl (S(O)), SO₂ means sulfonyl (S(O)₂), NO₂ means nitro, OMe means methoxy, OEt means ethoxy, SMe means methylthio, CN and -CN mean cyano, Ph means phenyl, Py means pyridinyl, TMS means trimethylsilyl, S(O)Me means methylsulfinyl, thien means thiophene, and S(O)₂Me means methylsulfonyl. In the Tables, "(R^{u})ₚ" denotes up to 5 instances of the substituent R^{u} as listed in the Tables.

**TABLE 1A**

| | | | |
|---|---|---|---|
| R³ | is H. | | 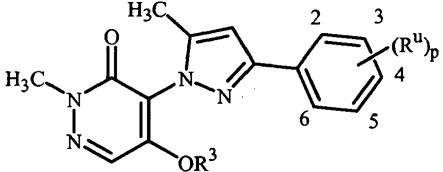 |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-I | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH2 | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCH₃ | 4-NHCOOMe | 3,4-di-F |
| 4-CF₂H | 4-SCF₂H | 4-NHSO₂Me | |
| R³ is CO₂-*i*-Pr. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-I | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4₋SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |

**TABLE 1B**

| | | | |
|---|---|---|---|
| 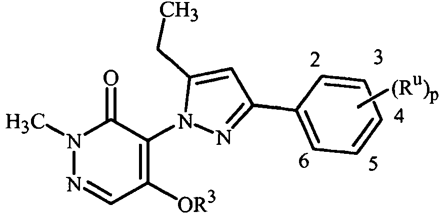 | | | |
| R³ is H. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH-CH₂ | 4-SOCF₃ | 4-Pyridin-3-yl |
| 4-I | 4-C≡ CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |
| R³ is CO₂-*i*-Pr. | | | |

| (R^{u})ₚ | (R^{u})ₚ | (R^{u})ₚ | (Ru)ₚ |
|---|---|---|---|
| 4-F | 4-CHO | 4-OCOMe | 4-SiMe₃ |
| 4-Cl | 4-CF₂H | 4-SMe | 4-NH₂ |
| 4-Br | 4-Me | 4-SCF₃ | 4-NMe₂ |
| 4-I | 4-Et | 4-SCF₂H | 4-NHCOMe |
| 4-CF₃ | 4-CH=CH₂ | 4-SOMe | 4-NHCOOMe |
| 4-CN | 4-C≡CH | 4-SO₂Me | 4-NHSO₂Me |
| 4-NO₂ | 4-COMe | 4-SOCF₃ | 4-Ph |
| 4-OH | 4-COCF₃ | 4-SOCF₂H | 4-Pyridin-2-yl |
| 4-OMe | 4-COOMe | 4-SO₂CF₃ | 4-Pyridin-3-yl |
| 4-OCF₃ | 4-CONH₂ | 4-SO₂CF₂H | 4-Pyridin-4-yl |
| 4-OCF₂H | 4-CONHMe | 4-SO₂NH₂ | 4-Thien-2-yl |
| 4-OCF₂CF₂H | 4-CONMe₂ | 4-SO₂NMe₂ | 4-SF₅ |
| 2-Cl | 2-F | 2,4-di-Cl | 3,4-di-F |
| 3-Cl | 3-F | 3,4-di-Cl | |

**TABLE 1C**

| | | | |
|---|---|---|---|
| 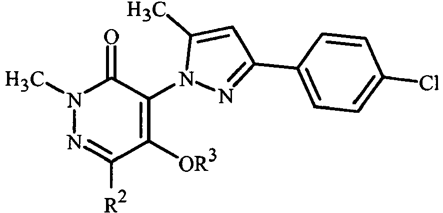 | | | |
| R² is H. | | | |

| R³ | R³ | R³ | R³ |
|---|---|---|---|
| COMe | COO-*i*-Pr | SO₂CH=CF | PO(Et)OEt |
| COCH=CH₂ | COO-*t*-Bu | SO₂-*c*-Pentyl | PS(OEt)₂ |
| COC≡CH | COO-*i*-Bu | SO₂-*c*-Pr | PS(Me)OEt |
| COCF₃ | CSSMe | SO₂-(2,2-di-Cl-*c*-Pr) | PS(Me)SMe |
| COCH₂CH=CF₂ | COSMe | SO₂-(1-Me-*c*-Pr) | PO(Me)SMe |
| CO-*c*-Pentyl | CSOMe | SO₂-*c*-(3-hexen-1-yl) | PO(OMe)₂ |
| CO-*c*-Pr | COOCH₂CH=CH₂ | SO₂CH₂CH₂O | PS(OMe)₂ |
| CO-(2,2-di-Cl-*c*-Pr) | COOCH₂C≡ CH | SO₂CH₂CH₂SMe | PO(NMe₂)₂ |
| CO-(1-Me-*c*-Pr) | COCH₂CF₃ | SO₂CH₂CH₂SOMe | PS(NMe₂)₂ |
| CO-*c*-(3-hexen-1-yl) | COOCH₂CH=CF₂ | SO₂CH₂CH₂SO₂Me | CO-2-Cl-Ph |
| COCH₂CH₂OMe | COO-*c*-Pentyl | SO₂CH₂CH₂NMe | CO-3-Cl-Ph |
| COCH₂CH₂SMe | COOCH₂-*c*-Pr | SO₂Et | CO-4-Cl-Ph |
| COCH₂CH₂SOMe | COCH₂-(2,2-di-Cl-*c*- | SO₂-*n*-Pr | CO-2-F-Ph |
| COCH₂CH₂SO₂Me | Pr) | SO₂-*i*-Pr | CO-3-F-Ph |
| COCH₂CH₂NMe₂ | COCH₂-(1-Me-*c*-Pr) | SO₂-*t*-Bu | CO-4-F-Ph |
| COEt | COO-*c*-(3-hexen-1-yl) | SO₂-*i*-Bu | CO-2-CF₃-Ph |
| CO-*n*-Pr | COOCH₂CH₂OMe | SO₂CH₂C(CH₃)₃ | CO-3-CF₃-Ph |
| CO-*i*-Pr | COOCH₂CH₂SMe | SO₂Ph | CO-4-CF₃-Ph |
| CO-*t*-Bu | COOCH₂CH₂SOMe | SO₂CH₂Ph | SO₂-2-Cl-Ph |
| CO-*i*-Bu | COOCH₂CH₂SO₂M | SO₂NHMe | SO₂-3-Cl-Ph |
| COCH₂C(CH₃)₃ | COOCH₂CH₂NMe₂ | SO₂NHCH₂CF₃ | SO₂-4-Cl-Ph |
| COPh | SO₂Me | SO₂NMe₂ | SO₂-2-F-Ph |
| COCH₂Ph | SO₂CH=CH₂ | SO₂NH-*c*-Pr | SO₂-3-F-Ph |
| COOMe | SO₂C≡ CH | PO(OEt)₂ | SO₂-4-F-Ph |
| COOEt | SO₂CF₃ | PO(Me)OEt | SO₂-2-CF₃-Ph |

| R³ | R³ | R³ | |
|---|---|---|---|
| SO₂-3-CF₃-Ph | CO-2-OMe-Ph | CO-4-OMe-Ph | |
| SO₂-4-CF₃-Ph | CO-3-OM e-Ph | | |
| R² is CH₃. | | | |

| R³ | R³ | R³ | R³ |
|---|---|---|---|
| COMe | COO-*i*-Bu | SO₂-(1-Me-*c*-Pr) | PS(OMe)₂ |
| COCH=CH₂ | CSSMe | SO₂-*c*-(3-hexen-1-yl) | PO(NMe2)₂ |
| COC≡CH | COSMe | SO₂CH₂CH₂OMe | PS(NMe₂)₂ |
| COCF₃ | CSOMe | SO₂CH₂CH₂SMe | CO-2-Cl-Ph |
| COCH₂CH=CF₂ | COOCH₂CH=CH₂ | SO₂CH₂CH₂SOMe | CO-3-Cl-Ph |
| CO-*c*-Pentyl | COOCH₂C≡CH | SO₂CH₂CH₂SO₂Me | CO-4-Cl-Ph |
| CO-*c*-Pr | COCH₂CF₃ | SO₂CH₂CH₂NMe₂ | CO-2-F-Ph |
| CO-(2,2-di-Cl-*c*-Pr) | COOCH₂CH=CF₂ | SO₂Et | CO-3-F-Ph |
| CO-(1-Me-*c*-Pr) | COO-*c*-Pentyl | SO₂-*n*-Pr | CO-4-F-Ph |
| CO-*c*-(3-hexen-1-yl) | COOCH₂-*c*-Pr | SO₂-*i*-Pr | CO-2-CF₃-Ph |
| COCH₂CH₂OMe | COCH₂-(2,2-di-Cl-*c-*Pr) | SO₂-*t*-Bu | CO-3-CF₃-Ph |
| COCH₂CH₂SMe | | SO₂-*i*-Bu | CO-4-CF₃-Ph |
| COCH₂CH₂SOMe | COCH₂-(1-Me-*c*-Pr) | SO₂CH₂C(CH₃)₃ | SO₂-2-Cl-Ph |
| COCH₂CH₂SO₂Me | COO-*c*-(3-hexen-1-yl) | SO₂Ph | SO₂-3-Cl-Ph |
| COCH₂CH₂NMe₂ | COOCH₂CH₂OMe | SO₂CH₂Ph | SO₂-4-Cl-Ph |
| COEt | COOCH₂CH₂SMe | SO₂NHMe | SO₂-2-F-Ph |
| CO-*n*-Pr | COOCH₂CH₂SOMe | SO₂NHCH₂CF₃ | SO₂-3-F-Ph |
| CO-*i*-Pr | COOCH₂CH₂SO₂Me | SO₂NMe₂ | SO₂-4-F-Ph |
| CO-*t*-Bu | COOCH₂CH₂NMe₂ | SO₂NH-*c*-Pr | SO₂-2-CF₃-Ph |
| CO-*i*-Bu | SO₂Me | PO(OEt)₂ | SO₂-3-CF₃-Ph |
| COCH₂C(CH₃)₃ | SO₂CH=CH₂ | PO(Me)OEt | SO₂-4-CF₃-Ph |
| COPh | SO₂C≡CH | PO(Et)OEt | CO-2-OMe-Pb |
| COCH₂Ph | SO₂CF₃ | PS(OEt)₂ | CO-3-OMe-Ph |
| COOMe | SO₂CH=CF₂ | PS(Me)OEt | CO-4-OMe-Ph |
| COOEt | SO₂-*c*-Penty | PS(Me)SMe | |
| COO-*i*-Pr | SO₂-*c*-Pr | PO(Me)SMe | |
| COO-*t*-Bu | SO₂-(2,2-di-Cl-*c*-Pr) | PO(OMe)₂ | |

**TABLE 1D**

| | | | | | | | |
|---|---|---|---|---|---|---|---|
| 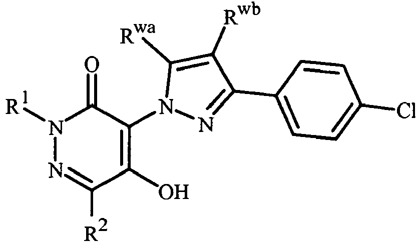 | | | | | | | |

| R¹ | R² | R^{wa} | R^{wb} | R¹ | R² | R^{wa} | R^{wb} |
|---|---|---|---|---|---|---|---|
| Me | Et | Me | H | CH₂C≡CH | H | Me | H |
| Me | *n*-Pr | Me | H | CH₂CF₃ | H | Me | H |
| Me | *i*-Pr | Me | H | *c*-Pr | H | Me | H |
| Me | *i*-Bu | Me | H | CH₂-*c*-Pr | H | Me | H |
| Me | *t*-Bu | Me | H | *c*-(3-hexen-1-yl) | H | Me | H |
| Me | *n*-Bu | Me | H | CH₂CH₂OMe | H | Me | H |
| Me | *c*-Pr | Me | H | CH₂OMe | H | Me | H |
| Me | CH₂CH=CH₂ | Me | H | CH₂CH₂SMe | H | Me | H |
| Me | C≡CH | Me | H | CH₂CH₂SOMe | H | Me | H |
| Me | F | Me | H | CH₂CH₂SO₂Me | H | Me | H |
| Me | Cl | Me | H | Me | H | Me | Me |
| Me | Br | Me | H | Me | H | Me | Cl |
| Me | CN | Me | H | Me | H | Me | Br |
| Me | CONH₂ | Me | H | Me | H | Me | F |
| Me | COOMe | Me | H | Me | H | Me | CF₃ |
| Me | CH₂OMe | Me | H | Me | H | Me | H |
| Me | CH₂SMe | Me | H | Me | H | Me | CN |
| Me | CH₂SOMe | Me | H | Me | H | Me | NO₂ |
| Me | CH₂SO₂Me | Me | H | Me | H | Me | SMe |
| Me | OMe | Me | H | Me | H | Me | SCF₃ |
| Me | OEt | Me | H | Me | H | H | H |
| Me | OCF₂H | Me | H | Me | H | Et | H |
| Me | SMe | Me | H | Me | H | *n*-Pr | H |
| Me | SCH₂CF₃ | Me | H | Me | H | *c*-Pr | H |
| H | H | Me | H | Me | H | *i-*Pr | H |
| Et | H | Me | H | Me | H | F | H |
| *n*-Pr | H | Me | H | Me | H | Cl | H |
| CH₂CH=CH₂ | H | Me | H | Me | H | Br | H |
| Me | H | CN | H | Me | OMe | *c*-Pr | H |
| Me | H | SMe | H | Me | OMe | Cl | H |
| Me | H | CF₃ | H | Me | OMe | Br | H |
| Me | H | Et | Me | Me | OMe | Et | Me |
| Me | H | *n*-Pr | Me | Me | OMe | Me | Me |
| Me | H | *i*-Pr | Me | Me | OEt | Et | H |
| Me | H | *i*-Bu | Me | Me | OEt | Et | Me |
| Me | H | *t*-Bu | Me | Me | NO₂ | Et | H |
| Me | H | *n*-Bu | Me | Me | NO₂ | Me | H |
| Me | H | *c*-Pr | Me | Me | F | Et | H |
| Me | H | F | Me | Me | Cl | Et | H |
| Me | H | Cl | Me | Me | Br | Et | H |
| Me | H | Br | Me | Et | H | Et | H |
| Me | H | CN | Me | Et | H | *n*-Pr | H |
| Me | H | SMe | Me | Et | H | *i*-Pr | H |
| Me | H | CF₃ | Me | Et | H | *i*-Bu | H |
| Me | H | OMe | Me | Et | H | *t*-Bu | H |
| Me | H | Et | Cl | Et | H | *n*-Bu | H |
| Me | H | *n*-Pr | Cl | Et | H | *c*-Pr | H |
| Me | H | *i*-Pr | Cl | Et | H | F | H |
| Me | H | *i*-Bu | Cl | Et | H | Cl | H |
| Me | H | *t*-Bu | Cl | Et | H | Br | H |
| Me | H | *n*-Bu | Cl | Et | H | Et | Me |
| Me | H | *c*-Pr | Cl | H | H | Et | H |
| Me | H | F | Cl | H | H | *n*-Pr | H |
| Me | H | Cl | Cl | H | H | *i*-Pr | H |
| Me | H | Br | Cl | H | H | *i*-Bu | H |
| Me | H | CN | Cl | H | H | *t*-Bu | H |
| Me | H | SMe | Cl | H | H | *n*-Bu | H |
| Me | H | CF₃ | Cl | H | H | *c*-Pr | H |
| Me | H | OMe | Cl | H | H | F | H |
| Me | H | Et | F | H | H | Cl | H |
| Me | H | *n*-Pr | F | H | H | Br | H |
| Me | OMe | Et | H | H | H | Et | Me |
| Me | OMe | *n*-Pr | H | | | | |

**TABLE 1E**

| |
|---|
| Table 1E is constructed the same as Table 1D, except that the chemical structure under the Table 1D heading is replaced with the following structure: |
| 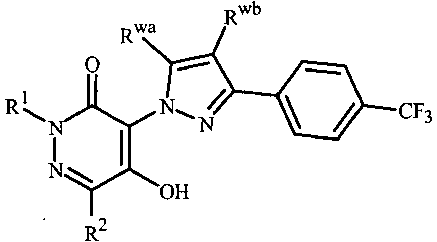 |

**TABLE 1F**

| |
|---|
| Table 1F is constructed the same as Table 1D, except that the chemical structure under the Table 1D heading is replaced with the following structure: |
| 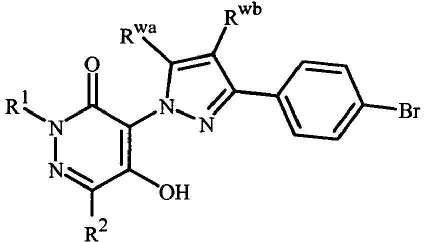 |

**TABLE 1G**

| | | | | | |
|---|---|---|---|---|---|
| 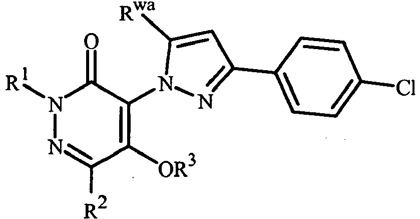 | | | | | |
| R² is H. | | | | | |

| R¹ | R³ | R^{wa} | R¹ | R³ | R^{wa} |
|---|---|---|---|---|---|
| COEt | COEt | Et | CO-*i*-Bu | CO-*i*-Bu | Et |
| CO-*n*-Pr | CO-*n*-Pr | Et | COCH₂C(CH₃)₃ | COCH₂C(CH₃)₃ | Et |
| CO-*i*-Pr | CO-*i*-Pr | Et | COPh | COPh | Et |
| CO-*t*-Bu | CO-*t*-Bu | Et | COOMe | COOMe | Et |
| COOEt | COOEt | Et | H | SO₂-*n*-Pr | Et |
| COO-*i*-Pr | COO-*i*-Pr | Et | H | SO₂-*i*-Pr | Et |
| COO-*t*-Bu | COO-*t*-Bu | Et | H | SO₂-*t*-Bu | Et |
| COO-*i*-Bu | COO-*i*-Bu | Et | H | SO₂*-i*-Bu | Et |
| COCH₂CF₃ | COCH₂CF₃ | Et | H | SO₂CH₂C(CH₃)₃ | Et |
| SO₂Me | SO₂Me | Et | H | SO₂Ph | Et |
| SO₂CF₃ | SO₂CF₃ | Et | H | SO₂NMe₂ | Et |
| SO₂-*c*-Pr | SO₂-*c*-Pr | Et | H | PO(OEt)₂ | Et |
| SO₂Et | SO₂Et | Et | H | PO(Me)OEt | Et |
| SO₂-*n*-Pr | SO₂-*n*-Pr | Et | H | PS(OEt)₂ | Et |
| SO₂-*i*-Pr | SO₂*-i*-Pr | Et | H | PO(OMe)₂ | Et |
| SO₂-*t*-Bu | SO₂-*t*-Bu | Et | COEt | COEt | Me |
| SO₂-*i*-Bu | SO₂-*i*-Bu | Et | CO-*n*-Pr | CO-*n*-Pr | Me |
| SO₂CH₂C(CH₃)₃ | SO₂CH₂C(CH₃)₃ | Et | CO-*i*-Pr | CO*-i*-Pr | Me |
| SO₂Ph | SO₂Ph | Et | CO-*t*-Bu | CO-*t*-Bu | Me |
| SO₂NMe₂ | SO₂NMe₂ | Et | CO-*i*-Bu | CO*-i*-Bu | Me |
| PO(OEt)₂ | PO(OEt)₂ | Et | COCH₂C(CH₃)₃ | COCH₂C(CH₃)₃ | Me |
| PO(Me)OEt | PO(Me)OEt | Et | COPh | COPh | Me |
| PS(OEt)₂ | PS(OEt)₂ | Et | COOMe | COOMe | Me |
| PO(OMe)₂ | PO(OMe)₂ | Et | COOEt | COOEt | Me |
| H | COEt | Et | COO-*i*-Pr | COO-*i*-Pr | Me |
| H | CO-*n*-Pr | Et | COO-*t*-Bu | COO-*t*-Bu | Me |
| H | CO-*i*-Pr | Et | COO*-i*-Bu | COO*-i*-Bu | Me |
| H | CO-*t*-Bu | Et | COCH₂CF₃ | COCH₂CF₃ | Me |
| H | CO-*i*-Bu | Et | SO₂Me | SO₂Me | Me |
| H | COCH₂C(CH₃)₃ | Et | SO₂CF₃ | SO₂CF₃ | Me |
| H | COPh | Et | SO₂-*c*-Pr | SO₂-*c*-Pr | Me |
| H | COOMe | Et | SO₂Et | SO₂Et | Me |
| H | COOEt | Et | SO₂-*n*-Pr | SO₂-*n*-Pr | Me |
| H | COO-*i*-Pr | Et | SO₂-*i*-Pr | SO₂*-i*-Pr | Me |
| H | COO-*t*-Bu | Et | SO₂-*t*-Bu | SO₂-*t*-Bu | Me |
| H | COO-*i*-Bu | Et | SO₂-*i*-Bu | SO₂*-i*-Bu | Me |
| H | COCH₂CF₃ | Et | SO₂CH₂C(CH₃)₃ | SO₂CH₂C(CH₃)₃ | Me |
| H | SO₂Me | Et | SO₂Ph | SO₂Ph | Me |
| H | SO₂CF₃ | Et | SO₂NMe₂ | SO₂NMe₂ | Me |
| H | SO₂-*c*-Pr | Et | PO(OEt)₂ | PO(OEt)₂ | Me |
| H | SO₂Et | Et | PO(Me)OEt | PO(Me)OEt | Me |
| PS(OEt)₂ | PS(OEt)₂ | Me | H | SO₂Me | Me |
| PO(OMe)₂ | PO(OMe)₂ | Me | H | SO₂CF₃ | Me |
| H | COEt | Me | H | SO₂-*c*-Pr | Me |
| H | CO-*n*-Pr | Me | H | SO₂Et | Me |
| H | CO-*i*-Pr | Me | H | SO₂-*n*-Pr | Me |
| H | CO-*t*-Bu | Me | H | SO₂-*i*-Pr | Me |
| H | CO-*i*-Bu | Me | H | SO₂-*t*-Bu | Me |
| H | COCH₂C(CH₃)₃ | Me | H | SO₂-*i*-Bu | Me |
| H | COPh | Me | H | SO₂CH₂C(CH₃)₃ | Me |
| H | COOMe | Me | H | SO₂Ph | Me |
| H | COOEt | Me | H | SO₂NMe₂ | Me |
| H | COO-*i*-Pr | Me | H | PO(OEt)₂ | Me |
| H | COO-*t*-Bu | Me | H | PO(Me)OEt | Me |
| H | COO-*i*-Bu | Me | H | PS(OEt)₂ | Me |
| H | COCH₂CF₃ | Me | H | PO(OMe)₂ | Me |

**TABLE 1GA**

| |
|---|
| Table 1GA is constructed the same as Table 1G, except that "R² is H." below the chemical structure under the Table 1G heading is replaced with "R² is Me.". |

**TABLE 1 GB**

| |
|---|
| Table 1GB is constructed the same as Table 1G, except that "R² is H." below the chemical structure under the Table 1G heading is replaced with "R² is OMe.". |

**TABLE 1H**

| |
|---|
| Table 1H is constructed the same as Table 1G, except that the chemical structure under the Table 1G heading is replaced with the following structure: |
| 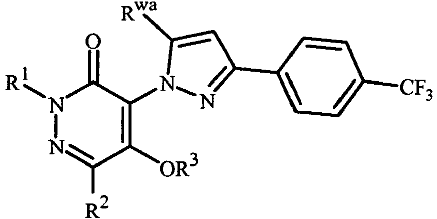 |

**TABLE 1HA**

| |
|---|
| Table 1HA is constructed the same as Table 1H, except that "R² is H." is replaced with "R² is Me.". |

**TABLE 1HB**

| |
|---|
| Table 1HB is constructed the same as Table 1H, except that "R² is H." is replaced with "R² is OMe.". |

**TABLE 1I**

| |
|---|
| Table 1I is constructed the same as Table 1G, except that the chemical structure under the Table 1G heading is replaced with the following structure: |
| 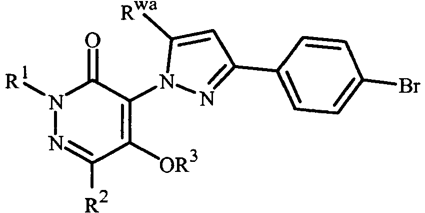 |

**TABLE 1IA**

| |
|---|
| Table 1IA is constructed the same as Table 1I, except that "R² is H." is replaced with "R² is Me.". |

**TABLE 1IB**

| |
|---|
| Table 1IB is constructed the same as Table 1I, except that "R² is H." is replaced with "R² is OMe.". |

**TABLE 2A**

| | | | |
|---|---|---|---|
| 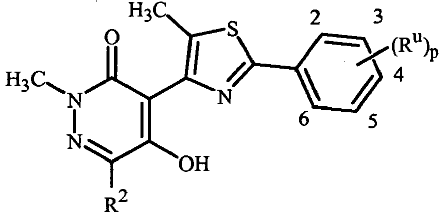 | | | |
| R² is H. | | | |

| (R^{u})ₚ | (R^{u})ₚ | (R^{u})ₚ | (R^{u})ₚ |
|---|---|---|---|
| 4-F | 4-Br | 4-CF₃ | 4-NO₂ |
| 4-Cl | 4-I | 4-CN | 4-OH |
| 4-OMe | 4-COOMe | 4-SO₂CF₃ | 4-Pyridin-3-yl |
| 4-OCF₃ | 4-CONH₂ | 4-SO₂CF₂H | 4-Pyridin-4-yl |
| 4-OCF₂H | 4-CONHMe | 4-SO₂NH₂ | 4-Thien-2-yl |
| 4-OCF₂CF₂H | 4-CONMe₂ | 4-SO₂NMe₂ | 4-SF₅ |
| 4-CHO | 4-OCOMe | 4-SiMe₃ | 2-Cl |
| 4-CF₂H | 4-SMe | 4-NH₂ | 3-Cl |
| 4-Me | 4-SCF₃ | 4-NMe₂ | 2-F |
| 4-Et | 4-SCF₂H | 4-NHCOMe | 3-F |
| 4-CH=CH₂ | 4-SOMe | 4-NHCOOMe | 2,4-di-Cl |
| 4-C≡CH | 4-SO₂Me | 4-NHSO₂Me | 3,4-di-Cl |
| 4-COMe | 4-SOCF₃ | 4-Ph | 3,4-di-F |
| 4-COCF₃ | 4-SOCF₂H | 4-Pyridin-2-yl | |
| R² is CH₃. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-1 | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |

**TABLE 2B**

| | | | | | |
|---|---|---|---|---|---|
| 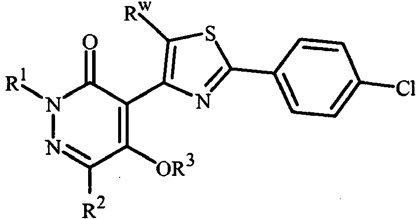 | | | | | |
| R¹ is Me and R^{W} is Me. | | | | | |

| R² | R³ | | R² | R³ | |
|---|---|---|---|---|---|
| H | COMe | | *n*-Bu | H | |
| H | COCH=CH₂ | | *c*-Pr | H | |
| H | COC≡CH | | CH₂CH=CH₂ | H | |
| H | COCF₃ | | C≡CH | H | |
| H | COCH₂CH=CF₂ | | F | H | |
| H | CO-*c*-Pentyl | | Cl | H | |
| H | CO-*c*-Pr | | Br | H | |
| H | CO-(2,2-di-Cl-*c*-Pr) | | -CN | H | |
| H | CO-(1-Me-*c*-Pr) | | CONH₂ | H | |
| H | CO-*c*-(3-hexen-1-yl) | | COOMe | H | |
| H | COCH₂CH₂OMe | | CH₂OMe | H | |
| H | COCH₂CH₂SMe | | CH₂SMe | H | |
| H | COCH₂CH₂SOMe | | CH₂SOMe | H | |
| H | COCH₂CH₂SO₂Me | | CH₂SO₂Me | H | |
| H | COCH₂CH₂NMe₂ | | OMe | H | |
| Et | H | | OEt | H | |
| *n*-Pr | H | | OCF₂H | H | |
| *i*-Pr | H | | SMe | H | |
| *i*-Bu | H | | SCH₂CF₃ | H | |
| *t*-Bu | H | | | | |
| R² is H and R³ is H. | | | | | |

| R¹ | R^{w} | R¹ | R^{w} | R¹ | R^{w} |
|---|---|---|---|---|---|
| H | Me | CH₂CH₂OMe | Me | Me | *i*-Pr |
| Et | Me | CH₂OMe | Me | Me | F |
| *n*-Pr | Me | CH₂CH₂SMe | Me | Me | Cl |
| CH₂CH=CH₂ | Me | CH₂CH₂SOMe | Me | Me | Br |
| CH₂C≡CH | Me | CH₂CH₂SO₂Me | Me | Me | -CN |
| CH₂CF₃ | Me | Me | H | Me | SMe |
| *c*-Pr | Me | Me | Et | Me | CF₃ |
| CH₂-c-Pr | Me | Me | *n*-Pr | | |
| *c*-(3-hexen-1-yl) | Me | Me | *c*-Pr | | |

**TABLE 3A**

| | | | | | |
|---|---|---|---|---|---|
| 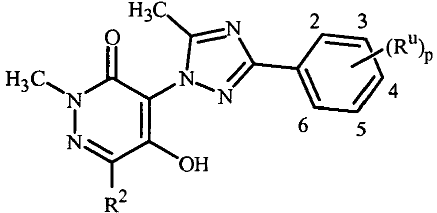 | | | | | |
| R² is H. | | | | | |

| (R^{u})ₚ | (R^{u})ₚ | (R^{u})ₚ | (R^{u})ₚ | | |
|---|---|---|---|---|---|
| 4-F | 4-Me | 4-SOMe | 4-Ph | | |
| 4-Cl | 4-Et | 4-SO₂Me | 4-Pyridin-2-yl | | |
| 4-Br | 4-CH≡CH₂ | 4-SOCF₃ | 4-Pyridin-3-yl | | |
| 4-I | 4-C≡CH | 4-SOCF₂H | 4-Pyridin-4-yl | | |
| 4-CF₃ | 4-COMe | 4-SO₂CF₃ | 4-Thien-2-yl | | |
| 4-CN | 4-COCF₃ | 4-SO₂CF₂H | 4-SF₅ | | |
| 4-NO₂ | 4-COOMe | 4-SO₂NH₂ | 2-Cl | | |
| 4-OH | 4-CONH₂ | 4-SO₂NMe₂ | 3-Cl | | |
| 4-OMe | 4-CONHMe | 4-SiMe₃ | 2-F | | |
| 4-OCF₃ | 4-CONMe₂ | 4-NH₂ | 3-F | | |
| 4-OCF₂H | 4-OCOMe | 4-NMe₂ | 2,4-di-Cl | | |
| 4-OCF₂CF₂H | 4-SMe | 4-NHCOMe | 3,4-di-Cl | | |
| 4-CHO | 4-SCF₃ | 4-NHCOOMe | 3,4-di-F | | |
| 4-CF₂H | 4-SCF₂H | 4-NHSO₂Me | | | |
| R² is CH₃. | | | | | |

| (R^{u})ₚ | (R^{u})ₚ | (R^{u})ₚ | (R^{u})ₚ | | |
|---|---|---|---|---|---|
| 4-F | 4-CHO | 4-OCOMe | 4-SiMe₃ | | |
| 4-Cl | 4-CF₂H | 4-SMe | 4-NH₂ | | |
| 4-Br | 4-Me | 4-SCF₃ | 4-NMe₂ | | |
| 4-1 | 4-Et | 4-SCF₂H | 4-NHCOMe | | |
| 4-CF₃ | 4-CH=CH₂ | 4-SOMe | 4-NHCOOMe | | |
| 4-CN | 4-C≡CH | 4-SO₂Me | 4-NHSO₂Me | | |
| 4-NO₂ | 4-COMe | 4-SOCF₃ | 4-Ph | | |
| 4-OH | 4-COCF₃ | 4-SOCF₂H | 4-Pyridin-2-yl | | |
| 4-OMe | 4-COOMe | 4-SO₂CF₃ | 4-Pyridin-3-yl | | |
| 4-OCF₃ | 4-CONH₂ | 4-SO₂CF₂H | 4-Pyridin-4-yl | | |
| 4-OCF₂H | 4-CONHMe | 4-SO₂NH₂ | 4-Thien-2-yl | | |
| 4-OCF₂CF₂H | 4-CONMe₂ | 4-SO₂NMe₂ | 4-SF₅ | | |

| | (R^{u})ₚ | (R^{u})ₚ | (R^{u})ₚ | (R^{u})ₚ | |
|---|---|---|---|---|---|
| | 2-Cl | 2-F | 2,4-di-Cl | 3,4-di-F | |
| | 3-Cl, | 3-F | 3,4-di-Cl | | |

**TABLE 3B**

| | | | | | | | |
|---|---|---|---|---|---|---|---|
| 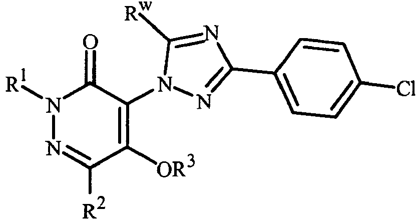 | | | | | | | |
| R¹ is Me and R^{w} is Me. | | | | | | | |

| R¹ | R² | R³ | R^{w} | R¹ | R² | R³ | R^{w} |
|---|---|---|---|---|---|---|---|
| Me | H | COMe | Me | Me | *n*-Bu | H | Me |
| Me | H | COCH=CH₂ | Me | Me | *c*-Pr | H | Me |
| Me | H | COC≡CH | Me | Me | CH₂CH=CH₂ | H | Me |
| Me | H | COCF₃ | Me | Me | C≡CH | H | Me |
| Me | H | COCH₂CH=CF₂ | Me | Me | F | H | Me |
| Me | H | CO-*c*-Pentyl | Me | Me | Cl | H | Me |
| Me | H | CO-*c*-Pr | Me | Me | Br | H | Me |
| Me | H | CO-(2,2-di-Cl-*c*-Pr) | Me | Me | -CN | H | Me |
| Me | H | CO-(1-Me-*c*-Pr) | Me | Me | CONH₂ | H | Me |
| Me | H | CO-*c*-(3-hexen-1-yl) | Me | Me | COOMe | H | Me |
| Me | H | COCH₂CH₂OMe | Me | Me | CH₂OMe | H | Me |
| Me | H | COCH₂CH₂SMe | Me | Me | CH₂SMe | H | Me |
| Me | H | COCH₂CH₂SOMe | Me | Me | CH₂SOMe | H | Me |
| Me | H | COCH₂CH₂SO₂Me | Me | Me | CH₂SO₂Me | H | Me |
| Me | H | COCH₂CH₂NMe₂ | Me | Me | OMe | H | Me |
| Me | Et | H | Me | Me | OEt | H | Me |
| Me | *n*-Pr | H | Me | Me | OCF₂H | H | Me |
| Me | *i*-Pr | H | Me | Me | SMe | H | Me |
| Me | *i*-Bu | H | Me | Me | SCH₂CF₃ | H | Me |
| Me | *t*-Bu | H | Me | | | | |
| R² is H and R³ is H. | | | | | | | |

| | R¹ | R^{w} | R¹ | | R^{w} | R¹ | R^{w} |
|---|---|---|---|---|---|---|---|
| | H | Me | *n*-Pr | | Me | CH₂C≡CH Me | |
| | Et | Me | CH₂CH=CH₂ | | Me | CH₂CF₃ | Me |

| R¹ | R^{w} | R¹ | R^{w} | R¹ | R^{w} | | |
|---|---|---|---|---|---|---|---|
| *c*-Pr | Me | CH₂CH₂SO₂Me | Me | Me | Cl | | |
| CH₂-*c*-Pr | Me | Me | H | Me | Br | | |
| *c*-(3-hexen-1-yl) | Me | Me | Et | Me | CN | | |
| CH₂CH₂OMe | Me | Me | *n*-Pr | Me | SMe | | |
| CH₂OMe | Me | Me | *c*-Pr | Me | CF₃ | | |
| CH₂CH₂SMe | Me | Me | *i*-Pr | Et | Me | | |
| CH₂CH₂SOMe | Me | Me | F | | | | |

**TABLE 4A**

| | | | |
|---|---|---|---|
| 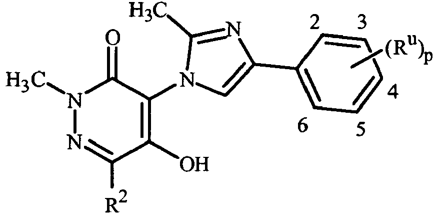 | | | |
| R² is H. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-1 | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |
| R² is CH₃. | | | |

| (R^{u})ₚ | (R^{u})ₚ | (R^{u})ₚ | (R^{u})ₚ |
|---|---|---|---|
| 4-F | 4-I | 4-NO₂ | 4-OCF₃ |
| 4-Cl | 4-CF₃ | 4-OH | 4-OCF₂H |
| 4-Br | 4-CN | 4-OMe | 4-OCF₂CF₂H |
| 4-CHO | 4-CONMe₂ | 4-SO₂NH₂ | 4-Pyridin-4-yl |
| 4-CF₂H | 4-OCOMe | 4-SO₂NMe₂ | 4-Thien-2-yl |
| 4-Me | 4-SMe | 4-SiMe₃ | 4-SF₅ |
| 4-Et | 4-SCF₃ | 4-NH₂ | 2-Cl |
| 4-CH=CH₂ | 4-SCF₂H | 4-NMe₂ | 3-Cl |
| 4-C≡CH | 4-SOMe | 4-NHCOMe | 2-F |
| 4-COMe | 4-SO₂Me | 4-NHCOOMe | 3-F |
| 4-COCF₃ | 4-SOCF₃ | 4-NHSO₂Me | 2,4-di-Cl |
| 4-COOMe | 4-SOCF₂H | 4-Ph | 3,4-di-Cl |
| 4-CONH₂ | 4-SO₂CF₃ | 4-Pyridin-2-yl | 3,4-di-F |
| 4-CONHMe | 4-SO₂CF₂H | 4-Pyridin-3-yl | |

**TABLE 4B**

| | | | | | | | |
|---|---|---|---|---|---|---|---|
| 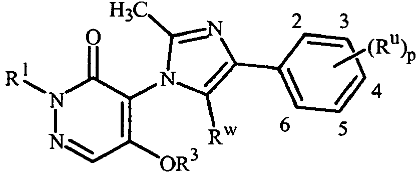 | | | | | | | |

| R¹ | R³ | R^{w} | (R^{u})ₚ | R¹ | R³ | R^{w} | (R^{u})ₚ |
|---|---|---|---|---|---|---|---|
| Me | H | Me | 4-Cl | Me | H | Me | 2-Cl |
| Me | H | Me | 4-F | Me | H | Me | 3-Cl |
| Me | H | Me | 4-Br | Me | H | Me | 2-F |
| Me | H | Me | 4-CF₃ | Me | H | Me | 3-F |
| Me | H | Me | 4-CN | Me | H | Me | 2,4-di-Cl |
| Me | H | Me | 4-NO₂ | Me | H | Me | 3,4-di-Cl |
| Me | H | Me | 4-OMe | Me | H | Me | 3,4-di-F |
| Me | H | Me | 4-OCF₃ | Me | COEt | H | 4-Cl |
| Me | H | Me | 4-OCF₂H | Me | CO-*n*-Pr | H | 4-Cl |
| Me | H | Me | 4-OCF₂CF₂H | Me | CO-*i*-Pr | H | 4-Cl |
| Me | H | Me | 4-CHO | Me | CO-*t*-Bu | H | 4-Cl |
| Me | H | Me | 4-CF₂H | Me | CO-*i*-Bu | H | 4-Cl |
| Me | H | Me | 4-Me | Me | COCH₂C(CH₃)₃ | H | 4-Cl |
| Me | H | Me | 4-Et | Me | COPh | H | 4-Cl |
| Me | H | Me | 4-SMe | Me | COOMe | H | 4-Cl |
| Me | H | Me | 4-SCF₃ | Me | COOEt | H | 4-Cl |
| Me | H | Me | 4-SCF₂H | Me | COO-*i*-Pr | H | 4-Cl |
| Me | COO-*t*-Bu | H | 4-Cl | Me | SO₂-*i*-Bu | H | 4-Cl |
| Me | COO-*i*-Bu | H | 4-Cl | Me | SO₂CH₂C(CH₃)₃ | H | 4-Cl |
| Me | COCH₂CF₃ | H | 4-Cl | Me | SO₂Ph | H | 4-Cl |
| Me | SO₂Me | H | 4-Cl | Me | SO₂NMe₂ | H | 4-Cl |
| Me | SO₂CF₃ | H | 4-Cl | Me | PO(OEt)₂ | H | 4-Cl |
| Me | SO₂-*c*-Pr | H | 4-Cl | Me | PO(Me)OEt | H | 4-Cl |
| Me | SO₂Et | H | 4-Cl | Me | PS(OEt)₂ | H | 4-Cl |
| Me | SO₂-*n*-Pr | H | 4-Cl | Me | PO(OMe)₂ | H | 4-Cl |
| Me | SO₂-*i*-Pr | H | 4-Cl | Et | H | H | 4-Cl |
| Me | SO₂-*t*-Bu | H | 4-Cl | | | | |

**TABLE 5A**

| | | | |
|---|---|---|---|
| 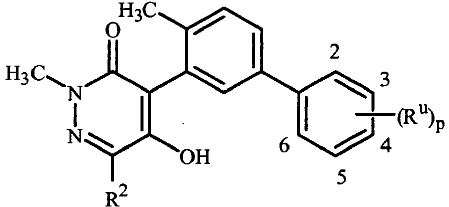 | | | |
| R² is H. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-I | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |
| R² is CH₃. | | | |
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-I | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |

**TABLE 5B**

| | | | | | | | |
|---|---|---|---|---|---|---|---|
| 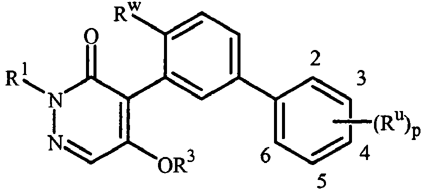 | | | | | | | |

| R¹ | R³ | R^{w} | (R^{u})ₚ | R¹ | R³ | R^{w} | (R^{u})ₚ |
|---|---|---|---|---|---|---|---|
| Me | COEt | Me | 4-Cl | Me | SO₂Me | Me | 4-Cl |
| Me | CO-*n*-Pr | Me | 4-Cl | Me | SO₂CF₃ | Me | 4-Cl |
| Me | CO-*i*-Pr | Me | 4-Cl | Me | SO₂-*c*-Pr | Me | 4-Cl |
| Me | CO-*t*-Bu | Me | 4-Cl | Me | SO₂Et | Me | 4-Cl |
| Me | CO-*i*-Bu | Me | 4-Cl | Me | SO₂-*n*-Pr | Me | 4-Cl |
| Me | COCH₂C(CH₃)₃ | Me | 4-Cl | Me | SO₂-*i*-Pr | Me | 4-Cl |
| Me | COPh | Me | 4-Cl | Me | SO₂-*t*-Bu | Me | 4-Cl |
| Me | COOMe | Me | 4-Cl | Me | SO₂-*i*-Bu | Me | 4-Cl |
| Me | COOEt | Me | 4-Cl | Me | SO₂CH₂C(CH₃)₃ | Me | 4-Cl |
| Me | COO-*i*-Pr | Me | 4-Cl | Me | SO₂Ph | Me | 4-Cl |
| Me | COO-*t*-Bu | Me | 4-Cl | Me | SO₂NMe₂ | Me | 4-Cl |
| Me | COO-*i*-Bu | Me | 4-Cl | Me | PO(OEt)₂ | Me | 4-Cl |
| Me | COCH₂CF₃ | Me | 4-Cl | Me | PO(Me)OEt | Me | 4-Cl |
| Me | PS(OEt)₂ | Me | 4-Cl | Me | H | Et | 4-Cl |
| Me | PO(OMe)₂ | Me | 4-Cl | Me | H | Et | 4-F |
| Me | H | Cl | 4-Cl | Me | H | Et | 4-Br |
| Me | H | Cl | 4-F | Me | H | Et | 4-CF₃ |
| Me | H | Cl | 4-Br | Me | H | Et | 4-CN |
| Me | H | Cl | 4-CF₃ | Me | H | Et | 4-NO₂ |
| Me | H | Cl | 4-CN | Me | H | Et | 4-OMe |
| Me | H | Cl | 4-NO₂ | Me | H | Et | 4-OCF₃ |
| Me | H | Cl | 4-OMe | Me | H | Et | 4-OCF₂H |
| Me | H | Cl | 4-OCF₃ | Me | H | Et | 4-OCF₂CF₂H |
| Me | H | Cl | 4-OCF₂H | Me | H | Et | 4-CHO |
| Me | H | Cl | 4-OCF₂CF₂H | Me | H | Et | 4-CF₂H |
| Me | H | Cl | 4-CHO | Me | H | Et | 4-Me |
| Me | H | Cl | 4-CF₂H | Me | H | Et | 4-Et |
| Me | H | Cl | 4-Me | Me | H | Et | 4-SMe |
| Me | H | Cl | 4-Et | Me | H | Et | 4-SCF₃ |
| Me | H | Cl | 4-SMe | Me | H | Et | 4-SCF₂H |
| Me | H | Cl | 4-SCF₃ | Me | H | Et | 2-Cl |
| Me | H | Cl | 4-SCF₂H | Me | H | Et | 3-Cl |
| Me | H | Cl | 2-Cl | Me | H | Et | 2-F |
| Me | H | Cl | 3-Cl | Me | H | Et | 3-F |
| Me | H | Cl | 2-F | Me | H | Et | 2,4-di-Cl |
| Me | H | Cl | 3-F | Me | H | Et | 3,4-di-Cl |
| Me | H | Cl | 2,4-di-Cl | Me | H | Et | 3,4-di-F |
| Me | H | Cl | 3,4-di-Cl | Et | H | Me | 4-Cl |
| Me | H | Cl | 3,4-di-F | | | | |

**TABLE 5C**

| | | | | | | | |
|---|---|---|---|---|---|---|---|
| R² is H. | | | | | | | 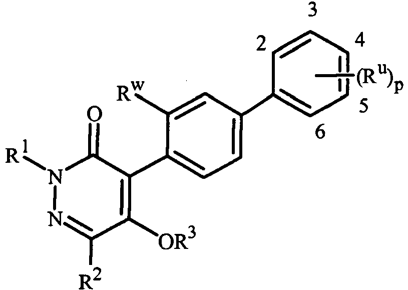 |

| R¹ | R³ | R^{W} | (R^{u})ₚ | R¹ | R³ | R^{w} | (R^{u})ₚ |
|---|---|---|---|---|---|---|---|
| Me | COEt | Me | 4-Cl | Me | H | Cl | 4-OCF₂CF₂H |
| Me | CO-*n*-Pr | Me | 4-Cl | Me | H | Cl | 4-CHO |
| Me | CO-*i*-Pr | Me | 4-Cl | Me | H | Cl | 4-CF₂H |
| Me | CO-*t*-Bu | Me | 4-Cl | Me | H | Cl | 4-Me |
| Me | CO-*i*-Bu | Me | 4-Cl | Me | H | Cl | 4-Et |
| Me | COCH₂C(CH₃)₃ | Me | 4-Cl | Me | H | Cl | 4-SMe |
| Me | COPh | Me | 4-Cl | Me | H | Cl | 4-SCF₃ |
| Me | COOMe | Me | 4-Cl | Me | H | Cl | 4-SCF₂H |
| Me | COOEt | Me | 4-Cl | Me | H | Cl | 2-Cl |
| Me | COO-*i*-Pr | Me | 4-Cl | Me | H | Cl | 3-Cl |
| Me | COO-*t-*Bu | Me | 4-Cl | Me | H | Cl | 2-F |
| Me | COO-*i*-Bu | Me | 4-Cl | Me | H | Cl | 3-F |
| Me | COCH₂CF₃ | Me | 4-Cl | Me | H | Cl | 2,4-di-Cl |
| Me | SO₂Me | Me | 4-Cl | Me | H | Cl | 3,4-di-Cl |
| Me | SO₂CF₃ | Me | 4-Cl | Me | H | Cl | 3,4-di-F |
| Me | SO₂-*c*-Pr | Me | 4-Cl | Me | H | Et | 4-Cl |
| Me | SO₂Et | Me | 4-Cl | Me | H | Et | 4-F |
| Me | SO₂-*n*-Pr | Me | 4-Cl | Me | H | Et | 4-Br |
| Me | SO₂-*i*-Pr | Me | 4-Cl | Me | H | Et | 4-CF₃ |
| Me | SO₂-*t*-Bu | Me | 4-Cl | Me | H | Et | 4-CN |
| Me | SO₂-*i*-Bu | Me | 4-Cl | Me | H | Et | 4-NO₂ |
| Me | SO₂CH₂C(CH₃)₃ | Me | 4-Cl | Me | H | Et | 4-OMe |
| Me | SO₂Ph | Me | 4-Cl | Me | H | Et | 4-OCF₃ |
| Me | SO₂NMe₂ | Me | 4-Cl | Me | H | Et | 4-OCF₂H |
| Me | PO(OEt)₂ | Me | 4-Cl | Me | H | Et | 4-OCF₂CF₂H |
| Me | PO(Me)OEt | Me | 4-Cl | Me | H | Et | 4-CHO |
| Me | PS(OEt)₂ | Me | 4-Cl | Me | H | Et | 4-CF₂H |
| Me | PO(OMe)₂ | Me | 4-Cl | Me | H | Et | 4-Me |
| Me | H | Cl | 4-Cl | Me | H | Et | 4-Et |
| Me | H | Cl | 4-F | Me | H | Et | 4-SMe |
| Me | H | Cl | 4-Br | Me | H | Et | 4-SCF₃ |
| Me | H | Cl | 4-CF₃ | Me | H | Et | 4-SCF₂H |
| Me | H | Cl | 4-CN | Me | H | Et | 2-Cl |
| Me | H | Cl | 4-NO₂ | Me | H | Et | 3-Cl |
| Me | H | Cl | 4-OMe | Me | H | Et | 2-F |
| Me | H | Cl | 4-OCF₃ | Me | H | Et | 3-F |
| Me | H | Cl | 4-OCF₂H | Me | H | Et | 2,4-di-Cl |

| R¹ | R³ | R^{w} | (R^{u})ₚ | R¹ | R³ | R^{w} | (R^{u})ₚ |
|---|---|---|---|---|---|---|---|
| Me | H | Et | 3,4-di-Cl | Me | H | Me | 4-Cl |
| Me | H | Et | 3,4-di-F | Me | H | Me | 4-Cl |
| Et | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | Me | H | Me | 4-Cl |
| Me | H | Me | 4-Cl | | | | |

**TABLE 5 CA**

| |
|---|
| Table 5CA is constructed the same as Table 5C, except that "R² is H." below the chemical structure under the Table 5C heading is replaced with "R² is Me.". |

**TABLE 5D**

| | | | | | |
|---|---|---|---|---|---|
| 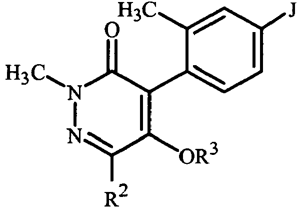 | | | | | |

| R² | R³ | J | R² | R³ | J |
|---|---|---|---|---|---|
| H | H | 3-CF₃-1*H*-pyrazol-1-yl | H | H | 4-Cl-1*H*-pyrazol-1-yl |
| H | H | 3-Br-1*H*-pyrazol-1-yl | H | H | 4-CN-1*H*-pyrazol-1-yl |
| H | H | 3-Me-1*H*-pyrazol-1-yl | H | H | 3-Me-1*H*-1,2,4-triazol-1-yl |
| H | H | 3-Cl-1*H*-pyrazol-1-yl | H | H | 3-Br-1*H*-1,2,4-triazol-1-yl |
| H | H | 4-CF₃-1*H*-pyrazol-1-yl | H | H | 3-CF₃-1*H*-1,2,4-triazol-1-yl |
| H | H | 4-Br-1*H*-pyrazol-1-yl | H | H | 4-CF₃-1*H*-imidazol-1-yl |
| H | H | 4-Me-1*H*-pyrazol-1-yl | H | H | 4-Me-1*H*-imidazol-1-yl |
| H | H | 4-Cl-1*H*-imidazol-1-yl | Me | H | 3-Br-1*H*-1,2,4-triazol-1-yl |
| H | H | 5-CF₃-pyridin-2-yl | Me | H | 3-CF₃-1*H*-1,2,4-triazol-1-yl |
| H | H | 5-Cl-pyridin-2-yl | Me | H | 4-CF₃-1*H*-imidazol-1-yl |
| H | H | 2-Cl-pyridin-5-yl | Me | H | 4-Me-1*H*-imidazol-1-yl |
| H | H | 5-Cl-thien-2-yl | Me | H | 4-Cl-1*H*-imidazol-1-yl |
| Me | H | 3-CF₃-1*H*-pyrazol-1-yl | Me | H | 5-CF₃-pyridin-2-yl |
| Me | H | 3-Br-1*H*-pyrazol-1-yl | Me | H | 5-Cl-pyridin-2-yl |
| Me | H | 3-Me-1*H*-pyrazol-1-yl | Me | H | 2-Cl-pyridin-5-yl |
| Me | H | 3-Cl-1*H*-pyrazol-1-yl | Me | H | 5-Cl-thien-2-yl |
| Me | H | 4-CF₃-1*H*-pyrazol-1-yl | H | COO-*i*-Pr | 3-CF₃-1*H*-pyrazol-1-yl |
| Me | H | 4-Br-1*H*-pyrazol-1-yl | H | CO-*i*-P*r* | 3-CF₃-1*H*-pyrazol-1-yl |
| Me | H | 4-Me-1*H*-pyrazol-1-yl | H | SO₂Me | 3-CF₃-1*H*-pyrazol-1-yl |
| Me | H | 4-Cl-1*H*-pyrazol-1-yl | H | SO₂Ph | 3-CF₃-1*H-*pyrazol-1-yl |
| Me | H | 4-CN-1*H*-pyrazol-1-yl | H | CO-*t*-Bu | 3-CF₃-1*H*-pyrazol-1-yl |
| Me | H | 3-Me-1*H*-1,2,4-triazol-1-yl | | | |

**TABLE 5E**

| | | | | | |
|---|---|---|---|---|---|
| 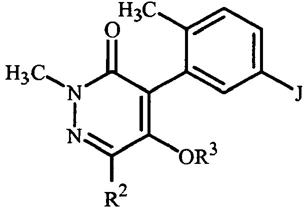 | | | | | |

| R² | R³ | J | R² | R³ | J |
|---|---|---|---|---|---|
| H | H | 3-CF₃-1*H*-pyrazol-1-yl | H | H | 4-CF₃-1*H*-imidazol-1-yl |
| H | H | 3-Br-1*H*-pyrazol-1-yl | H | H | 4-Me-1*H*-imidazol-1-yl |
| H | H | 3-Me-1*H*-pyrazol-1-yl | H | H | 4-Cl-1*H*-imidazol-1-yl |
| H | H | 3-Cl-1*H*-pyrazol-1-yl | H | H | 5-CF₃-pyridin-2-yl |
| H | H | 4-CF₃-1*H*-pyrazol-1-yl | H | H | 5-Cl-pyridin-2-yl |
| H | H | 4-Br-1*H*-pyrazol-1-yl | H | H | 2-Cl-pyridin-5-yl |
| H | H | 4-Me-1*H*-pyrazol-1-yl | H | H | 5-Cl-thien-2-yl |
| H | H | 4-Cl-1*H*-pyrazol-1-yl | Me | H | 3-CF₃-1*H*-pyrazol-1-yl |
| H | H | 4-CN-1*H*-pyrazol-1-yl | Me | H | 3-Br-1*H*-pyrazol-1-yl |
| H | H | 3-Me-1*H*-1,2,4-triazol-1-yl | Me | H | 3-Me-1*H*-pyrazol-1-yl |
| H | H | 3-Br-1*H*-1,2,4-triazol-1-yl | Me | H | 3-Cl-1*H*-pyrazol-1-yl |
| H | H | 3-CF₃-1*H*-1,2,4-triazol-1-yl | Me | H | 4-CF₃-1*H*-pyrazol-1-yl |
| Me | H | 4-Br-1*H*-pyrazol-1-yl | Me | H | 5-CF₃-pyridin-2-yl |
| Me | H | 4-Me-1*H*-pyrazol-1-yl | Me | H | 5-Cl-pyridin-2-yl |
| Me | H | 4-Cl-1*H*-pyrazol-1-yl | Me | H | 2-Cl-pyridin-5-yl |
| Me | H | 4-CN-1*H*-pyrazol-1-yl | Me | H | 5-Cl-thien-2-yl |
| Me | H | 3-Me-1*H*-1,2,4-triazol-1-yl | H | COO-*i*-Pr | 3-CF₃-1*H*-pyrazol-1-yl |
| Me | H | 3-Br-1*H*-1,2,4-triazol-1-yl | H | CO-*i*-Pr | 3-CF₃-1*H*-pyrazol-1-yl |
| Me | H | 3-CF₃-1*H*-1,2,4-triazol-1-yl | H | SO₂Me | 3-CF₃-1*H*-pyrazol-1-yl |
| Me | H | 4-CF₃-1*H*-imidazol-1-yl | H | SO₂Ph | 3-CF₃-1*H*-pyrazol-1-yl |
| Me | H | 4-Me-1*H*-imidazol-1-yl | H | CO-*t*-Bu | 3-CF₃-1*H*-pyrazol-1-yl |
| Me | H | 4-Cl-1*H*-imidazol-1-yl | | | |

**TABLE 6A**

| | | | |
|---|---|---|---|
| 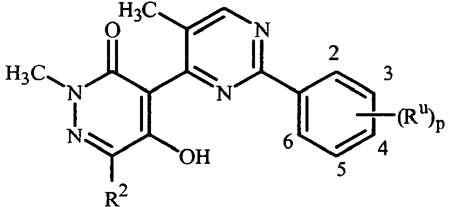 | | | |
| R² is H. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH-CH₂ | 4-SOCF₃ | 4-Pyridin-3-yl |
| 4-I | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |
| R² is CH₃. | | | |
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-I | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |

**TABLE 6B**

| | | | | | |
|---|---|---|---|---|---|
| 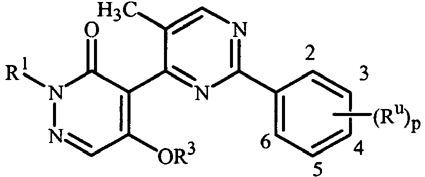 | | | | | |

| R¹ | R³ | (R^{u})ₚ | R¹ | R³ | (R^{u})ₚ |
|---|---|---|---|---|---|
| Me | COO-*i*-Pr | 4-Cl | Me | COO-*i*-Pr | 4-OCF₃ |
| Me | COO-*i*-Pr | 4-Br | Me | COO-*i*-Pr | 4-Me |
| Me | COO-*i*-Pr | 4-F | Et | H | 4-Cl |
| Me | COO-*i*-Pr | 4-CF₃ | | | |

**TABLE 7A**

| | | | |
|---|---|---|---|
| 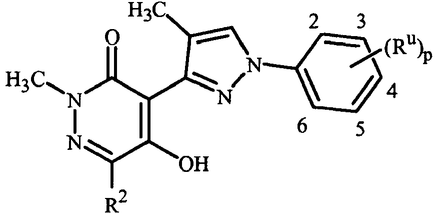 | | | |
| R² is H. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-1 | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |
| R² is CH₃. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-I | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |

**TABLE 7B**

| | | | | | | | |
|---|---|---|---|---|---|---|---|
| 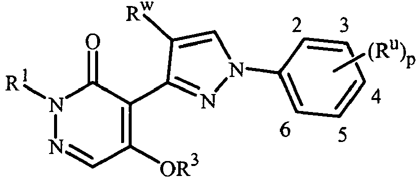 | | | | | | | |

| R¹ | R³ | R^{w} | (R^{u})ₚ | R¹ | R³ | R^{w} | (R^{u})ₚ |
|---|---|---|---|---|---|---|---|
| Me | H | Cl | 4-Cl | Me | H | Cl | 4-OCF₂CF₂H |
| Me | H | Br | 4-Cl | Me | H | Cl | 4-CHO |
| Me | H | Cl | 4-F | Me | H | Cl | 4-CF₂H |
| Me | H | Cl | 4-Br | Me | H | Cl | 4-Me |
| Me | H | Cl | 4-CF₃ | Me | COO-*i*-Pr | Me | 4-Cl |
| Me | H | Br | 4-CF₃ | Me | COO-*i*-Pr | Me | 4-Br |
| Me | H | Cl | 4-CN | Me | COO-*i*-Pr | Me | 4-F |
| Me | H | Cl | 4-NO₂ | Me | COO-*i*-Pr | Me | 4-CF₃ |
| Me | H | Cl | 4-OMe | Me | COO-*i*-Pr | Me | 4-OCF₃ |
| Me | H | Cl | 4-OCF₃ | Me | COO-*i*-Pr | Me | 4-Me |
| Me | H | Cl | 4-OCF₂H | Et | H | Me | 4-Cl |

**TABLE 8A**

| | | | |
|---|---|---|---|
| 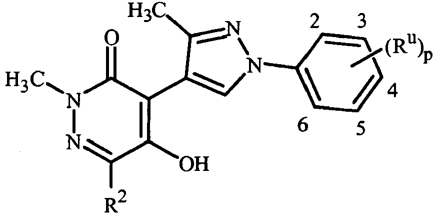 | | | |
| R² is H. | | | |

| (R^{u})ₚ | (R^{u})ₚ | (R^{u})ₚ | (R^{u})ₚ |
|---|---|---|---|
| 4-F | 4-OCF₃ | 4-COMe | 4-SCF₂H |
| 4-Cl | 4-OCF₂H | 4-COCF₃ | 4-SOMe |
| 4-Br | 4-OCF₂CF₂H | 4-COOMe | 4-SO₂Me |
| 4-I | 4-CHO | 4-CONH₂ | 4-SOCF₃ |
| 4-CF₃ | 4-CF₂H | 4-CONHMe | 4-SOCF₂H |
| 4-CN | 4-Me | 4-CONMe₂ | 4-SO₂CF₃ |
| 4-NO₂ | 4-Et | 4-OCOMe | 4-SO₂CF₂H |
| 4-OH | 4-CH=CH₂ | 4-SMe | 4-SO₂NH₂ |
| 4-OMe | 4-C≡CH | 4-SCF₃ | 4-SO₂NMe₂ |
| 4-SiMe₃ | 4-NHSO₂Me | 4-Thien-2-yl | 3-F |
| 4-NH₂ | 4-Ph | 4-SF₅ | 2,4-di-Cl |
| 4-NMe₂ | 4-Pyridin-2-yl | 2-Cl | 3,4-di-Cl |
| 4-NHCOMe | 4-Pyridin-3-yl | 3-Cl | 3,4-di-F |
| 4-NHCOOMe | 4-Pyridin-4-yl | 2-F | |
| R² is CH₃. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-1 | 4-C≡CH | 4-SOCF₂H | 4-Pyridin-4-yl |
| 4-CF₃ | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |

**TABLE 8B**

| | | | | | |
|---|---|---|---|---|---|
| 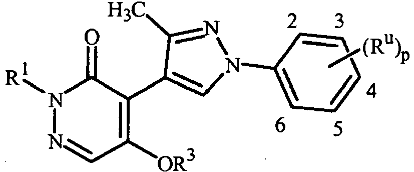 | | | | | |

| R¹ | R³ | (R^{u})ₚ | R¹ | R³ | (R^{u})ₚ |
|---|---|---|---|---|---|
| Me | COO-*i*-Pr | 4-Cl | Me | COO-*i*-Pr | 4-OCF₃ |
| Me | COO-*i*-Pr | 4-Br | Me | COO-*i*-Pr | 4-Me |
| Me | COO-*i*-Pr | 4-F | Et | H | 4-Cl |
| Me | COO-*i*-Pr | 4-CF₃ | | | |

**TABLE 9A**

| | | | |
|---|---|---|---|
| 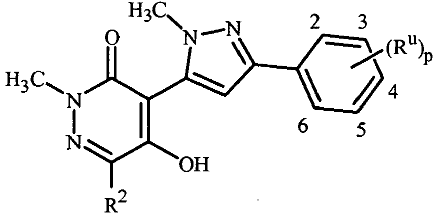 | | | |
| R² is H. | | | |

| (R^{u})ₚ | (R^{u})ₚ | (R^{u})ₚ | (R^{u})ₚ |
|---|---|---|---|
| 4-F | 4-Me | 4-SOMe | 4-Ph |
| 4-Cl | 4-Et | 4-SO₂Me | 4-Pyridin-2-yl |
| 4-Br | 4-CH=CH₂ | 4-SOCF₃ | 4-Pyridin-3-yl |
| 4-I | 4-C≡CH | 4-SOCF₂H | 4-Pyridin-4-yl |
| 4-CF3 | 4-COMe | 4-SO₂CF₃ | 4-Thien-2-yl |
| 4-CN | 4-COCF₃ | 4-SO₂CF₂H | 4-SF₅ |
| 4-NO₂ | 4-COOMe | 4-SO₂NH₂ | 2-Cl |
| 4-OH | 4-CONH₂ | 4-SO₂NMe₂ | 3-Cl |
| 4-OMe | 4-CONHMe | 4-SiMe₃ | 2-F |
| 4-OCF₃ | 4-CONMe₂ | 4-NH₂ | 3-F |
| 4-OCF₂H | 4-OCOMe | 4-NMe₂ | 2,4-di-Cl |
| 4-OCF₂CF₂H | 4-SMe | 4-NHCOMe | 3,4-di-Cl |
| 4-CHO | 4-SCF₃ | 4-NHCOOMe | 3,4-di-F |
| 4-CF₂H | 4-SCF₂H | 4-NHSO₂Me | |
| R² is CH₃. | | | |

| (R^{u})ₚ | (R^{u})ₚ | (R^{u})ₚ | (R^{u})ₚ |
|---|---|---|---|
| 4-F | 4-CHO | 4-OCOMe | 4-SiMe₃ |
| 4-Cl | 4-CF₂H | 4-SMe | 4-NH₂ |
| 4-Br | 4-Me | 4-SCF₃ | 4-NMe₂ |
| 4-I | 4-Et | 4-SCF₂H | 4-NHCOMe |
| 4-CF₃ | 4-CH=CH₂ | 4-SOMe | 4-NHCOOMe |
| 4-CN | 4-C≡CH | 4-SO₂Me | 4-NHSO₂Me |
| 4-NO₂ | 4-COMe | 4-SOCF₃ | 4-Ph |
| 4-OH | 4-COCF₃ | 4-SOCF₂H | 4-Pyridin-2-yl |
| 4-OMe | 4-COOMe | 4-SO₂CF₃ | 4-Pyridin-3-yl |
| 4-OCF₃ | 4-CONH₂ | 4-SO₂CF₂H | 4-Pyridin-4-yl |
| 4-OCF₂H | 4-CONHMe | 4-SO₂N-H₂ | 4-Thien-2-yl |
| 4-OCF₂CF₂H | 4-CONMe₂ | 4-SO₂NMe₂ | 4-SF₅ |
| 2-Cl | 2-F | 2,4-di-Cl | 3,4-di-F |
| 3-Cl | 3-F | 3,4-di-Cl | |

**TABLE 9B**

| | | | | | |
|---|---|---|---|---|---|
| 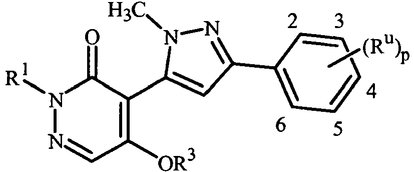 | | | | | |

| R¹ | R³ | (R^{u})ₚ | R¹ | R³ | (R^{u})ₚ |
|---|---|---|---|---|---|
| Me | COO-*i*-Pr | 4-Cl | Me | COO-*i*-Pr | 4-OCF₃ |
| Me | COO-*i*-Pr | 4-Br | Me | COO-*i*-Pr | 4-Me |
| Me | COO-*i*-Pr | 4-F | Et | H | 4-Cl |
| Me | COO-*i*-Pr | 4-CF₃ | | | |

**TABLE 10**

| | | | | | |
|---|---|---|---|---|---|
| 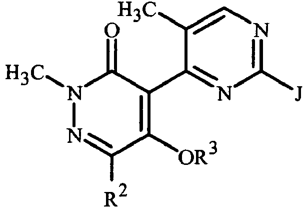 | | | | | |

| R² | R³ | J | R² | R³ | J |
|---|---|---|---|---|---|
| H | H | 3-CF₃-1*H*-pyrazol-1-yl | H | H | 4-CF₃-1*H* imidazol-1-yl |
| H | H | 3-Br-1*H*-pyrazol-1-yl | H | H | 4-Me-1*H*-imidazol-1-yl |
| H | H | 3-Me-1*H*-pyrazol-1-yl | H | H | 4-Cl-1*H*-imidazol-1-yl |
| H | H | 3-Cl-1*H*-pyrazol-1-yl | H | H | 5-CF₃-pyridin-2-yl |
| H | H | 4-CF₃-1*H*-pyrazol-1-yl | H | H | 5-Cl-pyridin-2-yl |
| H | H | 4-Br-1*H*-pyrazol-1-yl | H | H | 2-Cl-pyridin-5-yl |
| H | H | 4-Me-1*H*-pyrazol-1-yl | H | H | 5-Cl-thien-2-yl |
| H | H | 4-Cl-1*H-*pyrazol-1-yl | Me | H | 3-CF₃-1*H-*pyrazol-1-yl |
| H | H | 4-CN-1*H*-pyrazol-1-yl | Me | H | 3-Br-1*H*-pyrazol-1-yl |
| H | H | 3-Me-1*H*-1,2,4-triazol-1-yl | Me | H | 3-Me-1*H*-pyrazol-1-yl |
| H | H | 3-Br-1*H*-1,2,4-triazol-1-yl | Me | H | 3-Cl-1*H*-pyrazol-1-yl |
| H | H | 3-CF₃-1*H*-1,2,4-triazol-1-yl | Me | H | 4-CF₃-1*H*-pyrazol-1-yl |
| Me | H | 4-Br-1*H*-pyrazol-1-yl | Me | H | 5-CF₃-pyridin-2-yl |
| Me | H | 4-Me-1*H*-pyrazol-1-yl | Me | H | 5-Cl-pyridin-2-yl |
| Me | H | 4-Cl-1*H*-pyrazol-1-yl | Me | H | 2-Cl-pyridin-5-yl |
| Me | H | 4-CN-1*H*-pyrazol-1-yl | Me | H | 5-Cl-thien-2-yl |
| Me | H | 3-Me-1*H*-1,2,4-triazol-1-yl | H | COO-*i*-Pr | 3-CF₃-1*H*-pyrazol-1-yl |
| Me | H | 3-Br-1*H*-1,2,4-triazol-1-yl | H | CO-*i*-Pr | 3-CF₃-1*H*-pyrazol-1-yl |
| Me | H | 3-CF₃-1*H*-1,2,4-triazol-1-yl | H | SO₂Me | 3-CF₃-1*H*-pyrazol-1-yl |
| Me | H | 4-CF₃-1*H*-imidazol-1-yl | H | SO₂Ph | 3-CF₃-1*H*-pyrazol-1-yl |
| Me | H | 4-Me-1*H*-imidazol-1-yl | H | CO-*t*-Bu | 3-CF₃-1*H*-pyrazol-1-yl |
| Me | H | 4-Cl-1*H*-imidazol-1-yl | | | |

**TABLE 11**

| | | | | |
|---|---|---|---|---|
| 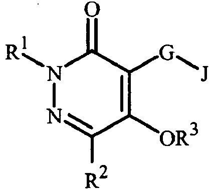 | | | | |

| R¹ | R² | R³ | G | J |
|---|---|---|---|---|
| Me | H | H | 1,2,4-thiadiazol-5-yl | 3-(4-chlorophenyl) |
| Me | H | H | 1,2,4-oxadiazol-5-yl | 3-(4-chlorophenyl) |
| Me | H | H | isoxazol-5-yl | 3-(4-chlorophenyl) |
| Me | H | H | isoxazol-3-yl | 5-(4-chlorophenyl) |
| Me | H | H | isothiazol-5-yl | 3-(4-chlorophenyl) |
| Me | H | H | isothiazol-3-yl | 5-(4-chlorophenyl) |
| Me | H | H | 5-Me-2*H*-1,2,3-triazol-4-yl | 2-(4-chlorophenyl) |
| Me | H | H | 1,3,4-thiadiazol-2-yl | 5-(4-chlorophenyl) |
| Me | H | H | 1,3,4-thiadiazol-5-yl | 2-(4-chlorophenyl) |
| Me | H | H | 1,3,5-thiadiazol-2-yl | 4-(4-chlorophenyl) |
| Me | H | H | 1,3,5-thiadiazol-4-yl | 2-(4-chlorophenyl) |
| Me | H | H | 1,3,4-oxadiazol-2-yl | 5-(4-chlorophenyl) |
| Me | H | H | 1,3,4-oxadiazol-5-yl | 2-(4-chlorophenyl) |
| Me | H | H | 1,3,5-oxadiazol-2-yl | 4-(4-chlorophenyl) |
| Me | H | H | 1,3,5-oxadiazol-4-yl | 2-(4-chlorophenyl) |
| Me | H | H | thiazol-2-yl | 5-(4-chlorophenyl) |
| Me | H | H | thiazol-2-yl | 4-(4-chlorophenyl) |
| Me | H | H | 5-Me-thiazol-4-yl | 2-(4-chlorophenyl) |
| Me | H | H | oxazol-2-yl | 5-(4-chlorophenyl) |
| Me | H | H | oxazol-2-yl | 4-(4-chlorophenyl) |
| Me | H | H | 5-Me-oxazol-4-yl | 2-(4-chlorophenyl) |
| Me | H | H | 4-Me-oxazol-5-yl | 2-(4-chlorophenyl) |
| Me | H | H | 1,2,4-triazin-3-yl | 5-(4-chlorophenyl) |
| Me | H | H | 6-Me-1,2,4-triazin-5-yl | 3-(4-chlorophenyl) |
| Me | H | H | 1,3,5-triazin-2-yl | 4-(4-chlorophenyl) |
| Me | H | H | 3-Me-pyridin-2-yl | 6-(4-chlorophenyl) |
| Me | H | H | 2-Me-pyridin-3-yl | 5-(4-chlorophenyl) |
| Me | H | H | 3-Cl-pyridin-2-yl | 4-(4-chlorophenyl) |
| Me | H | H | 5-Me-pyridin-4-yl | 2-(4-chlorophenyl) |
| Me | H | H | 2-Me-pyrazin-3-yl | 5-(4-chlorophenyl) |
| Me | H | H | 3-Me-pyridazin-4-yl | 6-(4-chlorophenyl) |
| Me | H | H | 3-Cl-pyridazin-4-yl | 6-(4-chlorophenyl) |
| Me | H | H | Pyrimidin-2-yl | 4-(4-chlorophenyl) |
| Me | H | H | 3-Me-thien-2-yl | 5-(4-chlorophenyl) |
| Me | H | H | 2-Me-thien-3-yl | 5-(4-chlorophenyl) |
| Me | H | H | 3-Me-furan-2-yl | 5-(4-chlorophenyl) |
| Me | H | H | 2-Me-furan-3-yl | 5-(4-chlorophenyl) |
| Me | Me | H | 5-Me-2*H*-1,2,3-triazol-4-yl | 2-(4-chlorophenyl) |
| Me | H | COO-*i*-Pr | 5-Me-2*H*-1,2,3-triazol-4-yl | 2-(4-chlorophenyl) |
| Me | Me | H | 6-Me-1,2,4-triazin-5-yl | 3-(4-chlorophenyl) |
| Me | H | SO₂Me | 6-Me-1,2,4-triazin-5-yl | 3-(4-chlorophenyl) |
| Me | Me | H | 5-Me-oxazol-4-yl | 2-(4-chlorophenyl) |
| Me | H | CO-*t*-Bu | 5-Me-oxazol-4-yl | 2-(4-chlorophenyl) |
| Et | H | H | 5-Me-2*H*-1,2,3-triazol-4-yl | 2-(4-chlorophenyl) |

### Formulation/Utility

A compound Formula **1** of this invention will generally be used as a herbicidal active ingredient in a composition, i.e. formulation, with at least one additional component selected from the group consisting of surfactants, solid diluents and liquid diluents, which serves as a carrier. The formulation or composition ingredients are selected to be consistent with the physical properties of the active ingredient, mode of application and environmental factors such as soil type, moisture and temperature.

Useful formulations include both liquid and solid compositions. Liquid compositions include solutions (including emulsifiable concentrates), suspensions, emulsions (including microemulsions and/or suspoemulsions) and the like, which optionally can be thickened into gels. The general types of aqueous liquid compositions are soluble concentrate, suspension concentrate, capsule suspension, concentrated emulsion, microemulsion and suspo-emulsion. The general types of nonaqueous liquid compositions are emulsifiable concentrate, microemulsifiable concentrate, dispersible concentrate and oil dispersion.

The general types of solid compositions are dusts, powders, granules, pellets, prills, pastilles, tablets, filled films (including seed coatings) and the like, which can be water-dispersible ("wettable") or water-soluble. Films and coatings formed from film-forming solutions or flowable suspensions are particularly useful for seed treatment. Active ingredient can be (micro)encapsulated and further formed into a suspension or solid formulation; alternatively the entire formulation of active ingredient can be encapsulated (or "overcoated"). Encapsulation can control or delay release of the active ingredient. An emulsifiable granule combines the advantages of both an emulsifiable concentrate formulation and a dry granular formulation. High-strength compositions are primarily used as intermediates for further formulation.

Sprayable formulations are typically extended in a suitable medium before spraying. Such liquid and solid formulations are formulated to be readily diluted in the spray medium, usually water. Spray volumes can range from about from about one to several thousand liters per hectare, but more typically are in the range from about ten to several hundred liters per hectare. Sprayable formulations can be tank mixed with water or another suitable medium for foliar treatment by aerial or ground application, or for application to the growing medium of the plant. Liquid and dry formulations can be metered directly into drip irrigation systems or metered into the furrow during planting.

The formulations will typically contain effective amounts of active ingredient, diluent and surfactant within the following approximate ranges which add up to 100 percent by weight.

| | Weight Percent | | |
|---|---|---|---|
| | Active Ingredient | Diluent | Surfactant |
| Water-Dispersible and Water-soluble Granules, Tablets and Powders | 0.001-90 | 0-99.999 | 0-15 |
| Oil Dispersions, Suspensions, Emulsions, Solutions (including Emulsifiable Concentrates) | 1-50 | 40-99 | 0-50 |
| Dusts | 1-25 | 70-99 | 0-5 |
| Granules and Pellets | 0.001-99 | 5-99.999 | 0-15 |
| High Strength Compositions | 90-99 | 0-10 | 0-2 |

Solid diluents include, for example, clays such as bentonite, montmorillonite, attapulgite and kaolin, gypsum, cellulose, titanium dioxide, zinc oxide, starch, dextrin, sugars (e.g., lactose, sucrose), silica, talc, mica, diatomaceous earth, urea, calcium carbonate, sodium carbonate and bicarbonate, and sodium sulfate. Typical solid diluents are described in Watkins et al., Handbook of Insecticide Dust Diluents and Carriers, 2nd Ed., Dorland Books, Caldwell, New Jersey.

Liquid diluents include, for example, water, *N,N*-dimethylalkanamides (e.g., *N,N*-dimethylformamide), limonene, dimethyl sulfoxide, *N*-alkylpyrrolidones (e.g., *N*-methylpyrrolidinone), ethylene glycol, triethylene glycol, propylene glycol, dipropylene glycol, polypropylene glycol, propylene carbonate, butylene carbonate, paraffins (e.g., white mineral oils, normal paraffins, isoparaffins), alkylbenzenes, alkylnaphthalenes, glycerine, glycerol triacetate, sorbitol, triacetin, aromatic hydrocarbons, dearomatized aliphatics, alkylbenzenes, alkylnaphthalenes, ketones such as cyclohexanone, 2-heptanone, isophorone and 4-hydroxy-4-methyl-2-pentanone, acetates such as isoamyl acetate, hexyl acetate, heptyl acetate, octyl acetate, nonyl acetate, tridecyl acetate and isobornyl acetate, other esters such as alkylated lactate esters, dibasic esters and γ-butyrolactone, and alcohols, which can be linear, branched, saturated or unsaturated, such as methanol, ethanol, *n*-propanol, isopropyl alcohol, *n*-butanol, isobutyl alcohol, *n*-hexanol, 2-ethylhexanol, *n*-octanol, decanol, isodecyl alcohol, isooctadecanol, cetyl alcohol, lauryl alcohol, tridecyl alcohol, oleyl alcohol, cyclohexanol, tetrahydrofurfuryl alcohol, diacetone alcohol and benzyl alcohol. Liquid diluents also include glycerol esters of saturated and unsaturated fatty acids (typically C₆-C₂₂), such as plant seed and fruit oils (e.g., oils of olive, castor, linseed, sesame, corn (maize), peanut, sunflower, grapeseed, safflower, cottonseed, soybean, rapeseed, coconut and palm kernel), animal-sourced fats (e.g., beef tallow, pork tallow, lard, cod liver oil, fish oil), and mixtures thereof. Liquid diluents also include alkylated fatty acids (e.g., methylated, ethylated, butylated) wherein the fatty acids may be obtained by hydrolysis of glycerol esters from plant and animal sources, and can be purified by distillation. Typical liquid diluents are described in Marsden, Solvents Guide, 2nd Ed., Interscience, New York, 1950.

The solid and liquid compositions of the present invention often include one or more surfactants. When added to a liquid, surfactants (also known as "surface-active agents") generally modify, most often reduce, the surface tension of the liquid. Depending on the nature of the hydrophilic and lipophilic groups in a surfactant molecule, surfactants can be useful as wetting agents, dispersants, emulsifiers or defoaming agents.

Surfactants can be classified as nonionic, anionic or cationic. Nonionic surfactants useful for the present compositions include, but are not limited to: alcohol alkoxylates such as alcohol alkoxylates based on natural and synthetic alcohols (which may be branched or linear) and prepared from the alcohols and ethylene oxide, propylene oxide, butylene oxide or mixtures thereof; amine ethoxylates, alkanolamides and ethoxylated alkanolamides; alkoxylated triglycerides such as ethoxylated soybean, castor and rapeseed oils; alkylphenol alkoxylates such as octylphenol ethoxylates, nonylphenol ethoxylates, dinonyl phenol ethoxylates and dodecyl phenol ethoxylates (prepared from the phenols and ethylene oxide, propylene oxide, butylene oxide or mixtures thereof); block polymers prepared from ethylene oxide or propylene oxide and reverse block polymers where the terminal blocks are prepared from propylene oxide; ethoxylated fatty acids; ethoxylated fatty esters and oils; ethoxylated methyl esters; ethoxylated tristyrylphenol (including those prepared from ethylene oxide, propylene oxide, butylene oxide or mixtures thereof); fatty acid esters, glycerol esters, lanolin-based derivatives, polyethoxylate esters such as polyethoxylated sorbitan fatty acid esters, polyethoxylated sorbitol fatty acid esters and polyethoxylated glycerol fatty acid esters; other sorbitan derivatives such as sorbitan esters; polymeric surfactants such as random copolymers, block copolymers, alkyd peg (polyethylene glycol) resins, graft or comb polymers and star polymers; polyethylene glycols (pegs); polyethylene glycol fatty acid esters; silicone-based surfactants; and sugar-derivatives such as sucrose esters, alkyl polyglycosides and alkyl polysaccharides.

Useful anionic surfactants include, but are not limited to: alkylaryl sulfonic acids and their salts; carboxylated alcohol or alkylphenol ethoxylates; diphenyl sulfonate derivatives; lignin and lignin derivatives such as lignosulfonates; maleic or succinic acids or their anhydrides; olefin sulfonates; phosphate esters such as phosphate esters of alcohol alkoxylates, phosphate esters of alkylphenol alkoxylates and phosphate esters of styryl phenol ethoxylates; protein-based surfactants; sarcosine derivatives; styryl phenol ether sulfate; sulfates and sulfonates of oils and fatty acids; sulfates and sulfonates of ethoxylated alkylphenols; sulfates of alcohols; sulfates of ethoxylated alcohols; sulfonates of amines and amides such as *N,N*-alkyltaurates; sulfonates of benzene, cumene, toluene, xylene, and dodecyl and tridecylbenzenes; sulfonates of condensed naphthalenes; sulfonates of naphthalene and alkyl naphthalene; sulfonates of fractionated petroleum; sulfosuccinamates; and sulfosuccinates and their derivatives such as dialkyl sulfosuccinate salts.

Useful cationic surfactants include, but are not limited to: amides and ethoxylated amides; amines such as *N*-alkyl propanediamines, tripropylenetriamines and dipropylenetetramines, and ethoxylated amines, ethoxylated diamines and propoxylated amines (prepared from the amines and ethylene oxide, propylene oxide, butylene oxide or mixtures thereof); amine salts such as amine acetates and diamine salts; quaternary ammonium salts such as quaternary salts, ethoxylated quaternary salts and diquatemary salts; and amine oxides such as alkyldimethylamine oxides and bis-(2-hydroxyethyl)-alkylamine oxides.

Also useful for the present compositions are mixtures of nonionic and anionic surfactants or mixtures of nonionic and cationic surfactants. Nonionic, anionic and cationic surfactants and their recommended uses are disclosed in a variety of published references including McCutcheon's Emulsifiers and Detergents, annual American and International Editions published by McCutcheon's Division, The Manufacturing Confectioner Publishing Co.; Sisely and Wood, Encyclopedia of Surface Active Agents, Chemical Publ. Co., Inc., New York, 1964; and A. S. Davidson and B. Milwidsky, Synthetic Detergents, Seventh Edition, John Wiley and Sons, New York, 1987.

Compositions of this invention may also contain formulation auxiliaries and additives, known to those skilled in the art as formulation aids (some of which may be considered to also function as solid diluents, liquid diluents or surfactants). Such formulation auxiliaries and additives may control: pH (buffers), foaming during processing (antifoams such polyorganosiloxanes), sedimentation of active ingredients (suspending agents), viscosity (thixotropic thickeners), in-container microbial growth (antimicrobials), product freezing (antifreezes), color (dyes/pigment dispersions), wash-off (film formers or stickers), evaporation (evaporation retardants), and other formulation attributes. Film formers include, for example, polyvinyl acetates, polyvinyl acetate copolymers, polyvinylpyrrolidone-vinyl acetate copolymer, polyvinyl alcohols, polyvinyl alcohol copolymers and waxes. Examples of formulation auxiliaries and additives include those listed in McCutcheon's Volume 2: Functional Materials, annual International and North American editions published by McCutcheon's Division, The Manufacturing Confectioner Publishing Co.; and PCT Publication WO 03/024222.

The compound of Formula **1** and any other active ingredients are typically incorporated into the present compositions by dissolving the active ingredient in a solvent or by grinding in a liquid or dry diluent. Solutions, including emulsifiable concentrates, can be prepared by simply mixing the ingredients. If the solvent of a liquid composition intended for use as an emulsifiable concentrate is water-immiscible, an emulsifier is typically added to emulsify the active-containing solvent upon dilution with water. Active ingredient slurries, with particle diameters of up to 2,000 µm can be wet milled using media mills to obtain particles with average diameters below 3 µm. Aqueous slurries can be made into finished suspension concentrates (see, for example, U.S. 3,060,084) or further processed by spray drying to form water-dispersible granules. Dry formulations usually require dry milling processes, which produce average particle diameters in the 2 to 10 µm range. Dusts and powders can be prepared by blending and usually grinding (such as with a hammer mill or fluid-energy mill). Granules and pellets can be prepared by spraying the active material upon preformed granular carriers or by agglomeration techniques. See Browning, "Agglomeration", Chemical Engineering, December 4, 1967, pp 147-48, Perry's Chemical Engineer's Handbook, 4th Ed., McGraw-Hill, New York, 1963, pages 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see T. S. Woods, "The Formulator's Toolbox - Product Forms for Modem Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pp 81-96; Hance et al., Weed Control Handbook, 8th Ed., Blackwell Scientific Publications, Oxford, 1989; and Developments in formulation technology, PJB Publications, Richmond, UK, 2000.

In the following Examples, all percentages are by weight and all formulations are prepared in conventional ways. Compound numbers refer to compounds in Index Tables A-B. Without further elaboration, it is believed that one skilled in the art using the preceding description can utilize the present invention to its fullest extent. The following Examples are, therefore, to be construed as merely illustrative, and not limiting of the disclosure in any way whatsoever. Percentages are by weight except where otherwise indicated.

### Example A

| High Strength Concentrate | | |
|---|---|---|
| | Compound 1 | 98.5% |
| | silica aerogel | 0.5% |
| | synthetic amorphous fine silica | 1.0% |

### Example B

| Wettable Powder | | |
|---|---|---|
| | Compound 2 | 65.0% |
| | dodecylphenol polyethylene glycol ether | 2.0% |
| | sodium ligninsulfonate | 4.0% |
| | sodium silicoaluminate | 6.0% |
| | montmorillonite (calcined) | 23.0% |

### Example C

| Granule | | |
|---|---|---|
| | Compound 3 | 10.0% |
| | attapulgite granules (low volatile matter, 0.71/0.30 mm; U.S.S. No. 25-50 sieves) | 90.0% |

### Example D

| Extruded Pellet | | |
|---|---|---|
| | Compound 4 | 25.0% |
| | anhydrous sodium sulfate | 10.0% |
| | crude calcium ligninsulfonate | 5.0% |
| | sodium alkylnaphthalenesulfonate | 1.0% |
| | calcium/magnesium bentonite | 59.0% |

### Example E

| Emulsifiable Concentrate | | |
|---|---|---|
| | Compound 5 | 10.0% |
| | polyoxyethylene sorbitol hexoleate | 20.0% |
| | C₆-C₁₀ fatty acid methyl ester | 70.0% |

### Example F

| Microemulsion | | |
|---|---|---|
| | Compound 6 | 5.0% |
| | polyvinylpyrrolidone-vinyl acetate copolymer | 30.0% |
| | alkylpolyglycoside | 30.0% |
| | glyceryl monooleate | 15.0% |
| | water | 20.0% |

Test results indicate that the compounds of the present invention are highly active preemergent and/or postemergent herbicides and/or plant growth regulants. Many of them have utility for broad-spectrum pre- and/or postemergence weed control in areas where complete control of all vegetation is desired such as around fuel storage tanks, industrial storage areas, parking lots, drive-in theaters, air fields, river banks, irrigation and other waterways, around billboards and highways and railroad structures. Many of the compounds of this invention, by virtue of selective metabolism in crops versus weeds, or by selective activity at the locus of physiological inhibition in crops and weeds, or by selective placement on or within the environment of a mixture of crops and weeds, are useful for the selective control of grass and broadleaf weeds within a crop/weed mixture. One skilled in the art will recognize that the preferred combination of these selectivity factors within a compound or group of compounds can readily be determined by performing routine biological and/or biochemical assays. Compounds of this invention may show tolerance to important agronomic crops including, but not limited to, alfalfa, barley, cotton, wheat, rape, sugar beets, corn (maize), sorghum, soybeans, rice, oats, peanuts, vegetables, tomato, potato, perennial plantation crops including coffee, cocoa, oil palm, rubber, sugarcane, citrus, grapes, fruit trees, nut trees, banana, plantain, pineapple, hops, tea and forests such as eucalyptus and conifers (e.g., loblolly pine), and turf species (e.g., Kentucky bluegrass, St. Augustine grass, Kentucky fescue and Bermuda grass). Compounds of the present invention are particularly useful for selective weed control in crops of corn, rice (both upland and paddy), soybeans and wheat. Compounds of this invention can be used in crops genetically transformed or bred to incorporate resistance to herbicides, express proteins toxic to invertebrate pests (such as *Bacillus thuringiensis* toxin), and/or express other useful traits. Those skilled in the art will appreciate that not all compounds are equally effective against all weeds. Alternatively, the subject compounds are useful to modify plant growth.

As the compounds of the invention have both preemergent and postemergent herbicidal activity, to control undesired vegetation by killing or injuring the vegetation or reducing its growth, the compounds can be usefully applied by a variety of methods involving contacting a herbicidally effective amount of a compound of the invention, or a composition comprising said compound and at least one of a surfactant, a solid diluent or a liquid diluent, to the foliage or other part of the undesired vegetation or to the environment of the undesired vegetation such as the soil or water in which the undesired vegetation is growing or which surrounds the seed or other propagule of the undesired vegetation.

A herbicidally effective amount of the compounds of this invention is determined by a number of factors. These factors include: formulation selected, method of application, amount and type of vegetation present, growing conditions, etc. In general, a herbicidally effective amount of compounds of this invention is about 0.0001 to 20 kg/ha with a preferred range of about 0.001 to 5 kg/ha and a more preferred range of about 0.004 to 3 kg/ha. One skilled in the art can easily determine the herbicidally effective amount necessary for the desired level of weed control.

Compounds of this invention can also be mixed with one or more other biologically active compounds or agents including herbicides, herbicide safeners, fungicides, insecticides, nematocides, bactericides, acaricides, growth regulators such as insect molting inhibitors and rooting stimulants, chemosterilants, semiochemicals, repellents, attractants, pheromones, feeding stimulants, plant nutrients, other biologically active compounds or entomopathogenic bacteria, virus or fungi to form a multi-component pesticide giving an even broader spectrum of agricultural protection. Mixtures of the compounds of the invention with other herbicides can broaden the spectrum of activity against additional weed species, and suppress the proliferation of any resistant biotypes. Thus the present invention also pertains to a composition comprising a herbicidally effective amount of a compound of Formula **1** and a biologically effective amount of at least one additional biologically active compound or agent and can further comprise at least one of a surfactant, a solid diluent or a liquid diluent. The other biologically active compounds or agents can be formulated in compositions comprising at least one of a surfactant, solid or liquid diluent. For mixtures of the present invention, one or more other biologically active compounds or agents can be formulated together with a compound of Formula **1**, to form a premix, or one or more other biologically active compounds or agents can be formulated separately from the compound of Formula **1**, and the formulations combined together before application (e.g., in a spray tank) or, alternatively, applied in succession.

A mixture of one or more of the following herbicides with a compound of this invention may be particularly useful for weed control: acetochlor, acifluorfen and its sodium salt, aclonifen, acrolein (2-propenal), alachlor, alloxydim, ametryn, amicarbazone, amidosulfuron, aminocyclopyrachlor and its esters (e.g., methyl, ethyl) and salts (e.g., sodium, potassium), aminopyralid, amitrole, ammonium sulfamate, anilofos, asulam, atrazine, azimsulfuron, beflubutamid, benazolin, benazolin-ethyl, bencarbazone, benfluralin, benfuresate, bensulfuron-methyl, bensulide, bentazone, benzobicyclon, benzofenap, bifenox, bilanafos, bispyribac and its sodium salt, bromacil, bromobutide, bromofenoxim, bromoxynil, bromoxynil octanoate, butachlor, butafenacil, butamifos, butralin, butroxydim, butylate, cafenstrole, carbetamide, carfentrazone-ethyl, catechin, chlomethoxyfen, chloramben, chlorbromuron, chlorflurenol-methyl, chloridazon, chlorimuron-ethyl, chlorotoluron, chlorpropham, chlorsulfuron, chlorthal-dimethyl, chlorthiamid, cinidon-ethyl, cinmethylin, cinosulfuron, clefoxydim, clethodim, clodinafop-propargyl, clomazone, clomeprop, clopyralid, clopyralid-olamine, cloransulam-methyl, cumyluron, cyanazine, cycloate, cyclosulfamuron, cycloxydim, cyhalofop-butyl, 2,4-D and its butotyl, butyl, isoctyl and isopropyl esters and its dimethylammonium, diolamine and trolamine salts, daimuron, dalapon, dalapon-sodium, dazomet, 2,4-DB and its dimethylammonium, potassium and sodium salts, desmedipham, desmetryn, dicamba and its diglycolammonium, dimethylammonium, potassium and sodium salts, dichlobenil, dichlorprop, diclofop-methyl, diclosulam, difenzoquat metilsulfate, diflufenican, diflufenzopyr, dimefuron, dimepiperate, dimethachlor, dimethametryn, dimethenamid, dimethenamid-P, dimethipin, dimethylarsinic acid and its sodium salt, dinitramine, dinoterb, diphenamid, diquat dibromide, dithiopyr, diuron, DNOC, endothal, EPTC, esprocarb, ethalfluralin, ethametsulfuron-methyl, ethiozin, ethofumesate, ethoxyfen, ethoxysulfuron, etobenzanid, fenoxaprop-ethyl, fenoxaprop-P-ethyl, fentrazamide, fenuron, fenuron-TCA, flamprop-methyl, flamprop-M-isopropyl, flamprop-M-methyl, flazasulfuron, florasulam, fluazifop-butyl, fluazifop-P-butyl, flucarbazone, flucetosulfuron, fluchloralin, flufenacet, flufenpyr, flufenpyr-ethyl, fluazolate, flumetsulam, flumiclorac-pentyl, flumioxazin, fluometuron, fluoroglycofen-ethyl, flupoxam, flupyrsulfuron-methyl and its sodium salt, flurenol, flurenol-butyl, fluridone, flurochloridone, fluroxypyr, flurtamone, fluthiacet-methyl, fomesafen, foramsulfuron, fosamine-ammonium, glufosinate, glufosinate-ammonium, glyphosate and its salts such as ammonium, isopropylammonium, potassium, sodium (including sesquisodium) and trimesium (alternatively named sulfosate), halosulfuron-methyl, haloxyfop-etotyl, haloxyfop-methyl, hexazinone, imazamethabenz-methyl, imazamox, imazapic, imazapyr, imazaquin, imazaquin-ammonium, imazethapyr, imazethapyr-ammonium, imazosulfuron, indanofan, iodosulfuron-methyl, ioxynil, ioxynil octanoate, ioxynil-sodium, isoproturon, isouron, isoxaben, isoxaflutole, isoxachlortole, lactofen, lenacil, linuron, maleic hydrazide, MCPA and its dimethylammonium, potassium and sodium salts, MCPA-isoctyl, MCPA-thioethyl, MCPB and its sodium salt, MCPB-ethyl, mecoprop, mecoprop-P, mefenacet, mefluidide, mesosulfuron-methyl, mesotrione, metam-sodium, metamifop, metamitron, metazachlor, methabenzthiazuron, methylarsonic acid and its calcium, monoammonium, monosodium and disodium salts, methyldymron, metobenzuron, metobromuron, metolachlor, S-metolachlor, metosulam, metoxuron, metribuzin, metsulfuron-methyl, molinate, monolinuron, naproanilide, napropamide, naptalam, neburon, nicosulfuron, norflurazon, orbencarb, orthosulfamuron, oryzalin, oxadiargyl, oxadiazon, oxasulfuron, oxaziclomefone, oxyfluorfen, paraquat dichloride, pebulate, pelargonic acid, pendimethalin, penoxsulam, pentanochlor, pentoxazone, perfluidone, pethoxamid, pethoxyamid, phenmedipham, picloram, picloram-potassium, picolinafen, pinoxaden, piperophos, pretilachlor, primisulfuron-methyl, prodiamine, profoxydim, prometon, prometryn, propachlor, propanil, propaquizafop, propazine, propham, propisochlor, propoxycarbazone, propyzamide, prosulfocarb, prosulfuron, pyraclonil, pyraflufen-ethyl, pyrasulfotole, pyrazogyl, pyrazolynate, pyrazoxyfen, pyrazosulfuron-ethyl, pyribenzoxim, pyributicarb, pyridate, pyriftalid, pyriminobac-methyl, pyrimisulfan, pyrithiobac, pyrithiobac-sodium, pyroxasulfone, pyroxsulam, quinclorac, quinmerac, quinoclamine, quizalofop-ethyl, quizalofop-P-ethyl, quizalofop-P-tefuryl, rimsulfuron, sethoxydim, siduron, simazine, simetryn, sulcotrione, sulfentrazone, sulfometuron-methyl, sulfosulfuron, 2,3,6-TBA, TCA, TCA-sodium, tebutam, tebuthiuron, tefuryltrione, tembotrione, tepraloxydim, terbacil, terbumeton, terbuthylazine, terbutryn, thenylchlor, thiazopyr, thiencarbazone, thifensulfuron-methyl, thiobencarb, tiocarbazil, topramezone, tralkoxydim, tri-allate, triasulfuron, triaziflam, tribenuron-methyl, triclopyr, triclopyr-butotyl, triclopyr-triethylammonium, tridiphane, trietazine, trifloxysulfuron, trifluralin, triflusulfuron-methyl, tritosulfuron and vemolate. Other herbicides also include bioherbicides such as *Alternaria destruens* Simmons, *Colletotrichum gloeosporiodes* (Penz.) Penz. & Sacc., *Drechsiera monoceras* (MTB-951), *Myrothecium verrucaria* (Albertini & Schweinitz) Ditmar: Fries, *Phytophthora palmivora* (Butl.) Butl. and *Puccinia thlaspeos* Schub. In certain instances, combinations of a compound of this invention with other biologically active (particularly herbicidal) compounds or agents (i.e. active ingredients) can result in a greater-than-additive (i.e. synergistic) effect on weeds and/or a less-than-additive effect (i.e. safening) on crops or other desirable plants. Reducing the quantity of active ingredients released in the environment while ensuring effective pest control is always desirable. Ability to use greater amounts of active ingredients to provide more effective weed control without excessive crop injury is also desirable. When synergism of herbicidal active ingredients occurs on weeds at application rates giving agronomically satisfactory levels of weed control, such combinations can be advantageous for reducing crop production cost and decreasing environmental load. When safening of herbicidal active ingredients occurs on crops, such combinations can be advantageous for increasing crop protection by reducing weed competition.

Of note is a combination of a compound of Formula **1** with at least one other herbicidal active ingredient. Of particular note is such a combination where the other herbicidal active ingredient has a different site of action from the compound of Formula **1**. In certain instances, a combination with at least one other herbicidal active ingredient having a similar spectrum of control but a different site of action will be particularly advantageous for resistance management. Thus, a composition of the present invention can further comprise a biologically effective amount of at least one additional herbicidal active ingredient having a similar spectrum of control but a different site of action. Herbicidally effective amounts of compounds of the invention as well as herbicidally effective amounts of other herbicides can be easily determined by one skilled in the art through simple experimentation.

Preferred for better control of undesired vegetation (e.g., lower use rate, broader spectrum of weeds controlled, or enhanced crop safety) or for preventing the development of resistant weeds are mixtures of a compound of this invention with a herbicide selected from the group consisting of 2,4-D, aminocyclopyrachlor, atrazine, chlorimuron-ethyl, chlorsulfuron, clomazone, diflufenican, dimethenamid, flufenacet, flumetsulam, flumioxazin, flupyrsulfuron-methyl, flupyrsulfuron-methyl-sodium, glyphosate (particularly glyphosate-isopropylammonium, glyphosate-sodium, glyphosate-potassium, glyphosate-trimesium), imazamethabenz-methyl, imazaquin, imazethapyr, iodosulfuron-methyl, isoproturon, lactofen, mesosulfuron-methyl, mesotrione, metribuzin, metsulfuron-methyl, nicosulfuron, rimsulfuron, S-metolachlor, sulfentrazone, thifensulfuron-methyl, and tribenuron-methyl. Specifically preferred mixtures (compound numbers refer to compounds in Index Table A) are selected from the group: compound 1 and 2,4-D; compound 2 and 2,4-D; compound 3 and 2,4-D; compound 4 and 2,4-D; compound 5 and 2,4-D; compound 6 and 2,4-D; compound 7 and 2,4-D; compound 1 and aminocyclopyrachlor; compound 2 and aminocyclopyrachlor; compound 3 and aminocyclopyrachlor; compound 4 and aminocyclopyrachlor; compound 5 and aminocyclopyrachlor; compound 6 and aminocyclopyrachlor; compound 7 and aminocyclopyrachlor; compound 1 and atrazine; compound 2 and atrazine; compound 3 and atrazine; compound 4 and atrazine; compound 5 and atrazine; compound 6 and atrazine; compound 7 and atrazine; compound 1 and chlorimuron-ethyl; compound 2 and chlorimuron-ethyl; compound 3 and chlorimuron-ethyl; compound 4 and chlorimuron-ethyl; compound 5 and chlorimuron-ethyl; compound 6 and chlorimuron-ethyl; compound 7 and chlorimuron-ethyl; compound 1 and chlorsulfuron; compound 2 and chlorsulfuron; compound 3 and chlorsulfuron; compound 4 and chlorsulfuron; compound 5 and chlorsulfuron; compound 6 and chlorsulfuron; compound 7 and chlorsulfuron; compound 1 and clomazone; compound 2 and clomazone; compound 3 and clomazone; compound 4 and clomazone; compound 5 and clomazone; compound 6 and clomazone; compound 7 and clomazone; compound 1 and diflufenican; compound 2 and diflufenican; compound 3 and diflufenican; compound 4 and diflufenican; compound 5 and diflufenican; compound 6 and diflufenican; compound 7 and diflufenican; compound 1 and dimethenamid; compound 2 and dimethenamid; compound 3 and dimethenamid; compound 4 and dimethenamid; compound 5 and dimethenamid; compound 6 and dimethenamid; compound 7 and dimethenamid; compound 1 and flufenacet; compound 2 and flufenacet; compound 3 and flufenacet; compound 4 and flufenacet; compound 5 and flufenacet; compound 6 and flufenacet; compound 7 and flufenacet; compound 1 and flumetsulam; compound 2 and flumetsulam; compound 3 and flumetsulam; compound 4 and flumetsulam; compound 5 and flumetsulam; compound 6 and flumetsulam; compound 7 and flumetsulam; compound 1 and flumioxazin; compound 2 and flumioxazin; compound 3 and flumioxazin; compound 4 and flumioxazin; compound 5 and flumioxazin; compound 6 and flumioxazin; compound 7 and flumioxazin; compound 1 and flupyrsulfuron-methyl; compound 2 and flupyrsulfuron-methyl; compound 3 and flupyrsulfuron-methyl; compound 4 and flupyrsulfuron-methyl; compound 5 and flupyrsulfuron-methyl; compound 6 and flupyrsulfuron-methyl; compound 7 and flupyrsulfuron-methyl; compound 1 and flupyrsulfuron-methyl-sodium; compound 2 and flupyrsulfuron-methyl-sodium; compound 3 and flupyrsulfuron-methyl-sodium; compound 4 and flupyrsulfuron-methyl-sodium; compound 5 and flupyrsulfuron-methyl-sodium; compound 6 and flupyrsulfuron-methyl-sodium; compound 7 and flupyrsulfuron-methyl-sodium; compound 1 and glyphosate; compound 2 and glyphosate; compound 3 and glyphosate; compound 4 and glyphosate; compound 5 and glyphosate; compound 6 and glyphosate; compound 7 and glyphosate; compound 1 and imazamethabenz-methyl; compound 2 and imazamethabenz-methyl; compound 3 and imazamethabenz-methyl; compound 4 and imazamethabenz-methyl; compound 5 and imazamethabenz-methyl; compound 6 and imazamethabenz-methyl; compound 7 and imazamethabenz-methyl; compound 1 and imazaquin; compound 2 and imazaquin; compound 3 and imazaquin; compound 4 and imazaquin; compound 5 and imazaquin; compound 6 and imazaquin; compound 7 and imazaquin; compound 1 and imazethapyr; compound 2 and imazethapyr; compound 3 and imazethapyr; compound 4 and imazethapyr; compound 5 and imazethapyr; compound 6 and imazethapyr; compound 7 and imazethapyr; compound 1 and iodosulfuron-methyl; compound 2 and iodosulfuron-methyl; compound 3 and iodosulfuron-methyl; compound 4 and iodosulfuron-methyl; compound 5 and iodosulfuron-methyl; compound 6 and iodosulfuron-methyl; compound 7 and iodosulfuron-methyl; compound 1 and isoproturon; compound 2 and isoproturon; compound 3 and isoproturon; compound 4 and isoproturon; compound 5 and isoproturon; compound 6 and isoproturon; compound 7 and isoproturon; compound 1 and lactofen; compound 2 and lactofen; compound 3 and lactofen; compound 4 and lactofen; compound 5 and lactofen; compound 6 and lactofen; compound 7 and lactofen; compound 1 and mesosulfuron-methyl; compound 2 and mesosulfuron-methyl; compound 3 and mesosulfuron-methyl; compound 4 and mesosulfuron-methyl; compound 5 and mesosulfuron-methyl; compound 6 and mesosulfuron-methyl; compound 7 and mesosulfuron-methyl; compound 1 and mesotrione; compound 2 and mesotrione; compound 3 and mesotrione; compound 4 and mesotrione; compound 5 and mesotrione; compound 6 and mesotrione; compound 7 and mesotrione; compound 1 and metribuzin; compound 2 and metribuzin; compound 3 and metribuzin; compound 4 and metribuzin; compound 5 and metribuzin; compound 6 and metribuzin; compound 7 and metribuzin; compound 1 and metsulfuron-methyl; compound 2 and metsulfuron-methyl; compound 3 and metsulfuron-methyl; compound 4 and metsulfuron-methyl; compound 5 and metsulfuron-methyl; compound 6 and metsulfuron-methyl; compound 7 and metsulfuron-methyl; compound 1 and nicosulfuron; compound 2 and nicosulfuron; compound 3 and nicosulfuron; compound 4 and nicosulfuron; compound 5 and nicosulfuron; compound 6 and nicosulfuron; compound 7 and nicosulfuron; compound 1 and rimsulfuron; compound 2 and rimsulfuron; compound 3 and rimsulfuron; compound 4 and rimsulfuron; compound 5 and rimsulfuron; compound 6 and rimsulfuron; compound 7 and rimsulfuron; compound 1 and S-metolachlor; compound 2 and S-metolachlor; compound 3 and S-metolachlor; compound 4 and S-metolachlor; compound 5 and S-metolachlor; compound 6 and S-metolachlor; compound 7 and S-metolachlor; compound 1 and sulfentrazone; compound 2 and sulfentrazone; compound 3 and sulfentrazone; compound 4 and sulfentrazone; compound 5 and sulfentrazone; compound 6 and sulfentrazone; compound 7 and sulfentrazone; compound 1 and thifensulfuron-methyl; compound 2 and thifensulfuron-methyl; compound 3 and thifensulfuron-methyl; compound 4 and thifensulfuron-methyl; compound 5 and thifensulfuron-methyl; compound 6 and thifensulfuron-methyl; compound 7 and thifensulfuron-methyl; compound 1 and tribenuron-methyl; compound 2 and tribenuron-methyl; compound 3 and tribenuron-methyl; compound 4 and tribenuron-methyl; compound 5 and tribenuron-methyl; compound 6 and tribenuron-methyl; compound 7 and tribenuron-methyl.

Also specifically preferred mixtures (compound numbers refer to compounds in Index Table A) are selected from the group: compound 11 and 2,4-D; compound 12 and 2,4-D; compound 21 and 2,4-D; compound 26 and 2,4-D; compound 31 and 2,4-D; compound 35 and 2,4-D; compound 37 and 2,4-D; compound 38 and 2,4-D; compound 57 and 2,4-D; compound 61 and 2,4-D; compound 66 and 2,4-D; compound 72 and 2,4-D; compound 74 and 2,4-D; compound 78 and 2,4-D; compound 82 and 2,4-D; compound 11 and aminocyclopyrachlor; compound 12 and aminocyclopyrachlor, compound 21 and aminocyclopyrachlor, compound 26 and aminocyclopyrachlor, compound 31 and aminocyclopyrachlor, compound 35 and aminocyclopyrachlor, compound 37 and aminocyclopyrachlor, compound 38 and aminocyclopyrachlor, compound 57 and aminocyclopyrachlor, compound 61 and aminocyclopyrachlor, compound 66 and aminocyclopyrachlor, compound 72 and aminocyclopyrachlor, compound 74 and aminocyclopyrachlor, compound 78 and aminocyclopyrachlor, compound 82 and aminocyclopyrachlor, compound 12 and atrazine; compound 21 and atrazine; compound 26 and atrazine; compound 31 and atrazine; compound 35 and atrazine; compound 37 and atrazine; compound 38 and atrazine; compound 57 and atrazine; compound 61 and atrazine; compound 66 and atrazine; compound 72 and atrazine; compound 74 and atrazine; compound 78 and atrazine; compound 82 and atrazine; compound 11 and bromoxynil; compound 12 and bromoxynil; compound 21 and bromoxynil; compound 26 and bromoxynil; compound 31 and bromoxynil; compound 35 and bromoxynil; compound 37 and bromoxynil; compound 38 and bromoxynil; compound 57 and bromoxynil; compound 61 and bromoxynil; compound 66 and bromoxynil; compound 72 and bromoxynil; compound 74 and bromoxynil; compound 78 and bromoxynil; compound 82 and bromoxynil; compound 11 and bromoxynil octanoate; compound 12 and bromoxynil octanoate; compound 21 and bromoxynil octanoate; compound 26 and bromoxynil octanoate; compound 31 and bromoxynil octanoate; compound 35 and bromoxynil octanoate; compound 37 and bromoxynil octanoate; compound 38 and bromoxynil octanoate; compound 57 and bromoxynil octanoate; compound 61 and bromoxynil octanoate; compound 66 and bromoxynil octanoate; compound 72 and bromoxynil octanoate; compound 74 and bromoxynil octanoate; compound 78 and bromoxynil octanoate; compound 82 and bromoxynil octanoate; compound 11 and carfentrazone-ethyl; compound 12 and carfentrazone-ethyl; compound 21 and carfentrazone-ethyl; compound 26 and carfentrazone-ethyl; compound 31 and carfentrazone-ethyl; compound 35 and carfentrazone-ethyl; compound 37 and carfentrazone-ethyl; compound 38 and carfentrazone-ethyl; compound 57 and carfentrazone-ethyl; compound 61 and carfentrazone-ethyl; compound 66 and carfentrazone-ethyl; compound 72 and carfentrazone-ethyl; compound 74 and carfentrazone-ethyl; compound 78 and carfentrazone-ethyl; compound 82 and carfentrazone-ethyl; compound 11 and chlorimuron-ethyl; compound 12 and chlorimuron-ethyl; compound 21 and chlorimuron-ethyl; compound 26 and chlorimuron-ethyl; compound 31 and chlorimuron-ethyl; compound 35 and chlorimuron-ethyl; compound 37 and chlorimuron-ethyl; compound 38 and chlorimuron-ethyl; compound 57 and chlorimuron-ethyl; compound 61 and chlorimuron-ethyl; compound 66 and chlorimuron-ethyl; compound 72 and chlorimuron-ethyl; compound 74 and chlorimuron-ethyl; compound 78 and chlorimuron-ethyl; compound 82 and chlorimuron-ethyl; compound 11 and chlorsulfuron; compound 12 and chlorsulfuron; compound 21 and chlorsulfuron; compound 26 and chlorsulfuron; compound 31 and chlorsulfuron; compound 35 and chlorsulfuron; compound 37 and chlorsulfuron; compound 38 and chlorsulfuron; compound 57 and chlorsulfuron; compound 61 and chlorsulfuron; compound 66 and chlorsulfuron; compound 72 and chlorsulfuron; compound 74 and chlorsulfuron; compound 78 and chlorsulfuron; compound 82 and chlorsulfuron; compound 11 and clomazone; compound 12 and clomazone; compound 21 and clomazone; compound 26 and clomazone; compound 31 and clomazone; compound 35 and clomazone; compound 37 and clomazone; compound 38 and clomazone; compound 57 and clomazone; compound 61 and clomazone; compound 66 and clomazone; compound 72 and clomazone; compound 74 and clomazone; compound 78 and clomazone; compound 82 and clomazone; compound 11 and clopyralid; compound 12 and clopyralid; compound 21 and clopyralid; compound 26 and clopyralid; compound 31 and clopyralid; compound 35 and clopyralid; compound 37 and clopyralid; compound 38 and clopyralid; compound 57 and clopyralid; compound 61 and clopyralid; compound 66 and clopyralid; compound 72 and clopyralid; compound 74 and clopyralid; compound 78 and clopyralid; compound 82 and clopyralid; compound 11 and dicamba; compound 12 and dicamba; compound 21 and dicamba; compound 26 and dicamba; compound 31 and dicamba; compound 35 and dicamba; compound 37 and dicamba; compound 38 and dicamba; compound 57 and dicamba; compound 61 and dicamba; compound 66 and dicamba; compound 72 and dicamba; compound 74 and dicamba; compound 78 and dicamba; compound 82 and dicamba; compound 11 and diflufenican; compound 12 and diflufenican; compound 21 and diflufenican; compound 26 and diflufenican; compound 31 and diflufenican; compound 35 and diflufenican; compound 37 and diflufenican; compound 38 and diflufenican; compound 57 and diflufenican; compound 61 and diflufenican; compound 66 and diflufenican; compound 72 and diflufenican; compound 74 and diflufenican; compound 78 and diflufenican; compound 82 and diflufenican; compound 11 and dimethenamid; compound 12 and dimethenamid; compound 21 and dimethenamid; compound 26 and dimethenamid; compound 31 and dimethenamid; compound 35 and dimethenamid; compound 37 and dimethenamid; compound 38 and dimethenamid; compound 57 and dimethenamid; compound 61 and dimethenamid; compound 66 and dimethenamid; compound 72 and dimethenamid; compound 74 and dimethenamid; compound 78 and dimethenamid; compound 82 and dimethenamid; compound 11 and florasulam; compound 12 and florasulam; compound 21 and florasulam; compound 26 and florasulam; compound 31 and florasulam; compound 35 and florasulam; compound 37 and florasulam; compound 38 and florasulam; compound 57 and florasulam; compound 61 and florasulam; compound 66 and florasulam; compound 72 and florasulam; compound 74 and florasulam; compound 78 and florasulam; compound 82 and florasulam; compound 11 and flufenacet; compound 12 and flufenacet; compound 21 and flufenacet; compound 26 and flufenacet; compound 31 and flufenacet; compound 35 and flufenacet; compound 37 and flufenacet; compound 38 and flufenacet; compound 57 and flufenacet; compound 61 and flufenacet; compound 66 and flufenacet; compound 72 and flufenacet; compound 74 and flufenacet; compound 78 and flufenacet; compound 82 and flufenacet; compound 11 and flumetsulam; compound 12 and flumetsulam; compound 21 and flumetsulam; compound 26 and flumetsulam; compound 31 and flumetsulam; compound 35 and flumetsulam; compound 37 and flumetsulam; compound 38 and flumetsulam; compound 57 and flumetsulam; compound 61 and flumetsulam; compound 66 and flumetsulam; compound 72 and flumetsulam; compound 74 and flumetsulam; compound 78 and flumetsulam; compound 82 and flumetsulam; compound 11 and flumioxazin; compound 12 and flumioxazin; compound 21 and flumioxazin; compound 26 and flumioxazin; compound 31 and flumioxazin; compound 35 and flumioxazin; compound 37 and flumioxazin; compound 38 and flumioxazin; compound 57 and flumioxazin; compound 61 and flumioxazin; compound 66 and flumioxazin; compound 72 and flumioxazin; compound 74 and flumioxazin; compound 78 and flumioxazin; compound 82 and flumioxazin; compound 11 and flupyrsulfuron-methyl; compound 12 and flupyrsulfuron-methyl; compound 21 and flupyrsulfuron-methyl; compound 26 and flupyrsulfuron-methyl; compound 31 and flupyrsulfuron-methyl; compound 35 and flupyrsulfuron-methyl; compound 37 and flupyrsulfuron-methyl; compound 38 and flupyrsulfuron-methyl; compound 57 and flupyrsulfuron-methyl; compound 61 and flupyrsulfuron-methyl; compound 66 and flupyrsulfuron-methyl; compound 72 and flupyrsulfuron-methyl; compound 74 and flupyrsulfuron-methyl; compound 78 and flupyrsulfuron-methyl; compound 82 and flupyrsulfuron-methyl; compound 11 and flupyrsulfuron-methyl-sodium; compound 12 and flupyrsulfuron-methyl-sodium; compound 21 and flupyrsulfuron-methyl-sodium; compound 26 and flupyrsulfuron-methyl-sodium; compound 31 and flupyrsulfuron-methyl-sodium; compound 35 and flupyrsulfuron-methyl-sodium; compound 37 and flupyrsulfuron-methyl-sodium; compound 38 and flupyrsulfuron-methyl-sodium; compound 57 and flupyrsulfuron-methyl-sodium; compound 61 and flupyrsulfuron-methyl-sodium; compound 66 and flupyrsulfuron-methyl-sodium; compound 72 and flupyrsulfuron-methyl-sodium; compound 74 and flupyrsulfuron-methyl-sodium; compound 78 and flupyrsulfuron-methyl-sodium; compound 82 and flupyrsulfuron-methyl-sodium; compound 11 and fluroxypyr; compound 12 and fluroxypyr; compound 21 and fluroxypyr; compound 26 and fluroxypyr; compound 31 and fluroxypyr; compound 35 and fluroxypyr; compound 37 and fluroxypyr; compound 38 and fluroxypyr; compound 57 and fluroxypyr; compound 61 and fluroxypyr; compound 66 and fluroxypyr; compound 72 and fluroxypyr; compound 74 and fluroxypyr; compound 78 and fluroxypyr; compound 82 and fluroxypyr; compound 11 and glyphosate; compound 12 and glyphosate; compound 21 and glyphosate; compound 26 and glyphosate; compound 31 and glyphosate; compound 35 and glyphosate; compound 37 and glyphosate; compound 38 and glyphosate; compound 57 and glyphosate; compound 61 and glyphosate; compound 66 and glyphosate; compound 72 and glyphosate; compound 74 and glyphosate; compound 78 and glyphosate; compound 82 and glyphosate; compound 11 and imazamethabenz-methyl; compound 12 and imazamethabenz-methyl; compound 21 and imazamethabenz-methyl; compound 26 and imazamethabenz-methyl; compound 31 and imazamethabenz-methyl; compound 35 and imazamethabenz-methyl; compound 37 and imazamethabenz-methyl; compound 38 and imazamethabenz-methyl; compound 57 and imazamethabenz-methyl; compound 61 and imazamethabenz-methyl; compound 66 and imazamethabenz-methyl; compound 72 and imazamethabenz-methyl; compound 74 and imazamethabenz-methyl; compound 78 and imazamethabenz-methyl; compound 82 and imazamethabenz-methyl; compound 11 and imazaquin; compound 12 and imazaquin; compound 21 and imazaquin; compound 26 and imazaquin; compound 31 and imazaquin; compound 35 and imazaquin; compound 37 and imazaquin; compound 38 and imazaquin; compound 57 and imazaquin; compound 61 and imazaquin; compound 66 and imazaquin; compound 72 and imazaquin; compound 74 and imazaquin; compound 78 and imazaquin; compound 82 and imazaquin; compound 11 and imazethapyr; compound 12 and imazethapyr; compound 21 and imazethapyr; compound 26 and imazethapyr; compound 31 and imazethapyr; compound 35 and imazethapyr; compound 37 and imazethapyr; compound 38 and imazethapyr; compound 57 and imazethapyr; compound 61 and imazethapyr; compound 66 and imazethapyr; compound 72 and imazethapyr; compound 74 and imazethapyr; compound 78 and imazethapyr; compound 82 and imazethapyr; compound 11 and iodosulfuron-methyl; compound 12 and iodosulfuron-methyl; compound 21 and iodosulfuron-methyl; compound 26 and iodosulfuron-methyl; compound 31 and iodosulfuron-methyl; compound 35 and iodosulfuron-methyl; compound 37 and iodosulfuron-methyl; compound 38 and iodosulfuron-methyl; compound 57 and iodosulfuron-methyl; compound 61 and iodosulfuron-methyl; compound 66 and iodosulfuron-methyl; compound 72 and iodosulfuron-methyl; compound 74 and iodosulfuron-methyl; compound 78 and iodosulfuron-methyl; compound 82 and iodosulfuron-methyl; compound 11 and isoproturon; compound 12 and isoproturon; compound 21 and isoproturon; compound 26 and isoproturon; compound 31 and isoproturon; compound 35 and isoproturon; compound 37 and isoproturon; compound 38 and isoproturon; compound 57 and isoproturon; compound 61 and isoproturon; compound 66 and isoproturon; compound 72 and isoproturon; compound 74 and isoproturon; compound 78 and isoproturon; compound 82 and isoproturon; compound 11 and lactofen; compound 12 and lactofen; compound 21 and lactofen; compound 26 and lactofen; compound 31 and lactofen; compound 35 and lactofen; compound 37 and lactofen; compound 38 and lactofen; compound 57 and lactofen; compound 61 and lactofen; compound 66 and lactofen; compound 72 and lactofen; compound 74 and lactofen; compound 78 and lactofen; compound 82 and lactofen; compound 11 and MCPA; compound 12 and MCPA; compound 21 and MCPA; compound 26 and MCPA; compound 31 and MCPA; compound 35 and MCPA; compound 37 and MCPA; compound 38 and MCPA; compound 57 and MCPA; compound 61 and MCPA; compound 66 and MCPA; compound 72 and MCPA; compound 74 and MCPA; compound 78 and MCPA; compound 82 and MCPA; compound 11 and MCPA-isoctyl; compound 12 and MCPA-isoctyl; compound 21 and MCPA-isoctyl; compound 26 and MCPA-isoctyl; compound 31 and MCPA-isoctyl; compound 35 and MCPA-isoctyl; compound 37 and MCPA-isoctyl; compound 38 and MCPA-isoctyl; compound 57 and MCPA-isoctyl; compound 61 and MCPA-isoctyl; compound 66 and MCPA-isoctyl; compound 72 and MCPA-isoctyl; compound 74 and MCPA-isoctyl; compound 78 and MCPA-isoctyl; compound 82 and MCPA-isoctyl; compound 11 and MCPA-thioethyl; compound 12 and MCPA-thioethyl; compound 21 and MCPA-thioethyl; compound 26 and MCPA-thioethyl; compound 31 and MCPA-thioethyl; compound 35 and MCPA-thioethyl; compound 37 and MCPA-thioethyl; compound 38 and MCPA-thioethyl; compound 57 and MCPA-thioethyl; compound 61 and MCPA-thioethyl; compound 66 and MCPA-thioethyl; compound 72 and MCPA-thioethyl; compound 74 and MCPA-thioethyl; compound 78 and MCPA-thioethyl; compound 82 and MCPA-thioethyl; compound 11 and mesosulfuron-methyl; compound 12 and mesosulfuron-methyl; compound 21 and mesosulfuron-methyl; compound 26 and mesosulfuron-methyl; compound 31 and mesosulfuron-methyl; compound 35 and mesosulfuron-methyl; compound 37 and mesosulfuron-methyl; compound 38 and mesosulfuron-methyl; compound 57 and mesosulfuron-methyl; compound 61 and mesosulfuron-methyl; compound 66 and mesosulfuron-methyl; compound 72 and mesosulfuron-methyl; compound 74 and mesosulfuron-methyl; compound 78 and mesosulfuron-methyl; compound 82 and mesosulfuron-methyl; compound 11 and mesotrione; compound 12 and mesotrione; compound 21 and mesotrione; compound 26 and mesotrione; compound 31 and mesotrione; compound 35 and mesotrione; compound 37 and mesotrione; compound 38 and mesotrione; compound 57 and mesotrione; compound 61 and mesotrione; compound 66 and mesotrione; compound 72 and mesotrione; compound 74 and mesotrione; compound 78 and mesotrione; compound 82 and mesotrione; compound 11 and metribuzin; compound 12 and metribuzin; compound 21 and metribuzin; compound 26 and metribuzin; compound 31 and metribuzin; compound 35 and metribuzin; compound 37 and metribuzin; compound 38 and metribuzin; compound 57 and metribuzin; compound 61 and metribuzin; compound 66 and metribuzin; compound 72 and metribuzin; compound 74 and metribuzin; compound 78 and metribuzin; compound 82 and metribuzin; compound 11 and metsulfuron-methyl; compound 12 and metsulfuron-methyl; compound 21 and metsulfuron-methyl; compound 26 and metsulfuron-methyl; compound 31 and metsulfuron-methyl; compound 35 and metsulfuron-methyl; compound 37 and metsulfuron-methyl; compound 38 and metsulfuron-methyl; compound 57 and metsulfuron-methyl; compound 61 and metsulfuron-methyl; compound 66 and metsulfuron-methyl; compound 72 and metsulfuron-methyl; compound 74 and metsulfuron-methyl; compound 78 and metsulfuron-methyl; compound 82 and metsulfuron-methyl; compound 11 and nicosulfuron; compound 12 and nicosulfuron; compound 21 and nicosulfuron; compound 26 and nicosulfuron; compound 31 and nicosulfuron; compound 35 and nicosulfuron; compound 37 and nicosulfuron; compound 38 and nicosulfuron; compound 57 and nicosulfuron; compound 61 and nicosulfuron; compound 66 and nicosulfuron; compound 72 and nicosulfuron; compound 74 and nicosulfuron; compound 78 and nicosulfuron; compound 82 and nicosulfuron; compound 11 and oxyfluorfen; compound 12 and oxyfluorfen; compound 21 and oxyfluorfen; compound 26 and oxyfluorfen; compound 31 and oxyfluorfen; compound 35 and oxyfluorfen; compound 37 and oxyfluorfen; compound 38 and oxyfluorfen; compound 57 and oxyfluorfen; compound 61 and oxyfluorfen; compound 66 and oxyfluorfen; compound 72 and oxyfluorfen; compound 74 and oxyfluorfen; compound 78 and oxyfluorfen; compound 82 and oxyfluorfen; compound 11 and pendimethalin; compound 12 and pendimethalin; compound 21 and pendimethalin; compound 26 and pendimethalin; compound 31 and pendimethalin; compound 35 and pendimethalin; compound 37 and pendimethalin; compound 38 and pendimethalin; compound 57 and pendimethalin; compound 61 and pendimethalin; compound 66 and pendimethalin; compound 72 and pendimethalin; compound 74 and pendimethalin; compound 78 and pendimethalin; compound 82 and pendimethalin; compound 11 and pronamide; compound 12 and pronamide; compound 21 and pronamide; compound 26 and pronamide; compound 31 and pronamide; compound 35 and pronamide; compound 37 and pronamide; compound 38 and pronamide; compound 57 and pronamide; compound 61 and pronamide; compound 66 and pronamide; compound 72 and pronamide; compound 74 and pronamide; compound 78 and pronamide; compound 82 and pronamide; compound 11 and prosulfuron; compound 12 and prosulfuron; compound 21 and prosulfuron; compound 26 and prosulfuron; compound 31 and prosulfuron; compound 35 and prosulfuron; compound 37 and prosulfuron; compound 38 and prosulfuron; compound 57 and prosulfuron; compound 61 and prosulfuron; compound 66 and prosulfuron; compound 72 and prosulfuron; compound 74 and prosulfuron; compound 78 and prosulfuron; compound 82 and prosulfuron; compound 11 and pyrasulfotole; compound 12 and pyrasulfotole; compound 21 and pyrasulfotole; compound 26 and pyrasulfotole; compound 31 and pyrasulfotole; compound 35 and pyrasulfotole; compound 37 and pyrasulfotole; compound 38 and pyrasulfotole; compound 57 and pyrasulfotole; compound 61 and pyrasulfotole; compound 66 and pyrasulfotole; compound 72 and pyrasulfotole; compound 74 and pyrasulfotole; compound 78 and pyrasulfotole; compound 82 and pyrasulfotole; compound 11 and pyroxsulam; compound 12 and pyroxsulam; compound 21 and pyroxsulam; compound 26 and pyroxsulam; compound 31 and pyroxsulam; compound 35 and pyroxsulam; compound 37 and pyroxsulam; compound 38 and pyroxsulam; compound 57 and pyroxsulam; compound 61 and pyroxsulam; compound 66 and pyroxsulam; compound 72 and pyroxsulam; compound 74 and pyroxsulam; compound 78 and pyroxsulam; compound 82 and pyroxsulam; compound 11 and quinclorac; compound 12 and quinclorac; compound 21 and quinclorac; compound 26 and quinclorac; compound 31 and quinclorac; compound 35 and quinclorac; compound 37 and quinclorac; compound 38 and quinclorac; compound 57 and quinclorac; compound 61 and quinclorac; compound 66 and quinclorac; compound 72 and quinclorac; compound 74 and quinclorac; compound 78 and quinclorac; compound 82 and quinclorac; compound 11 and rimsulfuron; compound 12 and rimsulfuron; compound 21 and rimsulfuron; compound 26 and rimsulfuron; compound 31 and rimsulfuron; compound 35 and rimsulfuron; compound 37 and rimsulfuron; compound 38 and rimsulfuron; compound 57 and rimsulfuron; compound 61 and rimsulfuron; compound 66 and rimsulfuron; compound 72 and rimsulfuron; compound 74 and rimsulfuron; compound 78 and rimsulfuron; compound 82 and rimsulfuron; compound 11 and S-metolachlor; compound 12 and S-metolachlor; compound 21 and S-metolachlor; compound 26 and S-metolachlor; compound 31 and S-metolachlor; compound 35 and S-metolachlor; compound 37 and S-metolachlor; compound 38 and S-metolachlor; compound 57 and S-metolachlor; compound 61 and S-metolachlor; compound 66 and S-metolachlor; compound 72 and S-metolachlor; compound 74 and S-metolachlor; compound 78 and S-metolachlor; compound 82 and S-metolachlor; compound 11 and sulfentrazone; compound 12 and sulfentrazone; compound 21 and sulfentrazone; compound 26 and sulfentrazone; compound 31 and sulfentrazone; compound 35 and sulfentrazone; compound 37 and sulfentrazone; compound 38 and sulfentrazone; compound 57 and sulfentrazone; compound 61 and sulfentrazone; compound 66 and sulfentrazone; compound 72 and sulfentrazone; compound 74 and sulfentrazone; compound 78 and sulfentrazone; compound 82 and sulfentrazone; compound 11 and thifensulfuron-methyl; compound 12 and thifensulfuron-methyl; compound 21 and thifensulfuron-methyl; compound 26 and thifensulfuron-methyl; compound 31 and thifensulfuron-methyl; compound 35 and thifensulfuron-methyl; compound 37 and thifensulfuron-methyl; compound 38 and thifensulfuron-methyl; compound 57 and thifensulfuron-methyl; compound 61 and thifensulfuron-methyl; compound 66 and thifensulfuron-methyl; compound 72 and thifensulfuron-methyl; compound 74 and thifensulfuron-methyl; compound 78 and thifensulfuron-methyl; compound 82 and thifensulfuron-methyl; compound 11 and triasulfuron; compound 12 and triasulfuron; compound 21 and triasulfuron; compound 26 and triasulfuron; compound 31 and triasulfuron; compound 35 and triasulfuron; compound 37 and triasulfuron; compound 38 and triasulfuron; compound 57 and triasulfuron; compound 61 and triasulfuron; compound 66 and triasulfuron; compound 72 and triasulfuron; compound 74 and triasulfuron; compound 78 and triasulfuron; compound 82 and triasulfuron; compound 11 and tribenuron-methyl; compound 12 and tribenuron-methyl; compound 21 and tribenuron-methyl; compound 26 and tribenuron-methyl; compound 31 and tribenuron-methyl; compound 35 and tribenuron-methyl; compound 37 and tribenuron-methyl; compound 38 and tribenuron-methyl; compound 57 and tribenuron-methyl; compound 61 and tribenuron-methyl; compound 66 and tribenuron-methyl; compound 72 and tribenuron-methyl; compound 74 and tribenuron-methyl; compound 78 and tribenuron-methyl; compound 82 and tribenuron-methyl; compound 11 and triclopyr; compound 12 and triclopyr; compound 21 and triclopyr; compound 26 and triclopyr; compound 31 and triclopyr; compound 35 and triclopyr; compound 37 and triclopyr; compound 38 and triclopyr; compound 57 and triclopyr; compound 61 and triclopyr; compound 66 and triclopyr; compound 72 and triclopyr; compound 74 and triclopyr; compound 78 and triclopyr; compound 82 and triclopyr; compound 11 and triclopyr-butotyl; compound 12 and triclopyr-butotyl; compound 21 and triclopyr-butotyl; compound 26 and triclopyr-butotyl; compound 31 and triclopyr-butotyl; compound 35 and triclopyr-butotyl; compound 37 and triclopyr-butotyl; compound 38 and triclopyr-butotyl; compound 57 and triclopyr-butotyl; compound 61 and triclopyr-butotyl; compound 66 and triclopyr-butotyl; compound 72 and triclopyr-butotyl; compound 74 and triclopyr-butotyl; compound 78 and triclopyr-butotyl; compound 82 and triclopyr-butotyl; compound 11 and triclopyr-triethylammonium; compound 12 and triclopyr-triethylammonium; compound 21 and triclopyr-triethylammonium; compound 26 and triclopyr-triethylammonium; compound 31 and triclopyr-triethylammonium; compound 35 and triclopyr-triethylammonium; compound 37 and triclopyr-triethylammonium; compound 38 and triclopyr-triethylammonium; compound 57 and triclopyr-triethylammonium; compound 61 and triclopyr-triethylammonium; compound 66 and triclopyr-triethylammonium; compound 72 and triclopyr-triethylammonium; compound 74 and triclopyr-triethylammonium; compound 78 and triclopyr-triethylammonium; compound 82 and triclopyr-triethylammonium;

Compounds of this invention can also be used in combination with herbicide safeners such as benoxacor, BCS (1-bromo-4-[(chloromethyl)sulfonyl]benzene), cloquintocet-mexyl, cyometrinil, cyprosulfamide, dichlormid, dicyclonon, dietholate, 2-(dichloromethyl)-2-methyl-1,3-dioxolane (MG 191), fenchlorazole-ethyl, fenclorim, flurazole, fluxofenim, furilazole, isoxadifen-ethyl, mefenpyr-diethyl, mephenate, methoxyphenone ((4-methoxy-3-methylphenyl)(3-methylphenyl)methanone), naphthalic anhydride (1,8-naphthalic anhydride) and oxabetrinil to increase safety to certain crops. Antidotally effective amounts of the herbicide safeners can be applied at the same time as the compounds of this invention, or applied as seed treatments. Therefore an aspect of the present invention relates to a herbicidal mixture comprising a compound of this invention and an antidotally effective amount of a herbicide safener. Seed treatment is particularly useful for selective weed control, because it physically restricts antidoting to the crop plants. Therefore a particularly useful embodiment of the present invention is a method for selectively controlling the growth of undesired vegetation in a crop comprising contacting the locus of the crop with a herbicidally effective amount of a compound of this invention wherein seed from which the crop is grown is treated with an antidotally effective amount of safener. Antidotally effective amounts of safeners can be easily determined by one skilled in the art through simple experimentation.

Compounds of this invention can also be used in combination with plant growth regulators such as aviglycine, *N*-(phenylmethyl)-1*H*-purin-6-amine, epocholeone, gibberellic acid, gibberellin A₄ and A₇, harpin protein, mepiquat chloride, prohexadione calcium, prohydrojasmon, sodium nitrophenolate and trinexapac-methyl, and plant growth modifying organisms such as *Bacillus cereus* strain BP01.

General references for agricultural protectants (i.e. herbicides, herbicide safeners, insecticides, fungicides, nematocides, acaricides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Farnham, Surrey, U.K., 2001.

For embodiments where one or more of these various mixing partners are used, the weight ratio of these various mixing partners (in total) to the compound of Formula 1 is typically between about 1:3000 and about 3000:1. Of note are weight ratios between about 1:300 and about 300:1 (for example ratios between about 1:30 and about 30:1). One skilled in the art can easily determine through simple experimentation the biologically effective amounts of active ingredients necessary for the desired spectrum of biological activity. It will be evident that including these additional components may expand the spectrum of weeds controlled beyond the spectrum controlled by the compound of Formula 1 alone.

The following Tests demonstrate the control efficacy of the compounds of this invention against specific weeds. The weed control afforded by the compounds is not limited, however, to these species. See Index Table A for compound descriptions. The following abbreviations are used in the Index Tables which follow: *t* is tertiary, *i* is iso, *c* is cyclo, Me is methyl, CH₃O or OMe is methoxy, Pr is propyl, *i*-Pr is isopropyl, *c*-Pr is cyclopropyl, *t*-Bu is *tert*-butyl SO₂ means sulfonyl (S(O)₂), and naphthyl means naphthalenyl, CF₃ is trifluoromethyl, CF₃O is trifluoromethoxy, THP means tetrahydropyranyl, Ph means phenyl, NO₂ is nitro, Cl is chloro, F is fluoro and Br is bromo. The abbreviation "Ex." stands for "Example" and is followed by a number indicating in which example the compound is prepared.

**INDEX TABLE A**

| | | | | | | |
|---|---|---|---|---|---|---|
| 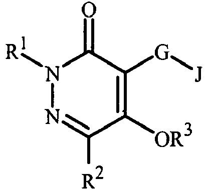 | | | | | | |

| Cmpd. No. | R¹ | R² | R³ | G | J | M.P. (°C) |
|---|---|---|---|---|---|---|
| 1 | Me | H | H | 5-Me-1*H*-pyrazol-1-yl | 3-[4-(CF₃)-Ph] | * |
| (Ex 1) | | | | | | |
| 2 | Me | H | CO-*t*-Bu | 5-Me-1*H*-pyrazol-1-yl | 3-[4-(CF₃)-Ph] | * |
| 3 | Me | H | H | 5-Me-1*H*-pyrazol-1-yl | 3-(4-Cl-Ph) | * |
| (Ex 2) | | | | | | |
| 4 | Me | H | H | 5-Me-1*H*-pyrazol-1-yl | 3-(4-Br-Ph) | * |
| 5 | Me | H | CO-*c*-Pr | 5-Me-1*H*-pyrazol-1-yl | 3-(4-Br-Ph) | * |
| 6 | Me | H | CO₂-*i*-Pr | 5-Me-1*H*-pyrazol-1-yl | 3-(4-Br-Ph) | * |
| 7 | Me | H | CO₂-*i*-Pr | 5-Me-1*H*-pyrazol-1-yl | 3-(4-Cl-Ph) | * |
| (Ex 3) | | | | | | |
| 8 | Me | H | H | 5-Me-1*H*-pyrazol-1-yl | 3-[3-(CF₃)-Ph] | * |
| 9 | Me | H | CO₂-*i*-Pr | 2-Me-1*H*-imidazol-1-yl | 4-(4-Cl-Ph) | * |
| 10 | Me | H | H | 2-Me-1*H*-imidazol-1-yl | 4-(4-Cl-Ph) | * |
| 11 | Me | H | H | 5-Et-1*H*-pyrazol-1-yl | 3-[4-(CF₃)-Ph] | 202-204 |
| 12 | Me | H | CO₂-*i*-Pr | 5-Et-1*H*-pyrazol-1-yl | 3-[4-(CF₃)-Ph] | 179-182 |
| 13 | Me | H | CO₂-*i*-Pr | 5-Me-1*H*-pyrazol-1-yl | 3-[4-(CF₃O)-Ph] | * |
| 14 | Me | H | H | 5-Me-1*H*-pyrazol-1-yl | 3-[4-(CF₃O)-Ph] | * |
| 15 | Me | Me | H | 5-Me-1*H*-pyrazol-1-yl | 3-(4-Cl-Ph) | * |
| 16 | Me | Me | H | 5-Et-1*H*-pyrazol-1-yl | 3-[4-(CF₃)-Ph] | * |
| 17 | Me | H | H | 5-Me-1*H*-triazol-1-yl | 3-(4-Cl-Ph) | * |
| 18 | Me | H | CO-*t*-Bu | 5-Et-1*H*-pyrazol-1-yl | 3-[4-(CF₃)-Ph] | * |
| 19 | Me | H | SO₂Me | 5-Et-1*H*-pyrazol-1-yl | 3-[4-(CF₃)-Ph] | * |
| 20 | Me | H | H | 5-Et-1*H*-pyrazol-1-yl | 3-(3,4-di-Cl-Ph) | * |
| 21 | Me | H | H | 5-Et-1*H*-pyrazol-1-yl | 3-(4-Cl-Ph) | * |
| 22 | Me | H | CO₂-*i*-Pr | 5-Et-1*H*-pyrazol-1-yl | 3-(3,4-di-Cl-Ph) | * |
| 23 | Me | H | H | 5-SMe-1*H*-pyrazol-1-yl | 3-(4-F-Ph) | * |
| 24 | Me | H | H | 4-Me-thiazol-5-yl | 2-[4-(CF₃)-Ph] | 209-213 |
| 25 | Me | H | H | 5-Et-1*H*-pyrazol-1-yl | 3-(4-F-Ph) | * |
| 26 | Me | H | H | 4-Me-5-Et-1*H-*pyrazol-1-yl | 3-(4-Cl-Ph) | 227-229 |
| 27 | Me | i-Pr | H | 5-Me-1*H*-pyrazol-1-yl | 3-[4-(CF₃)-Ph] | * |
| 28 | Me | H | H | 4-Cl-1*H*-pyrazol-3-yl | 1-(4-Cl-Ph) | 235-240 |
| 29 | Me | H | H | 3-Me-1*H*-pyrazol-4-yl | 1-(4-Cl-Ph) | 243-247 |
| 30 | Me | H | H | 4-Me-1*H*-pyrazol-3-yl | 1-(4-Cl-Ph) | 147-151 |
| 31 | Me | H | H | 4-Br-1*H*-pyrazol-3-yl | 1-(4-Cl-Ph) | 189-191 |
| 32 | Me | H | CO-(3-Cl-Ph) | 5-Me-1*H*-pyrazol-1-yl | 3-(4-Cl-Ph) | 170-175 |
| 33 | Me | H | CO₂Me | 5-Me-1*H*-pyrazol-1-yl | 3-(4-Cl-Ph) | 149-151 |
| 34 | Me | H | CO-*i*-Pr | 5-Me-1*H*-pyrazol-1-yl | 3-(4-Cl-Ph) | 107-110 |
| 35 | Me | H | SO₂Ph | 5-Me-1*H*-pyrazol-1-yl | 3-(4-Cl-Ph) | 136-139 |
| 36 | Me | H | SO₂Me | 5-Me-1*H*-pyrazol-1-yl | 3-(4-Cl-Ph) | 122-125 |
| 37 | Me | H | CO-*i*-Bu | 5-Me-1*H*-pyrazol-1-yl | 3-(4-Cl-Ph) | 125-129 |
| 38 | Me | H | CO-(2-Cl-Ph) | 5-Me-1*H*-pyrazol-1-yl | 3-(4-Cl-Ph) | 103-106 |
| 39 | Me | H | SO₂Me | 5-Et-1*H*-pyrazol-1-yl | 3-(4-Cl-Ph) | * |
| 40 | Me | H | CO-*t*-Bu | 5-Et-1*H*-pyrazol-1-yl | 3-(4-Cl-Ph) | * |
| 41 | Me | H | CO₂-*i*-Pr | 5-Et-1*H*-pyrazol-1-yl | 3-(4-Cl-Ph) | * |
| 42 | Me | CF₃ | H | 5-Me-1*H*-pyrazol-1-yl | 3-[4-(CF₃)-Ph] | * |
| 43 | Me | H | H | 5-n-Pr-1*H*-pyrazol-1-yl | 3-(4-Cl-Ph) | * |
| 44 | Me | CF₃ | H | 5-Me-1*H*-pyrazol-1-yl | 3-[3-F-4-(CF₃)-Ph] | * |
| 45 | Me | H | H | 5-c-Pr-1*H*-pyrazol-1-yl | 3-(4-Cl-Ph) | * |
| 46 | Me | H | H | 2-Me-Ph | 5-(4-Cl-Ph) | 235-238 |
| 47 | Me | H | SO₂-1-Naphthyl | 5-Me-1*H*-pyrazol-1-yl | 3-(4-Cl-Ph) | 156-160 |
| 48 | Me | H | CO-Ph | 5-Me-1*H*-pyrazol-1-yl | 3-(4-Cl-Ph) | 165-168 |
| 49 | Me | H | SO₂-*c*-Pr | 5-Me-1*H*-pyrazol-1-yl | 3-(4-Cl-Ph) | 152-155 |
| 50 | Me | H | SO₂-*i*-Pr | 5-Me-1*H*-pyrazol-1-yl | 3-(4-Cl-Ph) | 130-134 |
| 51 | Me | H | SO₂-(2-NO₂-Ph) | 5-Me-1*H*-pyrazol-1-yl | 3-(4-Cl-Ph) | 186-189 |
| 52 | Me | H | CO-2-Thienyl | 5-Me-1*H*-pyrazol-1-yl | 3-(4-Cl-Ph) | 150-153 |
| 53 | Me | H | CO₂Et | 5-Me-1*H*-pyrazol-1-yl | 3-(4-Cl-Ph) | 131-135 |
| 54 | Me | H | CO₂Ph | 5-Me-1*H*-pyrazol-1-yl | 3-(4-Cl-Ph) | 169-173 |
| 55 | Me | H | SO₂-(2-Cl-thien-5-yl) | 5-Me-1*H*-pyrazol-1-yl | 3-(4-Cl-Ph) | 128-131 |
| 56 | Me | H | CO-*n*-Pr | 5-Me-1*H*-pyrazol-1-yl | 3-(4-Cl-Ph) | 109-114 |
| 57 | Me | H | CO₂-*i*-Bu | 5-Me-1*H*-pyrazol-1-yl | 3-(4-Cl-Ph) | 171-174 |
| 58 | Me | H | CO₂CH₂C≡ CH | 5-Me-1*H*-pyrazol-1-yl | 3-(4-Cl-Ph) | 190-192 |
| 59 | Me | Me | H | 5-Cl-1*H*-pyrazol-1-yl | 3-(4-Cl-Ph) | * |
| 60 | Me | H | H | 2-Me-Ph | 4-(4-Cl-1*H*-pyrazol-1-yl) | * |
| 61 | Me | H | H | 5-Et-1*H*-pyrazol-1-yl | 3-(4-Br-Ph) | * |
| 62 | Me | H | H | 5-Me-1*H*-pyrazol-1-yl | 3-[4-(CH₃O)-Ph] | * |
| 63 | Me | H | H | 5-Et-1*H*-pyrazol-1-yl | 3-(2-Cl-thien-5-yl) | * |
| 64 | Me | H | CONMe₂ | 5-Me-1*H*-pyrazol-1-yl | 3-(4-Cl-Ph) | 191-194 |
| 65 | Et | H | H | 3-Me-1*H*-pyrazol-4-yl | 1-(4-Cl-Ph) | 160-165 |
| 66 | Me | OMe | H | 5-Et-1*H*-pyrazol-1-yl | 3-[4-(CF₃)-Ph] | * |
| 67 | Et | H | H | 5-Et-1*H*-pyrazol-1-yl | 3-[4-(CF₃)-Ph] | * |
| 68 | Me | H | H | 5-CH₂OMe-1*H-*pyrazol-1-yl | 3-(4-Br-Ph) | 117-120 |
| 69 | Me | H | CSNMe₂ | 5-Me-1*H*-pyrazol-1-yl | 3-(4-Cl-Ph) | 119-123 |
| 70 | Me | H | H | 4-Br-5-Me-1H pyrazol-1-yl | 3-(4-Br-Ph) | * |
| 71 | Me | H | H | 4-Br-1*H*-pyrazol-3-yl | 1-[5-(CF₃)-pyridin-2-yl] | * |
| 72 | Me | H | H | 5-*n*-Pr-1*H*-pyrazol-1-yl | 3-[4-(CF₃)-Ph] | * |
| 73 | CHCF₃ | H | H | 5-Et-1*H*-pyrazol-1-yl | 3-(4-Br-Ph) | * |
| 74 | Me | Me | H | 5-Et-1*H*-pyrazol-1-yl | 3-(4-Br-Ph) | 203-205 |
| 75 | Me | Me | H | 5-Me-1*H*-pyrazol-1-yl | 3-[4-(CF₃)-Ph] | 191-194 |
| 76 | Me | H | H | 5-*n*-Pr-1*H*-pyrazol-1-yl | 3-(4-Cl-Ph) | 194-198 |
| 77 | 2-THP | H | H | 5-Me-1*H*-pyrazol-1-yl | 3-(4-Br-Ph) | * |
| (Ex 9) | | | | | | |
| 78 | H | H | H | 5-Me-1*H*-pyrazol-1-yl | 3-(4-Br-Ph) | 325-335 |
| (Ex 10) | | | | | | |
| 79 | Me | Me | H | 5-Me-thiazol-4-yl | 2-(4-Cl-Ph) | * |
| (Ex 4) | | | | | | |
| 80 | CO-c- | H | CO-c-Pr | 5-Me-1*H*-pyrazol-1-yl | 3-(4-Br-Ph) | ** |
| (Ex 11) | Pr | | | | | |
| 81 | Me | Me | H | 5-Me-1*H*-pyrazol-1-yl | 3-(4-Br-Ph) | 200-204 |
| 82 | Me | OMe | H | 5-Me-1*H*-pyrazol-1-yl | 3-(4-Br-Ph) | 222-225 |
| 83 | Me | H | H | 4-Me-5-Et-1*H-*pyrazol-1-yl | 3-(4-Br-Ph) | * |
| 84 | Me | H | H | 4-Me-1*H*-pyrazol-3-yl | 1-(5-Cl-pyridin-2-yl) | 238-241 |
| 85 | Me | H | H | 4-Me-1H-pyrazol-3-yl | 1-[4-(CF₃)-Ph] | 150-154 |
| (Ex 7) | | | | | | |
| 86 | Me | Me | H | 5-Et-1*H*-pyrazol-1-yl | 3-(4-Cl-Ph) | * |
| 87 | Me | H | H | 4-Br-1*H*-pyrazol-1-yl | 3-[4-(CF₃)-Ph] | 237-241 |
| (Ex 6) | | | | | | |
| 88 | Me | H | H | 4-Cl-1*H*-pyrazol-3-yl | 1-[4-(CF₃)-Ph] | 176-179 |
| 89 | Me | OMe | H | 5-Et-1*H*-pyrazol-1-yl | 3-(4-Br-Ph) | 214-216 |
| 90 | Me | H | H | 5-Me-Pyrimdin-4-yl | 3-[4-(CF₃)-Ph] | 142-145 |
| 91 | Me | H | H | 5-*n*-Bu-1*H*-pyrazol-1-yl | 3-(4-Cl-Ph) | * |
| 92 | Me | H | H | 5-*i*-Bu-1*H*-pyrazol-1-yl | 3-(4-Cl-Ph) | * |
| 93 | Me | OMe | H | 5-Me-1*H*-pyrazol-1-yl | 3-(4-Br-Ph) | 208-211 |
| 94 | Me | H | CO-(6-Cl-pyridin-3-yl) | 5-Et-1*H*-pyrazol-1-yl | 3-[4-(CF₃)-Ph] | * |
| 95 | Me | H | CO-*c*-hexyl | 5-Et-1*H*-pyrazol-1-yl | 3-[4-(CF₃)-Ph] | * |
| 96 | Me | H | SO₂N(Me)₂ | 5-Et-1*H*-pyrazol-1-yl | 3-[4-(CF₃)-Ph] | * |
| 97 | Me | H | CO-4-Morpholinyl | 5-Et-1*H*-pyrazol-1-yl | 3-[4-(CF₃)-Ph] | * |

| | | | | | | |
|---|---|---|---|---|---|---|
| * See Index Table B for ¹H NMR or mass spectra data. ** See synthesis example for ¹H NMR data. Cmpd. No. means Compound Number. | | | | | | |

**INDEX TABLE B**

| Cmpd. No. | ¹H NMR Data (CDCl₃ solution unless indicated otherwise)^{a} or mass spectra data^{b} |
|---|---|
| 1 | δ 7.89 (s, 3H), 7.86 (m, 2H), 7.68 (m, 2H), 6.60 (s, 1H), 3.81 (s, 3H), 2.35 (s, 3H) |
| 2 | δ 7.87 (s, 1H), 7.82 (m, 2H), 7.62 (m, 2H), 6.54 (s, 1H), 3.89 (s, 3H), 1.09 (s, 9H) |
| 3 | δ 7.77 (s, 1H), 7.66 (m, 2H), 7.40 (m, 2H), 6.46 (s, 1H), 3.80 (s, 3H), 2.27 (s, 3H) |
| 4 | δ 7.77 (s, 1H), 7.55 (m, 4H), 6.45 (s, 1H), 3.80 (s, 3H), 2.26 (s, 3H) |
| 5 | δ 7.84 (s, 1H), 7.67 (m, 2H), 7.52 (m, 2H), 6.46 (s, 1H), 3.87 (s, 3H), 2.30 (s, 3H), 1.65 (m, 1H), 0.92 (m, 2H), 0.86 (m, 2H) |
| 6 | δ 7.89 (s, 1H), 7.64 (m, 2H), 7.52 (m, 2H), 6.48 (s, 1H), 4.73 (m, 1H), 3.88 (s, 3H), 2.33 (s, 3H), 1.12 (m, 6H) |
| 7 | δ 7.89 (s, 1H), 7.69 (m, 2H), 7.35 (m, 2H), 6.45 (s, 1H), 4.75 (m, 1H), 3.88 (s, 3H), 2.33 (s, 3H), 1.12 (m, 6H) |
| 8 | δ 7.92 (s, 1H), 7.79 (s, 1H), 7.61 (m, 1H), 7.55 (m, 1H), 6.54 (s, 1H), 3.81 (s, 3H), 2.30 (s, 3H) |
| 9 | δ 7.93 (s, 1H), 7.68 (d, 2H), 7.32 (d, 2H), 7.26 (s, 1H), 4.64 - 4.94 (m, 1H), 3.90 (s, 3H), 2.36 (s, 3H), 1.08-1.34 (m, 6H) |
| 10 | DMSO-*d*₆ δ 7.76 (d, 2H), 7.68 (s, 1H), 7.62 (s, 1H), 7.46 (d, 2H), 3.58 (s, 3H), 2.23 (s, 3H) |
| 13 | δ 7.89 (s, 1H), 7.77 (m, 2H), 7.18 (m, 2H), 6.50 (s, 1H), 4.71 (m, 1H), 3.88 (s, 3H), 2.34 (s, 3H), 1.10 (m, 6H) |
| 14 | δ 7.77 (s, 1H), 7.73 (m, 2H), 7.26 (m, 2H), 6.42 (s, 1H), 3.79 (s, 3H), 2.18 (s, 3H) |
| 15 | δ 7.67 (d, 2H), 7.39 (d, 2H), 6.49 (s, 1H), 3.78 (s, 3H), 2.36 (s, 6H) |
| 16 | δ 7.89 (d, 2H), 7.67 (d, 2H), 6.62 (s, 1H), 3.78 (s, 3H), 2.80 (d, 2H), 2.37 (s, 3H), 1.28 (t, 3H) |
| 17 | δ 7.89 (d, 2H), 7.81 (s, 1H), 7.37 (d, 2H), 3.80 (s, 3H), 2.52 (s, 3H) |
| 18 | δ 7.89 (s, 1H), 7.84 (m, 2H), 7.62 (m, 2H), 6.56 (s, 1H), 3.88 (s, 3H), 2.70 (m, 1H), 2.49 (m, 1H), 1.30 (m, 3H), 1.09 (s, 9H) |
| 19 | δ 8.09 (s, 1H), 7.90 (m, 2H), 7.65 (m, 2H), 6.62 (s, 1H), 3.90 (s, 3H), 3.04 (s, 3H), 2.70 (br, 1H), 2.50 (br, 1H), 1.32 (m, 3H) |
| 20 | δ 7.85 (s, 1H), 7.79 (s, 1H), 7.59 (m, 1H), 7.50 (m, 1H), 6.52 (s, 1H), 3.82 (s, 3H), 2.73 (m, 2H), 1.25 (m, 3H) |
| 21 | δ 7.89 (s, 1H), 7.69 (m, 2H), 7.39 (m, 2H), 6.50 (s, 1H), 3.81 (s, 3H), 2.69 (m, 1H), 1.22 (m, 3H) |
| 22 | δ 7.91 (s, 1H), 7.88 (s, 1H), 7.61 (m, 1H), 7.45 (m, 1H), 6.50 (s, 1H), 4.78 (m, 1H) 3.87 (s, 3H), 2.64 (br, 2H), 1.25 (m, 3H), 1.16 (br, 6H) |
| 23 | δ 7.79 (s, 1H), 7.73 (m, 2H), 7.13 (m, 2H), 6.57 (s, 1H), 3.83 (s, 3H), 2.47 (s, 3H) |
| 24 | Acetone-*d*₆ δ 8.19 (m, 2H), 7.84 (m, 2H), 7.81 (s, 1H), 3.69 (s, 3H), 2.35 (s, 3H) |
| 25 | δ 7.74 (s, 1H), 7.71 (m, 2H), 7.11 (m, 2H), 6.45 (s, 1H), 3.79 (s, 3H), 2.63 (m, 2H), 1.20 (m, 3H) |
| 27 | δ 7.78 (d, 2H), 7.66 (d, 2H), 6.36 (s, 1H), 3.75 (s, 3H), 3.23 (m, 1H), 2.07 (s, 3H), 1.24 (d, 6H) |
| 32 | δ 8.01 (s, 1H), 7.92 (m, 1H), 7.79 (m, 1H), 7.45 (m, 3H), 7.22 (m, 3H), 6.40 (s, 1H), 3.92 (s, 3H), 2.36 (m, 2H) |
| 33 | δ 7.91 (s, 1H), 7.73 (m, 2H), 7.34 (m, 2H), 6.46 (s, 1H), 3.88 (m, 3H), 3.71 (m, 3H), 2.33 (s, 3H) |
| 39 | δ 8.07 (s, 1H), 7.70 (m, 2H), 7.37 (m, 2H), 6.56 (s, 1H), 3.88 (s, 3H), 3.02 (s, 3H), 2.67 (br, 1H), 2.48 (br, 1H), 1.30 (m, 3H) |
| 40 | δ 7.82 (s, 1H), 7.71 (m, 2H), 7.34 (m, 2H), 6.48 (s, 1H), 3.87 (s, 3H), 2.73 m, 1H), 2.46 (m, 1H), 1.29 (m, 3H), 1.09 (s, 9H) |
| 41 | δ 7.90 (s, 1H), 7.71 (m, 2H), 7.34 (m, 2H), 6.49 (s, 1H), 4.74 (m, 2H), 3.87 (s, 3H), 2.64 (br, 2H), 1.30 (m, 3H), 1.14 (m, 6H) |
| 42 | δ 7.84 (d, 2H) 7.69 (d, 2H) 6.60 (s, 1H) 3.86 (s, 3H) 2.38 (s, 3H) |
| 43 | δ 7.75 (s, 1H), 7.67 (m, 2H), 7.39 (m, 2H),), 6.47 (s, 1H), 3.79 (s, 3H), 2.59 (m, 2H), 1.60 (m, 2H), 0.95 (m, 3H) |
| 44 | δ 7.79 (s, 1H), 7.62 (m, 3H), 6.56 (s, 1H), 3.82 (s, 3H), 2.70 (m, 2H), 1.24 (m, 3H) |
| 45 | δ 7.74 (s, 1H), 7.62 (m, 2H), 7.37 (m, 2H), 6.19 (s, 1H), 3.79 (s, 3H), 1.97 (m, 1H), 0.91 (m, 2H), 0.59 (m, 2H) |
| 59 | δ 7.60 (m, 2H), 7.39 (m, 2H), 6.56 (s, 1H), 3.75 (s, 3H), 2.32 (s, 3H) |
| 60 | δ 9.95 (br, 1H), 7.82 (s, 1H), 7.58 (s, 1H), 7.49 (s, 1H), 7.39 (d, 1H), 7.26 (m, 1H), 7.04 (d, 1H), 3.65 (s, 3H), 2.04 (s, 3H) |
| 61 | δ 7.75 (s, 1H), 7.60 (m, 2H), 7.55 (m, 2H), 6.49 (s, 1H), 3.80 (s, 3H), 2.67 (m, 2H), 1.22 (m, 3H) |
| 62 | δ 7.71 (s, 1H), 7.63 (m, 2H), 6.95 (m, 2H), 6.39 (s, 1H), 3.84 (s, 3H), 3.79 (s, 3H), 2.20 (s, 3H) |
| 63 | δ 7.75 (s, 1H), 7.11 (m, 2H), 6.88 (m, 2H), 6.36 (s, 1H), 3.79 (s, 3H), 2.66 (m, 2H), 1.22 (m, 3H) |
| 66 | DMSO-*d*₆. δ 7.96 (m, 2H), 7.71 (m, 2H), 6.62 (s, 1H), 3.71 (s, 3H), 3.31 (s, 3H), 2.34 (m, 2H), 1.13 (m, 3H) |
| 67 | δ 7.87 (m, 2H), 7.82 (s, 1H), 7.66 (m, 2H), 6.61 (s, 1H), 4.24 (m, 2H), 2.76 (m, 2H), 1.39 (m, 3H), 1.27 (m, 3H) |
| 70 | δ 7.78 (m, 2H), 7.75 (s, 1H), 7.59 (m, 2H), 3.82 (s, 3H), 2.31 (s, 3H) |
| 71 | δ 8.82 (s, 1H), 8.74 (s, 1H), 8.11 (m, 1H), 7.97 (m, 1H), 7.77 (s, 1H), 3.83 (s, 3H) |
| 72 | Acetone-*d*₆ δ 8.06 (m, 2H), 7.88 (s, 1H), 7.75 (m, 2H), 6.79 (s, 1H), 3.72 (s, 3H), 2.56 (m, 2H), 1.68 (m, 2H), 0.96 (m, 3H) |
| 73 | δ 7.82 (s, 1H), 7.60 (m, 2H), 7.57 (m, 2H), 6.51 (s, 1H), 4.79 (m, 2H), 2.64 (m, 2H), 1.22 (m, 3H) |
| 77 | Mass Spec: AP+ = 432 |
| 83 | δ 7.70 (s, 1H), 7.57 (m, 2H), 7.48 (m, 2H), 3.78 (s, 3H), 2.69 (s, 3H), 2.16 (s, 3H), 1.05 (m, 3H) |
| 86 | δ 7.72 (m, 2H), 7.39 (m, 2H), 6.55 (s, 1H), 3.78 (s, 3H), 2.80 (m, 2H), 2.36 (s, 3H), 1.28 (m, 3H) |
| 91 | δ 7.74 - 7.80 (m, 1H), 7.68 (d, 2H), 7.39 (d, 2H), 6.51 (s, 1H), 3.80 (s, 3H), 2.66 (q, 2H), 1.52 - 1.63 (m, 2H), 1.30 - 1.43 (m, 2H), 0.92 (t, 3H) |
| 92 | DMSO-*d*₆ δ 7.88 (s, 1H), 7.81 (d, 2H), 7.41 - 7.50 (m, 2H), 6.66 - 6.76 (m, 1H), 3.65 (s, 3H), 2.29 (dd, 2H), 1.76 - 1.93 (m, 1H), 0.87 (d, 6H) |
| 94 | 8.84 (s, 1H), 8.03 (d, 1H), 8.03 (s, 1H), 7.62 (d, 2H), 7.53 (d, 2H), 7.25 (dd, 1H), 6.51 (s, 1H), 3.93 (s, 3H), 2.70 (m, 2H), 1.33 (t, 3H) |
| 95 | 7.90 (d, 2H) 7.83 (s, 1H), 7.62 (d, 2H), 6.55 (s, 1H), 3.87 (s, 3H), 2.80 - 2.50 (m, 2H), 2.35 (m,1H), 1.68 (m, 2H), 1.55 (m, 4H), 1.35 (t, 3H), 1.33 (m, 2H), 1.1 (m, 2H) |
| 96 | 8.18 (s, 1H), 7.90 (d, 2H), 7.59 (d, 2H), 6.60 (s, 1H), 3.89 (s, 3H), 2.72 (s, 6H), 2.71 - 2.50 (m, 2H), 1.30 (t, 3H) |
| 97 | 8.02 (s, 1H), 7.9 (d, 2H), 7.76 (d, 2H), 6.58 (s, 1H), 3.88 (s, 3H), 3.33 (m, 8H), 2.80 - 2.50 (m, 2H), 1.33 (t, 3H) |

| | |
|---|---|
| Cmpd. No. means Compound number. ^{a 1}H NMR data are in ppm downfield from tetramethylsilane. Couplings are designated by (s)-singlet, (m)-multiplet, (d)-doublet, (q)-quartet, (dd) doublet of doublets, (br)-broad and (t)-triplet. ^{b}Mass spectra are reported as the molecular weight of the highest isotopic abundance parent ion (M+1) formed by addition of H⁺ (molecular weight of 1) to the molecule, observed by mass spectrometry using atmospheric pressure chemical ionization (AP⁺). | |

### BIOLOGICAL EXAMPLES OF THE INVENTION

### TEST A

Seeds of species selected from barnyardgrass (*Echinochloa crus-galli*), large crabgrass (*Digitaria sanguinalis*), giant foxtail (*Setaria faberi*), blackgrass (*Alopecurus myosuroides*), canarygrass (*Phalaris minor*), morningglory (*Ipomoea coccinea*), pigweed (*Amaranthus retroflexus*), velvetleaf (*Abutilon theophrasti*), wheat *(Triticum aestivum*), and corn (*Zea mays*) were planted into a blend of loam soil and sand and treated preemergence with a directed soil spray using test chemicals formulated in a non-phytotoxic solvent mixture which included a surfactant. At the same time these species were also treated with postemergence applications of test chemicals formulated in the same manner.

Plants ranged in height from 2 to 10 cm and were in the one- to two-leaf stage for the postemergence treatment. Treated plants and untreated controls were maintained in a greenhouse for approximately 10 days, after which time all treated plants were compared to untreated controls and visually evaluated for injury. Plant response ratings, summarized in Table A, are based on a 0 to 100 scale where 0 is no effect and 100 is complete control. A dash (-) response means no test results.

| Table A | Compounds | | | | | Table A | | | | | Compounds | | | |
|---|---|---|---|---|---|---|---|---|---|---|---|---|---|---|
| 1000 g ai/ha | 1 | 2 | | | | 1000 g ai/ha | | | | | 1 | | 2 | |
| Postemergence | | | | | | Postemergence | | | | | | | | |
| Barnyardgrass | 100 | 100 | | | | Morningglory | | | | | 60 | | 0 | |
| Corn | 100 | 90 | | | | Pigweed | | | | | 30 | | 0 | |
| Crabgrass, Large | 100 | 80 | | | | Velvetleaf | | | | | 60 | | 40 | |
| Foxtail, Giant | 100 | 90 | | | | Wheat | | | | | 100 | | 90 | |

| Table A | Compounds | | | | | | | | | | | | | |
|---|---|---|---|---|---|---|---|---|---|---|---|---|---|---|
| 500 g ai/ha | 3 | 4 | 5 | 6 | 7 | 8 | 9 | 10 | 11 | 12 | 13 | 14 | 15 | 16 |
| Postemergence | | | | | | | | | | | | | | |
| Barnyardgrass | 100 | 100 | 100 | 100 | 100 | 0 | 0 | 0 | 100 | 100 | 90 | 100 | 100 | 100 |
| Corn | 100 | 100 | 100 | 100 | 100 | 0 | 0 | 0 | 90 | 90 | 60 | 80 | 90 | 100 |
| Crabgrass, Large | 100 | 100 | 100 | 90 | 90 | 0 | 0 | 0 | 100 | 100 | 80 | 90 | 100 | 100 |
| Foxtail, Giant | 100 | 100 | 100 | 90 | 90 | 0 | 0 | 0 | 100 | 100 | 80 | 100 | 100 | 100 |
| Morningglory | 60 | 20 | 0 | 50 | 20 | 50 | 0 | 0 | 0 | 0 | 0 | 0 | 0 | 0 |
| Pigweed | 10 | 20 | 0 | 30 | 0 | 0 | 0 | 0 | 0 | 0 | 0 | 0 | 0 | 0 |
| Velvetleaf | 50 | 60 | 50 | 50 | 40 | 40 | 0 | 0 | 0 | 0 | 0 | 30 | 0 | 0 |
| Wheat | 90 | 80 | 70 | 70 | 60 | 0 | 0 | 0 | 100 | 90 | 60 | 70 | 60 | 90 |

| 500 g ai/ha | 17 | 19 | 20 | 21 | 22 | 23 | 24 | 26 | 27 | 28 | 29 | 30 | 31 | 42 |
|---|---|---|---|---|---|---|---|---|---|---|---|---|---|---|
| Postemergence | | | | | | | | | | | | | | |
| Barnyardgrass | 90 | 100 | 100 | 100 | 90 | 70 | 10 | 100 | 90 | 100 | 50 | 90 | 90 | 0 |
| Corn | 40 | 90 | 90 | 90 | 90 | 20 | 20 | 100 | 80 | 90 | 0 | 70 | 70 | 20 |
| Crabgrass, Large | 80 | 100 | 100 | 100 | 80 | 20 | 10 | 100 | 80 | 90 | 50 | 90 | 90 | 0 |
| Foxtail, Giant | 90 | 100 | 100 | 100 | 80 | 80 | 40 | 100 | 90 | 90 | 80 | 90 | 90 | 10 |
| Morningglory | 0 | 0 | 0 | 0 | 0 | 0 | 0 | 20 | 0 | 30 | 0 | 0 | 0 | 0 |
| Pigweed | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 20 | 0 | 0 | 0 | 0 | 0 | 0 |
| Wheat | 50 | 90 | 70 | 80 | 60 | 0 | 20 | 60 | 90 | 70 | 20 | 50 | 60 | 10 |

| Table A | Compounds | | | | | Table A | | | | Compound | | | | |
|---|---|---|---|---|---|---|---|---|---|---|---|---|---|---|
| 500 g ai/ha | 60 | 62 | 65 | 77 | | 250 g ai/ha | | | | 18 | | | | |
| Postemergence | | | | | | Postemergence | | | | | | | | |
| Barnyardgrass | 90 | 100 | 0 | 100 | | Barnyardgrass | | | | 100 | | | | |
| Blackgrass | - | - | - | 100 | | Corn | | | | 90 | | | | |
| Canarygrass | - | - | - | 100 | | Crabgrass, Large | | | | 100 | | | | |
| Corn | 90 | 40 | 0 | 100 | | Foxtail, Giant | | | | 100 | | | | |
| Crabgrass, Large | 60 | 90 | 20 | 100 | | Morningglory | | | | 0 | | | | |
| Foxtail, Giant | 80 | 60 | 20 | 100 | | Pigweed | | | | 0 | | | | |
| Morningglory | 0 | 0 | 0 | - | | Velvetleaf | | | | 0 | | | | |
| Pigweed | 0 | 0 | 0 | 0 | | Wheat | | | | 80 | | | | |
| Velvetleaf | 0 | 0 | 0 | - | | | | | | | | | | |
| Wheat | 50 | 20 | 0 | 90 | | | | | | | | | | |

| Table A | Compounds | | | | | | | | | | | | | |
|---|---|---|---|---|---|---|---|---|---|---|---|---|---|---|
| 125 g ai/ha | 3 | 4 | 5 | 6 | 7 | 8 | 9 | 10 | 11 | 12 | 13 | 14 | 15 | 16 |
| Postemergence | | | | | | | | | | | | | | |
| Barnyardgrass | 100 | 100 | 100 | 90 | 80 | 0 | 0 | 0 | 100 | 90 | 50 | 70 | 100 | 90 |
| Corn | 90 | 90 | 100 | 100 | 50 | 0 | 0 | 0 | 90 | 90 | 30 | 40 | 70 | 90 |
| Crabgrass, Large | 100 | 100 | 100 | 90 | 80 | 0 | 0 | 0 | 90 | 90 | 20 | 60 | 90 | 90 |
| Foxtail, Giant | 100 | 100 | 100 | 90 | 90 | 0 | 0 | 0 | 90 | 80 | 70 | 80 | 90 | 100 |
| Morningglory | 50 | 0 | 0 | 30 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 |
| Velvetleaf | 0 | 20 | 20 | 40 | 20 | 20 | 0 | 0 | 0 | 0 | 0 | 0 | 0 | 0 |
| Wheat | 70 | 60 | 60 | 50 | 40 | 0 | 0 | 0 | 80 | 70 | 50 | 40 | 50 | 70 |

| Table A | Compounds | | | | | | | | | | | | | |
|---|---|---|---|---|---|---|---|---|---|---|---|---|---|---|
| 125 g ai/ha | 17 | 19 | 20 | 21 | 22 | 23 | 24 | 25 | 26 | 27 | 28 | 29 | 30 | 31 |
| Postemergence | | | | | | | | | | | | | | |
| Barnyardgrass | 50 | 100 | 80 | 100 | 50 | 0 | 0 | 100 | 100 | 20 | 60 | 10 | 50 | 60 |
| Corn | 0 | 90 | 70 | 80 | 20 | 0 | 0 | 80 | 60 | 50 | 40 | 0 | 0 | 10 |
| Crabgrass, Large | 40 | 90 | 80 | 90 | 10 | 0 | 0 | 90 | 80 | 60 | 80 | 0 | 60 | 60 |
| Foxtail, Giant | 80 | 100 | 90 | 100 | 60 | 20 | 0 | 100 | 90 | 50 | 90 | 0 | 80 | 90 |
| Morningglory | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 0 | 60 | 50 | 60 | 20 | 0 | 0 | 20 | 0 | 50 | 50 | 0 | 40 | 40 |

| Table A | Compounds | | | | | | | | | | | | | |
|---|---|---|---|---|---|---|---|---|---|---|---|---|---|---|
| 125 g ai/ha | 32 | 33 | 34 | 35 | 36 | 37 | 38 | 39 | 40 | 41 | 42 | 43 | 44 | 45 |
| Postemergence | | | | | | | | | | | | | | |
| Barnyardgrass | 70 | 100 | 100 | 90 | 100 | 100 | 100 | 100 | 100 | 100 | 0 | 100 | 100 | 100 |
| Corn | 50 | 90 | 90 | 60 | 80 | 100 | 90 | 100 | 100 | 90 | 20 | 80 | 90 | 80 |
| Crabgrass, Large | 70 | 100 | 100 | 90 | 90 | 90 | 100 | 100 | 100 | 90 | 0 | 90 | 100 | 100 |
| Foxtail, Giant | 90 | 100 | 100 | 90 | 90 | 100 | 90 | 100 | 100 | 90 | 0 | 100 | 100 | 100 |
| Morningglory | 0 | 0 | 0 | 0 | 0 | 0 | 0 | 0 | 0 | 0 | 0 | 0 | 0 | 0 |
| Pigweed | 10 | 0 | 10 | 0 | 10 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 20 | 70 | 60 | 60 | 60 | 70 | 60 | 80 | 90 | 70 | 0 | 80 | 80 | 90 |

| Table A | Compounds | | | | | | | | | | | | | |
|---|---|---|---|---|---|---|---|---|---|---|---|---|---|---|
| 125 g ai/ha | 46 | 47 | 48 | 49 | 50 | 51 | 52 | 53 | 54 | 55 | 56 | 57 | 58 | 59 |
| Postemergence | | | | | | | | | | | | | | |
| Barnyardgrass | 50 | 40 | 90 | 0 | 70 | 80 | 100 | 100 | 100 | 30 | 100 | 80 | 80 | 100 |
| Corn | 50 | 10 | 60 | 0 | 30 | 60 | 100 | 90 | 90 | 40 | 100 | 60 | 60 | 70 |
| Crabgrass, Large | 40 | 50 | 80 | 20 | 40 | 70 | 90 | 100 | 90 | 60 | 100 | 80 | 70 | 90 |
| Foxtail, Giant | 60 | 60 | 80 | 20 | 60 | 70 | 90 | 100 | 100 | 70 | 100 | 90 | 90 | 90 |
| Morningglory | 0 | 0 | 0 | 0 | 0 | 0 | 0 | 20 | 0 | 0 | 0 | 0 | 0 | 0 |
| Pigweed | 0 | 0 | 30 | 0 | 0 | 0 | 0 | 0 | 0 | 0 | 0 | 0 | 0 | 0 |
| Velvetleaf | 0 | 0 | 70 | 0 | 0 | 0 | 20 | 0 | 0 | 0 | 0 | 0 | 0 | 0 |
| Wheat | 30 | 10 | 60 | 0 | 20 | 40 | 60 | 70 | 60 | 30 | 80 | 50 | 60 | 60 |

| Table A | Compounds | | | | | | | | | | | | | |
|---|---|---|---|---|---|---|---|---|---|---|---|---|---|---|
| 125 g ai/ha | 60 | 61 | 62 | 63 | 64 | 65 | 66 | 67 | 68 | 69 | 70 | 71 | 72 | 73 |
| Postemergence | | | | | | | | | | | | | | |
| Barnyardgrass | 30 | 100 | 40 | 20 | 10 | 0 | 100 | 100 | 100 | 100 | 50 | 90 | 100 | 100 |
| Blackgrass | - | - | - | - | - | - | - | - | 80 | 60 | 10 | - | 100 | 90 |
| Canarygrass | - | - | - | - | - | - | - | - | 40 | 80 | 0 | - | 100 | 100 |
| Corn | 40 | 100 | 0 | 30 | 60 | 0 | 100 | 100 | 50 | 90 | 20 | 40 | 100 | 100 |
| Crabgrass, Large | 20 | 100 | 50 | 10 | 40 | 0 | 100 | 100 | 10 | 80 | 0 | 80 | 90 | 100 |
| Foxtail, Giant | 50 | 100 | 40 | 60 | 80 | 0 | 100 | 100 | 90 | 100 | 0 | 90 | 100 | 100 |
| Morningglory | 0 | 0 | 0 | 0 | 0 | 0 | 0 | 0 | - | - | - | 0 | - | - |
| Pigweed | 0 | 0 | 0 | 0 | 0 | 0 | 0 | 0 | 0 | 0 | 0 | 0 | 0 | 10 |
| Velvetleaf | 0 | 0 | 0 | 0 | 0 | 0 | 0 | 0 | - | - | - | 0 | - | - |
| Wheat | 0 | 90 | 0 | 20 | 0 | 0 | 100 | 100 | 0 | 30 | 0 | 80 | 90 | 70 |

| Table A | Compounds | | | | | | | | | | | | | |
|---|---|---|---|---|---|---|---|---|---|---|---|---|---|---|
| 125 g ai/ha | 74 | 75 | 76 | 77 | 78 | 79 | 80 | 81 | 82 | 83 | 84 | 85 | 86 | 87 |
| Postemergence | | | | | | | | | | | | | | |
| Barnyardgrass | 100 | 90 | 100 | 50 | 100 | 50 | 100 | 100 | 100 | 100 | 0 | 30 | 100 | 90 |
| Blackgrass | 100 | 90 | 100 | 100 | 100 | 100 | 100 | 100 | 100 | 100 | 0 | 40 | 100 | 80 |
| Canarygrass | 100 | 80 | 100 | 60 | 70 | 90 | 100 | 100 | 100 | 100 | 0 | 20 | 100 | 70 |
| Corn | 100 | 90 | 100 | 80 | 100 | 30 | 100 | 100 | 100 | 100 | 0 | 0 | 90 | 40 |
| Crabgrass, Large | 100 | 80 | 100 | 70 | 100 | 50 | 100 | 100 | 100 | 100 | 10 | 40 | 100 | 80 |
| Foxtail, Giant | 100 | 80 | 90 | 80 | 100 | 70 | 100 | 100 | 100 | 100 | 10 | 60 | 90 | 80 |
| Pigweed | 0 | 0 | 0 | 0 | 0 | 0 | 0 | 0 | 0 | 0 | 0 | 0 | 0 | 0 |
| Wheat | 80 | 70 | 50 | 70 | 90 | 50 | 70 | 70 | 100 | 60 | 0 | 20 | 50 | 50 |

| Table A | Compounds | | | | | | | Table A | | | | Compound | | |
|---|---|---|---|---|---|---|---|---|---|---|---|---|---|---|
| 125 g ai/ha | 88 | 89 | 90 | 92 | 93 | 94 | 95 | 62 g ai/ha | | | | | 18 | |
| Postemergence | | | | | | | | Postemergence | | | | | | |
| Barnyardgrass | 90 | 100 | 0 | 80 | 100 | 100 | 100 | Barnyardgrass | | | | | 90 | |
| Blackgrass | 70 | 100 | 0 | 50 | 100 | 100 | 100 | Corn | | | | | 80 | |
| Canarygrass | 70 | 100 | 0 | 30 | 100 | 100 | 100 | Crabgrass, Large | | | | | 80 | |
| Corn | 50 | 100 | 0 | 20 | 100 | 100 | 100 | Foxtail, Giant | | | | | 90 | |
| Crabgrass, Large | 90 | 100 | 0 | 10 | 100 | 100 | 100 | Morningglory | | | | | 0 | |
| Foxtail, Giant | 90 | 100 | 0 | 60 | 100 | 100 | 100 | Pigweed | | | | | 0 | |
| Pigweed | 0 | 0 | 0 | 0 | 0 | 0 | 0 | Velvetleaf | | | | | 0 | |
| Wheat | 60 | 90 | 0 | 30 | 80 | 80 | 80 | Wheat | | | | | 70 | |

| Table A | Compounds | | | | | | | | | | | | | |
|---|---|---|---|---|---|---|---|---|---|---|---|---|---|---|
| 31 g ai/ha | 25 | 32 | 33 | 34 | 35 | 36 | 37 | 38 | 39 | 40 | 41 | 43 | 44 | 45 |
| Postemergence | | | | | | | | | | | | | | |
| Barnyardgrass | 80 | 0 | 90 | 90 | 60 | 80 | 90 | 40 | 100 | 100 | 80 | 90 | 100 | 80 |
| Corn | 20 | 0 | 60 | 70 | 0 | 50 | 60 | 20 | 70 | 90 | 60 | 50 | 70 | 40 |
| Crabgrass, Large | 60 | 10 | 90 | 70 | 40 | 80 | 50 | 40 | 60 | 70 | 20 | 40 | 50 | 40 |
| Foxtail, Giant | 90 | 10 | 90 | 90 | 60 | 90 | 80 | 80 | 90 | 100 | 70 | 80 | 90 | 90 |
| Morningglory | 0 | 0 | 0 | 0 | 0 | - | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 0 | 0 | 20 | 40 | 0 | 0 | 20 | 0 | 40 | 70 | 60 | 40 | 70 | 70 |

| Table A | Compounds | | | | | | | | | | | | | |
|---|---|---|---|---|---|---|---|---|---|---|---|---|---|---|
| 31 g ai/ha | 46 | 47 | 48 | 49 | 50 | 51 | 52 | 53 | 54 | 55 | 56 | 57 | 58 | 59 |
| Postemergence | | | | | | | | | | | | | | |
| Barnyardgrass | 0 | 0 | 20 | 0 | 0 | 20 | 80 | 90 | 80 | 0 | 100 | 0 | 0 | 90 |
| Corn | 20 | 0 | 20 | 0 | 0 | 20 | 30 | 80 | 40 | 0 | 90 | 0 | 0 | 30 |
| Crabgrass, Large | 0 | 10 | 20 | 0 | 0 | 20 | 60 | 80 | 70 | 10 | 80 | 0 | 0 | 50 |
| Foxtail, Giant | 20 | 10 | 30 | 0 | 0 | 20 | 80 | 90 | 90 | 20 | 100 | 0 | 20 | 90 |
| Morningglory | 0 | 0 | 0 | 0 | 0 | 0 | 0 | 0 | 0 | 0 | 0 | 0 | 0 | 0 |
| Pigweed | 0 | 0 | 20 | 0 | 0 | 0 | 0 | 0 | 0 | 0 | 0 | 0 | 0 | 0 |
| Velvetleaf | 0 | 0 | 20 | 0 | 0 | 0 | 0 | 0 | 0 | 0 | 0 | 0 | 0 | 0 |
| Wheat | 0 | 0 | 0 | 0 | 0 | 0 | 30 | 20 | 30 | 0 | 70 | 0 | 0 | 0 |

| Table A | Compounds | | | | | | | | | | | | | |
|---|---|---|---|---|---|---|---|---|---|---|---|---|---|---|
| 31 g ai/ha | 61 | 63 | 64 | 66 | 67 | 68 | 69 | 70 | 71 | 72 | 73 | 74 | 75 | 76 |
| Postemergence | | | | | | | | | | | | | | |
| Barnyardgrass | 100 | 0 | 0 | 100 | 100 | 10 | 90 | 0 | 10 | 50 | 60 | 100 | 10 | 90 |
| Blackgrass | - | - | - | - | - | 0 | 10 | 0 | - | 80 | 90 | 100 | 40 | 90 |
| Canarygrass | - | - | - | - | - | 0 | 0 | 0 | - | 40 | 20 | 100 | 10 | 40 |
| Corn | 100 | 0 | 0 | 100 | 90 | 0 | 60 | 0 | 0 | 80 | 90 | 60 | 0 | 20 |
| Crabgrass, Large | 100 | 10 | 0 | 100 | 80 | 10 | 40 | 0 | 20 | 20 | 40 | 60 | 20 | 20 |
| Foxtail, Giant | 100 | 0 | 30 | 100 | 100 | 50 | 90 | 0 | 30 | 90 | 50 | 70 | 50 | 70 |
| Morningglory | 0 | 0 | 0 | 0 | 0 | - | - | - | 0 | - | - | - | - | - |
| Pigweed | 0 | 0 | 0 | 0 | 0 | 0 | 0 | 0 | 0 | 0 | 10 | 0 | 0 | 0 |
| Velvetleaf | 0 | 0 | 0 | 0 | 0 | - | - | - | 0 | - | - | - | - | - |
| Wheat | 80 | 0 | 0 | 90 | 90 | 0 | 0 | 0 | 0 | 60 | 40 | 70 | 10 | 20 |

| Table A | Compounds | | | | | | | | | | | | | |
|---|---|---|---|---|---|---|---|---|---|---|---|---|---|---|
| 31 g ai/ha | 78 | 79 | 80 | 81 | 82 | 83 | 84 | 85 | 86 | 87 | 88 | 89 | 90 | 92 |
| Postemergence | | | | | | | | | | | | | | |
| Barnyardgrass | 90 | 0 | 100 | 90 | 100 | 100 | 0 | 0 | 100 | 0 | 10 | 100 | 0 | 0 |
| Blackgrass | 90 | 60 | 30 | 100 | 100 | 90 | 0 | 0 | 80 | 30 | 10 | 100 | 0 | 0 |
| Canarygrass | 40 | 40 | 40 | 60 | 100 | 70 | 0 | 0 | - | 10 | 30 | 100 | 0 | 0 |
| Corn | 60 | 0 | 60 | 30 | 80 | 0 | 0 | 0 | 30 | 0 | 0 | 100 | 0 | 0 |
| Crabgrass, Large | 80 | 30 | 60 | 90 | 100 | 60 | 0 | 0 | 70 | 10 | 40 | 100 | 0 | 0 |
| Foxtail, Giant | 80 | 10 | 90 | 80 | 100 | 90 | 0 | 0 | 70 | 20 | 40 | 100 | 0 | 10 |
| Pigweed | 0 | 0 | 0 | 0 | 0 | 0 | 0 | 0 | 0 | 0 | 0 | - | 0 | 0 |
| Wheat | 70 | 0 | 30 | 0 | 90 | 0 | 0 | 0 | 20 | 20 | 20 | 80 | 0 | 0 |

| Table A | Compound | | | | | | Table A | | | | Compounds | | | |
|---|---|---|---|---|---|---|---|---|---|---|---|---|---|---|
| 31 g ai/ha | 93 | 94 | 95 | | | | 1000 g ai/ha | | | | 1 | | 2 | |
| Postemergence | | | | | | | Preemergence | | | | | | | |
| Barnyardgrass | 100 | 100 | 100 | | | | Barnyardgrass | | | | 100 | | 100 | |
| Blackgrass | 90 | 80 | 70 | | | | Corn | | | | 100 | | 80 | |
| Canarygrass | 80 | 70 | 90 | | | | Crabgrass, Large | | | | 90 | | 100 | |
| Corn | 80 | 100 | 90 | | | | Foxtail, Giant | | | | 100 | | 100 | |
| Crabgrass, Large | 100 | 90 | 90 | | | | Morningglory | | | | 0 | | 0 | |
| Foxtail, Giant | 100 | 100 | 100 | | | | Pigweed | | | | 20 | | 0 | |
| Pigweed | 0 | 0 | 0 | | | | Velvetleaf | | | | 0 90 | | 60 | |
| Wheat | 70 | 50 | 70 | | | | Wheat | | | | | | 90 | |

| Table A | Compounds | | | | | | | | | | | | | |
|---|---|---|---|---|---|---|---|---|---|---|---|---|---|---|
| 500 g ai/ha | 3 | 4 | 5 | 6 | 7 | 8 | 9 | 10 | 11 | 12 | 13 | 14 | 15 | 16 |
| Preemergence | | | | | | | | | | | | | | |
| Barnyardgrass | 100 | 100 | 100 | 100 | 100 | 0 | 0 | 0 | 100 | 100 | 100 | 100 | 100 | 100 |
| Corn | 90 | 90 | 90 | 90 | 90 | 0 | 0 | 0 | 100 | 90 | 70 | 60 | 90 | 90 |
| Crabgrass, Large | 100 | 100 | 100 | 100 | 100 | 0 | 0 | 0 | 100 | 100 | 90 | 90 | 100 | 100 |
| Foxtail, Giant | 100 | 100 | 100 | 100 | 100 | 0 | 0 | 0 | 100 | 100 | 100 | 100 | 100 | 100 |
| Morningglory | 0 | 0 | 0 | 40 | 20 | 0 | 0 | 0 | 30 | 0 | 0 | 0 | 0 | 0 |
| Pigweed | 0 | 0 | 0 | 40 | 0 | 0 | 0 | 0 | 30 | 0 | 0 | 0 | 0 | 0 |
| Velvetleaf | 10 | 10 | 20 | 50 | 30 | 0 | 0 | 0 | 0 | 0 | 20 | 20 | 0 | 0 |
| Wheat | 90 | 100 | 100 | 90 | 80 | 0 | 0 | 0 | 100 | 100 | 70 | 80 | 80 | 100 |

| Table A | Compounds | | | | | | | | | | | | | |
|---|---|---|---|---|---|---|---|---|---|---|---|---|---|---|
| 500 g ai/ha | 17 | 19 | 20 | 21 | 22 | 23 | 24 | 26 | 27 | 28 | 29 | 30 | 31 | 42 |
| Preemergence | | | | | | | | | | | | | | |
| Barnyardgrass | 100 | 100 | 100 | 100 | 100 | 10 | 30 | 100 | 100 | 100 | 70 | 100 | 100 | 0 |
| Corn | 40 | 90 | 90 | 100 | 70 | 20 | 0 | 90 | 80 | 90 | 0 | 70 | 70 | 0 |
| Crabgrass, Large | 70 | 100 | 100 | 100 | 90 | 0 | 60 | 100 | 100 | 100 | 40 | 100 | 100 | 0 |
| Foxtail, Giant | 90 | 100 | 100 | 100 | 100 | 70 | 80 | 100 | 90 | 100 | 100 | 100 | 100 | 0 |
| Morningglory | 0 | 0 | 0 | 0 | 0 | 0 | 0 | 0 | 0 | 0 | 0 | 0 | 0 | 0 |
| Pigweed | 0 | 0 | 0 | 0 | 0 | 0 | 0 | 0 | 0 | 0 | 0 | 0 | 20 | 0 |
| Velvetleaf | 0 | 0 | 0 | 0 | 0 | 0 | 0 | 0 | 0 | 20 | 0 | 0 | 0 | 0 |
| Wheat | 50 | 100 | 80 | 100 | 70 | 0 | 0 | 70 | 100 | 100 | 0 | 90 | 90 | 0 |

| Table A | Compounds | | | | | | Table A | | | | Compound | | | |
|---|---|---|---|---|---|---|---|---|---|---|---|---|---|---|
| 500 g ai/ha | 60 | 62 | 65 | 77 | | | 250 g ai/ha | | | | 18 | | | |
| Preemergence | | | | | | | Preemergence | | | | | | | |
| Barnyardgrass | 100 | 90 | 0 | 100 | | | Barnyardgrass | | | | 100 | | | |
| Blackgrass | - | - | | - 100 | | | Corn | | | | 90 | | | |
| Canarygrass | - | - | - | 90 | | | Crabgrass, Large | | | | 100 | | | |
| Corn | 90 | 20 | 0 | 80 | | | Foxtail, Giant | | | | 100 | | | |
| Crabgrass, Large | 80 | 60 | 0 | 100 | | | Morningglory | | | | 0 | | | |
| Foxtail, Giant | 100 | 30 | 90 | 100 | | | Pigweed | | | | 0 | | | |
| Morningglory | 0 | 0 | 0 | - | | | Velvetleaf | | | | 0 | | | |
| Pigweed | 0 | 0 | 0 | 0 | | | Wheat | | | | 100 | | | |
| Velvetleaf | 0 | 0 | 0 | - | | | | | | | | | | |
| Wheat | 50 | 0 | 0 | 80 | | | | | | | | | | |

| Table A | Compounds | | | | | | | | | | | | | |
|---|---|---|---|---|---|---|---|---|---|---|---|---|---|---|
| 125 g ai/ha | 3 | 4 | 5 | 6 | 7 | 8 | 9 | 10 | 11 | 12 | 13 | 14 | 15 | 16 |
| Preemergence | | | | | | | | | | | | | | |
| Barnyardgrass | 90 | 100 | 90 | 90 | 100 | 0 | 0 | 0 | 100 | 100 | 60 | 70 | 100 | 100 |
| Corn | 80 | 70 | 80 | 80 | 60 | 0 | 0 | 0 | 90 | 80 | 20 | 0 | 50 | 70 |
| Crabgrass, Large | 100 | 100 | 100 | 100 | 100 | 0 | 0 | 0 | 100 | 100 | 60 | 70 | 80 | 90 |
| Foxtail, Giant | 100 | 100 | 100 | 100 | 100 | 0 | 0 | 0 | 100 | 100 | 70 | 80 | 90 | 100 |
| Morningglory | 0 | 0 | 0 | 30 | 20 | 0 | 0 | 0 | 0 | 0 | 0 | 0 | 0 | 0 |
| Pigweed | 0 | 0 | 0 | 20 | 0 | 0 | 0 | 0 | 0 | 0 | 0 | 0 | 0 | 0 |
| Velvetleaf | 0 | 0 | 0 | 40 | 0 | 0 | 0 | 0 | 0 | 0 | 0 | 0 | 0 | 0 |
| Wheat | 70 | 70 | 90 | 80 | 60 | 0 | 0 | 0 | 100 | 90 | 20 | 10 | 60 | 70 |

| Table A | Compounds | | | | | | | | | | | | | |
|---|---|---|---|---|---|---|---|---|---|---|---|---|---|---|
| 125 g ai/ha | 17 | 19 | 20 | 21 | 22 | 23 | 24 | 25 | 26 | 27 | 28 | 29 | 30 | 31 |
| Preemergence | | | | | | | | | | | | | | |
| Barnyardgrass | 40 | 100 | 90 | 100 | 30 | 0 | 0 | 100 | 100 | 80 | 100 | 0 | 100 | 100 |
| Corn | 0 | 80 | 60 | 90 | 30 | 0 | 0 | 50 | 50 | 20 | 80 | 0 | 0 | 50 |
| Crabgrass, Large | 0 | 100 | 90 | 100 | 50 | 0 | 0 | 90 | 90 | 80 | 90 | 0 | 100 | 100 |
| Foxtail, Giant | 40 | 100 | 90 | 100 | 90 | 20 | 0 | 100 | 100 | 80 | 100 | 60 | 100 | 100 |
| Morningglory | 0 | 0 | 0 | 0 | 0 | 0 | 0 | 0 | 0 | 0 | - | 0 | 0 | 0 |
| Pigweed | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 20 | 90 | 60 | 90 | 40 | 0 | 0 | 50 | 20 | 40 | 70 | 0 | 40 | 60 |

| Table A | Compounds | | | | | | | | | | | | | |
|---|---|---|---|---|---|---|---|---|---|---|---|---|---|---|
| 125 g ai/ha | 32 | 33 | 34 | 35 | 36 | 37 | 38 | 39 | 40 | 41 | 42 | 43 | 44 | 45 |
| Preemergence | | | | | | | | | | | | | | |
| Barnyardgrass | 90 | 100 | 100 | 100 | 100 | 100 | 100 | 100 | 100 | 100 | 0 | 100 | 100 | 100 |
| Corn | 60 | 90 | 60 | 80 | 80 | 90 | 70 | 90 | 90 | 90 | 0 | 90 | 80 | 60 |
| Crabgrass, Large | 90 | 100 | 90 | 100 | 100 | 100 | 100 | 100 | 100 | 100 | 0 | 90 | 100 | 90 |
| Foxtail, Giant | 100 | 100 | 100 | 100 | 100 | 100 | 100 | 100 | 100 | 100 | 0 | 100 | 100 | 100 |
| Morningglory | 0 | 0 | 0 | 0 | 0 | 0 | 0 | 0 | 0 | 0 | 0 | 0 | 0 | 0 |
| Pigweed | 0 | 0 | 0 | 30 | 30 | 0 | 0 | 0 | - | 0 | 0 | 0 | 0 | 0 |
| Velvetleaf | 10 | 0 | 0 | 0 | 0 | 0 | 0 | 0 | 0 | 0 | 0 | 0 | 0 | 0 |
| Wheat | 60 | 80 | 70 | 80 | 90 | 90 | 60 | 80 | 90 | 80 | 0 | 70 | 80 | 80 |

| Table A | Compounds | | | | | | | | | | | | | |
|---|---|---|---|---|---|---|---|---|---|---|---|---|---|---|
| 125 g ai/ha | 46 | 47 | 48 | 49 | 50 | 51 | 52 | 53 | 54 | 55 | 56 | 57 | 58 | 59 |
| Preemergence | | | | | | | | | | | | | | |
| Barnyardgrass | 100 | 80 | 100 | 0 | 100 | 100 | 100 | 100 | 100 | 80 | 100 | 100 | 100 | 100 |
| Corn | 0 | 40 | 80 | 0 | 20 | 80 | 70 | 70 | 60 | 40 | 80 | 50 | 20 | 60 |
| Crabgrass, Large | 90 | 90 | 100 | 40 | 90 | 100 | 100 | 100 | 100 | 80 | 100 | 100 | 90 | 50 |
| Foxtail, Giant | 100 | 90 | 100 | 80 | 90 | 100 | 100 | 100 | 100 | 90 | 100 | 100 | 100 | 90 |
| Morningglory | 0 | 0 | 0 | 0 | 0 | 0 | 20 | 0 | 20 | 0 | 0 | 0 | 0 | 0 |
| Pigweed | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 50 | 40 | 80 | 0 | 50 | 80 | 80 | 90 | 60 | 50 | 80 | 70 | 70 | 50 |

| Table A | Compounds | | | | | | | | | | | | | |
|---|---|---|---|---|---|---|---|---|---|---|---|---|---|---|
| 125 g ai/ha | 60 | 61 | 62 | 63 | 64 | 65 | 66 | 67 | 68 | 69 | 70 | 71 | 72 | 73 |
| Preemergence | | | | | | | | | | | | | | |
| Barnyardgrass | 70 | 100 | 10 | 10 | 0 | 0 | 100 | 100 | 90 | 70 | 0 | 40 | 90 | 10 |
| Blackgrass | - | - | - | - | - | - | - | - | 50 | 40 | 0 | - | 100 | 90 |
| Canarygrass | - | - | - | - | - | - | - | - | 20 | 80 | 0 | - | 80 | - |
| Corn | 50 | 90 | 0 | 0 | 30 | 0 | 80 | 80 | 0 | 30 | 0 | 20 | 50 | 20 |
| Crabgrass, Large | 20 | 100 | 20 | 0 | 50 | 0 | 70 | 90 | 10 | 90 | 0 | 60 | 60 | 50 |
| Foxtail, Giant | 80 | 100 | 0 | 0 | 50 | 0 | 100 | 100 | 100 | 90 | 0 | 100 | 90 | 30 |
| Morningglory | 0 | 0 | 0 | 0 | 0 | 0 | 0 | 0 | - | - | - | 0 | - | - |
| Pigweed | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | - | - | - | 0 | - | - |
| Wheat | 0 | 90 | 0 | 0 | 0 | 0 | 90 | 90 | - | 0 | 0 | 50 | 80 | 70 |

| Table A | Compounds | | | | | | | | | | | | | |
|---|---|---|---|---|---|---|---|---|---|---|---|---|---|---|
| 125 g ai/ha | 74 | 75 | 76 | 77 | 78 | 79 | 80 | 81 | 82 | 83 | 84 | 85 | 86 | 87 |
| Preemergence | | | | | | | | | | | | | | |
| Barnyardgrass | 100 | 80 | 100 | 0 | 100 | 100 | 100 | 100 | 100 | 100 | 0 | 40 | 100 | 50 |
| Blackgrass | 100 | 80 | 90 | 80 | 100 | 100 | 100 | 100 | 100 | 100 | 0 | 40 | 90 | 90 |
| Canarygrass | 100 | 80 | 70 | - | 100 | 100 | 100 | 90 | 100 | 90 | 0 | 70 | 100 | 90 |
| Corn | 60 | 0 | 50 | 30 | 90 | 20 | 60 | 50 | 50 | 40 | 0 | 0 | 40 | 20 |
| Crabgrass, Large | 90 | 40 | 80 | 10 | 100 | 100 | 100 | 100 | 100 | 70 | 0 | 70 | 90 | 70 |
| Foxtail, Giant | 100 | 80 | 100 | 30 | 100 | 100 | 100 | 100 | 100 | 100 | 0 | 100 | 100 | 90 |
| Pigweed | 0 | 0 | 0 | 0 | 0 | 0 | 0 | 0 | 0 | 30 | 0 | 0 | 0 | 0 |
| Wheat | 60 | 20 | 0 | 0 | 90 | 60 | 80 | 50 | 90 | 30 | 0 | 20 | 40 | 20 |

| Table A | Compounds | | | | | | | | Table A | | | | Compound | |
|---|---|---|---|---|---|---|---|---|---|---|---|---|---|---|
| 125 g ai/ha | 88 | 89 | 90 | 92 | 93 | 94 | 95 | | 62 g ai/ha | | | | 18 | |
| Preemergence | | | | | | | | | Preemergence | | | | | |
| Barnyardgrass | 90 | 100 | 0 | 20 | 100 | 100 | 100 | | Barnyardgrass | | | | 100 | |
| Blackgrass | 80 | 100 | 0 | 0 | 100 | 100 | 100 | | Corn | | | | 90 | |
| Canarygrass | 70 | 100 | 0 | 10 | 90 | 90 | 100 | | Crabgrass, Large | | | | 100 | |
| Corn | 20 | 70 | 0 | 20 | 60 | 90 | 90 | | Foxtail, Giant | | | | 100 | |
| Crabgrass, Large | 90 | 100 | 0 | 20 | 100 | 100 | 100 | | Morningglory | | | | 0 | |
| Foxtail, Giant | 90 | 100 | 0 | 50 | 100 | 100 | 100 | | Pigweed | | | | 0 | |
| Pigweed | 0 | 0 | 0 | 0 | 0 | 0 | 0 | | Velvetleaf | | | | 0 | |
| Wheat | 40 | 90 | 0 | 0 | 80 | 90 | 90 | | Wheat | | | | 90 | |

| Table A | Compounds | | | | | | | | | | | | | |
|---|---|---|---|---|---|---|---|---|---|---|---|---|---|---|
| 31 g ai/ha | 25 | 32 | 33 | 34 | 35 | 36 | 37 | 38 | 39 | 40 | 41 | 43 | 44 | 45 |
| Preemergence | | | | | | | | | | | | | | |
| Barnyardgrass | 50 | 20 | 100 | 70 | 80 | 90 | 90 | 90 | 100 | 100 | 100 | 100 | 80 | 90 |
| Corn | 0 | 0 | 30 | 20 | 0 | 50 | 30 | 0 | 30 | 30 | 30 | 0 | 30 | 0 |
| Crabgrass, Large | 20 | 90 | 90 | 80 | 90 | 70 | 80 | 90 | 60 | 70 | 80 | 10 | 20 | 30 |
| Foxtail, Giant | 40 | 90 | 100 | 100 | 100 | 100 | 100 | 90 | 90 | 100 | 90 | 90 | 80 | 90 |
| Morningglory | 0 | 0 | 0 | 0 | 0 | 0 | 0 | 0 | 0 | 0 | 0 | 0 | 0 | 0 |
| Pigweed | 0 | - | - | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 0 | 0 | 0 | 0 |
| Wheat | 0 | 0 | 50 | 30 | 20 | 50 | 30 | 20 | 40 | 50 | 50 | 20 | 40 | 50 |

| Table A | Compounds | | | | | | | | | | | | | |
|---|---|---|---|---|---|---|---|---|---|---|---|---|---|---|
| 31 g ai/ha | 46 | 47 | 48 | 49 | 50 | 51 | 52 | 53 | 54 | 55 | 56 | 57 | 58 | 59 |
| Preemergence | | | | | | | | | | | | | | |
| Barnyardgrass | 40 | 20 | 60 | 0 | 10 | 80 | 70 | 100 | 70 | 0 | 80 | 60 | 50 | 90 |
| Corn | 0 | 0 | - | 0 | 0 | 20 | 0 | 0 | 0 | 0 | 40 | 0 | 0 | 0 |
| Crabgrass, Large | 10 | 10 | 60 | 0 | 50 | 90 | 30 | 90 | 60 | 20 | 70 | 50 | 60 | 20 |
| Foxtail, Giant | 50 | 70 | 90 | 0 | 70 | 90 | 100 | 100 | 90 | 80 | 90 | 90 | 90 | 70 |
| Morningglory | 0 | 0 | 0 | 0 | 0 | 0 | 0 | 0 | 0 | 0 | 0 | 0 | 0 | 0 |
| Pigweed | 0 | 0 | 0 | 0 | 0 | - | 0 | 0 | 0 | 0 | 0 | 0 | 0 | 0 |
| Velvetleaf | 0 | 0 | 0 | 0 | 0 | 0 | - | 0 | 0 | 0 | 0 | 0 | 0 | 0 |
| Wheat | 0 | 0 | 30 | 0 | 0 | 40 | 10 | 50 | 0 | 0 | 20 | 0 | 20 | 0 |

| 31 g ai/ha | 61 | 63 | 64 | 66 | 67 | 68 | 69 | 70 | 71 | 72 | 73 | 74 | 75 | 76 |
|---|---|---|---|---|---|---|---|---|---|---|---|---|---|---|
| Preemergence | | | | | | | | | | | | | | |
| Barnyardgrass | 90 | 0 | 0 | 10 | 0 | 0 | 20 | 0 | 0 | 20 | 0 | 100 | 0 | 40 |
| Blackgrass | - | - | - | - | - | 0 | 20 | 0 | - | 70 | 60 | 100 | 40 | - |
| Canarygrass | - | - | - | - | - | 0 | - | 0 | - | 50 | 30 | 80 | 10 | 30 |
| Corn | 70 | 0 | 0 | 40 | 20 | 0 | 20 | 0 | 0 | 20 | 0 | - | 0 | 0 |
| Crabgrass, Large | 80 | 0 | 0 | 0 | 20 | 0 | 0 | 0 | 10 | 0 | 0 | 50 | 0 | 20 |
| Foxtail, Giant | 100 | 0 | 0 | 90 | 40 | 10 | 20 | 0 | 0 | 70 | 0 | 90 | 20 | 70 |
| Morningglory | 0 | 0 | 0 | 0 | 0 | - | - | - | 0 | - | - | - | - | - |
| Pigweed | 0 | 0 | 0 | 0 | 0 | 0 | 0 | 0 | 0 | 0 | 0 | 0 | 0 | 0 |
| Velvetleaf | 0 | 0 | 0 | 0 | 0 | - | - | - | 0 | - | - | - | - | - |
| Wheat | 70 | 0 | 0 | 60 | 20 | 0 | 0 | 0 | 0 | 50 | 20 | 20 | 0 | 0 |

| Table A | Compounds | | | | | | | | | | | | | |
|---|---|---|---|---|---|---|---|---|---|---|---|---|---|---|
| 31 g ai/ha | 78 | 79 | 80 | 81 | 82 | 83 | 84 | 85 | 86 | 87 | 88 | 89 | 90 | 92 |
| Preemergence | | | | | | | | | | | | | | |
| Barnyardgrass | 0 | 0 | 0 | 70 | 50 | 20 | 0 | 0 | 50 | 0 | 0 | 30 | 0 | 0 |
| Blackgrass | 0 | 80 | 70 | 70 | 70 | 20 | 0 | 0 | 50 | 30 | 40 | 90 | 0 | 0 |
| Canarygrass | 0 | 0 | 60 | 50 | 20 | - | 0 | 40 | - | 0 | 0 | 50 | 0 | 0 |
| Corn | 0 | 0 | 40 | 30 | 40 | 0 | 0 | 0 | 0 | 0 | 0 | 50 | 0 | 0 |
| Crabgrass, Large | 10 | 10 | 10 | 70 | 50 | 0 | 0 | 10 | 10 | 20 | 0 | 70 | 0 | 0 |
| Foxtail, Giant | 20 | 10 | 80 | 80 | 90 | 0 | 0 | 0 | 10 | 20 | 40 | 90 | 0 | 0 |
| Pigweed | 0 | 0 | 0 | 0 | 0 | 0 | 0 | 0 | 0 | 0 | 0 | 0 | 0 | 0 |
| Wheat | 0 | 0 | 20 | 20 | 50 | 0 | 0 | 0 | 0 | 0 | 0 | 40 | 0 | 0 |

| Table A | Compound | | | | | Table A | | | | Compound | | | | |
|---|---|---|---|---|---|---|---|---|---|---|---|---|---|---|
| 31 g ai/ha | 93 | 94 | 95 | | | 31 g ai/ha | | | | | 93 | 94 | 95 | |
| Preemergence | | | | | | Preemergence | | | | | | | | |
| Barnyardgrass | 20 | 50 | 50 | | | Crabgrass, Large | | | | | 20 | 70 | 90 | |
| Blackgrass | 30 | 60 | 90 | | | Foxtail, Giant | | | | | 90 | 90 | 100 | |
| Canarygrass | 10 | 70 | 70 | | | Pigweed | | | | | 0 | 0 | 0 | |
| Corn | 30 | - | 40 | | | Wheat | | | | | 50 | 50 | 80 | |

### TEST B

Seeds of plant species selected from blackgrass (*Alopecurus myosuroides*), downy bromegrass (*Bromus tectorum*), green foxtail (*Setaria viridis*), Italian ryegrass (*Lolium multiflorum*), wheat *(Triticum aestivum),* wild oat (*Avena fatua*), catchweed bedstraw (*Galium aparine*), bermudagrass (*Cynodon dactylon*), surinam grass (*Brachiaria decumbens*), cocklebur (*Xanthium strumarium*), corn (*Zea mays*), large crabgrass (*Digitaria sanguinalis*), woolly cupgrass (*Eriochloa villosa*), giant foxtail (*Setaria faberii*), goosegrass (*Eleusine indica*), johnsongrass (*Sorghum halepense*), kochia (*Kochia scoparia*), lambsquarters (*Chenopodium album*), morningglory (*Ipomoea coccinea*), eastern black (E.B.) nightshade (*Solanum ptycanthum*), yellow nutsedge (*Cyperus esculentus*), pigweed (*Amaranthus retroflexus*), common ragweed (*Ambrosia elatior*), Russian thistle (*Salsola iberica*), soybean (*Glycine max*), common (oilseed) sunflower (*Helianthus annuus*), and velvetleaf (*Abutilon theophrasti*) were planted and treated preemergence with test chemicals formulated in a non-phytotoxic solvent mixture which included a surfactant.

At the same time, plants selected from these crop and weed species and also winter barley (*Hordeum vulgare*), canarygrass (*Phalaris minor*), chickweed (*Stellaria media*) and windgrass (*Apera spica-venti*) were treated with postemergence applications of some of the test chemicals formulated in the same manner. Plants ranged in height from 2 to 18 cm (1-to 4-leaf stage) for postemergence treatments.

Plant species in the flooded paddy test consisted of rice (*Oryza sativa*), umbrella sedge (*Cyperus difformis*), ducksalad (*Heteranthera limosa*) and barnyardgrass (*Echinochloa crusgalli*) grown to the 2-leaf stage for testing. At time of treatment, test pots were flooded to 3 cm above the soil surface, treated by application of test compounds directly to the paddy water, and then maintained at that water depth for the duration of the test. Treated plants and controls were maintained in a greenhouse for 13 to 15 days, after which time all species were compared to controls and visually evaluated. Plant response ratings, summarized in Table B, are based on a scale of 0 to 100 where 0 is no effect and 100 is complete control. A dash (-) response means no test result.

| Table B | Compounds | | | | | | | | | | | | | |
|---|---|---|---|---|---|---|---|---|---|---|---|---|---|---|
| 250 g ai/ha | 1 | 2 | 3 | 4 | 5 | 6 | 7 | 11 | 12 | 13 | 14 | 15 | 16 | 17 |
| Flood | | | | | | | | | | | | | | |
| Barnyardgrass | 60 | 45 | 80 | 75 | 90 | 90 | 85 | 75 | 80 | 0 | 20 | 60 | 60 | 0 |
| Ducksalad | 30 | 40 | 40 | 85 | 65 | 70 | 40 | 40 | 50 | 0 | 40 | 100 | 0 | 95 |
| Rice | 50 | 45 | 80 | 80 | 80 | 90 | 65 | 70 | 80 | 0 | 20 | 70 | 80 | 40 |
| Sedge, Umbrella | 60 | - | 80 | 100 | 80 | 95 | 80 | 40 | 60 | 0 | 60 | 70 | 45 | 70 |

| Table B | Compounds | | | | | | | | | | | | | |
|---|---|---|---|---|---|---|---|---|---|---|---|---|---|---|
| 250 g ai/ha | 19 | 20 | 21 | 22 | 25 | 26 | 27 | 28 | 29 | 30 | 31 | 32 | 33 | 34 |
| Flood | | | | | | | | | | | | | | |
| Barnyardgrass | 75 | 0 | 100 | 0 | 0 | 20 | 30 | 30 | 0 | 0 | 0 | 70 | 100 | 100 |
| Ducksalad | 25 | 0 | 65 | 0 | 0 | 70 | 75 | 0 | 0 | 0 | 0 | 40 | 50 | 40 |
| Rice | 70 | 0 | 70 | 0 | 0 | 0 | 0 | 60 | 0 | 0 | 0 | 65 | 80 | 80 |
| Sedge, Umbrella | 70 | 0 | 30 | 0 | 0 | 75 | 65 | 0 | 0 | 0 | 0 | 30 | 60 | 0 |

| Table B | Compounds | | | | | | | | | | | | | |
|---|---|---|---|---|---|---|---|---|---|---|---|---|---|---|
| 250 g ai/ha | 36 | 37 | 38 | 39 | 40 | 41 | 42 | 43 | 44 | 45 | 46 | 47 | 48 | 49 |
| Flood | | | | | | | | | | | | | | |
| Barnyardgrass | 0 | 100 | 95 | 85 | 100 | 95 | 0 | - | 50 | 65 | 40 | 0 | 30 | 0 |
| Ducksalad | 0 | 75 | 40 | 80 | 0 | 70 | 0 | 30 | 70 | 75 | 85 | 0 | 30 | 0 |
| Rice | 0 | 90 | 85 | 80 | 80 | 80 | 0 | 20 | 40 | 45 | 35 | 0 | 0 | 0 |
| Sedge, Umbrella | 0 | 60 | 70 | 85 | 0 | 85 | 0 | 0 | 30 | 65 | 75 | 0 | 50 | 0 |

| Table B | Compounds | | | | | | | | | | | | | |
|---|---|---|---|---|---|---|---|---|---|---|---|---|---|---|
| 250 g ai/ha | 50 | 51 | 52 | 53 | 54 | 55 | 56 | 57 | 58 | 59 | 60 | 61 | 62 | 63 |
| Flood | | | | | | | | | | | | | | |
| Barnyardgrass | 0 | 30 | 75 | 40 | 80 | 65 | 60 | 0 | 70 | 50 | 30 | 100 | 0 | 0 |
| Ducksalad | 0 | 0 | 0 | 50 | 0 | 75 | 75 | 70 | 45 | 20 | 0 | 70 | 0 | 0 |
| Rice | 0 | 0 | 60 | 30 | 80 | 30 | 10 | 15 | 60 | 25 | 30 | 60 | 0 | 0 |
| Sedge, Umbrella | 0 | 70 | 0 | 70 | 0 | 80 | 85 | 80 | 0 | 30 | 0 | 30 | 0 | 0 |

| Table B | Compounds | | | | | | | | | | | | | |
|---|---|---|---|---|---|---|---|---|---|---|---|---|---|---|
| 250 g ai/ha | 64 | 65 | 66 | 67 | 68 | 69 | 72 | 73 | 75 | 76 | 77 | 80 | 81 | 83 |
| Flood | | | | | | | | | | | | | | |
| Barnyardgrass | 0 | 0 | 20 | 50 | 0 | 0 | 70 | 0 | 0 | 0 | 0 | 0 | 0 | 0 |
| Ducksalad | 0 | 40 | 0 | 75 | 0 | 0 | 40 | 0 | 0 | 0 | 0 | 0 | 0 | - |
| Rice | 0 | 15 | 30 | 15 | 0 | 0 | 65 | 0 | 0 | 0 | 0 | 0 | 0 | 0 |
| Sedge, Umbrella | 0 | 80 | 0 | 0 | 0 | 0 | 65 | 0 | 0 | 0 | 0 | 0 | 0 | 0 |

| Table B | Compounds | | | | | | | | | | | | | |
|---|---|---|---|---|---|---|---|---|---|---|---|---|---|---|
| 250 g ai/ha | 84 | 85 | 86 | 87 | | | | | | | | | | |
| Flood | | | | | | | | | | | | | | |
| Barnyardgrass | 0 | 0 | 40 | 0 | | | | | | | | | | |
| Ducksalad | 0 | 0 | 65 | 0 | | | | | | | | | | |
| Rice | 0 | 0 | 35 | 0 | | | | | | | | | | |
| Sedge, Umbrella | 0 | 0 | 55 | 0 | | | | | | | | | | |

| Table B | Compounds | | | | | | | | | | | | | |
|---|---|---|---|---|---|---|---|---|---|---|---|---|---|---|
| 125 g ai/ha | 1 | 2 | 3 | 4 | 5 | 6 | 7 | 11 | 12 | 13 | 14 | 15 | 16 | 17 |
| Flood | | | | | | | | | | | | | | |
| Barnyardgrass | 20 | 20 | 70 | 70 | 70 | 80 | 65 | 65 | 60 | 0 | 20 | 40 | 40 | 0 |
| Ducksalad | 0 | 0 | 0 | 85 | 50 | 60 | 20 | 0 | 40 | 0 | 40 | 80 | 0 | 40 |
| Rice | 0 | 15 | 75 | 70 | 75 | 80 | 45 | 60 | 70 | 0 | 0 | 15 | 45 | 20 |
| Sedge, Umbrella | 40 | 60 | 80 | 90 | 80 | 85 | 80 | 40 | 60 | 0 | - | 60 | 30 | 40 |

| 125 g ai/ha | 19 | 20 | 21 | 22 | 26 | 27 | 28 | 29 | 30 | 31 | 32 | 33 | 34 | 35 |
|---|---|---|---|---|---|---|---|---|---|---|---|---|---|---|
| Flood | | | | | | | | | | | | | | |
| Barnyardgrass | 60 | 0 | 80 | 0 | 0 | 0 | 0 | 0 | 0 | 0 | 65 | 85 | 100 | 0 |
| Ducksalad | 0 | 0 | 0 | 0 | - | 60 | 0 | 0 | 0 | 0 | 40 | 40 | 30 | 0 |
| Rice | 60 | 0 | 20 | 0 | 0 | 0 | 0 | 0 | 0 | 0 | 30 | 75 | 70 | 0 |
| Sedge, Umbrella | 60 | 0 | 0 | 0 | 0 | 30 | 0 | 0 | 0 | 0 | 30 | 35 | 0 | 0 |

| Table B | Compounds | | | | | | | | | | | | | |
|---|---|---|---|---|---|---|---|---|---|---|---|---|---|---|
| 125 g ai/ha | 36 | 37 | 38 | 39 | 40 | 41 | 42 | 43 | 44 | 45 | 46 | 47 | 48 | 49 |
| Flood | | | | | | | | | | | | | | |
| Barnyardgrass | 0 | 100 | 95 | 65 | 100 | 80 | 0 | 75 | 30 | 60 | 30 | 0 | 0 | 0 |
| Ducksalad | 0 | 70 | 30 | 0 | 0 | 40 | 0 | 0 | 70 | 0 | 45 | 0 | 0 | - |
| Rice | 0 | 80 | 75 | 50 | 70 | 50 | 0 | 20 | 20 | 45 | 20 | 0 | 0 | 0 |
| Sedge, Umbrella | 0 | 0 | 70 | 0 | 0 | 0 | 0 | 0 | 20 | 0 | 60 | 0 | 0 | 0 |

| Table B | Compounds | | | | | | | | | | | | | |
|---|---|---|---|---|---|---|---|---|---|---|---|---|---|---|
| 125 g ai/ha | 50 | 51 | 53 | 55 | 56 | 57 | 61 | 62 | 63 | 65 | 66 | 67 | 68 | 69 |
| Flood | | | | | | | | | | | | | | |
| Barnyardgrass | 0 | 10 | 0 | 0 | 20 | 0 | 50 | 0 | 0 | 0 | 20 | 35 | 0 | 0 |
| Ducksalad | 0 | 0 | 50 | 0 | 40 | 0 | 0 | 0 | 0 | 0 | 0 | 0 | 0 | 0 |
| Rice | 0 | 0 | 0 | 0 | 0 | 10 | 15 | 0 | 0 | 0 | 0 | 0 | 0 | 0 |
| Sedge, Umbrella | 0 | 60 | 40 | 75 | 85 | 75 | 0 | 0 | 0 | 0 | 0 | 0 | 0 | 0 |
| Table B | Compounds | | | | | | | | | | | | | |
| 125 g ai/ha | 72 | 73 | 77 | 80 | 81 | 83 | | | | | | | | |
| Flood | | | | | | | | | | | | | | |
| Barnyardgrass | 40 | 0 | 0 | 0 | 0 | 0 | | | | | | | | |
| Ducksalad | 0 | 0 | 0 | 0 | 0 | 0 | | | | | | | | |
| Rice | 45 | 0 | 0 | 0 | 0 | 0 | | | | | | | | |
| Sedge, Umbrella | 60 | 0 | 0 | 0 | 0 | 0 | | | | | | | | |

| Table B | Compounds | | | | | | | | | | | | | |
|---|---|---|---|---|---|---|---|---|---|---|---|---|---|---|
| 62 g ai/ha | 1 | 2 | 3 | 4 | 5 | 6 | 7 | 11 | 12 | 13 | 14 | 15 | 16 | 17 |
| Flood | | | | | | | | | | | | | | |
| Barnyardgrass | 0 | 0 | 60 | 60 | 50 | 40 | 45 | 50 | 30 | 0 | 0 | 15 | 20 | 0 |
| Ducksalad | 0 | 0 | 0 | 75 | 40 | 50 | 0 | 0 | 0 | 0 | 0 | 0 | 0 | 40 |
| Rice | 0 | 15 | 60 | 40 | 60 | 30 | 30 | 30 | 20 | 0 | 0 | 0 | 20 | 15 |
| Sedge, Umbrella | 40 | 50 | 60 | 60 | 70 | 70 | 80 | 30 | 40 | 0 | 60 | 50 | 30 | 40 |

| 62 g ai/ha | 19 | 20 | 21 | 22 | 25 | 26 | 27 | 28 | 29 | 30 | 31 | 32 | 33 | 34 |
|---|---|---|---|---|---|---|---|---|---|---|---|---|---|---|
| Flood | | | | | | | | | | | | | | |
| Barnyardgrass | 30 | 0 | 75 | 0 | 0 | 0 | 0 | 0 | 0 | 0 | 0 | 20 | 60 | 50 |
| Ducksalad | 0 | 0 | 0 | 0 | 0 | 50 | 40 | 0 | 0 | 0 | 0 | 30 | 0 | 0 |
| Rice | 20 | 0 | 0 | 0 | 0 | 0 | 0 | 0 | 0 | 0 | 0 | 0 | 65 | 40 |
| Sedge, Umbrella | 30 | 0 | 0 | 0 | 0 | 0 | 20 | 0 | 0 | 0 | 0 | 30 | 20 | 0 |

| Table B | Compounds | | | | | | | | | | | | | |
|---|---|---|---|---|---|---|---|---|---|---|---|---|---|---|
| 62 g ai/ha | 35 | 36 | 37 | 38 | 39 | 40 | 41 | 42 | 43 | 44 | 45 | 46 | 47 | 48 |
| Flood | | | | | | | | | | | | | | |
| Barnyardgrass | 0 | 0 | 60 | 95 | 80 | 85 | 75 | 0 | 50 | 0 | 20 | 0 | 0 | 0 |
| Ducksalad | 0 | 0 | 70 | - | 0 | 0 | 0 | 0 | 0 | 0 | 0 | 30 | 0 | 0 |
| Rice | 0 | 0 | 70 | 75 | 0 | 40 | 20 | 0 | 15 | 15 | 0 | 0 | 0 | 0 |
| Sedge, Umbrella | 0 | 0 | 0 | - | 0 | 0 | 0 | 0 | 0 | 0 | 0 | 50 | 0 | 0 |

| Table B | Compounds | | | | | | | | | | | | | |
|---|---|---|---|---|---|---|---|---|---|---|---|---|---|---|
| 62 g ai/ha | 49 | 50 | 51 | 52 | 53 | 54 | 55 | 56 | 57 | 58 | 59 | 60 | 61 | 62 |
| Flood | | | | | | | | | | | | | | |
| Barnyardgrass | 0 | 0 | 0 | 15 | 0 | 20 | 0 | 0 | 0 | 30 | 0 | 0 | 0 | 0 |
| Ducksalad | 0 | 0 | 0 | 0 | 0 | 0 | 0 | 0 | 0 | 0 | 0 | 0 | 0 | 0 |
| Rice | 0 | 0 | 0 | 10 | 0 | 15 | 0 | 0 | 0 | 0 | 10 | 0 | 0 | 0 |
| Sedge, Umbrella | 0 | 0 | 40 | 0 | 0 | 0 | 70 | 75 | 65 | 0 | 0 | 0 | 0 | 0 |

| Table B | Compounds | | | | | | | | | | | | | |
|---|---|---|---|---|---|---|---|---|---|---|---|---|---|---|
| 62 g ai/ha | 63 | 64 | 65 | 66 | 67 | 68 | 69 | 72 | 73 | 75 | 76 | 77 | 80 | 81 |
| Flood | | | | | | | | | | | | | | |
| Barnyardgrass | 0 | 0 | 0 | 0 | 25 | 0 | 0 | 20 | 0 | 0 | 0 | 0 | 0 | 0 |
| Ducksalad | 0 | 0 | 0 | 0 | 0 | 0 | 0 | 0 | 0 | 0 | 0 | 0 | 0 | 0 |
| Rice | 0 | 0 | 0 | 0 | 0 | 0 | 0 | 15 | 0 | 0 | 0 | 0 | 0 | 0 |
| Sedge, Umbrella | 0 | 0 | 0 | 0 | 0 | 0 | 0 | 0 | 0 | 0 | 0 | 0 | 0 | 0 |

| Table B | Compounds | | | | | | | | | | | | | |
|---|---|---|---|---|---|---|---|---|---|---|---|---|---|---|
| 62 g ai/ha | 83 | 84 | 85 | 86 | 87 | | | | | | | | | |
| Flood | | | | | | | | | | | | | | |
| Barnyardgrass | 0 | 0 | 0 | 0 | 0 | | | | | | | | | |
| Ducksalad | 0 | 0 | 0 | 30 | 0 | | | | | | | | | |
| Rice | 0 | 0 | 0 | 30 | 0 | | | | | | | | | |
| Sedge, Umbrella | 0 | 0 | 0 | 0 | 0 | | | | | | | | | |

| 31 g ai/ha | 1 | 2 | 3 | 4 | 5 | 6 | 7 | 11 | 12 | 13 | 14 | 15 | 16 | 17 |
|---|---|---|---|---|---|---|---|---|---|---|---|---|---|---|
| Flood | | | | | | | | | | | | | | |
| Barnyardgrass | 0 | 0 | 0 | 0 | 20 | 0 | 20 | 40 | 0 | 0 | 0 | 0 | 15 | 0 |
| Ducksalad | 0 | 0 | 0 | 0 | 30 | 40 | 0 | 0 | 0 | 0 | 0 | 0 | 0 | 40 |
| Rice | 0 | 0 | 20 | 15 | 15 | 20 | 20 | 0 | 0 | 0 | 0 | 0 | 0 | 0 |
| Sedge, Umbrella | 0 | 0 | 60 | 40 | 70 | 30 | 70 | 30 | 0 | 0 | 0 | 0 | 0 | 30 |

| Table B | Compounds | | | | | | | | | | | | | |
|---|---|---|---|---|---|---|---|---|---|---|---|---|---|---|
| 31 g ai/ha | 19 | 20 | 21 | 22 | 26 | 27 | 28 | 29 | 30 | 31 | 32 | 33 | 34 | 35 |
| Flood | | | | | | | | | | | | | | |
| Barnyardgrass | 0 | 0 | 55 | 0 | 0 | 0 | 0 | 0 | 0 | 0 | 10 | 30 | 40 | 0 |
| Ducksalad | 0 | 0 | 0 | 0 | 40 | 0 | 0 | 0 | 0 | 0 | 30 | 0 | 0 | 0 |
| Rice | 0 | 0 | 0 | 0 | 0 | 0 | 0 | 0 | 0 | 0 | 0 | 30 | 0 | 0 |
| Sedge, Umbrella | 0 | 0 | 0 | 0 | 0 | 20 | 0 | 0 | 0 | 0 | 20 | 0 | 0 | 0 |

| Table B | Compounds | | | | | | | | | | | | | |
|---|---|---|---|---|---|---|---|---|---|---|---|---|---|---|
| 31 g ai/ha | 36 | 37 | 38 | 39 | 40 | 41 | 42 | 43 | 44 | 45 | 46 | 47 | 48 | 49 |
| Flood | | | | | | | | | | | | | | |
| Barnyardgrass | 0 | 30 | 20 | 0 | 60 | 60 | 0 | 40 | 0 | 20 | 0 | 0 | 0 | 0 |
| Ducksalad | 0 | - | 40 | 0 | 0 | 0 | 0 | 0 | - | 0 | - | 0 | 0 | 0 |
| Rice | 0 | 0 | 0 | 0 | 35 | 15 | 0 | 0 | 0 | 0 | 0 | 0 | 0 | 0 |
| Sedge, Umbrella | 0 | - | 20 | 0 | 0 | 0 | 0 | 0 | 0 | 0 | 40 | 0 | 0 | 0 |

| Table B | Compounds | | | | | | | | | | | | | |
|---|---|---|---|---|---|---|---|---|---|---|---|---|---|---|
| 31 g ai/ha | 50 | 51 | 53 | 55 | 56 | 57 | 61 | 62 | 63 | 65 | 66 | 67 | 68 | 69 |
| Flood | | | | | | | | | | | | | | |
| Barnyardgrass | 0 | 0 | 0 | 0 | 0 | 0 | 0 | 0 | 0 | 0 | 0 | 0 | 0 | 0 |
| Ducksalad | 0 | 0 | 0 | 0 | 0 | - | 0 | 0 | 0 | 0 | 0 | 0 | 0 | 0 |
| Rice | 0 | 0 | 0 | 0 | 0 | 0 | 0 | 0 | 0 | 0 | 0 | 0 | 0 | 0 |
| Sedge, Umbrella | 0 | 0 | 0 | 45 | 70 | 45 | 0 | 0 | 0 | 0 | 0 | 0 | 0 | 0 |

| Table B | Compounds | | | | | | | | | | | | | |
|---|---|---|---|---|---|---|---|---|---|---|---|---|---|---|
| 31 g ai/ha | 72 | 73 | 77 | 80 | 81 | 83 | | | | | | | | |
| Flood | | | | | | | | | | | | | | |
| Barnyardgrass | 0 | 0 | 0 | 0 | 0 | 0 | | | | | | | | |
| Ducksalad | 0 | 0 | 0 | 0 | 0 | 0 | | | | | | | | |
| Rice | 10 | 0 | 0 | 0 | 0 | 0 | | | | | | | | |
| Sedge, Umbrella | 0 | 0 | 0 | 0 | 0 | 0 | | | | | | | | |

| Table B | Compounds | | | | | | | | | | | | | |
|---|---|---|---|---|---|---|---|---|---|---|---|---|---|---|
| 250 g ai/ha | 1 | 3 | 4 | 5 | 6 | 7 | 15 | 16 | | | | | | |
| Postemergence | | | | | | | | | | | | | | |
| Barley | 30 | 0 | 95 | 95 | 80 | 0 | - | 98 | | | | | | |
| Bermudagrass | 100 | 100 | 100 | 100 | 100 | 100 | 100 | 100 | | | | | | |
| Blackgrass | 100 | 100 | 100 | 100 | 100 | 100 | 98 | 100 | | | | | | |
| Bromegrass, Downy | 100 | 5 | 40 | - | 10 | 0 | 60 | 100 | | | | | | |
| Canarygrass | 40 | 90 | 100 | 100 | 100 | 90 | - | - | | | | | | |
| Chickweed | 5 | 0 | 20 | 0 | 0 | 0 | 0 | 0 | | | | | | |
| Cocklebur | 0 | 0 | 0 | 20 | 40 | 0 | 0 | 0 | | | | | | |
| Corn | 100 | 100 | 100 | 100 | 100 | 90 | - | 100 | | | | | | |
| Crabgrass, Large | 100 | 100 | 100 | 100 | 100 | 100 | 100 | 100 | | | | | | |
| Cupgrass, Woolly | 100 | 100 | 100 | 100 | 100 | 100 | 100 | 100 | | | | | | |
| Foxtail, Giant | 100 | 100 | 100 | 100 | 100 | 100 | 100 | 100 | | | | | | |
| Foxtail, Green | 100 | 100 | 100 | 100 | 100 | 100 | 100 | 100 | | | | | | |
| Goosegrass | 85 | 100 | 100 | 100 | 100 | 100 | 100 | 80 | | | | | | |
| Johnsongrass | 100 | - | - | 90 | - | 0 | 100 | 100 | | | | | | |
| Kochia | 20 | 70 | 70 | 70 | 20 | 10 | 0 | 100 | | | | | | |
| Lambsquarters | 50 | 20 | 80 | 80 | 70 | 10 | 0 | 0 | | | | | | |
| Morningglory | 0 | 0 | 0 | 0 | 50 | 0 | 0 | 0 | | | | | | |
| Nutsedge, Yellow | 0 | 0 | 0 | - | 0 | 0 | 0 | 0 | | | | | | |
| Oat, Wild | 100 | 100 | 100 | 100 | 100 | 95 | 100 | 100 | | | | | | |
| Pigweed | 40 | 25 | 50 | 45 | 40 | 20 | 0 | 0 | | | | | | |
| Ragweed | 55 | 50 | 80 | 65 | 50 | 0 | 40 | 0 | | | | | | |
| Ryegrass, Italian | 100 | 100 | 100 | 100 | 100 | 100 | 100 | 100 | | | | | | |
| Soybean | 60 | 60 | 90 | 70 | 90 | - | 0 | 0 | | | | | | |
| Surinam Grass | 100 | 100 | 100 | 100 | 100 | 100 | 100 | 100 | | | | | | |
| Velvetleaf | 50 | 0 | 45 | 10 | 10 | 0 | 0 | 0 | | | | | | |
| Wheat | 95 | 100 | 100 | 100 | 100 | - | 85 | 100 | | | | | | |
| Windgrass | 100 | 100 | 100 | 100 | 100 | 100 | 100 | 100 | | | | | | |

| Table B | Compounds | | | | | | | | | | | | | |
|---|---|---|---|---|---|---|---|---|---|---|---|---|---|---|
| 125 g ai/ha | 1 | 3 | 4 | 5 | 6 | 7 | 11 | 12 | 15 | 16 | | | | |
| Postemergence | | | | | | | | | | | | | | |
| Barley | 0 | 0 | 90 | 95 | 80 | 0 | 100 | 100 | 90 | 95 | | | | |
| Bermudagrass | 100 | 100 | 100 | 100 | 100 | 100 | 100 | 100 | 100 | 100 | | | | |
| Blackgrass | 100 | 100 | 100 | 100 | 100 | 100 | 100 | 100 | 95 | 100 | | | | |
| Bromegrass, Downy | 95 | 5 | 20 | 50 | 5 | 0 | 100 | - | 45 | 90 | | | | |
| Canarygrass | 0 | 85 | 100 | 100 | 100 | 90 | 100 | 100 | 85 | - | | | | |
| Chickweed | 0 | 0 | 0 | 0 | 0 | 0 | 0 | 0 | 0 | 0 | | | | |
| Cocklebur | 0 | 0 | 0 | 0 | 0 | 0 | 0 | 0 | 0 | 0 | | | | |
| Corn | 100 | 100 | 100 | 100 | 100 | 90 | 100 | 100 | 95 | 100 | | | | |
| Crabgrass, Large | 100 | 100 | 100 | 100 | 100 | 100 | 100 | 100 | 98 | 100 | | | | |
| Cupgrass, Woolly | 100 | 100 | 100 | 100 | 100 | 100 | 100 | 100 | 100 | 100 | | | | |
| Foxtail, Giant | 100 | 100 | 100 | 100 | 100 | 100 | 100 | 100 | 100 | 100 | | | | |
| Foxtail, Green | 100 | 100 | 100 | 100 | 100 | 100 | 100 | 100 | 100 | 100 | | | | |
| Goosegrass | 50 | 100 | 100 | 100 | 100 | 100 | 100 | 100 | 90 | 75 | | | | |
| Johnsongrass | 100 | - | 100 | 85 | - | 0 | 100 | 100 | 100 | 100 | | | | |
| Kochia | 0 | 60 | 60 | 0 | 20 | 0 | 0 | 0 | 0 | 98 | | | | |
| Lambsquarters | 40 | 15 | 60 | 80 | 20 | 0 | 0 | 40 | 0 | 0 | | | | |
| Morningglory | 0 | 0 | 0 | 0 | 0 | 0 | 0 | 40 | 0 | 0 | | | | |
| Nutsedge, Yellow | 0 | 0 | 0 | 60 | 0 | 0 | 0 | 0 | 0 | 0 | | | | |
| Oat, Wild | 100 | 100 | 100 | 100 | 100 | 95 | 100 | 100 | 98 | 100 | | | | |
| Pigweed | 0 | 10 | 35 | 45 | 0 | 0 | 5 | 50 | 0 | 0 | | | | |
| Ragweed | 50 | 0 | 50 | 60 | 0 | 0 | 0 | 0 | 0 | 0 | | | | |
| Ryegrass, Italian | 100 | 100 | 100 | 100 | 100 | 100 | 100 | 100 | 100 | 100 | | | | |
| Soybean | 50 | 40 | 90 | 65 | 75 | - | 0 | 0 | 0 | 0 | | | | |
| Surinam Grass | 100 | 100 | 100 | 100 | 100 | 100 | 100 | 100 | 100 | 100 | | | | |
| Velvetleaf | 0 | 0 | 20 | 0 | 0 | 0 | 0 | 30 | 0 | 0 | | | | |
| Wheat | 90 | 100 | 100 | 100 | 100 | 95 | 100 | 100 | 70 | 100 | | | | |
| Windgrass | 100 | 100 | 100 | 100 | 100 | 100 | 100 | 100 | 100 | 100 | | | | |

| Table B | Compounds | | | | | | | | | | | | | |
|---|---|---|---|---|---|---|---|---|---|---|---|---|---|---|
| 62 g ai/ha | 1 | 3 | 4 | 5 | 6 | 7 | 11 | 12 | 15 | 16 | 19 | 20 | 21 | 25 |
| Postemergence | | | | | | | | | | | | | | |
| Barley | 0 | 0 | 10 | 90 | 30 | 0 | 100 | 100 | 10 | 90 | 90 | 0 | 100 | 0 |
| Bermudagrass | 100 | 100 | 100 | 100 | 100 | 100 | 100 | 100 | 100 | 100 | 100 | 0 | 100 | 60 |
| Blackgrass | 90 | 100 | 100 | 100 | 100 | 90 | 100 | 100 | 95 | 100 | 95 | 60 | 100 | 95 |
| Bromegrass, Downy | 60 | 5 | 5 | 50 | 5 | 0 | 98 | 85 | 15 | 70 | 90 | 50 | 85 | 15 |
| Canarygrass | 0 | 85 | 90 | 100 | 95 | - | 100 | 100 | 35 | 95 | 100 | - | 100 | 10 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 100 | 100 | 95 | 100 | 95 | 90 | 100 | 100 | 95 | 98 | 95 | - | 100 | - |
| Crabgrass, Large | 90 | 100 | 100 | 100 | 100 | 100 | 100 | 100 | 90 | 95 | 100 | | 90 100 | 80 |
| Cupgrass, Woolly | 100 | 100 | 100 | 100 | 100 | 100 | 100 | 100 | 100 | 95 | 100 | 100 | 100 | 100 |
| Foxtail, Giant | 100 | 100 | 100 | 100 | 100 | 100 | 100 | 100 | 100 | 98 | 100 | 95 | 100 | 95 |
| Foxtail, Green | 100 | 100 | 100 | 100 | 100 | 100 | 100 | 100 | 95 | 98 | 100 | 95 | 100 | 98 |
| Goosegrass | 30 | 95 | 100 | 95 | 100 | 100 | 100 | 100 | 60 | 65 | 90 | 80 | 100 | 50 |
| Johnsongrass | 90 | 80 | - | 0 | 0 | - | 100 | 100 | 80 | 100 | 100 | 100 | 100 | 30 |
| Kochia | 0 | 0 | 0 | 0 | 0 | 0 | 0 | 0 | 0 | 65 | 0 | 0 | 0 | 0 |
| Lambsquarters | 0 | 0 | 0 | 20 | 0 | 0 | 0 | 0 | 0 | 0 | 0 | 40 | 0 | 10 |
| Morningglory | 0 | 0 | 0 | 0 | 0 | 0 | 0 | 0 | 0 | 0 | 0 | 0 | 0 | 20 |
| Nutsedge, Yellow | 0 | 0 | 0 | 0 | 0 | 0 | 0 | 0 | 0 | 0 | 30 | 0 | 0 | 0 |
| Oat, Wild | 100 | 95 | 100 | 100 | 100 | 95 | 100 | 100 | 98 | 100 | 100 | 90 | 100 | 95 |
| Pigweed | 0 | 0 | 0 | 0 | 0 | 0 | 0 | 5 | 0 | 0 | 0 | 0 | 0 | 10 |
| Ragweed | 0 | 0 | 0 | 0 | 0 | 0 | 0 | 0 | 0 | 0 | 0 | 0 | 0 | 0 |
| Ryegrass, Italian | 100 | 100 | 100 | 100 | 100 | 100 | 100 | 100 | 100 | 100 | 100 | 90 | 100 | 95 |
| Soybean | 25 | 40 | 30 | 50 | 60 | 0 | 0 | 0 | 0 | 0 | 0 | 0 | 10 | 0 |
| Surinam Grass | 100 | 100 | 100 | 100 | 100 | 100 | 100 | 100 | 100 | 100 | 100 | 100 | 100 | 100 |
| Velvetleaf | 0 | 0 | 5 | 0 | 0 | 0 | 0 | 0 | 0 | 0 | 0 | 0 | 0 | 0 |
| Wheat | 90 | 90 | 100 | 100 | 100 | 80 | 100 | 95 | 60 | 98 | 98 | 60 | 100 | 0 |
| Windgrass | 100 | 100 | 100 | 100 | 95 | 85 | 100 | 100 | 90 | 100 | 100 | 90 | 100 | 98 |

| Table B | Compounds | | | | | | | | | | | | | |
|---|---|---|---|---|---|---|---|---|---|---|---|---|---|---|
| 62 g ai/ha | 26 | 28 | 32 | 33 | 34 | 35 | 36 | 37 | 38 | 39 | 40 | 41 | 43 | 44 |
| Postemergence | | | | | | | | | | | | | | |
| Barley | 0 | - | 0 | 30 | 15 | 35 | 15 | 15 | 10 | 70 | 70 | 0 | 50 | 20 |
| Bermudagrass | 0 | 100 | 100 | 100 | 100 | 100 | 100 | 100 | 100 | 95 | 98 | 95 | - | 100 |
| Blackgrass | 80 | 100 | 70 | 95 | 90 | 90 | 80 | 90 | 98 | 100 | 90 | 95 | 100 | 98 |
| Bromegrass, Downy | 60 | 20 | 60 | 80 | 80 | 60 | 50 | 75 | 60 | 80 | 100 | 85 | 65 | 90 |
| Canarygrass | - | - | 30 | - | 20 | 45 | 10 | 40 | 30 | 50 | 80 | 0 | 95 | - |
| Chickweed | 0 | 0 | 20 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 100 | 40 | 0 | 100 | 98 | 100 | 40 | 100 | 100 | 100 | 100 | 95 | 100 | 95 |
| Crabgrass, Large | 80 | 98 | 98 | 100 | 100 | 100 | 100 | 100 | 100 | 100 | 100 | 100 | 85 | 98 |
| Cupgrass, Woolly | 98 | 95 | 100 | 100 | 100 | 100 | 95 | 100 | 100 | 100 | 100 | 100 | 95 | 100 |
| Foxtail, Giant | 100 | 100 | 100 | 100 | 100 | 100 | 60 | 100 | 100 | 100 | 100 | 100 | 100 | 100 |
| Foxtail, Green | 98 | 100 | 95 | 100 | 100 | 95 | 70 | 85 | 98 | 100 | 100 | 100 | 100 | 100 |
| Goosegrass | 20 | 90 | 70 | 100 | 80 | 75 | 100 | 100 | 100 | 100 | 100 | 98 | 85 | 90 |
| Johnsongrass | 20 | 98 | 98 | 90 | 100 | 95 | 100 | 100 | 100 | 95 | 100 | 75 | 100 | 100 |
| Kochia | 0 | 0 | 0 | 0 | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 100 | 98 | 95 | 98 | 100 | 95 | 90 | 98 | 100 | 100 | 100 | 100 | 100 | 100 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 100 | 98 | 95 | 100 | 100 | 98 | 95 | 100 | 98 | 100 | 100 | 100 | 100 | 100 |
| Soybean | 0 | 20 | 0 | 0 | 0 | 0 | 0 | 0 | 0 | 0 | 0 | 0 | 0 | 0 |
| Surinam Grass | 100 | 90 | 98 | 100 | 100 | 100 | 100 | 100 | 100 | 100 | 100 | 100 | 100 | 100 |
| Velvetleaf | 0 | 0 | 0 | 5 | 0 | 0 | 0 | 0 | 0 | 0 | 0 | 0 | 0 | 0 |
| Wheat | 5 | 90 | - | 98 | 90 | 85 | 85 | 95 | 95 | 98 | 100 | 85 | 100 | 95 |
| Windgrass | 95 | 85 | 50 | 70 | 80 | 40 | 65 | 85 | 80 | 100 | 100 | 85 | 100 | 100 |

| Table B | Compounds | | | | | | | | | | | | | |
|---|---|---|---|---|---|---|---|---|---|---|---|---|---|---|
| 62 g ai/ha | 45 | 48 | 52 | 53 | 54 | 56 | 57 | 59 | 61 | 66 | 67 | 68 | 69 | 72 |
| Postemergence | | | | | | | | | | | | | | |
| Barley | 5 | 10 | 0 | 0 | 0 | 75 | 40 | 50 | 90 | - | - | 0 | 0 | 95 |
| Bermudagrass | 98 | 100 | 100 | 100 | 100 | 100 | 100 | 20 | 100 | 100 | 75 | 0 | 98 | 80 |
| Blackgrass | 90 | 95 | 95 | 95 | 70 | 98 | 95 | 90 | 100 | 100 | 100 | 85 | 90 | 100 |
| Bromegrass, Downy | 90 | 90 | 70 | 90 | 70 | 0 | 60 | 10 | 98 | 98 | 100 | 0 | 45 | 30 |
| Canarygrass | 0 | - | 5 | 0 | 0 | 95 | 60 | 70 | 85 | - | - | 10 | 0 | 95 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 95 | 100 | 98 | 100 | 100 | 95 | 100 | 90 | 100 | 100 | 100 | 95 | 20 | 100 |
| Crabgrass, Large | 100 | 100 | 100 | 100 | 98 | 100 | 100 | 60 | 100 | 100 | 100 | 85 | 100 | 100 |
| Cupgrass, Woolly | 100 | 100 | 100 | 100 | 100 | 100 | 100 | 85 | 100 | 100 | 100 | 60 | 100 | 90 |
| Foxtail, Giant | 100 | 100 | 100 | 100 | 100 | 100 | 100 | 95 | 100 | 100 | 100 | 90 | 100 | 100 |
| Foxtail, Green | 100 | 100 | 100 | 100 | 100 | 100 | 100 | 95 | 100 | 100 | 100 | 95 | 95 | 100 |
| Goosegrass | 95 | 95 | 85 | 100 | 98 | 98 | 90 | 0 | 100 | 98 | 90 | 5 | 90 | 35 |
| Johnsongrass | 70 | 100 | 95 | 100 | 100 | 100 | 100 | 80 | 100 | 100 | 100 | 30 | 75 | 100 |
| Kochia | 0 | 0 | 0 | 0 | 0 | 0 | 0 | 0 | 45 | 0 | 0 | 0 | 98 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 100 | 100 | 100 | 98 | 98 | 90 | 98 | 80 | 100 | 100 | 100 | 40 | 90 | 100 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 100 | 100 | 100 | 100 | 100 | 65 | 95 | 85 | 100 | 100 | 100 | 95 | 100 | 100 |
| Soybean | 0 | 0 | 0 | 0 | 0 | 0 | 0 | 0 | - | 0 | 15 | 0 | 20 | 0 |
| Surinam Grass | 100 | 100 | 100 | 100 | 100 | 100 | 100 | 100 | 100 | 100 | 100 | 80 | 95 | 100 |
| Velvetleaf | 0 | 0 | 0 | 0 | 0 | 0 | 0 | 0 | 0 | 0 | 0 | 0 | 0 | 0 |
| Wheat | 98 | 98 | 85 | 85 | 60 | 90 | 90 | 50 | 100 | 100 | 100 | 0 | 80 | 95 |
| Windgrass | 65 | 80 | 80 | 60 | 80 | 20 | 70 | 50 | 100 | 100 | 100 | 35 | 40 | 100 |

| 62 g ai/ha | 73 | 74 | 75 | 76 | 78 | 79 | 80 | 81 | 82 | 83 | | | | |
|---|---|---|---|---|---|---|---|---|---|---|---|---|---|---|
| Postemergence | | | | | | | | | | | | | | |
| Barley | 70 | 95 | 60 | 25 | 95 | 40 | 98 | 98 | 100 | 45 | | | | |
| Bermudagrass | 10 | 98 | 90 | 90 | 100 | 100 | 100 | 100 | 100 | 90 | | | | |
| Blackgrass | 100 | 100 | 95 | 100 | 100 | 98 | 100 | 100 | 100 | 100 | | | | |
| Bromegrass, Downy | 60 | 60 | 50 | 20 | 100 | 50 | - | 70 | 100 | 85 | | | | |
| Canarygrass | 100 | 100 | 50 | 98 | 100 | 95 | 100 | 100 | 100 | 90 | | | | |
| Chickweed | 0 | 0 | 0 | 0 | 0 | 0 | 0 | 0 | 90 | 0 | | | | |
| Cocklebur | 0 | 0 | 0 | 0 | 0 | 0 | 0 | 0 | 20 | 0 | | | | |
| Corn | 100 | 100 | 100 | 100 | 100 | 35 | 100 | 100 | 100 | 100 | | | | |
| Crabgrass, Large | 100 | 100 | 60 | 100 | 100 | 98 | 100 | 100 | 100 | 100 | | | | |
| Cupgrass, Woolly | 100 | 98 | 75 | 90 | 100 | 80 | 100 | 98 | 100 | 100 | | | | |
| Foxtail, Giant | 100 | 98 | 85 | 98 | 100 | 90 | 100 | 100 | 100 | 100 | | | | |
| Foxtail, Green | 100 | 100 | 95 | 100 | 100 | 98 | 100 | 98 | 100 | 100 | | | | |
| Goosegrass | 90 | 85 | 75 | 75 | 100 | 90 | 98 | 85 | 90 | 60 | | | | |
| Johnsongrass | 100 | 100 | 100 | 98 | 100 | 100 | 100 | 100 | 100 | 100 | | | | |
| Kochia | 0 | 0 | 0 | 0 | - | - | - | - | - | 0 | | | | |
| Lambsquarters | 0 | 0 | 0 | 0 | 0 | 0 | 0 | 0 | 0 | 0 | | | | |
| Morningglory | 0 | 0 | 0 | 0 | 0 | 0 | 0 | 0 | 55 | 0 | | | | |
| Nutsedge, Yellow | 0 | 0 | 0 | 0 | 0 | 0 | 0 | 0 | 0 | 0 | | | | |
| Oat, Wild | 100 | 100 | 98 | 100 | 100 | 98 | 100 | 100 | 100 | 100 | | | | |
| Pigweed | 0 | 0 | 0 | 0 | 0 | 0 | 0 | 0 | 0 | 20 | | | | |
| Ragweed | 0 | 0 | 0 | 0 | 60 | 0 | 0 | 20 | 85 | 0 | | | | |
| Ryegrass, Italian | 100 | 100 | 98 | 100 | 100 | 90 | 100 | 100 | 100 | 100 | | | | |
| Soybean | 10 | 0 | 0 | 0 | 25 | 15 | 20 | 20 | 70 | 0 | | | | |
| Surinam Grass | 100 | 100 | 85 | 100 | 100 | 85 | 100 | 100 | 100 | 100 | | | | |
| Velvetleaf | 0 | 0 | 0 | 0 | 0 | 0 | 0 | 0 | 55 | 20 | | | | |
| Wheat | 95 | 95 | 85 | 35 | 100 | 85 | 98 | 100 | 100 | 95 | | | | |
| Windgrass | | 95 100 | 90 | 90 | 80 | 70 | 70 | 95 | 100 | 100 | | | | |

| Table B | Compounds | | | | | | | | | | | | | |
|---|---|---|---|---|---|---|---|---|---|---|---|---|---|---|
| 31 g ai/ha | 1 | 3 | 4 | 5 | 6 | 7 | 11 | 12 | 15 | 16 | 19 | 20 | 21 | 25 |
| Postemergence | | | | | | | | | | | | | | |
| Barley | 0 | 0 | 0 | 30 | 30 | - | 95 | 95 | 10 | 90 | 5 | 0 | - | 0 |
| Bermudagrass | 100 | 100 | 100 | 100 | 100 | 100 | 100 | 100 | 70 | 65 | 100 | 0 | 80 | 60 |
| Blackgrass | 20 | 100 | 90 | 95 | 100 | 85 | 100 | 100 | 90 | 98 | 90 | - | 100 | 75 |
| Bromegrass, Downy | 30 | 5 | 0 | 5 | 5 | 0 | 90 | 85 | 10 | 60 | 75 | 40 | 80 | 10 |
| Canarygrass | 0 | 85 | 80 | 80 | 95 | 90 | 100 | 100 | 35 | 70 | 20 | 50 | - | 0 |
| Chickweed | 0 | 0 | 0 | 0 | 0 | 0 | 0 | 0 | 0 | 0 | - | 0 | 0 | 0 |
| Cocklebur | 0 | 0 | 0 | 0 | 0 | 0 | 0 | 0 | 0 | 0 | 0 | 0 | 0 | 0 |
| Corn | 10 | 100 | 90 | 90 | 95 | 90 | 100 | 100 | 90 | 98 | 0 | 95 | 100 | 95 |
| Crabgrass, Large | 80 | 100 | 100 | 100 | 100 | 100 | 100 | 100 | 90 | 95 | 95 | 85 | 100 | 75 |
| Cupgrass, Woolly | 95 | 100 | 100 | 95 | 100 | 100 | 100 | 100 | - | 80 | 100 | 90 | 100 | 85 |
| Foxtail, Giant | 100 | 100 | 100 | 100 | 100 | 98 | 100 | 100 | 95 | 85 | 100 | 95 | 100 | 85 |
| Foxtail, Green | 95 | 90 | 95 | 95 | 100 | 75 | 100 | 100 | 95 | 95 | 100 | 85 | 100 | 80 |
| Goosegrass | 30 | 95 | 95 | 95 | 95 | 95 | 100 | 100 | 45 | 60 | 80 | 65 | 95 | 0 |
| Johnsongrass | 60 | 0 | 80 | 0 | 0 | - | 100 | 100 | 70 | 98 | 100 | 100 | 90 | 0 |
| Kochia | 0 | 0 | 0 | 0 | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 | 0 | 0 | 0 | 0 |
| Morningglory | - | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | - | 0 | 0 | 0 | 0 | 0 |
| Oat, Wild | 90 | 90 | 98 | 95 | 100 | 95 | 100 | 100 | 95 | 95 | 100 | 90 | 100 | 70 |
| Pigweed | 0 | 0 | 0 | 0 | 0 | 0 | 0 | 0 | 0 | 0 | 0 | 0 | 0 | 0 |
| Ragweed | 0 | - | 0 | 0 | 0 | 0 | 0 | 0 | 0 | 0 | 0 | 0 | 0 | 0 |
| Ryegrass, Italian | 95 | 95 | 100 | 100 | 100 | 95 | 100 | 100 | 98 | 100 | 100 | 90 | 100 | 85 |
| Soybean | 0 | 0 | 20 | 0 | 0 | 0 | 0 | 0 | 0 | 0 | 0 | 0 | 0 | 0 |
| Surinam Grass | 100 | 100 | 100 | 100 | 100 | 100 | 100 | 100 | 100 | 90 | 100 | 90 | 100 | 95 |
| Velvetleaf | 0 | 0 | 5 | 0 | 0 | 0 | 0 | 0 | 0 | 0 | 0 | 0 | 0 | 0 |
| Wheat | 45 | 80 | 90 | 90 | 90 | 60 | 100 | 95 | 5 | 95 | 85 | 20 | 90 | 0 |
| Windgrass | 95 | 80 | 90 | 95 | 90 | 80 | 100 | 100 | 90 | 90 | 100 | 85 | 100 | 60 |

| Table B | Compounds | | | | | | | | | | | | | |
|---|---|---|---|---|---|---|---|---|---|---|---|---|---|---|
| 31 g ai/ha | 26 | 28 | 32 | 33 | 34 | 35 | 36 | 37 | 38 | 39 | 40 | 41 | 43 | 44 |
| Postemergence | | | | | | | | | | | | | | |
| Barley | 0 | 50 | 0 | 30 | 0 | 5 | 15 | 5 | 10 | 40 | 70 | 0 | - | 20 |
| Bermudagrass | 0 | 100 | 100 | 100 | 100 | 100 | 100 | 100 | 100 | 75 | 65 | 25 | 65 | 95 |
| Blackgrass | 80 | 95 | 70 | 80 | 75 | 70 | 50 | 90 | 80 | 95 | 90 | 95 | 95 | 98 |
| Bromegrass, Downy | 30 | 10 | 45 | 70 | 50 | 20 | 40 | 55 | 50 | 45 | 80 | 60 | 60 | 70 |
| Canarygrass | 60 | 50 | 10 | 80 | 20 | 5 | 10 | 10 | 10 | 40 | 80 | 0 | 50 | 90 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 100 | 0 | 0 | 100 | 98 | 100 | 15 | 100 | 100 | 98 | 100 | 95 | 85 | 95 |
| Crabgrass, Large | 80 | 80 | 95 | 90 | 95 | 80 | 90 | 85 | 90 | 98 | 98 | 100 | 75 | 90 |
| Cupgrass, Woolly | 98 | 95 | 100 | 100 | 100 | 100 | 95 | 100 | 100 | 100 | 100 | 100 | 95 | 100 |
| Foxtail, Giant | 95 | 95 | 98 | 100 | 100 | 100 | 60 | 85 | 50 | 100 | 100 | 100 | 98 | 100 |
| Foxtail, Green | 80 | 98 | 90 | 100 | 100 | 95 | 60 | 80 | 80 | 85 | 100 | 100 | 100 | 95 |
| Goosegrass | 0 | 80 | 70 | 90 | 70 | 70 | 100 | 100 | 100 | 85 | 80 | 85 | 75 | 75 |
| Johnsongrass | 20 | 75 | 98 | 85 | 98 | 95 | 95 | 95 | 90 | 95 | 98 | 60 | 80 | 98 |
| Kochia | 0 | 0 | 0 | 0 | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 90 | 95 | 95 | 95 | 95 | 85 | 60 | 95 | 95 | 100 | 100 | 100 | 100 | 100 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 85 | 55 | 95 | 100 | 95 | 90 | 65 | 98 | 85 | 95 | 100 | 100 | 100 | 100 |
| Soybean | 0 | 0 | 0 | 0 | 0 | 0 | 0 | 0 | 0 | 0 | 0 | 0 | 0 | 0 |
| Surinam Grass | 100 | 70 | 85 | 100 | 100 | 100 | 100 | 100 | 100 | 100 | 100 | 100 | 95 | 100 |
| Velvetleaf | 0 | 0 | 0 | 0 | 0 | 0 | 0 | 0 | 0 | 0 | 0 | 0 | 0 | 0 |
| Wheat | 5 | 85 | 70 | 85 | 40 | 50 | 40 | 80 | 85 | 80 | 98 | 85 | 80 | 90 |
| Windgrass | 80 | 50 | 30 | 60 | 60 | 40 | 40 | 65 | 50 | 80 | 90 | 70 | 90 | 100 |

| Table B | Compounds | | | | | | | | | | | | | |
|---|---|---|---|---|---|---|---|---|---|---|---|---|---|---|
| 31 g ai/ha | 45 | 48 | 52 | 53 | 54 | 56 | 57 | 59 | 61 | 66 | 67 | 68 | 69 | 72 |
| Postemergence | | | | | | | | | | | | | | |
| Barley | 5 | - | 0 | 0 | 0 | 50 | 0 | 20 | 90 | 100 | 95 | 0 | 0 | 0 |
| Bermudagrass | 98 | 100 | 100 | 100 | 100 | 95 | 98 | 0 | 95 | 100 | 60 | 0 | 95 | 60 |
| Blackgrass | 85 | 85 | 95 | 95 | 70 | 95 | 80 | 90 | 100 | 100 | 100 | 50 | 35 | 98 |
| Bromegrass, Downy | 90 | 60 | 40 | 60 | 50 | 0 | 50 | 5 | 98 | 98 | 80 | 0 | 15 | 30 |
| Canarygrass | 0 | 90 | 0 | 0 | 0 | 90 | 0 | 55 | 85 | 95 | 95 | 0 | 0 | 30 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 95 | 100 | 98 | 100 | 25 | 95 | 98 | 85 | 100 | 90 | 100 | 30 | 0 | 90 |
| Crabgrass, Large | 100 | 90 | 100 | 90 | 98 | 95 | 98 | 55 | 100 | 98 | 100 | 80 | 98 | 100 |
| Cupgrass, Woolly | 100 | 100 | 95 | 100 | 98 | 100 | 100 | 65 | 100 | 100 | 100 | 30 | 95 | 90 |
| Foxtail, Giant | 100 | 100 | 100 | 100 | 100 | 98 | 98 | 90 | 100 | 100 | 100 | 65 | 100 | 100 |
| Foxtail, Green | 100 | 100 | 98 | 100 | 95 | 90 | 100 | 90 | 100 | 100 | 98 | 60 | 90 | 100 |
| Goosegrass | 80 | 75 | 80 | 65 | 70 | 75 | 85 | 0 | 100 | 85 | 85 | 0 | 75 | 35 |
| Johnsongrass | 65 | 100 | 90 | 98 | 80 | 85 | 90 | 60 | 100 | 100 | 100 | 0 | 70 | - |
| Kochia | 0 | 0 | 0 | 0 | 0 | 0 | 0 | 0 | 35 | 0 | 0 | 0 | 95 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 100 | 98 | 95 | 95 | 98 | 90 | 95 | 70 | 100 | 100 | 100 | 5 | 90 | 100 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 100 | 100 | 95 | 100 | 95 | 60 | 95 | 85 | 100 | 100 | 100 | 80 | 98 | 100 |
| Soybean | 0 | 0 | 0 | 0 | 0 | 0 | 0 | 0 | - | 0 | 0 | 0 | 0 | 0 |
| Surinam Grass | 98 | 100 | 100 | 100 | 98 | 98 | 100 | 90 | 100 | 100 | 100 | 80 | 80 | 98 |
| Velvetleaf | 0 | 0 | 0 | 0 | 0 | 0 | 0 | 0 | 0 | 0 | 0 | 0 | 0 | 0 |
| Wheat | 85 | 95 | 70 | 85 | 60 | 80 | 90 | 40 | 98 | 100 | 100 | 0 | 40 | 85 |
| Windgrass | 60 | 60 | 60 | 40 | 20 | 20 | 55 | 10 | 100 | 100 | 100 | 0 | 10 | 100 |

| Table B | Compounds | | | | | | | | | | | | | |
|---|---|---|---|---|---|---|---|---|---|---|---|---|---|---|
| 31 g ai/ha | 73 | 74 | 75 | 76 | 78 | 79 | 80 | 81 | 82 | 83 | | | | |
| Postemergence | | | | | | | | | | | | | | |
| Barley | 40 | 95 | 15 | 5 | 95 | 30 | 95 | 70 | 100 | 45 | | | | |
| Bermudagrass | 10 | 95 | 85 | 85 | 100 | 100 | 100 | 100 | 100 | 85 | | | | |
| Blackgrass | 100 | 98 | 65 | 100 | 100 | 90 | 95 | 98 | 100 | 95 | | | | |
| Bromegrass, Downy | 30 | 50 | 30 | 0 | 95 | 30 | 90 | 50 | 100 | 75 | | | | |
| Canarygrass | 90 | 100 | 0 | 90 | 100 | 85 | 100 | 98 | 100 | 90 | | | | |
| Chickweed | 0 | 0 | 0 | 0 | 0 | 0 | 0 | 0 | 0 | 0 | | | | |
| Cocklebur | 0 | 0 | 0 | 0 | 0 | 0 | 0 | 0 | 0 | 0 | | | | |
| Corn | 100 | 100 | 90 | 100 | 100 | 20 | 100 | 100 | 100 | 98 | | | | |
| Crabgrass, Large | 100 | 98 | 30 | 95 | 100 | 85 | 100 | 100 | 100 | 100 | | | | |
| Cupgrass, Woolly | 100 | 90 | 65 | 85 | 100 | 75 | 100 | 95 | 95 | 98 | | | | |
| Foxtail, Giant | 90 | 95 | 80 | 95 | 100 | 85 | 100 | 98 | 100 | 100 | | | | |
| Foxtail, Green | 70 | 98 | 80 | 95 | 80 | 98 | 100 | 95 | 100 | 98 | | | | |
| Goosegrass | 90 | 75 | 60 | 70 | 90 | 85 | 85 | 80 | 85 | 55 | | | | |
| Johnsongrass | 90 | 100 | 98 | 95 | 100 | 100 | 95 | - | 100 | 98 | | | | |
| Kochia | 0 | 0 | 0 | 0 | - | - | - | - | - | 0 | | | | |
| Lambsquarters | 0 | 0 | 0 | 0 | 0 | 0 | 0 | 0 | 0 | 0 | | | | |
| Morningglory | 0 | 0 | 0 | 0 | 0 | 0 | 0 | 0 | 0 | 0 | | | | |
| Nutsedge, Yellow | 0 | 0 | 0 | 0 | 0 | 0 | 0 | 0 | 0 | 0 | | | | |
| Oat, Wild | 100 | 100 | 85 | 100 | 100 | 60 | 100 | 100 | 95 | 100 | | | | |
| Pigweed | 0 | 0 | 0 | 0 | 0 | 0 | 0 | 0 | 0 | 0 | | | | |
| Ragweed | 0 | 0 | 0 | 0 | 0 | 0 | 0 | 0 | 65 | 0 | | | | |
| Ryegrass, Italian | 100 | 100 | 95 | 100 | 100 | 80 | 100 | 100 | 100 | 100 | | | | |
| Soybean | 10 | 0 | 0 | 0 | 20 | 15 | 15 | 15 | 55 | 0 | | | | |
| Surinam Grass | 90 | 100 | 75 | 100 | 100 | 75 | 100 | 100 | 100 | 100 | | | | |
| Velvetleaf | 0 | 0 | 0 | 0 | 0 | 0 | 0 | 0 | 0 | 0 | | | | |
| Wheat | 90 | 90 | 70 | 30 | 98 | 60 | 95 | 95 | 100 | 90 | | | | |
| Windgrass | 80 | 100 | 70 | 80 | 60 | 50 | 60 | 85 | 100 | 100 | | | | |

| 16 g ai/ha | 11 | 12 | 19 | 20 | 21 | 25 | 26 | 28 | 32 | 33 | 34 | 35 | 36 | 37 |
|---|---|---|---|---|---|---|---|---|---|---|---|---|---|---|
| Postemergence | | | | | | | | | | | | | | |
| Barley | 90 | 80 | 5 | 0 | 60 | 0 | 0 | 0 | 0 | 25 | 0 | 0 | 0 | 0 |
| Bermudagrass | 100 | 100 | 100 | 0 | 60 | 0 | 0 | 80 | 65 | 100 | 100 | 65 | 80 | 85 |
| Blackgrass | 100 | 100 | 85 | 50 | 95 | 40 | 50 | 70 | 35 | 70 | 75 | 60 | 50 | 75 |
| Bromegrass, Downy | 85 | 85 | 75 | 0 | 55 | 0 | 10 | 5 | 10 | 50 | 50 | 20 | 5 | 55 |
| Canarygrass | 80 | 100 | 20 | 30 | 90 | 0 | 10 | 0 | 10 | 35 | 10 | 0 | 0 | 10 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 100 | 100 | 0 | 90 | 100 | 50 | 0 | 0 | 0 | 95 | 98 | 90 | 15 | 95 |
| Crabgrass, Large | 98 | 100 | 85 | 70 | 100 | 60 | 60 | 75 | 85 | 90 | 80 | 70 | 65 | 70 |
| Cupgrass, Woolly | 95 | 100 | 90 | 90 | 100 | 80 | 95 | 55 | 85 | 100 | 90 | 95 | 90 | 98 |
| Foxtail, Giant | 100 | 100 | 100 | 90 | 100 | 85 | 95 | 80 | 80 | 100 | 90 | 98 | 35 | 70 |
| Foxtail, Green | 85 | 85 | 100 | 60 | 100 | 70 | 80 | 65 | 60 | 85 | 90 | 75 | 50 | 75 |
| Goosegrass | 100 | 100 | 80 | 20 | 80 | 0 | 0 | 75 | 65 | 70 | 55 | 40 | 100 | 100 |
| Johnsongrass | 100 | 100 | 80 | 75 | 90 | 0 | 0 | 65 | 70 | 70 | 95 | 60 | 50 | 55 |
| Kochia | 0 | 0 | 0 | 0 | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 100 | 100 | 98 | 90 | 100 | 55 | 90 | 80 | 85 | 95 | 90 | 70 | 50 | 95 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 100 | 100 | 98 | 55 | 100 | 80 | 85 | 55 | 85 | 80 | 85 | 40 | 30 | 90 |
| Soybean | 0 | 0 | 0 | 0 | 0 | 0 | 0 | 0 | 0 | 0 | 0 | 0 | 0 | 0 |
| Surinam Grass | 100 | 100 | 100 | 90 | 100 | 60 | 85 | 65 | 70 | 100 | 80 | 95 | 85 | 100 |
| Velvetleaf | 0 | 0 | 0 | 0 | 0 | 0 | 0 | 0 | 0 | 0 | 0 | 0 | 0 | 0 |
| Wheat | 90 | 90 | 60 | 20 | 85 | 0 | 0 | 40 | 30 | 70 | 40 | 35 | 5 | 50 |
| Windgrass | 100 | 100 | 95 | 70 | 98 | 60 | 80 | 35 | 0 | 50 | 10 | 0 | 0 | 60 |

| Table B | Compounds | | | | | | | | | | | | | |
|---|---|---|---|---|---|---|---|---|---|---|---|---|---|---|
| 16 g ai/ha | 38 | 39 | 40 | 41 | 43 | 44 | 45 | 48 | 52 | 53 | 54 | 56 | 57 | 59 |
| Postemergence | | | | | | | | | | | | | | |
| Barley | 0 | 5 | 40 | 0 | 50 | 20 | 5 | 0 | 0 | 0 | 0 | 40 | 0 | 0 |
| Bermudagrass | 85 | 60 | 60 | 0 | 45 | 90 | 95 | 98 | 98 | 100 | 100 | 70 | 85 | 0 |
| Blackgrass | 40 | 0 | 90 | 90 | 85 | 90 | 20 | 85 | 70 | 60 | 45 | 65 | 80 | 60 |
| Bromegrass, Downy | 45 | 10 | 60 | 50 | 45 | 60 | 60 | 50 | 30 | 45 | 50 | 0 | 20 | 5 |
| Canarygrass | 0 | 0 | 30 | 0 | 50 | 90 | 0 | 0 | 0 | 0 | 0 | 85 | 0 | 40 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 95 | 85 | 0 | 85 | 85 | 95 | 90 | 95 | 98 | 100 | 0 | 85 | 0 | 85 |
| Crabgrass, Large | 70 | 90 | 98 | 85 | 70 | 65 | 80 | 85 | 85 | 85 | 85 | 85 | 95 | 45 |
| Cupgrass, Woolly | 98 | 95 | 98 | 100 | 90 | 98 | 95 | 85 | 90 | 100 | 90 | 100 | 90 | 60 |
| Foxtail, Giant | 20 | 98 | 100 | 100 | 98 | 98 | 98 | 95 | 98 | 95 | 98 | 98 | 95 | 85 |
| Foxtail, Green | 75 | 80 | 95 | 100 | 75 | 95 | 95 | 98 | 95 | 95 | 95 | 90 | 95 | 90 |
| Goosegrass | 100 | 65 | 75 | 80 | 70 | 60 | 70 | 65 | 65 | 60 | 65 | 65 | 80 | 0 |
| Johnsongrass | 65 | 55 | 0 | 50 | 0 | 95 | 65 | 75 | 80 | 70 | 25 | 65 | 65 | 0 |
| Kochia | 0 | 0 | 0 | 0 | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 90 | 95 | 100 | 100 | 100 | 95 | 98 | 95 | 90 | 90 | 90 | 85 | 85 | 60 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 85 | 90 | 100 | 98 | 90 | 95 | 90 | 90 | 80 | 90 | 95 | 40 | 95 | 80 |
| Soybean | 0 | 0 | 0 | 0 | 0 | 0 | 0 | 0 | 0 | 0 | 0 | 0 | 0 | 0 |
| Surinam Grass | 90 | 100 | 100 | 100 | 95 | 100 | 85 | 90 | 85 | 98 | 95 | 75 | 75 | 85 |
| Velvetleaf | 0 | 0 | 0 | 0 | 0 | 0 | 0 | 0 | 0 | 0 | 0 | 0 | 0 | 0 |
| Wheat | 45 | 20 | 70 | 85 | 70 | 90 | 85 | 50 | 50 | 55 | 30 | 65 | 45 | 15 |
| Windgrass | 0 | 40 | 80 | 60 | 70 | 85 | 35 | 40 | 0 | 20 | 20 | 0 | 50 | 0 |

| Table B | Compounds | | | | | | | | | | | | | |
|---|---|---|---|---|---|---|---|---|---|---|---|---|---|---|
| 16 g ai/ha | 61 | 66 | 67 | 68 | 69 | 72 | 73 | 74 | 75 | 76 | 78 | 79 | 80 | 81 |
| Postemergence | | | | | | | | | | | | | | |
| Barley | 90 | 98 | 85 | 0 | 0 | 0 | 40 | 40 | 0 | 0 | 90 | 10 | 60 | 15 |
| Bermudagrass | 70 | 98 | 0 | 0 | 65 | 50 | 0 | 85 | 80 | 80 | 98 | 80 | 95 | 95 |
| Blackgrass | 100 | 100 | 100 | 0 | 35 | 95 | 100 | 95 | 50 | 85 | 95 | 50 | 85 | 90 |
| Bromegrass, Downy | 90 | 95 | 80 | 0 | 0 | 30 | 0 | 40 | 10 | 0 | 85 | 0 | 45 | 45 |
| Canarygrass | 85 | 90 | 80 | 0 | 0 | 30 | 90 | 90 | 0 | 60 | 90 | 55 | 98 | - |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 100 | 75 | 90 | 0 | 0 | 90 | 100 | 100 | 50 | 85 | 100 | 0 | 100 | 100 |
| Crabgrass, Large | 100 | 85 | 98 | 60 | 75 | 100 | 70 | 90 | 0 | 85 | 100 | 80 | 100 | 98 |
| Cupgrass, Woolly | 100 | 95 | 100 | 0 | 90 | 80 | 98 | 90 | 65 | 65 | 100 | 65 | 95 | 85 |
| Foxtail, Giant | 100 | 98 | 95 | 60 | 85 | 100 | 70 | 85 | 70 | 85 | 100 | 80 | 100 | 85 |
| Foxtail, Green | 100 | 100 | 70 | 50 | 80 | 98 | 70 | 95 | 40 | 85 | 60 | 85 | 60 | 90 |
| Goosegrass | 100 | 75 | 70 | 0 | 70 | 30 | 90 | 65 | 50 | 60 | 85 | 80 | 75 | 70 |
| Johnsongrass | 100 | 100 | 98 | 0 | 65 | 75 | 85 | 98 | 80 | 75 | 100 | 90 | 90 | 95 |
| Kochia | 0 | 0 | 0 | 0 | 0 | 0 | 0 | 0 | 0 | 0 | - | - | - | - |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 100 | 100 | 100 | 0 | 40 | 100 | 95 | 98 | 30 | 95 | 100 | 45 | 100 | 95 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 100 | 100 | 100 | 10 | 55 | 100 | 70 | 100 | 50 | 98 | 98 | 70 | 98 | 90 |
| Soybean | - | 0 | 0 | 0 | 0 | 0 | 0 | 0 | 0 | 0 | 15 | 0 | 0 | 0 |
| Surinam Grass | 100 | 98 | 85 | 35 | 75 | 95 | 90 | 100 | 70 | 90 | 100 | 70 | 100 | 98 |
| Velvetleaf | 0 | 0 | 0 | 0 | 0 | 0 | 0 | 0 | 0 | 0 | 0 | 0 | 0 | 0 |
| Wheat | 95 | 100 | 100 | 0 | 35 | 85 | 60 | 70 | 20 | 30 | 85 | 20 | 85 | 55 |
| Windgrass | 100 | 100 | 100 | 0 | 0 | 100 | 60 | 90 | 50 | 80 | 50 | 50 | 40 | 80 |

| Table B | Compounds | | | | | | Table B | | | | Compounds | | | |
|---|---|---|---|---|---|---|---|---|---|---|---|---|---|---|
| 16 g ai/ha | 82 | 83 | | | | | 16 g ai/ha | | | | 82 | | 83 | |
| Postemergence | | | | | | | Postemergence | | | | | | | |
| Barley | 98 | 0 | | | | | Kochia | | | | - | | 0 | |
| Bermudagrass | 100 | 65 | | | | | Lambsquarters | | | | 0 | | 0 | |
| Blackgrass | 98 | 95 | | | | | Morningglory | | | | 0 | | 0 | |
| Bromegrass, Downy | 95 | 55 | | | | | Nutsedge, Yellow | | | | 0 | | 0 | |
| Canarygrass | 95 | - | | | | | Oat, Wild | | | | 95 | | 100 | |
| Chickweed | 0 | 0 | | | | | Pigweed | | | | 0 | | 0 | |
| Cocklebur | 0 | 0 | | | | | Ragweed | | | | 40 | | 0 | |
| Corn | 100 | 95 | | | | | Ryegrass, Italian | | | | 98 | | 98 | |
| Crabgrass, Large | 100 | 95 | | | | | Soybean | | | | 45 | | 0 | |
| Cupgrass, Woolly | 90 | 95 | | | | | Surinam Grass | | | | 95 | | 100 | |
| Foxtail, Giant | 100 | 90 | | | | | Velvetleaf | | | | 0 | | 0 | |
| Foxtail, Green | 100 | 65 | | | | | Wheat | | | | 98 | | 55 | |
| Goosegrass | 65 | 45 | | | | | Windgrass | | | | 100 | | 98 | |
| Johnsongrass | 100 | 70 | | | | | | | | | | | | |

| Table B | Compounds | | | | | | | | | | | | | |
|---|---|---|---|---|---|---|---|---|---|---|---|---|---|---|
| 8 g ai/ha | 19 | 20 | 21 | 25 | 26 | 28 | 32 | 33 | 34 | 35 | 36 | 37 | 38 | 39 |
| Postemergence | | | | | | | | | | | | | | |
| Barley | 0 | 0 | 0 | 0 | 0 | 0 | 0 | 0 | 0 | 0 | 0 | 0 | 0 | 0 |
| Bermudagrass | 80 | 0 | 40 | 0 | 0 | 65 | 60 | 70 | 65 | 40 | 40 | 80 | 40 | 0 |
| Blackgrass | 80 | 30 | 95 | 0 | 0 | 60 | 30 | 70 | 0 | 0 | 0 | 10 | 0 | 0 |
| Bromegrass, Downy | 10 | 0 | 55 | 0 | 0 | 0 | 5 | 5 | 20 | 0 | 0 | 0 | 30 | 5 |
| Canarygrass | 0 | 5 | 10 | 0 | 10 | 0 | 0 | 0 | 10 | 0 | 0 | 0 | 0 | 0 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 0 | 0 | 50 | 0 | 0 | 0 | 0 | 80 | 0 | 40 | 10 | 45 | 90 | 0 |
| Crabgrass, Large | 80 | 5 | 95 | 5 | 45 | 60 | 60 | 70 | 70 | 60 | 50 | 70 | 70 | 70 |
| Cupgrass, Woolly | 90 | 45 | 100 | 60 | 85 | 50 | 75 | 98 | 75 | 80 | 85 | 90 | 70 | 80 |
| Foxtail, Giant | 95 | 80 | 98 | 70 | 80 | 65 | 75 | 100 | 80 | 90 | 0 | 30 | 0 | 80 |
| Foxtail, Green | 60 | 20 | 95 | 55 | 60 | 60 | 60 | 85 | 60 | 0 | 0 | 60 | 30 | 30 |
| Goosegrass | 50 | 5 | 65 | 0 | 0 | 65 | 0 | 50 | 50 | 20 | 0 | 100 | 100 | 20 |
| Johnsongrass | 40 | 30 | 60 | 0 | 0 | 20 | 50 | 70 | 75 | 0 | 0 | 0 | 60 | 40 |
| Kochia | 0 | 0 | 0 | 0 | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 90 | 50 | 98 | 25 | 85 | 45 | 60 | 90 | 70 | 50 | 0 | 85 | 80 | 95 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 80 | 40 | 98 | 60 | 70 | 40 | 5 | 75 | 70 | 40 | 10 | 60 | 10 | 30 |
| Soybean | 0 | 0 | 0 | 0 | 0 | 0 | 0 | 0 | 0 | 0 | 0 | 0 | 0 | 0 |
| Surinam Grass | 90 | 60 | 100 | 50 | 85 | 65 | 65 | 85 | 70 | 70 | 70 | 95 | 90 | 100 |
| Velvetleaf | 0 | 0 | 0 | 0 | 0 | 0 | 0 | 0 | 0 | 0 | 0 | 0 | 0 | 0 |
| Wheat | 0 | 0 | 50 | 0 | 0 | 20 | 0 | 20 | 0 | 0 | 0 | 15 | 10 | 20 |
| Windgrass | 90 | 20 | 50 | 40 | 50 | 0 | 0 | 10 | 0 | 0 | 0 | 10 | 0 | 0 |

| Table B | Compounds | | | | | | | | | | | | | |
|---|---|---|---|---|---|---|---|---|---|---|---|---|---|---|
| 8 g ai/ha | 40 | 41 | 43 | 44 | 45 | 48 | 52 | 53 | 54 | 56 | 57 | 59 | 61 | 66 |
| Postemergence | | | | | | | | | | | | | | |
| Barley | 35 | - | 20 | 15 | 5 | 0 | 0 | 0 | 0 | 0 | 0 | 0 | 70 | 90 |
| Bermudagrass | 0 | 0 | 20 | 65 | 60 | 65 | 40 | 70 | 100 | 65 | 65 | 0 | 55 | 20 |
| Blackgrass | 85 | 85 | 85 | 85 | 0 | 40 | 50 | 30 | 45 | 0 | 40 | 0 | 85 | 90 |
| Bromegrass, Downy | 50 | 30 | 40 | 40 | 30 | 5 | 30 | 10 | 5 | 0 | 0 | 0 | 80 | 80 |
| Canarygrass | 20 | - | 30 | 50 | 0 | 0 | 0 | 0 | 0 | 50 | 0 | 0 | 80 | 40 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 0 | 85 | 80 | 85 | 85 | 90 | 85 | 20 | 0 | 75 | 0 | 0 | 75 | 65 |
| Crabgrass, Large | 70 | 70 | 65 | 60 | 65 | 70 | 65 | 65 | 65 | 65 | 75 | 20 | 98 | 70 |
| Cupgrass, Woolly | 98 | 95 | 80 | 98 | 85 | 75 | 75 | 85 | 80 | 95 | 65 | 55 | 98 | 90 |
| Foxtail, Giant | 100 | 100 | 85 | 95 | 90 | 85 | 85 | 85 | 85 | 75 | 80 | 80 | 98 | 85 |
| Foxtail, Green | 85 | 85 | 60 | 75 | 60 | 70 | 80 | 95 | 70 | 50 | 80 | 50 | 95 | 80 |
| Goosegrass | 60 | 80 | 60 | 60 | 65 | 15 | 60 | 20 | 55 | 60 | 65 | 0 | 98 | 70 |
| Johnsongrass | 0 | 50 | 0 | 55 | 60 | 65 | 75 | 35 | 0 | 0 | 15 | 0 | 65 | 75 |
| Kochia | 0 | 0 | 0 | 0 | 0 | 0 | 0 | 0 | 0 | 0 | 0 | 0 | 0 | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 95 | 100 | 95 | 90 | 90 | 80 | 50 | 80 | 85 | 5 | 85 | 45 | 100 | 95 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Ryegrass, Italian | 80 | 90 | 80 | 90 | 70 | 40 | 70 | 85 | 75 | 0 | 80 | 80 | 100 | 60 |
| Soybean | 0 | 0 | 0 | 0 | 0 | 0 | 0 | 0 | 0 | 0 | 0 | 0 | - | 0 |
| Surinam Grass | 98 | 100 | 65 | 98 | 75 | 75 | 70 | 75 | 50 | 70 | 65 | 75 | 100 | 95 |
| Velvetleaf | 0 | 0 | 0 | 0 | 0 | 0 | 0 | 0 | 0 | 0 | 0 | 0 | 0 | 0 |
| Wheat | 70 | 80 | 30 | 55 | 60 | 0 | 0 | 5 | 0 | 5 | 30 | 0 | 90 | 95 |
| Windgrass | 70 | 30 | 50 | 85 | 30 | 0 | 0 | 0 | 0 | 0 | 50 | 0 | 90 | 90 |

| Table B | Compounds | | | | | | | | | | | | | |
|---|---|---|---|---|---|---|---|---|---|---|---|---|---|---|
| 8 g ai/ha | 67 | 68 | 69 | 72 | 73 | 74 | 75 | 76 | 78 | 79 | 80 | 81 | 82 | 83 |
| Postemergence | | | | | | | | | | | | | | |
| Barley | 35 | 0 | 0 | 0 | 0 | 20 | 0 | 0 | 45 | 0 | 30 | 10 | 90 | 0 |
| Bermudagrass | 0 | 0 | 20 | 0 | 0 | 20 | 65 | 70 | 80 | 70 | 85 | 40 | 98 | 40 |
| Blackgrass | 85 | 0 | 10 | 80 | 60 | 90 | 10 | 70 | 70 | 50 | 80 | 85 | 98 | 70 |
| Bromegrass, Downy | 55 | 0 | 0 | 20 | 0 | 10 | 0 | 0 | 55 | 0 | 40 | 0 | 85 | 10 |
| Canarygrass | 50 | 0 | 0 | - | 30 | 80 | 0 | 45 | 80 | 10 | 90 | 65 | 80 | 65 |
| Chickweed | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 75 | 0 | 0 | 50 | 95 | 98 | 0 | 75 | 40 | 0 | 100 | 45 | 40 | 65 |
| Crabgrass, Large | 70 | 20 | 55 | 95 | 45 | 80 | 0 | 20 | 98 | 75 | 98 | 50 | 100 | 80 |
| Cupgrass, Woolly | 90 | 0 | 20 | 55 | 85 | 85 | 20 | 60 | 98 | 60 | 80 | 75 | 85 | 65 |
| Foxtail, Giant | 85 | 10 | 60 | 95 | 30 | 80 | 50 | 75 | 98 | 75 | 95 | 80 | 95 | 80 |
| Foxtail, Green | 65 | 10 | 60 | 98 | 40 | 85 | 0 | 65 | 40 | 75 | 50 | 85 | 85 | 60 |
| Goosegrass | 65 | 0 | 60 | 30 | 70 | 60 | 30 | 20 | 70 | 60 | 20 | 65 | 60 | 0 |
| Johnsongrass | 80 | 0 | 60 | 70 | 70 | 98 | 75 | 65 | 75 | 85 | 75 | 75 | 100 | 65 |
| Kochia | 0 | 0 | 0 | 0 | 0 | 0 | 0 | 0 | - | - | - | - | - | 0 |
| Lambsquarters | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 100 | 0 | 20 | 100 | 70 | 98 | 0 | 80 | 98 | 30 | 85 | 80 | 95 | 98 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 20 | 0 |
| Ryegrass, Italian | 100 | 0 | 10 | 80 | 45 | 98 | 30 | 65 | 90 | 55 | 80 | 85 | 98 | 85 |
| Soybean | 0 | 0 | 0 | 0 | 0 | 0 | 0 | 0 | 0 | 0 | 0 | 0 | 0 | 0 |
| Surinam Grass | 70 | 35 | 65 | 95 | 60 | 85 | 60 | 65 | 98 | 65 | 95 | 90 | 75 | 85 |
| Velvetleaf | 0 | 0 | 0 | 0 | 0 | 0 | 0 | 0 | 0 | 0 | 0 | 0 | 0 | 0 |
| Wheat | 95 | 0 | 0 | 85 | 5 | 40 | 0 | 10 | 50 | 0 | 35 | 30 | 85 | 40 |
| Windgrass | 95 | 0 | 0 | 85 | 50 | 85 | 10 | 30 | 40 | 0 | 30 | 45 | 70 | 90 |

| Table B | Compounds | | | | | | | | | | | | | |
|---|---|---|---|---|---|---|---|---|---|---|---|---|---|---|
| 250 g ai/ha | 1 | 2 | 3 | 4 | 5 | 6 | 7 | 15 | 16 | | | | | |
| Preemergence | | | | | | | | | | | | | | |
| Bermudagrass | 100 | 100 | 100 | 100 | 100 | 100 | 100 | 100 | 100 | | | | | |
| Blackgrass | 100 | 100 | 100 | 100 | 100 | 100 | 100 | 100 | 100 | | | | | |
| Bromegrass, Downy | 95 | - | 98 | 100 | 100 | 95 | 60 | 98 | 100 | | | | | |
| Cocklebur | 0 | 0 | 0 | 30 | - | 0 | 0 | 10 | 0 | | | | | |
| Corn | 100 | 95 | 98 | 100 | 100 | 100 | 100 | 100 | 100 | | | | | |
| Crabgrass, Large | 100 | 100 | 100 | 100 | 100 | 100 | 100 | 100 | 40 | | | | | |
| Cupgrass, Woolly | 100 | 100 | 100 | 100 | 100 | 85 | 100 | 98 | 95 | | | | | |
| Foxtail, Giant | 100 | 100 | 100 | 100 | 100 | 100 | 100 | 100 | 70 | | | | | |
| Foxtail, Green | 100 | 100 | 100 | 100 | 100 | 100 | 100 | 100 | 100 | | | | | |
| Galium | 0 | 100 | 85 | 90 | 90 | 70 | 80 | 100 | 0 | | | | | |
| Goosegrass | 100 | 95 | 100 | 100 | 100 | 100 | 100 | 100 | 60 | | | | | |
| Johnsongrass | 100 | 80 | 100 | 100 | 100 | 100 | 100 | 100 | 100 | | | | | |
| Kochia | - | 50 | 30 | 0 | 60 | 60 | 0 | - | - | | | | | |
| Lambsquarters | 0 | 30 | - | 90 | 100 | 95 | 100 | 0 | - | | | | | |
| Morningglory | 0 | 0 | 0 | 30 | 0 | 0 | 0 | - | 0 | | | | | |
| Nightshade, E. B. | 0 | 0 | 100 | 80 | 100 | 90 | 100 | 0 | 0 | | | | | |
| Nutsedge, Yellow | 0 | 0 | 0 | - | - | 0 | 0 | 0 | 20 | | | | | |
| Oat, Wild | 98 | 60 | 95 | 95 | 95 | 85 | 85 | 85 | 100 | | | | | |
| Pigweed | 0 | 70 | 90 | 0 | 100 | 60 | - | 0 | 0 | | | | | |
| Ragweed | 0 | 0 | 0 | 0 | 10 | 20 | 20 | 0 | 0 | | | | | |
| Russian Thistle | - | - | - | - | - | - | - | 80 | 0 | | | | | |
| Ryegrass, Italian | 100 | 100 | 100 | 100 | 100 | 100 | 100 | 100 | 100 | | | | | |
| Soybean | 0 | 0 | 0 | 20 | - | 70 | 0 | 30 | 0 | | | | | |
| Sunflower | 60 | 0 | - | 70 | 0 | 0 | 0 | 60 | 0 | | | | | |
| Surinam Grass | 100 | 100 | 100 | 100 | 100 | 100 | 100 | 100 | 100 | | | | | |
| Velvetleaf | 50 | 10 | 20 | 50 | 0 | 40 | 0 | 0 | 0 | | | | | |
| Wheat | 95 | 75 | 98 | 100 | 98 | 90 | 95 | 80 | 100 | | | | | |

| 125 g ai/ha | 1 | 2 | 3 | 4 | 5 | 6 | 7 | 11 | 12 | 15 | 16 | | | |
|---|---|---|---|---|---|---|---|---|---|---|---|---|---|---|
| Preemergence | | | | | | | | | | | | | | |
| Bermudagrass | 100 | 100 | 100 | 100 | 100 | 100 | 90 | 100 | 100 | 100 | 98 | | | |
| Blackgrass | 100 | 100 | 100 | 100 | 100 | 100 | 100 | 100 | 100 | 100 | 100 | | | |
| Bromegrass, Downy | 80 | 70 | 95 | 98 | 98 | 65 | 60 | 100 | 75 | 90 | 100 | | | |
| Cocklebur | 0 | 0 | - | 0 | 0 | 0 | 0 | 0 | 0 | 0 | 0 | | | |
| Corn | 100 | 95 | 95 | 98 | 98 | 98 | 98 | 100 | 95 | 100 | 100 | | | |
| Crabgrass, Large | 100 | 100 | 100 | 100 | 100 | 100 | 100 | 100 | 100 | 100 | 20 | | | |
| Cupgrass, Woolly | 100 | 80 | 90 | 100 | 100 | 85 | 85 | 100 | 100 | 90 | 70 | | | |
| Foxtail, Giant | 100 | 100 | 100 | 100 | 100 | 100 | 100 | 100 | 100 | 100 | 40 | | | |
| Foxtail, Green | 100 | 100 | 100 | 100 | 100 | 100 | 100 | 100 | 100 | 100 | 100 | | | |
| Galium | 0 | - | 85 | 85 | 80 | 70 | 80 | 0 | 0 | 10 | 0 | | | |
| Goosegrass | 100 | 95 | 85 | 100 | 100 | 100 | 100 | 100 | 100 | 90 | 50 | | | |
| Johnsongrass | 100 | - | 100 | 100 | 100 | 95 | 100 | 100 | 100 | 98 | 100 | | | |
| Kochia | 20 | 0 | 30 | 0 | 50 | 20 | 0 | - | 0 | - | - | | | |
| Lambsquarters | 0 | 0 | - | 85 | 90 | 85 | 90 | 0 | 0 | 0 | - | | | |
| Morningglory | 0 | 0 | 0 | 0 | 0 | 0 | 0 | 0 | 0 | 0 | 0 | | | |
| Nightshade, E. B. | 0 | 0 | 100 | - | - | 90 | 100 | 0 | 85 | 0 | 0 | | | |
| Nutsedge, Yellow | - | 0 | 0 | 0 | 0 | 0 | 0 | 0 | 0 | - | 0 | | | |
| Oat, Wild | 90 | - | 80 | 85 | 80 | 85 | 80 | 100 | 98 | 80 | 98 | | | |
| Pigweed | 0 | 70 | 90 | - | 80 | 30 | 100 | - | - | 0 | 0 | | | |
| Ragweed | 0 | 0 | 0 | 0 | 0 | 20 | 0 | 0 | 0 | 0 | 0 | | | |
| Russian Thistle | - | - | - | - | - | - | - | - | - | - | 0 | | | |
| Ryegrass, Italian | 100 | 100 | 100 | 100 | 100 | 100 | 100 | 100 | 100 | 100 | 100 | | | |
| Soybean | - | 0 | 0 | 0 | 0 | 40 | 0 | 0 | 0 | - | - | | | |
| Sunflower | 0 | 0 | - | 0 | 0 | 0 | 0 | 0 | 0 | - | 0 | | | |
| Surinam Grass | 100 | - | 100 | 100 | 100 | 100 | 100 | 100 | 100 | 100 | 95 | | | |
| Velvetleaf | 0 | 0 | 0 | 40 | 0 | 0 | 0 | 0 | 0 | - | 0 | | | |
| Wheat | 90 | 60 | 95 | 100 | 95 | 90 | 80 | 100 | 95 | 60 | 98 | | | |

| Table B | Compounds | | | | | | | | | | | | | |
|---|---|---|---|---|---|---|---|---|---|---|---|---|---|---|
| 62 g ai/ha | 1 | 2 | 3 | 4 | 5 | 6 | 7 | 11 | 12 | 15 | 16 | 19 | 20 | 21 |
| Preemergence | | | | | | | | | | | | | | |
| Bermudagrass | 100 | 100 | 100 | 100 | 100 | 100 | 90 | 100 | 100 | 98 | 0 | 100 | 70 | 100 |
| Blackgrass | 100 | 80 | 100 | 100 | 100 | 100 | 85 | 100 | 100 | 100 | 100 | 100 | 90 | 100 |
| Bromegrass, Downy | 80 | 20 | 60 | 70 | 85 | 65 | 35 | 98 | 70 | 55 | 100 | 60 | 0 | 100 |
| Cocklebur | 0 | 0 | 0 | 0 | 0 | 0 | 0 | 0 | 0 | 0 | 0 | 0 | 0 | 0 |
| Corn | 95 | 90 | 95 | 95 | 95 | 98 | 95 | 95 | 95 | 98 | 100 | 98 | 95 | 98 |
| Crabgrass, Large | 100 | 100 | 90 | 100 | 100 | 100 | 100 | 100 | 100 | 100 | 0 | 100 | 60 | 100 |
| Cupgrass, Woolly | 100 | 80 | 50 | 85 | 90 | 85 | 70 | 100 | 98 | 85 | 25 | 100 | 98 | 100 |
| Foxtail, Giant | 100 | 100 | 98 | 100 | 100 | 100 | 100 | 100 | 100 | 100 | 0 | 100 | 100 | 100 |
| Foxtail, Green | 100 | 95 | 100 | 100 | 100 | 100 | 100 | 100 | 100 | 100 | 100 | 100 | 100 | 100 |
| Galium | 0 | 100 | 85 | 0 | - | - | 0 | 0 | 0 | 0 | 0 | 0 | 100 | 90 |
| Goosegrass | 50 | 60 | 85 | 100 | 100 | 95 | 90 | 100 | 100 | 90 | 0 | 100 | 70 | 100 |
| Johnsongrass | 95 | 80 | 95 | 30 | 100 | 90 | 98 | 100 | 100 | 95 | 70 | 100 | 95 | 100 |
| Kochia | 0 | 0 | - | - | 50 | 0 | 0 | - | 0 | 0 | - | 0 | 0 | 0 |
| Lambsquarters | 0 | 0 | - | 85 | - | - | 30 | - | - | 0 | - | 40 | 100 | 100 |
| Morningglory | 0 | 0 | 0 | 0 | 0 | 0 | 0 | 0 | 0 | - | 0 | 0 | 0 | 0 |
| Nightshade, E. B. | 0 | 0 | 100 | - | 0 | - | 30 | 0 | 50 | 0 | 0 | 0 | 0 | - |
| Nutsedge, Yellow | 0 | 0 | 0 | 0 | 0 | 0 | 0 | 0 | 0 | 0 | 0 | 0 | 0 | 0 |
| Oat, Wild | 85 | 50 | 70 | 70 | 70 | 50 | 70 | 98 | 95 | 70 | 95 | 95 | 60 | 95 |
| Pigweed | 0 | 0 | 90 | - | 0 | 0 | 100 | - | - | 0 | 0 | 0 | 0 | 0 |
| Ragweed | 0 | 0 | - | 0 | 0 | 0 | 0 | 0 | 0 | 0 | 0 | 0 | 0 | 0 |
| Russian Thistle | - | - | - | - | - | - | - | - | - | - | 0 | - | 0 | 0 |
| Ryegrass, Italian | 100 | 100 | 100 | 100 | 100 | 100 | 100 | 100 | 100 | 100 | 100 | 100 | 90 | 100 |
| Soybean | - | 0 | 0 | 0 | 0 | 0 | - | 0 | 0 | 0 | - | 0 | - | - |
| Sunflower | 0 | 0 | 40 | 0 | 0 | 0 | 0 | 0 | 0 | 0 | 0 | 0 | 40 | 0 |
| Surinam Grass | 100 | 85 | 100 | 100 | 100 | 98 | 100 | 100 | 100 | 100 | 55 | 100 | 100 | 100 |
| Velvetleaf | 0 | 0 | 0 | 0 | 0 | 0 | 0 | 0 | 0 | 0 | 0 | 0 | 0 | 0 |
| Wheat | 45 | 50 | 75 | 95 | 70 | 80 | 65 | 95 | 95 | 60 | 90 | 95 | 0 | 95 |

| Table B | Compounds | | | | | | | | | | | | | |
|---|---|---|---|---|---|---|---|---|---|---|---|---|---|---|
| 62 g ai/ha | 25 | 26 | 27 | 28 | 30 | 31 | 32 | 33 | 34 | 35 | 36 | 37 | 38 | 39 |
| Preemergence | | | | | | | | | | | | | | |
| Bermudagrass | 100 | 80 | 100 | 100 | 100 | 100 | 0 | 100 | 100 | 100 | 100 | 100 | 100 | 98 |
| Blackgrass | 90 | 100 | 100 | - | 100 | 100 | 98 | 100 | 100 | 100 | 100 | 100 | 100 | 90 |
| Bromegrass, Downy | 0 | 65 | 50 | 50 | 85 | 85 | 75 | 100 | 80 | 60 | 90 | 80 | 90 | 85 |
| Cocklebur | 0 | 10 | 0 | 0 | 0 | 0 | 0 | - | 0 | 0 | 0 | 0 | 0 | 0 |
| Corn | 75 | 98 | 85 | 90 | 5 | 90 | 95 | 95 | 95 | 90 | 95 | 98 | 95 | 100 |
| Crabgrass, Large | 80 | 95 | 0 | 100 | 100 | 100 | 100 | 98 | 100 | 100 | 100 | 100 | 100 | 100 |
| Cupgrass, Woolly | 80 | 100 | 55 | 98 | 100 | 100 | 85 | 100 | 95 | 100 | 100 | 100 | 100 | 98 |
| Foxtail, Giant | 100 | 100 | 20 | 100 | 100 | 100 | 100 | 100 | 100 | 100 | 100 | 100 | 100 | 100 |
| Foxtail, Green | 98 | 100 | 85 | 100 | 100 | 100 | 100 | 100 | 100 | 100 | 100 | 100 | 100 | 100 |
| Galium | 0 | 0 | - | - | 0 | 50 | 0 | 0 | 0 | 20 | 0 | 0 | 0 | 0 |
| Goosegrass | 85 | 95 | 95 | 100 | 100 | 100 | 100 | 98 | 100 | 100 | 100 | 100 | 95 | 100 |
| Johnsongrass | 0 | 50 | 85 | 100 | 98 | 75 | 90 | 100 | 100 | 80 | 100 | 80 | 80 | 95 |
| Kochia | 50 | - | - | - | 0 | 0 | 0 | 0 | 0 | - | - | 0 | - | 0 |
| Lambsquarters | 40 | 0 | 0 | 98 | 0 | 0 | 0 | 0 | 0 | 0 | 0 | 0 | 0 | 0 |
| Morningglory | 0 | 0 | 0 | 0 | - | 0 | 0 | 0 | 0 | 10 | 0 | 0 | 0 | 0 |
| Nightshade, E. B. | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 60 | 85 | 50 | 90 | 80 | 90 | 80 | 90 | 90 | 95 | 95 | 95 | 90 | 95 |
| Pigweed | 80 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Russian Thistle | 0 | 0 | 0 | 0 | 0 | 0 | 0 | 80 | 0 | 0 | 80 | 90 | 0 | 0 |
| Ryegrass, Italian | 100 | 100 | 95 | 100 | 100 | 100 | 100 | 98 | 100 | 100 | 100 | 100 | 100 | 100 |
| Soybean | 0 | 0 | 0 | 0 | 0 | 60 | 0 | 0 | 0 | 0 | 0 | 0 | 0 | 0 |
| Sunflower | 0 | 0 | 0 | 0 | 0 | 0 | 0 | 0 | 0 | 0 | 0 | 30 | 0 | 0 |
| Surinam Grass | 100 | 100 | 75 | 100 | 95 | 100 | 100 | 100 | 100 | 100 | 100 | 100 | 100 | 100 |
| Velvetleaf | 0 | 0 | 0 | 0 | 0 | 0 | 0 | - | 0 | 0 | 0 | 0 | 0 | 0 |
| Wheat | 15 | 0 | 45 | 90 | 10 | 80 | 70 | 80 | 40 | 30 | 60 | 70 | 90 | 80 |

| Table B | Compounds | | | | | | | | | | | | | |
|---|---|---|---|---|---|---|---|---|---|---|---|---|---|---|
| 62 g ai/ha | 40 | 41 | 43 | 44 | 45 | 46 | 47 | 48 | 50 | 51 | 52 | 53 | 54 | 55 |
| Preemergence | | | | | | | | | | | | | | |
| Bermudagrass | 100 | 0 | 95 | 0 | 0 | 100 | 100 | 0 | 100 | 100 | 85 | 0 | 0 | 100 |
| Blackgrass | 95 | 100 | 100 | 100 | 100 | 85 | 100 | 100 | 95 | 100 | 100 | 100 | 90 | 100 |
| Bromegrass, Downy | 90 | 80 | 80 | 85 | 90 | 5 | 90 | 80 | 0 | 85 | 80 | 90 | 85 | 60 |
| Cocklebur | 0 | 0 | 0 | 0 | 0 | 0 | 0 | 0 | 0 | 0 | 0 | 0 | 0 | 0 |
| Corn | 95 | 95 | 100 | 85 | 98 | 0 | 95 | 75 | 90 | 80 | 75 | 60 | 85 | 95 |
| Crabgrass, Large | 100 | 20 | 100 | 0 | 0 | 100 | 100 | 0 | 100 | 90 | 0 | 0 | 0 | 100 |
| Cupgrass, Woolly | 100 | 75 | 100 | 100 | 0 | 98 | 100 | 0 | 100 | 100 | 0 | 0 | 0 | 100 |
| Foxtail, Giant | 100 | 98 | 100 | 0 | 45 | 100 | 100 | 0 | 100 | 100 | 98 | 70 | 55 | 100 |
| Foxtail, Green | 100 | 100 | 100 | 100 | 100 | 100 | 100 | 100 | 100 | 100 | 100 | 100 | 100 | 100 |
| Galium | | 0 100 | 0 | 0 | 0 | 0 | - | - | - | 0 | 0 | - | 0 | 0 |
| Goosegrass | 100 | 0 | 100 | 0 | 0 | 100 | 100 | 20 | 100 | 90 | 0 | 0 | 0 | 100 |
| Johnsongrass | 98 | 95 | 98 | 95 | 98 | 90 | 95 | 80 | 100 | 80 | 85 | 100 | 90 | 95 |
| Kochia | 0 | 0 | 0 | 100 | 0 | - | - | 0 | - | - | 85 | - | - | - |
| Lambsquarters | 0 | 0 | 0 | 0 | 0 | 0 | 65 | 0 | 100 | 0 | 0 | 35 | 0 | 0 |
| Morningglory | 0 | 0 | 0 | 0 | 0 | 0 | 0 | 0 | 30 | 0 | 100 | 0 | 0 | 0 |
| Nightshade, E. B. | 0 | 0 | 0 | 55 | 0 | 100 | 0 | 20 | 100 | 0 | 0 | 20 | 0 | 100 |
| Nutsedge, Yellow | 0 | 0 | 0 | 0 | 0 | 0 | 0 | 0 | 0 | 0 | 0 | 0 | 0 | 0 |
| Oat, Wild | 95 | 95 | 95 | 90 | 95 | 35 | 70 | 85 | 70 | 90 | 90 | 85 | 95 | 70 |
| Pigweed | 0 | 0 | 0 | 0 | 0 | 100 | 0 | 0 | 0 | 0 | 0 | 0 | 0 | 0 |
| Ragweed | 0 | 0 | 0 | 20 | 0 | 0 | 0 | 0 | 0 | 0 | 0 | 0 | 0 | 0 |
| Russian Thistle | 0 | 0 | 0 | 0 | - | 0 | 40 | 0 | 0 | 0 | 0 | 0 | 0 | 0 |
| Ryegrass, Italian | 100 | 100 | 90 | 100 | 100 | 100 | 98 | 100 | 100 | 100 | 100 | 100 | 100 | 100 |
| Soybean | 0 | 0 | 0 | 90 | 65 | 0 | 0 | 0 | 100 | 0 | 0 | 65 | 0 | 0 |
| Sunflower | 0 | 0 | 0 | 0 | 0 | 0 | 0 | 0 | 20 | 0 | 0 | 0 | 0 | 0 |
| Surinam Grass | 100 | 100 | 100 | 20 | 90 | 0 | 100 | 75 | 98 | 100 | 100 | 95 | 85 | 100 |
| Velvetleaf | 0 | 0 | 0 | 0 | 0 | 0 | 0 | 0 | 0 | 0 | 0 | 0 | 0 | 0 |
| Wheat | 90 | 100 | 80 | 90 | 95 | 0 | 20 | 60 | 0 | 60 | 50 | 55 | 50 | 5 |

| Table B | Compounds | | | | | | | | | | | | | |
|---|---|---|---|---|---|---|---|---|---|---|---|---|---|---|
| 62 g ai/ha | 56 | 57 | 58 | 61 | 66 | 67 | 69 | 72 | 74 | 75 | 76 | 78 | 79 | 80 |
| Preemergence | | | | | | | | | | | | | | |
| Bermudagrass | 100 | 100 | 100 | 95 | 100 | 0 | 0 | 100 | 100 | 98 | 100 | 100 | 100 | 100 |
| Blackgrass | 98 | 100 | 100 | 100 | 100 | 100 | 70 | 100 | 100 | 90 | 90 | 100 | 100 | 100 |
| Bromegrass, Downy | 75 | 70 | 85 | 95 | 100 | 100 | 0 | 95 | 85 | 80 | 0 | 98 | 80 | 85 |
| Cocklebur | 0 | 0 | 0 | 0 | 0 | 0 | 0 | 0 | 0 | 0 | 0 | 0 | 0 | 0 |
| Corn | 95 | 95 | 80 | 100 | 100 | 100 | 0 | 100 | 98 | 95 | 85 | 100 | 100 | 100 |
| Crabgrass, Large | 100 | 100 | 100 | 100 | 80 | 0 | 0 | 100 | 100 | 55 | 98 | 100 | 100 | 100 |
| Cupgrass, Woolly | 100 | 100 | 100 | 100 | 98 | 0 | 85 | 100 | 100 | 75 | 98 | 100 | 100 | 100 |
| Foxtail, Giant | 100 | 100 | 100 | 100 | 95 | 0 | 0 | 100 | 100 | 95 | 100 | 100 | 100 | 100 |
| Foxtail, Green | 100 | 100 | 100 | 100 | 100 | 100 | 100 | 100 | 100 | 80 | 90 | 100 | 100 | 100 |
| Galium | 0 | 0 | 0 | 0 | 0 | 0 | 0 | 0 | 0 | 0 | 0 | 0 | 0 | 0 |
| Goosegrass | 100 | 100 | 100 | 100 | 70 | 60 | 0 | 100 | 100 | 98 | 100 | 100 | 100 | 100 |
| Johnsongrass | 100 | 100 | 60 | 100 | 100 | 100 | 0 | 100 | 100 | 95 | 85 | 100 | 100 | 100 |
| Kochia | 100 | 0 | - | 0 | - | - | - | - | - | - | - | - | - | - |
| Lambsquarters | 20 | 65 | 100 | 0 | - | - | - | 40 | 20 | 0 | 0 | 0 | 100 | 0 |
| Morningglory | 0 | 0 | 0 | 0 | 0 | 0 | 0 | 0 | 0 | 0 | 0 | 0 | 0 | 0 |
| Nightshade, E. B. | 20 | 0 | 0 | 0 | 0 | 0 | 0 | 30 | 65 | 0 | 0 | 55 | 0 | 0 |
| Nutsedge, Yellow | 0 | 0 | 0 | 0 | 100 | 0 | 0 | 0 | 0 | 0 | 100 | 0 | 0 | 100 |
| Oat, Wild | 90 | 80 | 95 | 100 | 100 | 100 | 40 | 98 | 80 | 50 | 80 | 85 | 95 | 70 |
| Pigweed | 0 | 0 | 0 | 0 | 95 | 0 | 0 | 50 | 20 | 0 | 0 | 0 | 0 | 55 |
| Ragweed | 0 | 0 | 0 | 0 | 0 | 0 | 100 | 10 | 0 | 0 | 0 | 0 | 0 | 0 |
| Russian Thistle | 0 | 0 | 60 | 0 | 0 | 0 | 0 | 0 | 0 | 0 | 0 | 0 | 0 | 0 |
| Ryegrass, Italian | 100 | 100 | 100 | 100 | 100 | 100 | 100 | 100 | 100 | 98 | 95 | 100 | 100 | 100 |
| Soybean | 0 | 0 | 0 | 0 | 0 | - | - | 0 | 0 | 0 | 0 | 0 | 100 | 0 |
| Sunflower | 0 | 0 | 0 | 0 | 0 | 0 | 0 | 0 | 0 | 0 | 0 | 0 | 0 | 0 |
| Surinam Grass | 100 | 100 | 100 | 100 | 98 | 55 | 35 | 100 | 100 | 98 | 100 | 100 | 100 | 100 |
| Velvetleaf | 0 | 0 | 0 | 0 | 0 | 0 | 0 | 0 | 0 | 0 | 0 | 0 | 0 | 0 |
| Wheat | 80 | 50 | 80 | 95 | 95 | 100 | 0 | 90 | 85 | 45 | 0 | 95 | 0 | 95 |

| Table B | Compounds | | | | | | Table B | | | | Compounds | | | |
|---|---|---|---|---|---|---|---|---|---|---|---|---|---|---|
| 62 g ai/ha | 81 | 82 | 83 | | | | 62 g ai/ha | | | | 81 | 82 | 83 | |
| Preemergence | | | | | | | Preemergence | | | | | | | |
| Bermudagrass | 100 | 100 | 100 | | | | Morningglory | | | | 100 | 25 | 0 | |
| Blackgrass | 100 | 100 | 98 | | | | Nightshade, E. B. | | | | 50 | 0 | 0 | |
| Bromegrass, Downy | 85 | 100 | 30 | | | | Nutsedge, Yellow | | | | 0 | 45 | 0 | |
| Cocklebur | 0 | 35 | 0 | | | | Oat, Wild | | | | 85 | 80 | 85 | |
| Corn | 100 | 98 | 95 | | | | Pigweed | | | | 55 | 0 | 0 | |
| Crabgrass, Large | 100 | 100 | 100 | | | | Ragweed | | | | 0 | 80 | 0 | |
| Cupgrass, Woolly | 98 | 100 | 100 | | | | Russian Thistle | | | | 0 | 0 | - | |
| Foxtail, Giant | 100 | 100 | 100 | | | | Ryegrass, Italian | | | | 100 | 100 | 100 | |
| Foxtail, Green | 100 | 100 | 100 | | | | Soybean | | | | 0 | 100 | 0 | |
| Galium | 0 | 50 | 0 | | | | Sunflower | | | | 20 | 85 | 0 | |
| Goosegrass | 100 | 100 | 100 | | | | Surinam Grass | | | | 100 | 100 | 100 | |
| Johnsongrass | 95 | 100 | 98 | | | | Velvetleaf | | | | 0 | 75 | 0 | |
| Kochia | - | - | 100 | | | | Wheat | | | | 85 | 100 | 20 | |
| Lambsquarters | 0 | 100 | 100 | | | | | | | | | | | |

| Table B | Compounds | | | | | | | | | | | | | |
|---|---|---|---|---|---|---|---|---|---|---|---|---|---|---|
| 31 g ai/ha | 1 | 2 | 3 | 4 | 5 | 6 | 7 | 11 | 12 | 15 | 16 | 19 | 20 | 21 |
| Preemergence | | | | | | | | | | | | | | |
| Bermudagrass | 100 | 100 | 30 | 100 | 98 | 100 | 60 | 100 | 100 | 98 | 0 | 98 | 0 | 85 |
| Blackgrass | 100 | 60 | 80 | 100 | 100 | 80 | 70 | 100 | 100 | 100 | 100 | 100 | 90 | 100 |
| Bromegrass, Downy | 40 | 0 | 50 | 70 | 40 | 50 | 35 | 95 | 0 | 50 | 90 | 30 | 0 | 60 |
| Cocklebur | 0 | 0 | 0 | 0 | 0 | 0 | 0 | - | 0 | 0 | 0 | 0 | 0 | 0 |
| Corn | 90 | 50 | 95 | 85 | 95 | 95 | 90 | 85 | 75 | 50 | 0 | 95 | 80 | 95 |
| Crabgrass, Large | 98 | 98 | 90 | 100 | 100 | 98 | 100 | 90 | 100 | 85 | 0 | 100 | 50 | 80 |
| Cupgrass, Woolly | 85 | 70 | 50 | 85 | 50 | 85 | 70 | 100 | 85 | 60 | 0 | 98 | 50 | - |
| Foxtail, Giant | 100 | 100 | 98 | 100 | 98 | 100 | 100 | 90 | 100 | 90 | 0 | 100 | 50 | 100 |
| Foxtail, Green | 100 | 90 | 100 | 100 | 98 | 100 | 100 | 100 | 100 | 100 | 100 | 100 | 100 | 100 |
| Galium | 0 | 100 | - | 0 | - | 0 | - | 0 | 0 | 0 | 0 | 0 | 50 | 0 |
| Goosegrass | 50 | 50 | 80 | 85 | 75 | 80 | 85 | 90 | 98 | 0 | 0 | 100 | - | 95 |
| Johnsongrass | 90 | - | 80 | 0 | 0 | 60 | 75 | 98 | 95 | 70 | 25 | 100 | 75 | 90 |
| Kochia | - | 0 | 0 | 0 | - | 0 | 0 | - | 0 | - | - | - | 0 | 0 |
| Lambsquarters | 0 | 0 | - | - | - | 0 | 30 | 0 | 0 | 0 | - | 0 | 100 | 100 |
| Morningglory | 0 | 0 | 0 | 0 | 0 | 0 | 0 | 0 | 0 | 0 | 0 | 0 | 0 | 0 |
| Nightshade, E. B. | 0 | 0 | 90 | - | - | 0 | 30 | 0 | 0 | 0 | 0 | - | - | 0 |
| Nutsedge, Yellow | 0 | 0 | 0 | 0 | - | 0 | 0 | 0 | 0 | 0 | 0 | 0 | 0 | 0 |
| Oat, Wild | 60 | 0 | 60 | 65 | 30 | 50 | 0 | 98 | 90 | 10 | 90 | 95 | 50 | 90 |
| Pigweed | - | 0 | 0 | - | 0 | - | 85 | - | - | 0 | - | 0 | 0 | 0 |
| Ragweed | 0 | 0 | 0 | 0 | 0 | 0 | 0 | 0 | 0 | 0 | 0 | 0 | - | 0 |
| Russian Thistle | - | - | - | - | - | - | - | - | - | 0 | 0 | 0 | 0 | 0 |
| Ryegrass, Italian | 80 | 75 | 98 | 100 | 100 | 90 | 90 | 100 | 95 | 70 | 100 | 100 | 40 | - |
| Soybean | 0 | 0 | 0 | 0 | 0 | 0 | - | 0 | 0 | 0 | - | - | - | 0 |
| Sunflower | 0 | 0 | 30 | 0 | 0 | 0 | 0 | 0 | 0 | 0 | 0 | 0 | 30 | 0 |
| Surinam Grass | 100 | 85 | 100 | 100 | 100 | 98 | 98 | 100 | 100 | 100 | 45 | 98 | 50 | - |
| Velvetleaf | 0 | 0 | 0 | 0 | 0 | 0 | 0 | 0 | 0 | 0 | 0 | 0 | 0 | 0 |
| Wheat | 35 | 30 | 45 | 60 | 30 | 35 | 0 | 95 | 90 | 0 | 15 | 90 | 0 | 85 |

| Table B | Compounds | | | | | | | | | | | | | |
|---|---|---|---|---|---|---|---|---|---|---|---|---|---|---|
| 31 g ai/ha | 25 | 26 | 27 | 28 | 30 | 31 | 32 | 33 | 34 | 35 | 36 | 37 | 38 | 39 |
| Preemergence | | | | | | | | | | | | | | |
| Bermudagrass | 80 | 0 | 100 | 100 | 100 | 100 | 0 | 100 | 98 | 100 | 100 | 100 | 100 | 95 |
| Blackgrass | - | 85 | 50 | - | 100 | 98 | 95 | 100 | 100 | 100 | 100 | 100 | 90 | 90 |
| Bromegrass, Downy | 0 | 10 | 50 | 50 | 10 | 65 | 0 | 80 | 55 | 40 | 50 | 60 | 60 | 85 |
| Cocklebur | 0 | 0 | 0 | 0 | 0 | 0 | 0 | 0 | 0 | 0 | 0 | 0 | 0 | 0 |
| Corn | 50 | 70 | 75 | 55 | 5 | 60 | 60 | 90 | 85 | 55 | 70 | 95 | 75 | 98 |
| Crabgrass, Large | 60 | 70 | 0 | 100 | 100 | 100 | 100 | 95 | 100 | 100 | 100 | 100 | 100 | 100 |
| Cupgrass, Woolly | 80 | 85 | 30 | 98 | 98 | 100 | 80 | 100 | 95 | 100 | 98 | 50 | 90 | 95 |
| Foxtail, Giant | 90 | 100 | 0 | 100 | 100 | 100 | 100 | 100 | 100 | 100 | 100 | 100 | 100 | 100 |
| Foxtail, Green | 98 | 100 | 60 | 100 | 100 | 100 | 100 | 100 | 100 | 100 | 100 | 100 | 100 | 100 |
| Galium | 0 | 0 | - | - | 0 | 0 | 0 | 0 | 0 | 0 | 0 | 0 | 0 | 0 |
| Goosegrass | 75 | 70 | 95 | 100 | 100 | 100 | 98 | 98 | 98 | 100 | 100 | 98 | 95 | 100 |
| Johnsongrass | 0 | 0 | 75 | 98 | 50 | 75 | 85 | 98 | 100 | 80 | 40 | 80 | 70 | 90 |
| Kochia | - | - | - | - | 0 | 0 | 0 | 0 | 0 | 0 | - | 0 | - | 0 |
| Lambsquarters | 0 | 0 | - | 95 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nightshade, E. B. | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 0 | 85 | 50 | 70 | 50 | 70 | 60 | 80 | 85 | 80 | 90 | 80 | 85 | 90 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Russian Thistle | 0 | 0 | 0 | 0 | 0 | 0 | 0 | 0 | 0 | 0 | - | 0 | 0 | 0 |
| Ryegrass, Italian | 90 | 100 | 70 | 100 | 50 | 90 | 100 | 95 | 100 | 100 | 100 | 98 | 95 | 100 |
| Soybean | 0 | 0 | 0 | 0 | 0 | 0 | 0 | 0 | 0 | 0 | 0 | 0 | 0 | 0 |
| Sunflower | 0 | 0 | 0 | 0 | 0 | 0 | 0 | 0 | 0 | 0 | 0 | 0 | 0 | 0 |
| Surinam Grass | 85 | 100 | 0 | 98 | 90 | 100 | 100 | 98 | 100 | 100 | 100 | 98 | 100 | 100 |
| Velvetleaf | 0 | 0 | 0 | 0 | 0 | 0 | 0 | 0 | 0 | 0 | 0 | 0 | 0 | 0 |
| Wheat | 0 | 0 | 15 | 15 | 0 | 0 | 20 | 50 | 30 | 0 | 5 | 5 | 0 | 70 |

| Table B | Compounds | | | | | | | | | | | | | |
|---|---|---|---|---|---|---|---|---|---|---|---|---|---|---|
| 31 g ai/ha | 40 | 41 | 43 | 44 | 45 | 46 | 47 | 48 | 50 | 51 | 52 | 53 | 54 | 55 |
| Preemergence | | | | | | | | | | | | | | |
| Bermudagrass | 100 | 0 | 65 | 0 | 0 | 85 | 100 | 0 | 100 | 100 | 0 | 0 | 0 | 100 |
| Blackgrass | 95 | 100 | 100 | 100 | 100 | 40 | 100 | 90 | 60 | 98 | 100 | 100 | 90 | 100 |
| Bromegrass, Downy | 80 | 75 | 70 | 60 | 80 | 0 | 0 | 70 | 0 | 45 | 35 | 70 | 70 | 30 |
| Cocklebur | 0 | 0 | 0 | 0 | 0 | 0 | 0 | 0 | 0 | 0 | 0 | 0 | 0 | 0 |
| Corn | 85 | 85 | 85 | 65 | 65 | 0 | 85 | 70 | - | 60 | 70 | 55 | 65 | 80 |
| Crabgrass, Large | 100 | 0 | 98 | 0 | 0 | 60 | 100 | 0 | 100 | 75 | 0 | 0 | 0 | 100 |
| Cupgrass, Woolly | 100 | 0 | 98 | 0 | - | 85 | 100 | 0 | 98 | 90 | 0 | 0 | 0 | 100 |
| Foxtail, Giant | 100 | 0 | 100 | 0 | 35 | 95 | 100 | 0 | 100 | 100 | 0 | 45 | 20 | 100 |
| Foxtail, Green | 100 | 100 | 100 | 100 | 100 | 80 | 100 | 100 | 98 | 98 | 100 | 100 | 100 | 100 |
| Galium | 0 | 50 | 0 | - | 0 | 0 | 0 | 0 | - | 0 | - | - | - | 0 |
| Goosegrass | 100 | 0 | 98 | 0 | 0 | 100 | 100 | 0 | 98 | 75 | 0 | 0 | 0 | 98 |
| Johnsongrass | 98 | 70 | 80 | 0 | 75 | 85 | 85 | 75 | 98 | 70 | 80 | 75 | 75 | 70 |
| Kochia | 0 | 0 | 0 | 0 | 0 | - | - | 0 | - | - | - | 0 | 0 | - |
| Lambsquarters | 0 | 0 | 0 | 0 | 0 | 0 | 0 | 0 | 100 | 0 | 0 | 0 | 0 | 0 |
| Morningglory | 0 | 0 | 0 | - | 0 | 0 | 0 | 0 | 0 | 0 | 90 | 0 | 0 | 0 |
| Nightshade, E. B. | 0 | 0 | 0 | 0 | 0 | 20 | 0 | 0 | 100 | 0 | 0 | 15 | 0 | 80 |
| Nutsedge, Yellow | 0 | 0 | 0 | 0 | 0 | 0 | 0 | 0 | 0 | 0 | 0 | 0 | 0 | 0 |
| Oat, Wild | 90 | 95 | 95 | 85 | 90 | 0 | 0 | 80 | 40 | 80 | 70 | 80 | 75 | 35 |
| Pigweed | 0 | 0 | 0 | 0 | 0 | 60 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Russian Thistle | 0 | 0 | 0 | 0 | 0 | 0 | 0 | 0 | 0 | 0 | 0 | 0 | 0 | 0 |
| Ryegrass, Italian | 95 | 100 | 90 | 90 | 100 | 40 | 90 | 100 | 5 | 100 | 95 | 100 | 90 | 90 |
| Soybean | 0 | 0 | 0 | 70 | - | 0 | 0 | 0 | 100 | 0 | - | 0 | 0 | 0 |
| Sunflower | 0 | 0 | 0 | - | 0 | 0 | 0 | 0 | 0 | 0 | 0 | 0 | 0 | 0 |
| Surinam Grass | 100 | 55 | 100 | 0 | 40 | 0 | 98 | 65 | 90 | 90 | 25 | 60 | 55 | 100 |
| Velvetleaf | 0 | 0 | 0 | 0 | 0 | 0 | 0 | 0 | 0 | 0 | 0 | 0 | 0 | 0 |
| Wheat | 60 | 60 | 15 | 10 | 70 | 0 | 0 | 5 | 0 | 10 | 20 | 0 | 10 | 0 |

| Table B | Compounds | | | | | | | | | | | | | |
|---|---|---|---|---|---|---|---|---|---|---|---|---|---|---|
| 31 g ai/ha | 56 | 57 | 58 | 61 | 66 | 67 | 69 | 72 | 74 | 75 | 76 | 78 | 79 | 80 |
| Preemergence | | | | | | | | | | | | | | |
| Bermudagrass | 100 | 100 | 100 | 90 | 98 | 0 | 0 | 100 | 100 | 65 | 100 | 100 | 100 | 100 |
| Blackgrass | 98 | 100 | 100 | 100 | 100 | 100 | 40 | 95 | 100 | 60 | 80 | 100 | 98 | 100 |
| Bromegrass, Downy | 45 | 65 | 65 | 95 | 100 | 100 | 0 | 50 | 85 | 50 | 0 | 80 | 55 | 85 |
| Cocklebur | 0 | 0 | 0 | 0 | 0 | 0 | 0 | 0 | 0 | 0 | 0 | 0 | 0 | 0 |
| Corn | 95 | 85 | 55 | 100 | 98 | 100 | 0 | 85 | 90 | 75 | 75 | 100 | 50 | 98 |
| Crabgrass, Large | 100 | 98 | 90 | 100 | 60 | 0 | 0 | 80 | 100 | 25 | 55 | 100 | 100 | 100 |
| Cupgrass, Woolly | 100 | 85 | 100 | 100 | 90 | 0 | 0 | 85 | 98 | 65 | 90 | 100 | 100 | 98 |
| Foxtail, Giant | 100 | 100 | 100 | 100 | 95 | 0 | 0 | 100 | 100 | 50 | 90 | 100 | 100 | 100 |
| Foxtail, Green | 100 | 100 | 100 | 100 | 100 | 100 | 40 | 100 | 100 | 50 | 90 | 100 | 98 | 100 |
| Galium | 0 | 0 | 0 | 0 | 0 | 0 | 0 | 0 | 0 | 0 | 0 | 0 | 0 | 0 |
| Goosegrass | 98 | 98 | 95 | 100 | 25 | 0 | 0 | 95 | 100 | 90 | 80 | 100 | 100 | 100 |
| Johnsongrass | 70 | 90 | 40 | 100 | 98 | 100 | 0 | 95 | 90 | 85 | 70 | 95 | 100 | 80 |
| Kochia | 98 | 0 | - | 0 | - | - | - | - | - | - | - | - | - | - |
| Lambsquarters | 0 | 20 | 95 | 0 | - | - | - | 30 | 0 | 0 | 0 | 0 | 20 | 0 |
| Morningglory | 0 | 0 | 0 | 0 | 0 | 0 | 0 | 0 | 0 | 0 | 0 | 0 | 0 | 0 |
| Nightshade, E. B. | 0 | 0 | 0 | 0 | 0 | 0 | 0 | 0 | 0 | 0 | 0 | 0 | - | 0 |
| Nutsedge, Yellow | 0 | 0 | 0 | 0 | 0 | 0 | 0 | 0 | 0 | 0 | 100 | 0 | 0 | 0 |
| Oat, Wild | 60 | 70 | 80 | 95 | 95 | 95 | 20 | 95 | 70 | 40 | 5 | 70 | 70 | 60 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | - | - | 100 | 0 | 0 | 0 | 0 | 0 | 0 | 0 |
| Russian Thistle | 0 | 0 | 20 | 0 | 0 | 0 | 0 | 0 | 0 | 0 | 0 | 0 | 0 | 0 |
| Ryegrass, Italian | 98 | 95 | 100 | 100 | 100 | 100 | 60 | 100 | 100 | 98 | 95 | 100 | 100 | 40 |
| Soybean | - | 0 | 0 | 0 | 0 | - | - | 0 | 0 | 0 | 0 | 0 | 98 | 0 |
| Sunflower | 0 | 0 | 0 | 0 | 0 | 0 | 0 | 0 | 0 | 0 | 0 | 0 | 0 | 0 |
| Surinam Grass | 100 | 100 | 98 | 100 | 98 | 0 | 15 | 85 | 100 | 80 | 95 | 100 | 100 | 98 |
| Velvetleaf | 0 | 0 | 0 | 0 | 0 | 0 | 0 | 0 | 0 | 0 | 0 | 0 | 0 | 0 |
| Wheat | 35 | 40 | 40 | 90 | 95 | 100 | 0 | 80 | 25 | 20 | 0 | 90 | 0 | 60 |

| Table B | Compounds | | | | | | Table B | | | | Compounds | | | |
|---|---|---|---|---|---|---|---|---|---|---|---|---|---|---|
| 31 g ai/ha | 81 | 82 | 83 | | | | 31 g ai/ha | | | | 81 | 82 | 83 | |
| Preemergence | | | | | | | Preemergence | | | | | | | |
| Bermudagrass | 100 | 100 | 100 | | | | Morningglory | | | | 0 | 0 | 0 | |
| Blackgrass | 100 | 100 | 80 | | | | Nightshade, E. B. | | | | 0 | 0 | 0 | |
| Bromegrass, Downy | 70 | 98 | 20 | | | | Nutsedge, Yellow | | | | 0 | 0 | 0 | |
| Cocklebur | 0 | 0 | 0 | | | | Oat, Wild | | | | 70 | 80 | 80 | |
| Corn | 98 | 98 | 95 | | | | Pigweed | | | | 0 | 0 | 0 | |
| Crabgrass, Large | 100 | 100 | 85 | | | | Ragweed | | | | 0 | 50 | 0 | |
| Cupgrass, Woolly | 95 | 98 | 100 | | | | Russian Thistle | | | | 0 | 0 | - | |
| Foxtail, Giant | 100 | 100 | 100 | | | | Ryegrass, Italian | | | | 100 | 100 | 100 | |
| Foxtail, Green | 100 | 100 | 80 | | | | Soybean | | | | 0 | 20 | 0 | |
| Galium | 0 | 50 | 0 | | | | Sunflower | | | | 15 | 70 | 0 | |
| Goosegrass | 100 | 98 | 95 | | | | Surinam Grass | | | | 100 | 95 | 98 | |
| Johnsongrass | 95 | 98 | 95 | Velvetleaf | | | | | | | | 0 | 45 | 0 |
| Kochia | - | - | 100 | Wheat | | | | | | | | 50 | 98 | 0 |
| Lambsquarters | 0 | 100 | 100 | | | | | | | | | | | |

| Table B | Compounds | | | | | | | | | | | | | |
|---|---|---|---|---|---|---|---|---|---|---|---|---|---|---|
| 16 g ai/ha | 11 | 12 | 19 | 20 | 21 | 25 | 26 | 27 | 28 | 30 | 31 | 32 | 33 | 34 |
| Preemergence | | | | | | | | | | | | | | |
| Bermudagrass | 100 | 100 | 90 | 0 | 80 | 70 | 0 | 0 | 100 | 90 | 100 | 0 | 98 | 98 |
| Blackgrass | 100 | 98 | 100 | - | 100 | - | - | 20 | - | 60 | 90 | 85 | 100 | 85 |
| Bromegrass, Downy | 50 | 0 | 0 | 0 | 50 | 0 | 0 | 0 | 40 | 10 | 0 | 0 | 0 | 55 |
| Cocklebur | - | 0 | 0 | 0 | 0 | 0 | 0 | 0 | 0 | 0 | 0 | 0 | 0 | 0 |
| Corn | 70 | 70 | 85 | 5 | 80 | 5 | 30 | 0 | 0 | 5 | 0 | 45 | 85 | 65 |
| Crabgrass, Large | 90 | 100 | 100 | 50 | 80 | 20 | 65 | 0 | 98 | 90 | 90 | 85 | 95 | 100 |
| Cupgrass, Woolly | 100 | 80 | 85 | 30 | 100 | 40 | 70 | 0 | 30 | 75 | 98 | 70 | 90 | 90 |
| Foxtail, Giant | 90 | 100 | 100 | 0 | 100 | 60 | 95 | 0 | 98 | 98 | 90 | 95 | 100 | 100 |
| Foxtail, Green | 100 | 100 | 100 | 95 | 100 | 98 | 100 | 0 | 100 | 0 | 98 | 100 | 100 | 100 |
| Galium | 0 | 0 | - | 0 | 0 | 0 | 0 | - | - | 0 | 0 | 0 | 0 | 0 |
| Goosegrass | 85 | 40 | 75 | 0 | 80 | 50 | 50 | 75 | 100 | 80 | 90 | 98 | 98 | 85 |
| Johnsongrass | 90 | 70 | 100 | 50 | 85 | 0 | 0 | 40 | 85 | 50 | 60 | 75 | 95 | 95 |
| Kochia | - | 0 | 0 | 0 | 0 | 0 | - | - | - | 0 | 0 | 0 | - | 0 |
| Lambsquarters | 0 | 0 | 0 | 20 | 0 | 0 | 0 | 0 | 95 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nightshade, E. B. | - | 0 | 0 | 0 | 0 | 0 | 0 | 0 | 0 | 0 | 0 | 0 | 0 | 0 |
| Nutsedge, Yellow | 0 | 0 | 0 | - | 0 | 0 | 0 | 0 | 0 | 0 | 0 | 0 | 0 | 0 |
| Oat, Wild | 80 | 60 | 75 | 0 | 85 | 0 | 80 | 0 | 50 | 50 | 45 | 0 | 80 | - |
| Pigweed | - | - | 0 | 0 | 0 | 0 | 0 | 0 | 0 | 0 | 0 | 0 | 0 | 0 |
| Ragweed | 0 | 0 | 0 | - | 0 | 0 | 0 | 0 | 0 | 0 | 0 | 0 | 0 | 0 |
| Russian Thistle | - | - | 0 | 0 | 0 | 0 | 0 | 0 | 0 | 0 | 0 | 0 | 0 | 0 |
| Ryegrass, Italian | 90 | 90 | 90 | 35 | 100 | 50 | 90 | 0 | 80 | 10 | 90 | 100 | 95 | 100 |
| Soybean | 0 | 0 | - | - | - | 0 | 0 | 0 | - | 0 | 0 | 0 | 0 | 0 |
| Sunflower | 0 | 0 | 0 | 0 | 0 | 0 | 0 | 0 | 0 | 0 | 0 | 0 | 0 | 0 |
| Surinam Grass | 100 | 100 | 80 | 20 | 100 | 85 | 85 | 0 | 65 | 60 | 80 | 75 | 90 | 100 |
| Velvetleaf | 0 | 0 | 0 | 0 | - | 0 | 0 | 0 | 0 | 0 | 0 | 0 | 0 | 0 |
| Wheat | 90 | 20 | 10 | 0 | 30 | 0 | 0 | 0 | 5 | 0 | 0 | 0 | 0 | 0 |

| Table B | Compounds | | | | | | | | | | | | | |
|---|---|---|---|---|---|---|---|---|---|---|---|---|---|---|
| 16 g ai/ha | 35 | 36 | 37 | 38 | 39 | 40 | 41 | 43 | 44 | 45 | 46 | 47 | 48 | 50 |
| Preemergence | | | | | | | | | | | | | | |
| Bermudagrass | 98 | 100 | 90 | 100 | 90 | 90 | 0 | 0 | 0 | 0 | 20 | 100 | 0 | 100 |
| Blackgrass | 85 | 80 | 100 | - | 90 | 95 | 100 | 95 | 100 | 98 | 30 | 70 | 90 | 0 |
| Bromegrass, Downy | 40 | 50 | 30 | 55 | 70 | 60 | 70 | 0 | 20 | 50 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 40 | 55 | 40 | 50 | 80 | 75 | 65 | 70 | 55 | 55 | 0 | 20 | 55 | 0 |
| Crabgrass, Large | 100 | 98 | 98 | 98 | 100 | 100 | 0 | 65 | 0 | 0 | 0 | 85 | 0 | 98 |
| Cupgrass, Woolly | 35 | 85 | 50 | 50 | 65 | 100 | 0 | 60 | 0 | 0 | 0 | 100 | 0 | 75 |
| Foxtail, Giant | 100 | 100 | 100 | 98 | 100 | 100 | 0 | 100 | 0 | 0 | 15 | 98 | 0 | 98 |
| Foxtail, Green | 100 | 100 | 98 | 100 | 100 | 100 | 100 | 100 | 98 | 100 | 10 | 80 | 80 | 95 |
| Galium | 0 | 0 | 0 | 0 | 0 | 0 | 0 | 0 | - | 0 | 0 | 0 | 0 | 0 |
| Goosegrass | 100 | 100 | 90 | 85 | 100 | 100 | 0 | 75 | 0 | 0 | 100 | 98 | 0 | 80 |
| Johnsongrass | 80 | - | 50 | - | 90 | 95 | 65 | 65 | 0 | 20 | 55 | 75 | 60 | 95 |
| Kochia | 0 | - | 0 | 0 | 0 | 0 | 0 | 0 | 0 | 0 | - | - | 0 | - |
| Lambsquarters | 0 | 0 | 0 | 0 | 0 | 0 | 0 | 0 | 0 | 0 | 0 | - | 0 | 65 |
| Morningglory | 0 | 0 | 0 | 0 | 0 | 0 | 0 | 0 | 0 | 0 | 0 | 0 | 0 | 0 |
| Nightshade, E. B. | 0 | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | - | 50 | 65 | 60 | 90 | 85 | 85 | 90 | 80 | 80 | 0 | 0 | 5 | 40 |
| Pigweed | 0 | 0 | 0 | 0 | 0 | 0 | 0 | 0 | 0 | 0 | 0 | - | 0 | 0 |
| Ragweed | 0 | 0 | 0 | 0 | 0 | 0 | 0 | 0 | 0 | 0 | 0 | 0 | 0 | 0 |
| Russian Thistle | 0 | 0 | 0 | 0 | 0 | 0 | - | 0 | 0 | 0 | 0 | 0 | 0 | 0 |
| Ryegrass, Italian | 100 | 98 | 90 | 80 | 95 | 95 | 85 | 90 | 90 | 90 | 10 | 80 | 85 | 0 |
| Soybean | 0 | 0 | 0 | 0 | 0 | 0 | 0 | 0 | - | 0 | 0 | 0 | 0 | 50 |
| Sunflower | 0 | 0 | 0 | 0 | 0 | 0 | 0 | 0 | 0 | 0 | 0 | 0 | 0 | 0 |
| Surinam Grass | 90 | 100 | 85 | 90 | 95 | 100 | 40 | 90 | 0 | 20 | 0 | 95 | 40 | 40 |
| Velvetleaf | 0 | 0 | 0 | 0 | 0 | 0 | 0 | 0 | 0 | 0 | 0 | 0 | 0 | 0 |
| Wheat | 0 | 0 | 0 | 0 | 30 | 60 | 15 | 10 | 0 | 30 | 0 | 0 | 0 | 0 |

| Table B | Compounds | | | | | | | | | | | | | |
|---|---|---|---|---|---|---|---|---|---|---|---|---|---|---|
| 16 g ai/ha | 51 | 52 | 53 | 54 | 55 | 56 | 57 | 58 | 61 | 66 | 67 | 69 | 72 | 74 |
| Preemergence | | | | | | | | | | | | | | |
| Bermudagrass | 100 | 0 | 0 | 0 | 100 | 100 | 100 | 100 | 80 | 85 | 0 | 0 | 100 | 100 |
| Blackgrass | 98 | 95 | 100 | 90 | 80 | 60 | 100 | 100 | 100 | 100 | 100 | 0 | 90 | 100 |
| Bromegrass, Downy | 0 | 35 | 50 | 0 | 0 | 20 | 0 | - | 90 | 85 | 90 | 0 | 30 | 85 |
| Cocklebur | 0 | 0 | 0 | 0 | - | 0 | 0 | 0 | 0 | 0 | 0 | 0 | 0 | 0 |
| Corn | - | 55 | 45 | 0 | 0 | 10 | 60 | 0 | 98 | 70 | 98 | 0 | 80 | 80 |
| Crabgrass, Large | 20 | 0 | 0 | 0 | 98 | 60 | 0 | 75 | 95 | 0 | 0 | 0 | 50 | 95 |
| Cupgrass, Woolly | 80 | 0 | 0 | 0 | 80 | 80 | 20 | 80 | 98 | 80 | 0 | 0 | - | 95 |
| Foxtail, Giant | 100 | 0 | 20 | 0 | 98 | 100 | 98 | 95 | 100 | 45 | 0 | 0 | 85 | 98 |
| Foxtail, Green | 90 | 90 | 100 | 95 | 90 | 65 | 100 | 100 | 100 | 100 | 100 | 0 | 100 | 100 |
| Galium | 0 | 0 | - | 0 | - | 0 | 0 | 0 | - | 0 | 0 | 0 | 0 | 0 |
| Goosegrass | 60 | 0 | 0 | 0 | 90 | 80 | 0 | 85 | 100 | 0 | 0 | 0 | 95 | 98 |
| Johnsongrass | 50 | 65 | 65 | 40 | 15 | 65 | 90 | 0 | 95 | 95 | 90 | 0 | 55 | 85 |
| Kochia | - | 0 | - | - | - | 20 | 0 | - | - | - | - | - | - | - |
| Lambsquarters | - | 0 | 0 | 0 | 0 | 0 | 0 | 0 | 0 | - | - | - | 0 | 0 |
| Morningglory | 0 | 0 | 0 | 0 | 0 | 0 | 0 | 0 | 0 | 0 | 0 | 0 | 0 | 0 |
| Nightshade, E. B, | - | 0 | 0 | 0 | 0 | 0 | 0 | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 0 |
| Oat, Wild | 10 | 30 | 65 | 70 | 0 | 50 | 60 | 80 | 90 | 95 | 95 | 0 | 80 | 70 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | - | 98 | 0 | 0 |
| Russian Thistle | 0 | 0 | 0 | 0 | 0 | 0 | 0 | 0 | 0 | 0 | 0 | 0 | 0 | 0 |
| Ryegrass, Italian | 100 | 95 | 100 | 90 | 70 | 98 | 70 | 100 | 100 | 100 | 100 | 20 | 90 | 98 |
| Soybean | 0 | 0 | 0 | 0 | 0 | 0 | 0 | 0 | 0 | 0 | - | - | 0 | 0 |
| Sunflower | 0 | 0 | 0 | 0 | 0 | 0 | 0 | 0 | 0 | 0 | 0 | 0 | 0 | 0 |
| Surinam Grass | 75 | 0 | 20 | 0 | 50 | 90 | 98 | 98 | 100 | 80 | 0 | 0 | 85 | 100 |
| Velvetleaf | 0 | 0 | 0 | 0 | 0 | 0 | 0 | 0 | 0 | 0 | 0 | 0 | 0 | 0 |
| Wheat | 0 | 0 | 0 | 0 | 0 | 10 | 15 | 0 | 30 | 90 | 70 | 0 | 35 | 0 |

| Table B | Compounds | | | | | | | | | | | | | |
|---|---|---|---|---|---|---|---|---|---|---|---|---|---|---|
| 16 g ai/ha | 75 | 76 | 78 | 79 | 80 | 81 | 82 | 83 | | | | | | |
| Preemergence | | | | | | | | | | | | | | |
| Bermudagrass | 0 | 20 | 100 | 100 | 100 | 50 | 100 | 65 | | | | | | |
| Blackgrass | 20 | 40 | 90 | 85 | 70 | 85 | 98 | 30 | | | | | | |
| Bromegrass, Downy | 20 | 0 | 60 | 0 | 30 | 70 | 50 | 0 | | | | | | |
| Cocklebur | 0 | 0 | 0 | 0 | 0 | 0 | 0 | 0 | | | | | | |
| Corn | 0 | 55 | 85 | 0 | 85 | 70 | 95 | 45 | | | | | | |
| Crabgrass, Large | 0 | 20 | 95 | 100 | 98 | 98 | 98 | 35 | | | | | | |
| Cupgrass, Woolly | 0 | 75 | 98 | 65 | 65 | 45 | 80 | 90 | | | | | | |
| Foxtail, Giant | 0 | 70 | 100 | 100 | 100 | 100 | 100 | 100 | | | | | | |
| Foxtail, Green | 30 | 65 | 100 | 60 | 98 | 80 | 100 | 70 | | | | | | |
| Galium | 0 | 0 | 0 | 0 | 0 | 0 | - | 0 | | | | | | |
| Goosegrass | 65 | 75 | 100 | 100 | 20 | 98 | 98 | 80 | | | | | | |
| Johnsongrass | 75 | 0 | 80 | 85 | 65 | 80 | 85 | 85 | | | | | | |
| Kochia | - | - | - | - | - | - | - | 100 | | | | | | |
| Lambsquarters | 0 | 0 | 0 | 0 | 0 | 0 | 0 | 100 | | | | | | |
| Morningglory | 0 | 0 | 0 | 0 | 0 | 0 | 0 | 0 | | | | | | |
| Nightshade, E. B. | 0 | 0 | 0 | 0 | - | 0 | 0 | 0 | | | | | | |
| Nutsedge, Yellow | 0 | 100 | 0 | 0 | 0 | 0 | 0 | 0 | | | | | | |
| Oat, Wild | 0 | 0 | 40 | 0 | 40 | 60 | 60 | 30 | | | | | | |
| Pigweed | 0 | 0 | 0 | 0 | 0 | 0 | - | 0 | | | | | | |
| Ragweed | 0 | 0 | 0 | 0 | 0 | 0 | 20 | 0 | | | | | | |
| Russian Thistle | 0 | 0 | 0 | 0 | 0 | 0 | 0 | - | | | | | | |
| Ryegrass, Italian | 30 | 75 | 98 | 90 | 40 | 100 | 98 | 85 | | | | | | |
| Soybean | 0 | 0 | 0 | 0 | 0 | 0 | 0 | 0 | | | | | | |
| Sunflower | 0 | 0 | 0 | 0 | 0 | 0 | 60 | 0 | | | | | | |
| Surinam Grass | 50 | 90 | 95 | 85 | 65 | 85 | 85 | 90 | | | | | | |
| Velvetleaf | 0 | 0 | 0 | 0 | 0 | 0 | 0 | 0 | | | | | | |
| Wheat | 0 | 0 | 80 | 0 | 20 | 5 | 65 | 0 | | | | | | |

| Table B | Compounds | | | | | | | | | | | | | |
|---|---|---|---|---|---|---|---|---|---|---|---|---|---|---|
| 8 g ai/ha | 19 | 20 | 21 | 25 | 26 | 27 | 28 | 30 | 31 | 32 | 33 | 34 | 35 | 36 |
| Preemergence | | | | | | | | | | | | | | |
| Bermudagrass | 0 | 0 | 60 | 0 | 0 | - | 0 | 60 | 95 | 0 | 80 | 50 | 98 | 98 |
| Blackgrass | 95 | 10 | 95 | 70 | - | - | - | 0 | 80 | - | 60 | 0 | 0 | 80 |
| Bromegrass, Downy | 0 | 0 | 0 | 0 | 0 | 0 | 20 | 0 | 0 | 0 | 0 | 0 | 40 | 0 |
| Cocklebur | 0 | 0 | 0 | 0 | 0 | 0 | 0 | 0 | 0 | 0 | 0 | 0 | 0 | 0 |
| Corn | 70 | 0 | 70 | 0 | 15 | 0 | 0 | 0 | - | 0 | 50 | 0 | 5 | 40 |
| Crabgrass, Large | 50 | 0 | 0 | 0 | 0 | 0 | 65 | 80 | 80 | 20 | 70 | 100 | 90 | 98 |
| Cupgrass, Woolly | 50 | 0 | 60 | 0 | 20 | 0 | 0 | 50 | 70 | 55 | 80 | 55 | 35 | 85 |
| Foxtail, Giant | 70 | 0 | 85 | 50 | 65 | 0 | 60 | 60 | 90 | 85 | 100 | 98 | 100 | 100 |
| Foxtail, Green | 85 | 20 | 100 | 50 | 98 | 0 | 30 | 0 | 65 | 70 | 100 | 100 | 30 | 50 |
| Galium | 0 | 0 | 0 | 0 | 0 | - | - | 0 | 0 | 0 | 0 | 0 | 0 | 0 |
| Goosegrass | 0 | 0 | 10 | 0 | 50 | 0 | 80 | 80 | 85 | 60 | 85 | 55 | 80 | 80 |
| Johnsongrass | 80 | 0 | 0 | 0 | 0 | 0 | 70 | 0 | 55 | 65 | 60 | 65 | 45 | 0 |
| Kochia | 0 | - | 0 | 0 | 0 | - | - | 0 | 0 | 0 | 0 | 0 | 0 | - |
| Lambsquarters | - | 0 | 0 | 0 | 0 | 0 | 80 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Nightshade, E. B. | 0 | 0 | 0 | 0 | 0 | 0 | 0 | 0 | 0 | 0 | 0 | 0 | 0 | 0 |
| Nutsedge, Yellow | 0 | 0 | - | 0 | 0 | 0 | 0 | 0 | 0 | 0 | 0 | 0 | 0 | 0 |
| Oat, Wild | 60 | 0 | 65 | 0 | - | 0 | 0 | 50 | - | 0 | 70 | 40 | 0 | 0 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Russian Thistle | 0 | - | 0 | 0 | 0 | 0 | 0 | 0 | 0 | 0 | 0 | 0 | 0 | 0 |
| Ryegrass, Italian | 50 | 30 | 90 | 50 | 50 | 0 | 0 | 0 | 50 | 90 | 85 | 100 | 100 | 20 |
| Soybean | 0 | - | - | 0 | 0 | 0 | - | 0 | 0 | 0 | 0 | 0 | 0 | 0 |
| Sunflower | 0 | 0 | - | 0 | 0 | 0 | 0 | 0 | 0 | 0 | 0 | 0 | 0 | 0 |
| Surinam Grass | 80 | 0 | 98 | 50 | 75 | 0 | 0 | 10 | 80 | 70 | 75 | 95 | 45 | 70 |
| Velvetleaf | 0 | 0 | 0 | 0 | 0 | 0 | 0 | 0 | 0 | 0 | 0 | 0 | 0 | 0 |
| Wheat | 0 | 0 | 0 | 0 | 0 | 0 | 0 | 0 | 0 | 0 | 0 | 0 | 0 | 0 |

| Table B | Compounds | | | | | | | | | | | | | |
|---|---|---|---|---|---|---|---|---|---|---|---|---|---|---|
| 8 g ai/ha | 37 | 38 | 39 | 40 | 41 | 43 | 44 | 45 | 46 | 47 | 48 | 50 | 51 | 52 |
| Preemergence | | | | | | | | | | | | | | |
| Bermudagrass | 90 | 60 | 0 | 0 | 0 | 0 | 0 | 0 | 0 | 60 | 0 | 90 | 60 | 0 |
| Blackgrass | 95 | 90 | 40 | 95 | 95 | 70 | 90 | - | 0 | 70 | 80 | 0 | 50 | 0 |
| Bromegrass, Downy | 0 | 55 | 0 | 0 | 70 | 0 | 0 | 0 | 0 | 0 | 0 | 0 | 0 | 0 |
| Cocklebur | 0 | 0 | 0 | 0 | 0 | 0 | 0 | 0 | 0 | 0 | 0 | 0 | 0 | 0 |
| Corn | 40 | 45 | 65 | 55 | 45 | 0 | 0 | 0 | 0 | 0 | 15 | 0 | - | 0 |
| Crabgrass, Large | 70 | 45 | 60 | 45 | 0 | 15 | 0 | 0 | 0 | 0 | 0 | 60 | 0 | 0 |
| Cupgrass, Woolly | 50 | 0 | 20 | 15 | 0 | 0 | - | 0 | 0 | 98 | 0 | 20 | 0 | 0 |
| Foxtail, Giant | 100 | 45 | 100 | 100 | 0 | 95 | 0 | 0 | 0 | 0 | 0 | 20 | 80 | 0 |
| Foxtail, Green | 40 | 30 | 80 | 90 | 100 | 100 | 40 | 100 | 0 | 5 | 30 | 0 | 50 | 90 |
| Galium | 0 | 0 | 0 | 0 | 0 | 0 | 0 | 0 | 0 | 0 | - | 0 | 0 | 0 |
| Goosegrass | 75 | 45 | 95 | 98 | 0 | 65 | 0 | 0 | 0 | 70 | 0 | 0 | 0 | 0 |
| Johnsongrass | 50 | 0 | 75 | 90 | 20 | 20 | 0 | 15 | - | 0 | 40 | 70 | - | 55 |
| Kochia | 0 | 0 | 0 | 0 | 0 | 0 | 0 | 0 | - | - | 0 | - | - | 0 |
| Lambsquarters | 0 | 0 | 0 | 0 | 0 | 0 | 0 | 0 | 0 | 0 | 0 | 0 | - | 0 |
| Morningglory | 0 | 0 | 0 | 0 | 0 | 0 | 0 | 0 | 0 | 0 | 0 | 0 | 0 | 0 |
| Nightshade, E. B. | 0 | 0 | 0 | 0 | 0 | 0 | 0 | 0 | 0 | 0 | 0 | 0 | - | 0 |
| Nutsedge, Yellow | 0 | 0 | 0 | 0 | 0 | 0 | 0 | 0 | 0 | 0 | 0 | 0 | 0 | 0 |
| Oat, Wild | 0 | 0 | 70 | 60 | 70 | 70 | 25 | 15 | 0 | 0 | 0 | 20 | 0 | 0 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | 0 | 0 | 0 | 0 | 0 | 0 |
| Russian Thistle | 0 | 0 | 0 | 0 | - | 0 | 0 | 0 | 0 | 0 | 0 | 0 | 0 | 0 |
| Ryegrass, Italian | 45 | 30 | 90 | 80 | 85 | 90 | 20 | 0 | 0 | 0 | 85 | 0 | 100 | 0 |
| Soybean | 0 | 0 | 0 | 0 | 0 | 0 | 0 | - | 0 | 0 | 0 | 0 | 0 | 0 |
| Sunflower | 0 | 0 | 0 | 0 | 0 | 0 | 0 | 0 | 0 | 0 | 0 | 0 | 0 | 0 |
| Surinam Grass | 65 | 90 | 90 | 80 | 0 | 0 | 0 | 0 | 0 | 80 | 0 | 20 | 30 | 0 |
| Velvetleaf | 0 | 0 | 0 | 0 | 0 | 0 | 0 | 0 | 0 | 0 | 0 | 0 | 0 | 0 |
| Wheat | 0 | 0 | 0 | 5 | 10 | 0 | 0 | 0 | 0 | 0 | 0 | 0 | 0 | 0 |

| Table B | Compounds | | | | | | | | | | | | | |
|---|---|---|---|---|---|---|---|---|---|---|---|---|---|---|
| 8 g ai/ha | 53 | 54 | 55 | 56 | 57 | 58 | 61 | 66 | 67 | 69 | 72 | 74 | 75 | 76 |
| Preemergence | | | | | | | | | | | | | | |
| Bermudagrass | 0 | 0 | 100 | 85 | 100 | 100 | 55 | 0 | 0 | 0 | 40 | 100 | - | 0 |
| Blackgrass | 95 | 60 | 0 | 40 | 40 | 80 | 40 | 90 | 100 | 0 | 60 | 85 | 0 | 40 |
| Bromegrass, Downy | 10 | 0 | 0 | 0 | 0 | 0 | 0 | 85 | 50 | 0 | 0 | 30 | 0 | 0 |
| Cocklebur | 0 | 0 | 0 | 0 | 0 | 0 | 0 | 0 | 0 | 0 | 0 | 0 | 0 | 0 |
| Corn | 0 | 0 | 0 | 0 | 0 | 0 | 95 | 0 | 60 | 0 | 70 | 75 | 0 | 0 |
| Crabgrass, Large | 0 | 0 | 20 | 0 | 0 | 50 | 50 | 0 | 0 | 0 | 30 | 65 | 0 | 0 |
| Cupgrass, Woolly | 0 | 0 | 15 | 0 | 0 | 75 | 80 | 0 | 0 | 0 | 70 | 65 | 0 | 55 |
| Foxtail, Giant | 0 | 0 | 90 | 95 | 98 | 55 | 75 | 0 | 0 | 0 | 70 | 80 | 0 | 0 |
| Foxtail, Green | 95 | 55 | 0 | 65 | 70 | 100 | 100 | 98 | 100 | 0 | 25 | 100 | 0 | 40 |
| Galium | 0 | 0 | 0 | 0 | 0 | 0 | 0 | 0 | 0 | 0 | 0 | 0 | 0 | 0 |
| Goosegrass | 0 | 0 | 55 | 75 | 0 | 70 | 80 | 0 | 0 | 0 | 95 | 95 | 50 | 20 |
| Johnsongrass | 45 | 0 | 0 | 60 | 65 | 0 | 90 | 70 | 70 | 0 | 50 | 70 | 0 | 0 |
| Kochia | - | - | - | 0 | 0 | - | - | - | - | - | - | - | - | - |
| Lambsquarters | 0 | 0 | 0 | 0 | 0 | 0 | 0 | - | - | - | 0 | 0 | 0 | 0 |
| Morningglory | 0 | 0 | 0 | 0 | 0 | 0 | 0 | 0 | 0 | 0 | 0 | 0 | 0 | 0 |
| Nightshade, E. B. | 0 | 0 | 0 | 0 | 0 | 0 | - | 0 | 0 | 0 | 0 | 0 | 0 | 0 |
| Nutsedge, Yellow | 0 | 0 | 0 | 0 | 0 | 0 | 0 | 0 | 0 | 0 | 0 | 0 | 0 | 100 |
| Oat, Wild | 40 | 40 | 0 | 0 | 0 | 80 | 40 | 90 | 90 | 0 | 60 | 50 | 0 | 0 |
| Pigweed | 0 | 0 | 0 | 0 | 0 | 0 | 0 | 0 | 0 | 0 | 0 | 0 | 0 | 0 |
| Ragweed | 0 | 0 | 0 | 0 | 0 | 0 | 0 | 0 | - | 0 | 0 | 0 | 0 | 0 |
| Russian Thistle | 0 | 0 | 0 | 0 | 0 | 0 | 0 | 0 | 0 | 0 | 0 | 0 | 0 | 0 |
| Ryegrass, Italian | 80 | 20 | 60 | 60 | 0 | 0 | 85 | 100 | 100 | 0 | 75 | 60 | 0 | 75 |
| Soybean | 0 | 0 | 0 | 0 | 0 | 0 | 0 | 0 | - | - | 0 | 0 | 0 | 0 |
| Sunflower | 0 | 0 | 0 | 0 | 0 | 0 | 0 | 0 | 0 | 0 | 0 | 0 | 0 | 0 |
| Surinam Grass | 0 | 0 | 15 | 75 | 60 | 95 | 85 | 0 | 0 | 0 | - | 100 | 45 | 65 |
| Velvetleaf | 0 | 0 | 0 | 0 | 0 | 0 | 0 | 0 | 0 | 0 | 0 | 0 | 0 | 0 |
| Wheat | 0 | 0 | 0 | 0 | 0 | 0 | 20 | 30 | 0 | 0 | 0 | 0 | 0 | 0 |

| Table B | Compounds | | | | | | | | | | | | | |
|---|---|---|---|---|---|---|---|---|---|---|---|---|---|---|
| 8 g ai/ha | 78 | 79 | 80 | 81 | 82 | 83 | | | | | | | | |
| Preemergence | | | | | | | | | | | | | | |
| Bermudagrass | 85 | 90 | 50 | 0 | 90 | 55 | | | | | | | | |
| Blackgrass 70 | | 85 | 70 | 80 | 80 | 0 | | | | | | | | |
| Bromegrass, Downy | 40 | 0 | 0 | 20 | 30 | 0 | | | | | | | | |
| Cocklebur | 0 | 0 | 0 | 0 | 0 | 0 | | | | | | | | |
| Corn | 55 | 0 | 20 | 65 | 95 | 0 | | | | | | | | |
| Crabgrass, Large | 15 | 20 | 0 | 65 | 95 | 0 | | | | | | | | |
| Cupgrass, Woolly | 65 | 20 | 0 | 20 | 45 | 65 | | | | | | | | |
| Foxtail, Giant | 98 | 55 | 0 | 80 | 98 | 80 | | | | | | | | |
| Foxtail, Green | 100 | 30 | 0 | 40 | 70 | 40 | | | | | | | | |
| Galium | 0 | 0 | 0 | 0 | - | 0 | | | | | | | | |
| Goosegrass | 85 | 98 | 0 | 80 | 95 | 55 | | | | | | | | |
| Johnsongrass | 60 | 65 | 60 | 60 | 70 | 55 | | | | | | | | |
| Kochia | - | - | - | - | - | 50 | | | | | | | | |
| Lambsquarters | 0 | 0 | 0 | 0 | 0 | 100 | | | | | | | | |
| Morningglory | 0 | 0 | 0 | 0 | 0 | 0 | | | | | | | | |
| Nightshade, E. B. | 0 | 0 | 0 | 0 | 0 | 0 | | | | | | | | |
| Nutsedge, Yellow | 0 | 0 | 0 | 0 | 0 | 0 | | | | | | | | |
| Oat, Wild | 30 | 0 | 30 | 45 | 0 | 0 | | | | | | | | |
| Pigweed | 0 | 0 | 0 | 0 | 0 | 0 | | | | | | | | |
| Ragweed | 0 | 0 | 0 | 0 | 0 | 0 | | | | | | | | |
| Russian Thistle | 0 | 0 | 0 | 0 | 0 | - | | | | | | | | |
| Ryegrass, Italian | 98 | 0 | 0 | 70 | 40 | 30 | | | | | | | | |
| Soybean | 0 | 0 | 0 | 0 | 0 | 0 | | | | | | | | |
| Sunflower | 0 | 0 | 0 | 0 | 0 | 0 | | | | | | | | |
| Surinam Grass | 95 | 75 | 0 | 60 | 80 | 55 | | | | | | | | |
| Velvetleaf | 0 | 0 | 0 | 0 | 0 | 0 | | | | | | | | |
| Wheat | 0 | 0 | 0 | 0 | 0 | 0 | | | | | | | | |

### TEST C

Seeds of plant species selected from annual bluegrass (*Poa annua*), blackgrass (*Alopecurus myosuroides*), canola (*Brassica rapa*), downy bromegrass (*Bromus tectorum*), green foxtail (*Setaria viridis*), Italian ryegrass (*Lolium multiflorum*), canarygrass (*Phalaris minor*), pigweed (*Amaranthus retroflexus*), spring barley (*Hordeum vulgare*), spring wheat (*Triticum aestivum*), wild mustard (*Sinapis arvensis*), wild oat (*Avena fatua*), windgrass (*Apera spica-venti*), winter barley (*Hordeum vulgare*), and winter wheat (*Triticum aestivum*) were planted and treated preemergence with test chemicals formulated in a non-phytotoxic solvent mixture which included a surfactant. At the same time, plants selected from these crop and weed species were treated with postemergence applications of some of the test chemicals formulated in the same manner. Plants ranged in height from 2 to 18 cm (1- to 4-leaf stage) for postemergence treatments.
Treated plants and controls were maintained in a controlled growth environment for 15 to 25 days after which time all species were compared to controls and visually evaluated. Plant response ratings, summarized in Table C, are based on a scale of 0 to 100 where 0 is no effect and 100 is complete control. A dash (-) response means no test result.

| Table C | Compounds | | | | | | | | | | | | | |
|---|---|---|---|---|---|---|---|---|---|---|---|---|---|---|
| 62 g ai/ha | 11 | 15 | 19 | 20 | 25 | 26 | 37 | 43 | 44 | 53 | 57 | | | |

| Postemergence | | | | | | | | | | | | | | |
|---|---|---|---|---|---|---|---|---|---|---|---|---|---|---|
| Barley, Spring | 98 | 50 | 100 | 50 | - | 50 | 100 | 90 | 98 | 100 | 40 | | | |
| Barley, Winter | 100 | 90 | 100 | 20 | - | 40 | 95 | 80 | 95 | 95 | 90 | | | |
| Blackgrass | 100 | 100 | 100 | 75 | 98 | 95 | 100 | 100 | 100 | 100 | - | | | |
| Bluegrass | 20 | 50 | 35 | 0 | 15 | 15 | 20 | 15 | 0 | 35 | 35 | | | |
| Bromegrass, Downy | 98 | 30 | 100 | 10 | 20 | 0 | 70 | 75 | 75 | 90 | 70 | | | |
| Canarygrass | 100 | - | - | 95 | 40 | 100 | 100 | 95 | 100 | - | - | | | |
| Canola | 0 | 0 | 0 | 0 | 0 | 0 | 0 | 0 | 0 | 0 | 0 | | | |
| Foxtail, Green | 100 | 98 | 100 | 98 | 98 | 98 | 100 | 98 | 100 | 100 | 100 | | | |
| Mustard, Wild | 40 | 20 | 20 | 0 | 0 | - | 0 | 25 | 0 | 0 | 0 | | | |
| Oat, Wild | 100 | 90 | 100 | 100 | 95 | 100 | 95 | 100 | 100 | 100 | 100 | | | |
| Pigweed | 20 | 0 | 0 | 0 | 20 | 20 | 0 | 0 | 0 | 0 | 0 | | | |
| Ryegrass, Italian | 100 | 100 | 100 | 98 | 95 | 100 | 100 | 100 | 100 | 100 | 100 | | | |
| Wheat, Spring | 98 | 70 | 100 | 60 | - | 30 | 95 | 90 | 100 | 98 | 75 | | | |
| Wheat, Winter | 100 | 25 | 100 | 60 | - | 35 | 90 | 65 | 98 | 95 | 50 | | | |
| Windgrass | 95 | 40 | 100 | 75 | 60 | 65 | 30 | 60 | 100 | 70 | 65 | | | |

| Table C | Compounds | | | | | | | | | | | | | |
|---|---|---|---|---|---|---|---|---|---|---|---|---|---|---|
| 31 g ai/ha | 11 | 15 | 19 | 20 | 25 | 26 | 37 | 43 | 44 | 53 | 57 | | | |
| Postemergence | | | | | | | | | | | | | | |
| Barley, Spring | 95 | 0 | 100 | 25 | 0 | 35 | 50 | 50 | 95 | 40 | 30 | | | |
| Barley, Winter | 95 | 10 | 100 | 20 | 0 | 10 | 25 | 40 | 90 | 40 | 15 | | | |
| Blackgrass | 100 | 98 | 100 | 60 | 90 | 70 | 100 | 100 | 100 | 100 | 98 | | | |
| Bluegrass | 15 | 40 | 25 | 0 | 15 | 0 | 20 | 0 | 0 | 0 | 30 | | | |
| Bromegrass, Downy | 98 | 20 | 98 | 0 | 0 | 0 | 50 | 0 | 65 | 30 | 30 | | | |
| Canarygrass | 100 | - | - | 75 | 0 | 75 | 75 | 85 | 90 | - | - | | | |
| Canola | 0 | 0 | 0 | 0 | 0 | 0 | 0 | 0 | 0 | 0 | 0 | | | |
| Foxtail, Green | 100 | 98 | 100 | 80 | 95 | 95 | 100 | 98 | 98 | 100 | 100 | | | |
| Mustard, Wild | 25 | 0 | 20 | 0 | 0 | - | 0 | 0 | 0 | 0 | 0 | | | |
| Oat, Wild | 100 | 90 | 100 | 100 | 90 | 98 | 90 | 100 | 100 | 100 | 90 | | | |
| Pigweed | 10 | 0 | 0 | 0 | 0 | 0 | 0 | 0 | 0 | 0 | 0 | | | |
| Ryegrass, Italian | 100 | 100 | 100 | 95 | 80 | 95 | 100 | 100 | 100 | 100 | 100 | | | |
| Wheat, Spring | 98 | 15 | 90 | 25 | 0 | 30 | 75 | 60 | 95 | 80 | 75 | | | |
| Wheat, Winter | 100 | 0 | 90 | 30 | 0 | 30 | 60 | 40 | 95 | 65 | 30 | | | |
| Windgrass | 95 | 0 | 100 | 50 | 35 | 20 | 15 | 60 | 95 | 50 | 0 | | | |

| Table C | | | | Compounds | | | | | | | | | | |
|---|---|---|---|---|---|---|---|---|---|---|---|---|---|---|
| 16 g ai/ha | | | | 11 | 15 | 19 | 20 | 25 | 26 | 37 | 43 | 44 | 53 | 57 |
| Postemergence | | | | | | | | | | | | | | |
| Barley, Spring | 95 | 0 | 90 | 25 | 0 | 0 | 20 | 35 | 95 | 40 | 20 | | | |
| Barley, Winter | 70 | 0 | 50 | 0 | 0 | 0 | 10 | 15 | 75 | 10 | 15 | | | |
| Blackgrass | 100 | 95 | 100 | 35 | 35 | 50 | 90 | 98 | 98 | 100 | 75 | | | |
| Bluegrass | 15 | 25 | 0 | 0 | 0 | 0 | 20 | 0 | 0 | 0 | 10 | | | |
| Bromegrass, Downy | 80 | 15 | 65 | 0 | 0 | 0 | 30 | 0 | 40 | 20 | 30 | | | |
| Canarygrass | 100 | - | - | 35 | 0 | 75 | 35 | 75 | 80 | - | - | | | |
| Canola | 0 | 0 | 0 | 0 | 0 | 0 | 0 | 0 | 0 | 0 | 0 | | | |
| Foxtail, Green | 98 | 95 | 100 | 70 | 80 | 95 | 100 | 95 | 95 | 98 | 100 | | | |
| Mustard, Wild | 25 | 0 | 20 | 0 | 0 | 0 | 0 | 0 | 0 | 0 | 0 | | | |
| Oat, Wild | 100 | 25 | 100 | 98 | 15 | 98 | 80 | 100 | 100 | 90 | 90 | | | |
| Pigweed | 10 | 0 | 0 | 0 | 0 | 0 | 0 | 0 | 0 | 0 | 0 | | | |
| Ryegrass, Italian | 98 | 100 | 100 | 80 | 60 | 85 | 95 | 98 | 98 | 100 | 98 | | | |
| Wheat, Spring | 90 | 0 | 80 | 25 | 0 | 10 | 50 | 15 | 95 | 50 | 25 | | | |
| Wheat, Winter | 100 | 0 | 80 | 20 | 0 | 0 | 30 | 25 | 80 | 10 | 30 | | | |
| Windgrass | 95 | 0 | 100 | 0 | 0 | 0 | 0 | 60 | 90 | 0 | 0 | | | |

| Table C | Compounds | | | | | | | | | | | | | |
|---|---|---|---|---|---|---|---|---|---|---|---|---|---|---|
| 8 g ai/ha | 11 | 15 | 19 | 20 | 25 | 26 | 37 | 43 | 44 | 53 | 57 | | | |
| Postemergence | | | | | | | | | | | | | | |
| Barley, Spring | 80 | 0 | 20 | 0 | 0 | 0 | 0 | 20 | 60 | 0 | 0 | | | |
| Barley, Winter | 60 | 0 | 20 | 0 | 0 | 0 | 10 | 5 | 20 | 0 | 0 | | | |
| Blackgrass | 100 | 75 | 75 | 0 | 5 | 50 | 65 | 90 | 90 | 65 | 35 | | | |
| Bluegrass | 0 | 0 | 0 | 0 | 0 | 0 | 0 | 0 | 0 | 0 | 0 | | | |
| Bromegrass, Downy | 50 | 0 | 40 | 0 | 0 | 0 | 20 | 0 | 15 | 0 | 0 | | | |
| Canarygrass | 65 | - | - | 10 | 0 | 25 | 0 | 65 | 75 | - | - | | | |
| Canola | 0 | 0 | 0 | 0 | 0 | 0 | 0 | 0 | 0 | 0 | 0 | | | |
| Foxtail, Green | 95 | 90 | 95 | 0 | 70 | 90 | 90 | 90 | 50 | 95 | 90 | | | |
| Mustard, Wild | 0 | 0 | 0 | 0 | 0 | 0 | 0 | 0 | 0 | 0 | 0 | | | |
| Oat, Wild | 100 | 0 | 100 | 10 | 0 | 70 | 25 | 100 | 100 | 25 | 10 | | | |
| Pigweed | 0 | 0 | 0 | 0 | 0 | 0 | 0 | 0 | 0 | 0 | 0 | | | |
| Ryegrass, Italian | 98 | 50 | 60 | 65 | 0 | 70 | 75 | 75 | 90 | 75 | 75 | | | |
| Wheat, Spring | 65 | 0 | 70 | 0 | 0 | 10 | 15 | 0 | 70 | 5 | 0 | | | |
| Wheat, Winter | 90 | 0 | 20 | 0 | 0 | 0 | 0 | 5 | 75 | 0 | 0 | | | |
| Windgrass | 95 | 0 | 80 | 0 | 0 | 0 | 0 | 30 | 50 | 0 | 0 | | | |

| Table C | Compounds | | | | | | | | | | | | | |
|---|---|---|---|---|---|---|---|---|---|---|---|---|---|---|
| 62 g ai/ha | 11 | 15 | 19 | 21 | 25 | 26 | 28 | 30 | 32 | 33 | 34 | 35 | 37 | 41 |
| Preemergence | | | | | | | | | | | | | | |
| Barley, Spring | 100 | 65 | 80 | 75 | 0 | 35 | 25 | 50 | 75 | 25 | 70 | 65 | 40 | 90 |
| Barley, Winter | 95 | 60 | 80 | 75 | 0 | 10 | 40 | 15 | 65 | 20 | 35 | 50 | 40 | 80 |
| Blackgrass | 100 | 98 | 100 | 100 | 75 | 75 | 85 | 98 | 98 | 100 | 85 | 75 | 98 | 100 |
| Bluegrass | 30 | 90 | - | - | 40 | 0 | 50 | 0 | 20 | 0 | 35 | 0 | 70 | - |
| Bromegrass, Downy | 100 | 70 | 95 | 95 | 35 | 20 | 75 | 0 | 60 | 60 | 60 | 60 | 85 | 80 |
| Canarygrass | 100 | 95 | 100 | 100 | 35 | 70 | 90 | 90 | 90 | 90 | 100 | 90 | 95 | 100 |
| Canola | 0 | 0 | 0 | 0 | 0 | 0 | 0 | 0 | - | 0 | 0 | - | 0 | 0 |
| Foxtail, Green | 100 | 100 | 100 | 100 | 100 | 100 | 100 | 100 | 100 | 100 | 100 | 100 | 100 | 100 |
| Mustard, Wild | 0 | - | 0 | 0 | - | 0 | 0 | - | - | - | 0 | 0 | 0 | 0 |
| Oat, Wild | 100 | 65 | 95 | 100 | 40 | 75 | 75 | 40 | 70 | 75 | 75 | 70 | 80 | 95 |
| Pigweed | 15 | 0 | 75 | 0 | 0 | 20 | 0 | 0 | 0 | 0 | 0 | 0 | 0 | 0 |
| Ryegrass, Italian | 100 | 100 | 100 | 100 | 75 | 100 | 95 | 75 | 75 | 100 | 100 | 100 | 100 | 100 |
| Wheat, Spring | 100 | 70 | 75 | 90 | 0 | 25 | 50 | 20 | 98 | 50 | 90 | 65 | 98 | 95 |
| Wheat, Winter | 100 | 25 | 90 | 95 | 0 | 25 | 60 | 0 | 65 | 40 | 60 | 70 | 90 | 98 |
| Windgrass | 100 | 95 | 100 | 100 | - | 100 | 85 | 35 | 75 | 50 | 60 | 80 | 80 | 100 |

| Table C | Compounds | | | | | | | | | | | | | |
|---|---|---|---|---|---|---|---|---|---|---|---|---|---|---|
| 62 g ai/ha | 43 | 44 | 47 | 48 | 51 | 52 | 53 | 54 | 55 | 57 | 58 | | | |
| Preemergence | | | | | | | | | | | | | | |
| Barley, Spring | 60 | 75 | 75 | 80 | 70 | 60 | 75 | 70 | 70 | 40 | 75 | | | |
| Barley, Winter | 65 | 75 | 40 | 70 | 40 | 50 | 50 | 75 | 60 | 60 | 75 | | | |
| Blackgrass | 100 | 100 | 100 | 98 | 100 | 95 | 98 | 100 | 95 | 95 | 100 | | | |
| Bluegrass | 25 | 0 | 50 | 0 | 10 | 10 | 65 | 25 | 15 | 65 | 0 | | | |
| Bromegrass, Downy | 75 | 80 | 35 | 40 | 50 | 80 | 85 | 80 | 40 | 80 | 75 | | | |
| Canarygrass | 95 | 85 | 90 | 95 | 90 | 95 | 95 | 95 | 90 | 90 | 95 | | | |
| Canola | 0 | 0 | - | - | - | - | 0 | - | - | 0 | - | | | |
| Foxtail, Green | 100 | 100 | 100 | 100 | 100 | 100 | 100 | 100 | 100 | 100 | 100 | | | |
| Mustard, Wild | 0 | - | 0 | 0 | 0 | 0 | 0 | 0 | 0 | 0 | 0 | | | |
| Oat, Wild | 98 | 90 | 80 | 70 | 50 | 60 | 85 | 70 | 65 | 80 | 75 | | | |
| Pigweed | 20 | 20 | 0 | 0 | 0 | 0 | 0 | 0 | - | 0 | 0 | | | |
| Ryegrass, Italian | 98 | 100 | 100 | 100 | 90 | 100 | 100 | 100 | 90 | 100 | 100 | | | |
| Wheat, Spring | 65 | 75 | 65 | 75 | 75 | 90 | 85 | 98 | 60 | 90 | 90 | | | |
| Wheat, Winter | 70 | 70 | 65 | 70 | 40 | 65 | 70 | 50 | 60 | 65 | 80 | | | |
| Windgrass | 100 | 100 | 65 | 80 | 80 | 70 | 90 | 75 | 60 | 75 | 70 | | | |

| Table C | Compounds | | | | | | | | | | | | | |
|---|---|---|---|---|---|---|---|---|---|---|---|---|---|---|
| 31 g ai/ha | 11 | 15 | 19 | 21 | 25 | 26 | 28 | 30 | 32 | 33 | 34 | 35 | 37 | 41 |
| Preemergence | | | | | | | | | | | | | | |
| Barley, Spring | 70 | 40 | 65 | 60 | 0 | 25 | 15 | 30 | 25 | 10 | 0 | 20 | 15 | 75 |
| Barley, Winter | 75 | 50 | 75 | 50 | 0 | 10 | 0 | 0 | 20 | 0 | 10 | 0 | 20 | 70 |
| Blackgrass | 90 | 95 | 100 | 100 | 75 | - | 15 | 90 | 75 | 80 | 75 | 75 | 90 | 100 |
| Bluegrass | - | 75 | - | - | 40 | 0 | - | 0 | 0 | 0 | 0 | 0 | 60 | - |
| Bromegrass, Downy | 70 | 35 | 75 | 75 | 20 | 0 | 20 | 0 | 20 | 35 | 10 | 50 | 60 | 50 |
| Canarygrass | 98 | 80 | 98 | 90 | 10 | 65 | 10 | 65 | 75 | 65 | 65 | 50 | 80 | 98 |
| Canola | 0 | 0 | 0 | 0 | 0 | 0 | 0 | 0 | - | 0 | 0 | - | 0 | 0 |
| Foxtail, Green | 100 | 100 | 100 | 100 | 100 | 75 | 100 | 80 | 100 | 100 | 100 | 40 | 100 | 100 |
| Mustard, Wild | 0 | 0 | 0 | 0 | 0 | 0 | 0 | 0 | 0 | - | 0 | 0 | 0 | 0 |
| Oat, Wild | 98 | 50 | 80 | 95 | 40 | 65 | 20 | 30 | 65 | 60 | 70 | 30 | 70 | 95 |
| Pigweed | 0 | 0 | 50 | 0 | 0 | 0 | 0 | 0 | 0 | 0 | 0 | 0 | 0 | 0 |
| Ryegrass, Italian | 100 | 100 | 100 | 100 | 60 | 100 | 90 | 60 | - | 100 | 100 | 98 | 98 | 100 |
| Wheat, Spring | 75 | 35 | 60 | 60 | 0 | 25 | 35 | 0 | 60 | 20 | 50 | 50 | 90 | 75 |
| Wheat, Winter | 100 | 20 | 60 | 60 | 0 | 0 | 30 | 0 | 35 | 15 | 25 | 25 | 25 | 60 |
| Windgrass | 100 | 75 | 100 | 100 | - | 98 | 75 | - | 0 | - | 20 | 15 | 60 | 100 |

| Table C | Compounds | | | | | | | | | | | | | |
|---|---|---|---|---|---|---|---|---|---|---|---|---|---|---|
| 31 g ai/ha | 43 | 44 | 47 | 48 | 51 | 52 | 53 | 54 | 55 | 57 | 58 | | | |
| Preemergence | | | | | | | | | | | | | | |
| Barley, Spring | 35 | 60 | 35 | 30 | 20 | 40 | 40 | 25 | 0 | 20 | 10 | | | |
| Barley, Winter | 50 | 75 | 40 | 0 | 0 | 0 | 50 | 15 | 0 | 20 | 0 | | | |
| Blackgrass | 98 | 100 | 90 | 65 | 100 | 95 | 98 | 100 | 80 | 95 | 98 | | | |
| Bluegrass | - | 0 | 50 | 0 | 0 | 10 | 40 | 0 | 0 | 50 | 0 | | | |
| Bromegrass, Downy | 40 | 50 | 35 | 0 | 0 | 15 | 80 | 15 | 0 | 40 | 70 | | | |
| Canarygrass | 75 | 75 | 50 | 85 | 80 | 90 | 95 | 80 | 20 | 80 | 90 | | | |
| Canola | 0 | 0 | - | - | - | - | - | - | - | - | - | | | |
| Foxtail, Green | 100 | 100 | 80 | 100 | 100 | 100 | 100 | 100 | 100 | 100 | 100 | | | |
| Mustard, Wild | 0 | 0 | 0 | 0 | 0 | 0 | 0 | 0 | 0 | 0 | - | | | |
| Oat, Wild | 80 | 80 | 75 | 50 | 35 | 60 | 75 | 40 | 0 | 60 | 65 | | | |
| Pigweed | 0 | 20 | 0 | 0 | 0 | 0 | 0 | 0 | 0 | 0 | - | | | |
| Ryegrass, Italian | 98 | 100 | 80 | 90 | - | 90 | 100 | 100 | - | 100 | 100 | | | |
| Wheat, Spring | 30 | 70 | 25 | 60 | 60 | 65 | 85 | 65 | 50 | 65 | 60 | | | |
| Wheat, Winter | 20 | - | 35 | 20 | 0 | 40 | 50 | 35 | 0 | 20 | 60 | | | |
| Windgrass | 80 | 100 | 30 | 35 | 20 | 70 | 75 | 60 | 0 | 50 | 50 | | | |

| Table C | Compounds | | | | | | | | | | | | | |
|---|---|---|---|---|---|---|---|---|---|---|---|---|---|---|
| 16 g ai/ha | 11 | 15 | 19 | 21 | 25 | 26 | 28 | 30 | 32 | 33 | 34 | 35 | 37 | 41 |
| Preemergence | | | | | | | | | | | | | | |
| Barley, Spring | 30 | 0 | 65 | 35 | 0 | 0 | 15 | 0 | 0 | 0 | 0 | 0 | 0 | 65 |
| Barley, Winter | 40 | 20 | 40 | 40 | 0 | 0 | 0 | 0 | 0 | 0 | 0 | - | 0 | 25 |
| Blackgrass | 90 | 80 | 100 | 98 | 50 | 50 | 5 | 20 | - | 50 | 20 | 30 | 75 | 100 |
| Bluegrass | 20 | 60 | 0 | 0 | 35 | 0 | 0 | 0 | 0 | 0 | 0 | 0 | 35 | 0 |
| Bromegrass, Downy | 65 | 20 | 65 | 40 | 20 | 0 | 0 | 0 | 20 | 35 | 0 | 0 | 0 | 50 |
| Canarygrass | 75 | 70 | 90 | 75 | 0 | 0 | 0 | 0 | 0 | 10 | 20 | 0 | 60 | 80 |
| Canola | 0 | 0 | 0 | 0 | 0 | - | 0 | 0 | - | 0 | 0 | - | - | 0 |
| Foxtail, Green | 100 | 98 | 100 | 100 | 25 | 0 | 20 | 25 | 75 | 95 | 100 | - | 90 | 100 |
| Mustard, Wild | 0 | 0 | 0 | 0 | 0 | 0 | 0 | - | 0 | - | 0 | - | 0 | - |
| Oat, Wild | 98 | - | 65 | 75 | 15 | 15 | 0 | 0 | 10 | 25 | 0 | 0 | 20 | 75 |
| Pigweed | 0 | 0 | 30 | 0 | 0 | 0 | 0 | 0 | 0 | 0 | 0 | - | 0 | 0 |
| Ryegrass, Italian | 98 | 100 | 100 | 100 | 30 | 75 | 15 | 30 | 20 | 65 | 100 | 35 | 80 | 100 |
| Wheat, Spring | 60 | 10 | 40 | 40 | 0 | 0 | 0 | 0 | 25 | 0 | 0 | 20 | 0 | 50 |
| Wheat, Winter | 40 | 0 | 50 | 50 | 0 | 0 | 10 | 0 | 20 | 0 | 10 | 10 | 15 | 40 |
| Windgrass | 100 | 25 | 100 | 100 | 75 | 30 | 30 | - | 0 | 0 | 0 | - | 20 | 98 |

| Table C | Compounds | | | | | | | | | | | | | |
|---|---|---|---|---|---|---|---|---|---|---|---|---|---|---|
| 16 g ai/ha | 43 | 44 | 47 | 48 | 51 | 52 | 53 | 54 | 55 | 57 | 58 | | | |
| Preemergence | | | | | | | | | | | | | | |
| Barley, Spring | 20 | 60 | 20 | 15 | 15 | 0 | 0 | 0 | 0 | 0 | 0 | | | |
| Barley, Winter | 20 | 50 | 20 | 0 | 0 | 0 | 15 | 10 | 0 | 0 | 0 | | | |
| Blackgrass | 90 | 25 | 0 | 50 | 35 | 60 | 75 | 70 | 40 | 85 | 65 | | | |
| Bluegrass | 0 | 0 | 20 | 0 | 0 | 0 | 20 | 0 | 0 | 30 | 0 | | | |
| Bromegrass, Downy | 25 | 20 | 15 | 0 | 0 | 0 | 20 | 0 | 0 | 20 | 15 | | | |
| Canarygrass | 65 | 65 | 0 | 0 | 0 | 65 | 0 | 70 | 0 | 75 | 60 | | | |
| Canola | - | 0 | - | - | - | - | - | - | - | - | - | | | |
| Foxtail, Green | 100 | 90 | 15 | 95 | 80 | 100 | 90 | 98 | 40 | 100 | 98 | | | |
| Mustard, Wild | 0 | 0 | 0 | - | 0 | 0 | - | 0 | 0 | 0 | 0 | | | |
| Oat, Wild | 70 | 75 | 0 | 0 | 0 | 40 | 0 | 0 | 0 | 15 | 50 | | | |
| Pigweed | 0 | 20 | 0 | 0 | 0 | 0 | 0 | 0 | 0 | 0 | - | | | |
| Ryegrass, Italian | 98 | 100 | 20 | 40 | 90 | 65 | 100 | 20 | 0 | 85 | 65 | | | |
| Wheat, Spring | 0 | 20 | 15 | 35 | 0 | 0 | 20 | 25 | 0 | 20 | 50 | | | |
| Wheat, Winter | 0 | 65 | 20 | 0 | 0 | 0 | 0 | 0 | 0 | 15 | 0 | | | |
| Windgrass | 25 | 100 | 0 | 0 | 10 | 0 | 30 | 0 | 0 | 0 | 20 | | | |

| Table C | Compounds | | | | | | | | | | | | | |
|---|---|---|---|---|---|---|---|---|---|---|---|---|---|---|
| 8 g ai/ha | 11 | 15 | 19 | 21 | 25 | 26 | 28 | 30 | 32 | 33 | 34 | 35 | 37 | 41 |
| Preemergence | | | | | | | | | | | | | | |
| Barley, Spring | 20 | 0 | 50 | 20 | 0 | 0 | 0 | 0 | 0 | 0 | 0 | 0 | 0 | 0 |
| Barley, Winter | 20 | 0 | 20 | 20 | 0 | 0 | 0 | 0 | 0 | 0 | 0 | 0 | 0 | 10 |
| Blackgrass | 85 | 70 | 80 | 95 | 0 | 20 | 0 | 0 | - | 0 | 0 | 0 | 35 | 100 |
| Bluegrass | 0 | 40 | - | 0 | 0 | 0 | 0 | 0 | 0 | 0 | 0 | 0 | 0 | 0 |
| Bromegrass, Downy | 35 | 0 | 30 | 0 | 20 | 0 | 0 | 0 | 0 | 0 | 0 | 0 | 0 | 0 |
| Canarygrass | 15 | 20 | 65 | 65 | 0 | 0 | 0 | 0 | 0 | 0 | 0 | 0 | 15 | 50 |
| Canola | 0 | 0 | 0 | 0 | 0 | - | 0 | 0 | - | 0 | 0 | - | 0 | 0 |
| Foxtail, Green | 100 | 60 | 20 | 50 | 15 | 0 | 0 | 10 | 40 | 15 | 30 | 15 | 75 | 98 |
| Mustard, Wild | 0 | 0 | 0 | 0 | 0 | 0 | 0 | - | 0 | - | - | - | 0 | 0 |
| Oat, Wild | 98 | 0 | 65 | 65 | 15 | 0 | 0 | 0 | 0 | 0 | 0 | 0 | 20 | 65 |
| Pigweed | 0 | 0 | 0 | 0 | 0 | 0 | 0 | 0 | 0 | - | 0 | 0 | 0 | 0 |
| Ryegrass, Italian | 85 | 15 | 80 | 98 | 25 | 15 | 0 | 0 | 0 | 10 | 30 | 0 | 0 | 100 |
| Wheat, Spring | 50 | 0 | 0 | 30 | 0 | 0 | 0 | 0 | 0 | 0 | 0 | 0 | 0 | 0 |
| Wheat, Winter | 20 | 0 | 10 | 0 | 0 | 0 | 0 | 0 | 0 | 0 | 0 | 0 | 0 | 0 |
| Windgrass | 100 | - | 100 | 100 | 50 | 30 | 20 | - | 0 | 0 | 0 | 0 | 0 | 65 |

| Table C | Compounds | | | | | | | | | | | | | |
|---|---|---|---|---|---|---|---|---|---|---|---|---|---|---|
| 8 g ai/ha | 43 | 44 | 47 | 48 | 51 | 52 | 53 | 54 | 55 | 57 | 58 | | | |
| Preemergence | | | | | | | | | | | | | | |
| Barley, Spring | 0 | 0 | 10 | 0 | 0 | 0 | 0 | 0 | 0 | 0 | 0 | | | |
| Barley, Winter | 0 | 0 | 0 | 0 | 0 | 0 | 0 | 0 | 0 | 0 | 0 | | | |
| Blackgrass | 85 | - | 0 | 35 | 0 | 40 | 70 | 35 | - | 75 | 0 | | | |
| Bluegrass | 0 | 0 | 0 | 0 | 0 | 0 | 0 | 0 | 0 | 0 | 0 | | | |
| Bromegrass, Downy | 10 | 0 | 0 | 0 | 0 | 0 | 10 | 0 | 0 | 0 | 0 | | | |
| Canarygrass | 50 | 30 | 0 | 0 | 0 | 0 | 0 | 0 | 0 | 0 | 0 | | | |
| Canola | 0 | 0 | - | - | - | - | - | - | - | 0 | - | | | |
| Foxtail, Green | 75 | 15 | 0 | 35 | 10 | 75 | 90 | 40 | 20 | 40 | 35 | | | |
| Mustard, Wild | 0 | 0 | 0 | - | - | 0 | 0 | - | 0 | 0 | 0 | | | |
| Oat, Wild | 35 | 50 | 0 | 0 | 0 | 0 | 0 | 0 | 0 | 0 | 0 | | | |
| Pigweed | 0 | 0 | 0 | 0 | 0 | 0 | 0 | 0 | 0 | 0 | 0 | | | |
| Ryegrass, Italian | 60 | 90 | 0 | 40 | 0 | 0 | 25 | 0 | 0 | 65 | 25 | | | |
| Wheat, Spring | 0 | 0 | 0 | 0 | 0 | 0 | 0 | 0 | 0 | 20 | 0 | | | |
| Wheat, Winter | 0 | 40 | 0 | 0 | 0 | 0 | 0 | 0 | 0 | 15 | 0 | | | |
| Windgrass | - | 100 | 0 | 0 | 0 | 0 | 0 | 0 | 0 | 0 | 0 | | | |

### TEST D

Three plastic pots (ca. 16-cm diameter) for each application rate were partially filled with sterilized Tama silt loam soil comprising a 35:50:15 ratio of sand, silt and clay and 2.6% organic matter. Separate plantings for each of the three pots were as follows. Seeds from the U.S. of ducksalad (*Heteranthera limosa*), umbrella sedge (*Cyperus difformis*) and purple redstem (*Ammannia coccinea*), were planted into one 16-cm pot for each rate. Seeds from the U.S. of rice flatsedge (*Cyperus iria*), bearded (Brdd.) sprangletop (*Leptochloa fascicularis*), one stand of 9 or 10 water-seeded rice seedlings (*Oryza sativa* cv. 'Japonica - M202'), and one stand of 6 transplanted rice seedlings (*Oryza sativa* cv. 'Japonica - M202') were planted into one 16-cm pot for each rate. Seeds from the U.S. of barnyardgrass (*Echinochloa crus-galli*), late watergrass (*Echinochloa oryzicola*), early watergrass (*Echinochloa oryzoides*) and junglerice (*Echinochloa colona*) were planted into one 16-cm pot for each rate. Plantings were sequential so that crop and weed species were at the 2.0 to 2.5-leaf stage at time of treatment.

Potted plants were grown in a greenhouse with day/night temperature settings of 30/27 °C, and supplemental balanced lighting was provided to maintain a 16-hour photoperiod. Test pots were maintained in the greenhouse until test completion.

At time of treatment, test pots were flooded to 3 cm above the soil surface, treated by application of test compounds directly to the paddy water, and then maintained at that water depth for the duration of the test. Effects of treatments on rice and weeds were visually evaluated by comparison to untreated controls after 21 days. Plant response ratings, summarized in Table D, are based on a scale of 0 to 100 where 0 is no effect and 100 is complete control. A dash (-) response means no test result.

| Table D | Compounds | | | | Table D | Compounds | |
|---|---|---|---|---|---|---|---|
| 250 g ai/ha | 21 | 43 | | | 125 g ai/ha | 21 | 43 |
| Flood | | | | | Flood | | |
| Barnyardgrass | 100 | 100 | | | Barnyardgrass | 100 | 100 |
| Ducksalad | 0 | 0 | | | Ducksalad | 0 | 0 |
| Flatsedge, Rice | 70 | 0 | | | Flatsedge, Rice | 70 | 0 |
| Junglerice | 100 | 100 | | | Junglerice | 100 | 100 |
| Redstem, Purple | 0 | 0 | | | Redstem, Purple | 0 | 0 |
| Rice, Transplanted | 65 | 35 | | | Rice, Transplanted | 50 | 10 |
| Rice, Water Seeded | 75 | 35 | | | Rice, Water Seeded | 65 | 30 |
| Sedge, Umbrella | 0 | 0 | | | Sedge, Umbrella | 0 | 0 |
| Sprangletop, Brdd. | 100 | 20 | | | Sprangletop, Brdd. | 100 | 0 |
| Watergrass, Early | 100 | 100 | | | Watergrass, Early | 100 | 70 |
| Watergrass, Late | 100 | 100 | | | Watergrass, Late | 100 | 65 |

| Table D | Compounds | | | | Table D | Compounds | |
|---|---|---|---|---|---|---|---|
| 64 g ai/ha | 21 | 43 | | | 32 g ai/ha | 21 | 43 |
| Flood | | | | | Flood | | |
| Barnyardgrass | 100 | 60 | | | Barnyardgrass | 60 | 20 |
| Ducksalad | 0 | 0 | | | Ducksalad | 0 | 0 |
| Flatsedge, Rice | 70 | 0 | | | Flatsedge, Rice | 0 | 0 |
| Junglerice | 100 | 60 | | | Junglerice | 70 | 0 |
| Redstem, Purple | 0 | 0 | | | Redstem, Purple | 0 | 0 |
| Rice, Transplanted | 0 | 0 | | | Rice, Transplanted | 0 | 0 |
| Rice, Water Seeded | 35 | 0 | | | Rice, Water Seeded | 20 | 0 |
| Sedge, Umbrella | 0 | 0 | | | Sedge, Umbrella | 0 | 0 |
| Sprangletop, Brdd. | 80 | 0 | | | Sprangletop, Brdd. | 30 | 0 |
| Watergrass, Early | 65 | 60 | | | Watergrass, Early | 40 | 0 |
| Watergrass, Late | 60 | 30 | | | Watergrass, Late | 30 | 0 |

| Table D | Compounds | | | | Table D | Compounds | |
|---|---|---|---|---|---|---|---|
| 16 g ai/ha | 21 | 43 | | | 16 g ai/ha | 21 | 43 |
| Flood | | | | | Flood | | |
| Barnyardgrass | 0 | 0 | | | Rice, Water Seeded | 0 | 0 |
| Ducksalad | 0 | 0 | | | Sedge, Umbrella | 0 | 0 |
| Flatsedge, Rice | 0 | 0 | | | Sprangletop, Brdd. | 30 | 0 |
| Junglerice | 0 | 0 | | | Watergrass, Early | 0 | 0 |
| Redstem, Purple | 0 | 0 | | | Watergrass, Late | 0 | 0 |
| Rice, Transplanted | 0 | 0 | | | | | |

### TEST E

Seeds of plant species selected from bermudagrass (*Cynodon dactylon*), Surinam grass (*Brachiaria decumbens*), large crabgrass (*Digitaria sanguinalis*), green foxtail (*Setaria viridis*), goosegrass (*Eleusine indica*), johnsongrass (*Sorghum halepense*), kochia (*Kochia scoparia*), pitted morningglory (*Ipomoea lacunosa*), purple nutsedge (*Cyperus rotundus*), common ragweed (*Ambrosia elatior*), black mustard (*Brassica nigra*), guineagrass (*Panicum maximum*), dallisgrass (*Paspalum dilatatum*), barnyardgrass (*Echinochloa crus-galli*), southern (S.) sandbur (*Cenchrus echinatus*), common sowthistle (*Sonchus oleraceous*), prickly sida (*Sida spinosa*), Italian ryegrass (*Lolium multiflorum*), common purslane (*Portulaca oleracea*), broadleaf signalgrass (*Brachiaria platyphylla*), common groundsel (*Senecio vulgaris*), common chickweed (*Stellaria media*), Virginia (V.) dayflower (*Commelina virginica*), tropical (T.) spiderwort (*Commelina benghalensis*), annual bluegrass (*Poa annua*), naked crabgrass (*Digitaria nuda*), itchgrass (*Rottboellia cochinchinensis*), quackgrass (*Elytrigia repens*), Canada horseweed (*Conyza canadensis*), field bindweed (*Convolvulus arvensis*), spanishneedles (*Bidens bipinnata*), common mallow (*Malva sylvestris*) and Russian thistle (*Salsola kali*) were planted and treated preemergence with test chemicals formulated in a non-phytotoxic solvent mixture which included a surfactant. At the same time, plants selected from these weed species were treated with postemergence applications of some of the test chemicals formulated in the same manner. Plants ranged in height from 2 to 18 cm (1- to 4-leaf stage) for postemergence treatments.

Treated plants and controls were maintained in a greenhouse for 14 to 21 days, after which time all species were compared to controls and visually evaluated. Plant response ratings, summarized in Table E, are based on a scale of 0 to 100 where 0 is no effect and 100 is complete control. A dash (-) response means no test result.

| Table E | Compounds | | | | | | | | | | | | | |
|---|---|---|---|---|---|---|---|---|---|---|---|---|---|---|
| 62 g ai/ha | 21 | 31 | 32 | 33 | 34 | 35 | 36 | 39 | 40 | 43 | 53 | 54 | | |
| Postemergence | | | | | | | | | | | | | | |
| Barnyardgrass | 100 | 100 | 100 | 100 | 100 | 100 | 100 | 100 | 100 | 100 | 100 | 100 | | |
| Bermudagrass | 75 | 95 | 75 | 95 | 95 | 90 | 90 | 65 | 70 | 20 | 98 | 80 | | |
| Mustard, Black | 0 | 0 | 0 | 0 | 0 | 0 | 0 | 0 | 0 | 0 | 0 | 0 | | |
| Bluegrass | 15 | 10 | 0 | 5 | 0 | 0 | 0 | 0 | 15 | 0 | 0 | 0 | | |
| Chickweed | - | - | - | - | - | - | - | 0 | - | - | - | - | | |
| Crabgrass, Large | 98 | 80 | 80 | 95 | 100 | 80 | 80 | 98 | 95 | 80 | 100 | 100 | | |
| Crabgrass, Naked | 100 | 90 | 90 | 98 | 100 | 95 | 98 | 98 | 100 | 100 | 100 | 90 | | |
| Dallisgrass | 98 | 70 | 40 | 65 | 70 | 60 | 50 | 75 | 95 | 50 | 75 | 80 | | |
| Dayflower, V. | - | - | 0 | - | 0 | - | - | - | - | 0 | 0 | 0 | | |
| Foxtail, Green | 100 | 75 | 90 | 95 | 98 | 75 | 75 | 95 | 98 | 98 | 100 | 98 | | |
| Goosegrass | 98 | 75 | 80 | 75 | 80 | 75 | 75 | 95 | 95 | 90 | 95 | 95 | | |
| Groundsel | 0 | - | 0 | 0 | 0 | - | - | 0 | - | 0 | 0 | - | | |
| Guineagrass | 100 | 90 | 100 | 95 | 100 | 95 | 90 | 95 | 100 | 100 | 100 | 100 | | |
| Itchgrass | 75 | 70 | 90 | 75 | 95 | 70 | 60 | 60 | 80 | 75 | 98 | 100 | | |
| Johnsongrass | 100 | 100 | 100 | 98 | 100 | 98 | 95 | 95 | 98 | - | 100 | 100 | | |
| Mallow | 0 | 0 | 0 | 0 | 0 | 0 | 0 | 0 | 0 | 0 | 0 | 0 | | |
| Morningglory | 0 | 0 | 50 | 0 | 30 | 0 | 0 | 0 | 0 | 0 | 30 | 0 | | |
| Prickly Sida | - | - | 0 | - | 0 | - | - | - | - | 0 | 0 | 0 | | |
| Nutsedge, Purple | 0 | 0 | 0 | 0 | 0 | 0 | 0 | 0 | 0 | 0 | 0 | 0 | | |
| Purslane | 0 | 0 | 0 | 0 | 0 | 0 | 0 | 0 | 0 | 0 | 0 | 0 | | |
| Quackgrass | 80 | 50 | 0 | 70 | 40 | 40 | 35 | 60 | 95 | 75 | 30 | 35 | | |
| Ryegrass, Italian | 100 | 90 | 98 | 95 | 100 | 95 | 80 | 100 | 100 | 100 | 100 | 100 | | |
| Sandbur, S. | 100 | 75 | 80 | 90 | 100 | 75 | 85 | 100 | 100 | 100 | 100 | 95 | | |
| Signalgrass | 100 | 75 | 100 | 100 | 100 | 100 | 100 | 100 | 100 | 98 | 100 | 100 | | |
| Sowthistle | - | - | 0 | - | 0 | - | - | - | - | 0 | 0 | 0 | | |
| Spiderwort, T. | 0 | 0 | - | 0 | - | 0 | 0 | 0 | 0 | - | - | - | | |
| Surinam Grass | 100 | 75 | 100 | 100 | 100 | 90 | 100 | 100 | 100 | 98 | 100 | 100 | | |

| Table E | Compounds | | | | | | | | | | | | | |
|---|---|---|---|---|---|---|---|---|---|---|---|---|---|---|
| 31 g ai/ha | 11 | 12 | 19 | 21 | 31 | 32 | 33 | 34 | 35 | 36 | 39 | 40 | 43 | 53 |
| Postemergence | | | | | | | | | | | | | | |
| Barnyardgrass | 100 | 100 | 100 | 100 | 75 | 100 | 100 | 100 | 100 | 95 | 100 | 100 | 100 | 100 |
| Bermudagrass | 95 | 95 | 80 | 65 | 90 | 40 | 90 | 80 | 80 | 70 | 50 | 60 | 20 | 80 |
| Mustard, Black | 0 | 0 | 0 | 0 | 0 | 0 | 0 | 0 | 0 | 0 | 0 | 0 | 0 | 0 |
| Bluegrass | 0 | 0 | 0 | 10 | 0 | 0 | 5 | 0 | 0 | 0 | 0 | 15 | 0 | 0 |
| Chickweed | 0 | 0 | - | 0 | 0 | - | 0 | - | 0 | - | 0 | 0 | - | - |
| Crabgrass, Large | 100 | 95 | 80 | 95 | 75 | 75 | 90 | 100 | 75 | 75 | 95 | 90 | 80 | 95 |
| Crabgrass, Naked | 98 | 95 | 98 | 98 | 75 | 80 | 90 | - | 95 | 90 | 80 | 95 | - | 98 |
| Dallisgrass | 90 | 85 | 75 | 85 | 60 | 30 | 65 | 20 | 50 | 30 | 75 | 80 | 20 | 30 |
| Dayflower, V. | 0 | 0 | 0 | - | - | 0 | - | 0 | - | - | - | - | 0 | 0 |
| Field Bindweed | 0 | 0 | 0 | - | - | - | - | - | - | - | - | - | - | - |
| Foxtail, Green | 100 | 100 | 100 | 95 | 70 | 85 | 75 | 95 | 65 | 75 | 75 | 95 | 80 | 90 |
| Goosegrass | 85 | 90 | 75 | 90 | 70 | 60 | 75 | 80 | 40 | 40 | 80 | 80 | 75 | 80 |
| Groundsel | 0 | 0 | 0 | - | - | 0 | 0 | 0 | - | - | 0 | - | 0 | 0 |
| Guineagrass | 98 | 95 | 98 | 100 | 85 | 98 | 90 | 100 | 80 | 90 | 90 | 95 | 100 | 100 |
| Horseweed | 0 | 0 | 0 | - | - | - | - | - | - | - | - | - | - | - |
| Itchgrass | 98 | 95 | 80 | 75 | 65 | 90 | 70 | 95 | 70 | 50 | 40 | 80 | 20 | 95 |
| Johnsongrass | 100 | 95 | 100 | 85 | 75 | 98 | 98 | 98 | 70 | 75 | 75 | 90 | 65 | 100 |
| Kochia | 0 | 0 | - | - | - | - | - | - | - | - | - | - | - | - |
| Mallow | 0 | 0 | 0 | 0 | 0 | 0 | 0 | 0 | 0 | 0 | 0 | 0 | 0 | 0 |
| Morningglory | 0 | 0 | 0 | 0 | 0 | 35 | 0 | 0 | 0 | 0 | 0 | 0 | 0 | 0 |
| Prickly Sida | 0 | 0 | 0 | - | - | 0 | - | 0 | - | - | - | - | 0 | 0 |
| Nutsedge, Purple | - | 0 | - | 0 | 0 | 0 | 0 | 0 | 0 | 0 | 0 | 0 | 0 | 0 |
| Purslane | 0 | 0 | 0 | 0 | 0 | 0 | 0 | 0 | 0 | 0 | 0 | 0 | - | 0 |
| Quackgrass | 95 | 95 | 80 | 40 | 30 | 0 | 60 | 30 | 30 | 30 | 60 | 70 | 20 | 0 |
| Ragweed | 0 | 0 | 0 | - | - | - | - | - | - | - | - | - | - | - |
| Ryegrass, Italian | 95 | 100 | 100 | 100 | 50 | 95 | 90 | 100 | 60 | 60 | 98 | 100 | 100 | 100 |
| Sandbur, S. | 100 | 100 | 100 | 95 | 75 | 80 | 80 | 90 | 75 | 80 | 95 | 95 | 98 | 95 |
| Signalgrass | 100 | 100 | 98 | 100 | 75 | 95 | 95 | 95 | 75 | 90 | 95 | 95 | 95 | 95 |
| Sowthistle | 0 | 0 | 0 | - | - | 0 | - | 0 | - | - | - | - | 0 | 0 |
| Spanishneedles | 0 | 0 | - | - | - | - | - | - | - | - | - | - | - | - |
| Spiderwort, T. | - | - | - | 0 | 0 | - | - | - | 0 | 0 | 0 | 0 | - | - |
| Surinam Grass | 100 | 98 | 100 | 100 | 50 | 100 | 100 | 100 | 80 | 80 | 100 | 100 | 98 | 100 |

| Table E | Compound | | | | | | Table E | | Compound | | | | | |
|---|---|---|---|---|---|---|---|---|---|---|---|---|---|---|
| 31 g ai/ha | 54 | | | | | | 31 g ai/ha | | 54 | | | | | |
| Postemergence | | | | | | | Postemergence | | | | | | | |
| Barnyardgrass | 100 | | | | | | Johnsongrass | | 100 | | | | | |
| Bermudagrass | 40 | | | | | | Kochia | | - | | | | | |
| Mustard, Black | 0 | | | | | | Mallow | | 0 | | | | | |
| Bluegrass | 0 | | | | | | Morningglory | | 0 | | | | | |
| Chickweed | - | | | | | | Prickly Sida | | 0 | | | | | |
| Crabgrass, Large | 95 | | | | | | Nutsedge, Purple | | 0 | | | | | |
| Crabgrass, Naked | 90 | | | | | | Purslane | | 0 | | | | | |
| Dallisgrass | 40 | | | | | | Quackgrass | | 0 | | | | | |
| Dayflower, V. | 0 | | | | | | Ragweed | | - | | | | | |
| Field Bindweed | - | | | | | | Ryegrass, Italian | | 100 | | | | | |
| Foxtail, Green | 80 | | | | | | Sandbur, S. | | 90 | | | | | |
| Goosegrass | 50 | | | | | | Signalgrass | | 98 | | | | | |
| Groundsel | 0 | | | | | | Sowthistle | | 0 | | | | | |
| Guineagrass | 95 | | | | | | Spanishneedles | | - | | | | | |
| Horseweed | - | | | | | | Spiderwort, T. | | - | | | | | |
| Itchgrass | 95 | | | | | | Surinam Grass | | 100 | | | | | |

| Table E | Compounds | | | | | | | | | | | | | |
|---|---|---|---|---|---|---|---|---|---|---|---|---|---|---|
| 16 g ai/ha | 11 | 19 | 21 | 31 | 32 | 33 | 34 | 35 | 36 | 39 | 40 | 43 | 53 | 54 |
| Postemergence | | | | | | | | | | | | | | |
| Barnyardgrass | 100 | 100 | 100 | 70 | 98 | 100 | 100 | 75 | 50 | 100 | 100 | 100 | 100 | 98 |
| Bermudagrass | 80 | 35 | 50 | 75 | 20 | 80 | 50 | 65 | 40 | 40 | 50 | 20 | 70 | 20 |
| Mustard, Black | 0 | 0 | 0 | 0 | 0 | 0 | 0 | 0 | 0 | 0 | 0 | 0 | 0 | 0 |
| Bluegrass | 0 | 0 | 0 | 0 | 0 | 0 | 0 | 0 | 0 | 0 | 0 | 0 | 0 | 0 |
| Chickweed | 0 | - | - | 0 | - | 0 | - | 0 | 0 | 0 | 0 | - | - | - |
| Crabgrass, Large | 95 | 60 | 90 | 65 | 75 | 75 | 75 | 60 | 70 | 90 | 85 | 60 | 95 | 80 |
| Crabgrass, Naked | 95 | 90 | 90 | 65 | - | 90 | 80 | 50 | 60 | 80 | 75 | 65 | 80 | 85 |
| Dallisgrass | 90 | 60 | 80 | 40 | 20 | 35 | 20 | 30 | 20 | 70 | 75 | - | 20 | 30 |
| Dayflower, V. | 0 | 0 | - | - | 0 | - | 0 | - | - | - | - | 0 | 0 | 0 |
| Field Bindweed | 0 | 0 | - | - | - | - | - | - | - | - | - | - | - | - |
| Foxtail, Green | 98 | 95 | 75 | 60 | 60 | 65 | 75 | 60 | 50 | 70 | 80 | 80 | 80 | 70 |
| Goosegrass | 80 | 60 | 80 | 50 | 35 | 35 | 20 | 30 | 35 | 70 | 65 | 75 | 65 | 0 |
| Groundsel | 0 | 0 | - | 0 | 0 | 0 | 0 | 0 | - | - | - | 0 | 0 | 0 |
| Guineagrass | 98 | 95 | 98 | 80 | 98 | 80 | 95 | 75 | 75 | 80 | 90 | 100 | 98 | 95 |
| Horseweed | 0 | 0 | - | - | - | - | - | - | - | - | - | - | - | - |
| Itchgrass | 85 | 70 | 65 | 60 | 75 | 65 | 80 | 40 | 40 | 25 | 40 | - | 90 | 90 |
| Johnsongrass | 100 | 100 | 65 | 60 | 80 | 75 | 80 | 50 | 50 | 75 | 60 | 25 | 100 | 60 |
| Kochia | 0 | 0 | - | - | - | - | - | - | - | - | - | - | - | - |
| Mallow | 0 | 0 | 0 | 0 | 0 | 0 | 0 | 0 | 0 | 0 | 0 | 0 | 0 | 0 |
| Morningglory | 0 | 0 | 0 | 0 | 35 | 0 | 0 | 0 | 0 | 0 | 0 | 0 | 0 | 0 |
| Prickly Sida | 0 | 0 | - | - | 0 | - | 0 | - | - | - | - | 0 | 0 | 0 |
| Nutsedge, Purple | - | 0 | 0 | 0 | 0 | 0 | 0 | 0 | 0 | 0 | 0 | 0 | 0 | 0 |
| Purslane | 0 | 0 | 0 | 0 | 0 | 0 | 0 | 0 | 0 | 0 | 0 | 0 | 0 | 0 |
| Quackgrass | 90 | 75 | 35 | 30 | 0 | 30 | 0 | 10 | 0 | 30 | 50 | 0 | 0 | 0 |
| Ragweed | 0 | 0 | - | - | - | - | - | - | - | - | - | - | - | - |
| Ryegrass, Italian | 95 | 100 | 98 | 35 | 95 | 75 | 95 | 20 | 35 | 98 | 90 | 80 | 100 | 80 |
| Sandbur, S. | 100 | 98 | 80 | 70 | 75 | 75 | 75 | 75 | 65 | 85 | 85 | 95 | 80 | 80 |
| Signalgrass | 90 | 98 | 90 | 65 | 80 | 75 | 85 | 70 | 50 | 95 | 90 | 80 | 95 | 90 |
| Sowthistle | 0 | 0 | - | - | 0 | - | 0 | - | - | - | - | 0 | 0 | 0 |
| Spanishneedles | 0 | 0 | - | - | - | - | - | - | - | - | - | - | - | - |
| Spiderwort, T. | - | - | 0 | 0 | - | 0 | - | 0 | 0 | 0 | 0 | - | - | - |
| Surinam Grass | 100 | 95 | 95 | 50 | - | 75 | 98 | 75 | 65 | 100 | 90 | 98 | 90 | 90 |

| Table E | Compounds | | | | | | | | | | | | | |
|---|---|---|---|---|---|---|---|---|---|---|---|---|---|---|
| 8 g ai/ha | 11 | 12 | 19 | 21 | 31 | 32 | 33 | 34 | 35 | 36 | 39 | 40 | 43 | 53 |
| Postemergence | | | | | | | | | | | | | | |
| Barnyardgrass | 100 | 100 | 98 | 100 | 35 | 98 | 95 | 95 | 60 | 50 | 100 | 100 | 100 | 100 |
| Bermudagrass | 35 | 70 | 35 | 40 | 65 | 5 | 70 | 20 | 35 | 40 | 35 | 30 | 0 | 50 |
| Mustard, Black | 0 | 0 | 0 | 0 | 0 | 0 | 0 | 0 | 0 | 0 | 0 | 0 | 0 | 0 |
| Bluegrass | 0 | 0 | 0 | 0 | 0 | 0 | 0 | 0 | 0 | 0 | 0 | 0 | 0 | 0 |
| Chickweed | 0 | 0 | - | - | 0 | - | 0 | - | 0 | 0 | 0 | 0 | - | - |
| Crabgrass, Large | 70 | 65 | 40 | 80 | 60 | 35 | 70 | 60 | 50 | 65 | 65 | 75 | 30 | 35 |
| Crabgrass, Naked | 90 | 90 | 85 | 80 | 30 | 60 | 75 | 40 | 50 | 40 | 65 | 50 | 65 | 30 |
| Dallisgrass | 75 | 75 | 30 | 75 | 35 | 0 | 35 | 10 | 30 | 0 | 60 | 65 | 0 | 0 |
| Dayflower, V. | 0 | 0 | 0 | - | - | 0 | - | 0 | - | - | - | - | 0 | 0 |
| Field Bindweed | 0 | 0 | 0 | - | - | - | - | - | - | - | - | - | - | - |
| Foxtail, Green | 90 | 90 | 90 | 65 | 50 | 40 | 60 | 50 | 40 | 35 | 60 | 70 | 75 | 70 |
| Goosegrass | 35 | 30 | 25 | 75 | 35 | 35 | 35 | 0 | 30 | 0 | 35 | 65 | 20 | 20 |
| Groundsel | 0 | 0 | 0 | 0 | 0 | 0 | 0 | 0 | 0 | - | - | - | 0 | 0 |
| Guineagrass | 95 | 95 | 90 | 95 | 75 | 90 | 80 | 95 | 75 | 35 | 80 | 80 | 100 | 90 |
| Horseweed | 0 | 0 | 0 | - | - | - | - | - | - | - | - | - | - | - |
| Itchgrass | 75 | 50 | 20 | 50 | 50 | 30 | 40 | 70 | 30 | 0 | 20 | 40 | 20 | 70 |
| Johnsongrass | 100 | 95 | 90 | 50 | 40 | 50 | 50 | 10 | 40 | 35 | 35 | 60 | 15 | 50 |
| Kochia | 0 | 0 | - | - | - | - | - | - | - | - | - | - | - | - |
| Mallow | 0 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 |
| Prickly Sida | 0 | 0 | 0 | - | - | 0 | - | 0 | - | - | - | - | 0 | 0 |
| Nutsedge, Purple | 0 | 0 | - | 0 | 0 | 0 | 0 | 0 | 0 | 0 | 0 | 0 | 0 | 0 |
| Purslane | 0 | 0 | 0 | 0 | 0 | 0 | 0 | 0 | 0 | 0 | 0 | 0 | 0 | 0 |
| Quackgrass | 65 | 30 | 15 | 20 | 0 | 0 | 0 | 0 | 0 | 0 | 0 | 30 | 0 | 0 |
| Ragweed | 0 | 0 | 0 | - | - | - | - | - | - | - | - | - | - | - |
| Ryegrass, Italian | 90 | 100 | 90 | 80 | 30 | 75 | 35 | 60 | 20 | 0 | 75 | 90 | 50 | 80 |
| Sandbur, S. | 95 | 98 | 98 | 80 | 0 | 60 | 65 | 75 | 40 | 40 | 75 | 75 | 80 | 65 |
| Signalgrass | 90 | 80 | 80 | 80 | 65 | 80 | 75 | 75 | 60 | 10 | 35 | 75 | 80 | 90 |
| Sowthistle | 0 | 0 | 0 | - | - | 0 | - | 0 | - | - | - | - | 0 | 0 |
| Spanishneedles | 0 | 0 | 0 | - | - | - | - | - | - | - | - | - | - | - |
| Spiderwort, T. | - | - | - | 0 | 0 | - | 0 | - | 0 | 0 | 0 | 0 | - | - |
| Surinam Grass | 80 | 90 | 70 | 95 | 20 | 80 | 75 | 90 | 70 | 65 | 95 | 90 | 95 | 90 |

| Table E | Compound | | | | | | Table E | | | | | Compounds | | |
|---|---|---|---|---|---|---|---|---|---|---|---|---|---|---|
| 8 g ai/ha | 54 | | | | | | 4 g ai/ha | | | | | 11 | 12 | 19 |
| Postemergence | | | | | | | Postemergence | | | | | | | |
| Barnyardgrass | 95 | | | | | | Barnyardgrass | | | | | 90 | 90 | 90 |
| Bermudagrass | 10 | | | | | | Bermudagrass | | | | | 10 | 15 | 15 |
| Mustard, Black | 0 | | | | | | Mustard, Black | | | | | 0 | 0 | 0 |
| Bluegrass | 0 | | | | | | Bluegrass | | | | | 0 | 0 | 0 |
| Chickweed | - | | | | | | Chickweed | | | | | 0 | 0 | 0 |
| Crabgrass, Large | 25 | | | | | | Crabgrass, Large | | | | | 60 | 0 | 30 |
| Crabgrass, Naked | 10 | | | | | | Crabgrass, Naked | | | | | 25 | 50 | 40 |
| Dallisgrass | 20 | | | | | | Dallisgrass | | | | | 60 | 65 | 0 |
| Dayflower, V. | 0 | | | | | | Dayflower, V. | | | | | 0 | 0 | 0 |
| Field Bindweed | - | | | | | | Field Bindweed | | | | | 0 | 0 | 0 |
| Foxtail, Green | 40 | | | | | | Foxtail, Green | | | | | 60 | 30 | 20 |
| Goosegrass | 0 | | | | | | Goosegrass | | | | | 10 | 10 | 0 |
| Groundsel | 0 | | | | | | Groundsel | | | | | 0 | 0 | 0 |
| Guineagrass | 95 | | | | | | Guineagrass | | | | | 90 | 95 | 80 |
| Horseweed | - | | | | | | Horseweed | | | | | 0 | 0 | 0 |
| Itchgrass | 10 | | | | | | Itchgrass | | | | | 0 | 0 | 0 |
| Johnsongrass | 20 | | | | | | Johnsongrass | | | | | 95 | 95 | 90 |
| Kochia | - | | | | | | Kochia | | | | | 0 | 0 | 0 |
| Mallow | 0 | | | | | | Mallow | | | | | 0 | 0 | 0 |
| Morningglory | 0 | | | | | | Morningglory | | | | | 0 | 0 | 0 |
| Prickly Sida | 0 | | | | | | Prickly Sida | | | | | 0 | 0 | 0 |
| Nutsedge, Purple | 0 | | | | | | Nutsedge, Purple | | | | | 0 | 0 | 0 |
| Purslane | 0 | | | | | | Purslane | | | | | 0 | 0 | 0 |
| Quackgrass | 0 | | | | | | Quackgrass | | | | | 40 | 20 | 0 |
| Ragweed | - | | | | | | | Ragweed | | | | 0 | 0 | 0 |
| Ryegrass, Italian | 80 | | | | | | | Ryegrass, Italian | | | | 75 | 75 | 50 |
| Sandbur, S. | 60 | | | | | | | Sandbur, S. | | | | 90 | 90 | 90 |
| Signalgrass | 75 | | | | | | | Signalgrass | | | | 90 | 65 | 65 |
| Sowthistle | 0 | | | | | | | Sowthistle | | | | 0 | 0 | 0 |
| Spanishneedles | - | | | | | | | Spanishneedles | | | | 0 | 0 | 0 |
| Spiderwort, T. | - | | | | | | | Surinam Grass | | | | 25 | 35 | 35 |
| Surinam Grass | 90 | | | | | | | | | | | | | |

| Table E | Compounds | | | | | | | | | | | | | |
|---|---|---|---|---|---|---|---|---|---|---|---|---|---|---|
| 62 g ai/ha | 21 | 31 | 33 | 34 | 35 | 36 | 39 | 40 | 43 | 53 | | | | |
| Preemergence | | | | | | | | | | | | | | |
| Barnyardgrass | 100 | 90 | 100 | 100 | 100 | 100 | 100 | 100 | 100 | 100 | | | | |
| Bermudagrass | 100 | 100 | 100 | 100 | 100 | 100 | 100 | 100 | 85 | 98 | | | | |
| Mustard, Black | 0 | 0 | 0 | 0 | 0 | 0 | 0 | 0 | 0 | 25 | | | | |
| Bluegrass | 0 | 0 | 0 | 15 | 0 | 0 | 0 | 95 | 0 | 60 | | | | |
| Chickweed | - | - | - | - | - | - | - | - | 0 | 0 | | | | |
| Crabgrass, Large | 100 | 95 | 100 | 100 | 100 | 98 | 100 | 100 | 80 | 100 | | | | |
| Crabgrass, Naked | 100 | 100 | 100 | 100 | 100 | 100 | 100 | 100 | 75 | 100 | | | | |
| Dallisgrass | 100 | 98 | 100 | 85 | 90 | 98 | 100 | 100 | 90 | 95 | | | | |
| Dayflower, V. | - | - | - | 0 | - | - | - | - | 0 | 0 | | | | |
| Foxtail, Green | 100 | 90 | 100 | 100 | 98 | 100 | 100 | 100 | 100 | 100 | | | | |
| Goosegrass | 100 | 100 | 100 | 100 | 100 | 100 | 100 | 100 | 98 | 100 | | | | |
| Groundsel | - | - | - | 0 | - | - | - | - | 0 | 0 | | | | |
| Guineagrass | 100 | 100 | 100 | 100 | 100 | 100 | 100 | 100 | 100 | 100 | | | | |
| Itchgrass | 80 | 75 | 80 | 90 | 75 | 90 | 80 | 85 | 85 | 90 | | | | |
| Johnsongrass | 95 | 98 | 98 | 90 | 90 | 95 | 98 | 98 | 60 | 95 | | | | |
| Kochia | - | - | - | 0 | - | - | - | - | 0 | - | | | | |
| Mallow | - | 0 | 0 | 0 | 0 | 0 | - | - | 0 | 0 | | | | |
| Morningglory | 0 | 0 | 0 | 0 | 0 | 0 | 0 | 0 | 30 | 35 | | | | |
| Prickly Sida | - | - | - | 0 | - | - | - | - | 0 | 0 | | | | |
| Nutsedge, Purple | 0 | 0 | 0 | 35 | 0 | 0 | - | 0 | 75 | 98 | | | | |
| Purslane | 0 | 0 | 0 | - | 0 | 0 | 0 | 0 | 0 | 0 | | | | |
| Quackgrass | 95 | 65 | 60 | 70 | 50 | 70 | 100 | 90 | 95 | 70 | | | | |
| Thistle, Russian | - | - | - | 0 | - | - | - | - | 0 | 0 | | | | |
| Ryegrass, Italian | 100 | 100 | 100 | 100 | 100 | 100 | - | 100 | 90 | 100 | | | | |
| Sandbur, S. | 100 | 90 | 100 | 100 | 100 | 100 | 100 | 100 | 98 | 100 | | | | |
| Signalgrass | 100 | 80 | 100 | 100 | 100 | 100 | 100 | 100 | - | 98 | | | | |
| Sowthistle | - | - | - | 0 | - | - | - | - | 0 | 0 | | | | |
| Spiderwort, T. | 0 | 0 | 0 | - | 0 | 0 | 0 | 0 | - | - | | | | |
| Surinam Grass | 100 | 95 | 100 | 100 | 100 | 100 | 100 | 100 | 100 | 100 | | | | |

| Table E | Compounds | | | | | | | | | | | | | |
|---|---|---|---|---|---|---|---|---|---|---|---|---|---|---|
| 31 g ai/ha | 11 | 12 | 19 | 21 | 31 | 33 | 34 | 35 | 36 | 39 | 40 | 43 | 53 | |
| Preemergence | | | | | | | | | | | | | | |
| Barnyardgrass | 98 | 95 | 100 | 100 | 75 | 100 | 100 | 95 | 95 | 100 | 100 | 100 | 100 | |
| Bermudagrass | 100 | 95 | 100 | 95 | 95 | 98 | 90 | 98 | 100 | 95 | 98 | 70 | 98 | |
| Mustard, Black | 0 | 0 | 0 | 0 | 0 | 0 | 0 | 0 | 0 | 0 | 0 | 0 | 0 | |
| Bluegrass | 0 | 0 | 0 | 0 | 0 | 0 | 0 | 0 | 0 | 0 | - | 0 | 0 | |
| Chickweed | - | - | - | - | - | - | - | - | - | - | - | 0 | - | |
| Crabgrass, Large | 100 | 98 | 100 | 100 | 75 | 100 | 50 | 95 | 98 | 98 | 100 | 70 | 95 | |
| Crabgrass, Naked | 100 | 100 | 98 | 100 | 98 | 100 | 100 | 100 | 100 | 98 | 100 | 35 | 100 | |
| Dallisgrass | 85 | 100 | 95 | 100 | 75 | 80 | 85 | 90 | 75 | 98 | 100 | 80 | 80 | |
| Dayflower, V. | 0 | 0 | - | - | - | - | 0 | - | - | - | - | 0 | 0 | |
| Field Bindweed | 0 | 0 | 0 | - | - | - | - | - | - | - | - | - | - | |
| Foxtail, Green | 98 | 100 | 100 | 100 | 60 | 100 | 75 | 90 | 95 | 98 | 98 | 85 | 65 | |
| Goosegrass | 90 | 75 | 90 | 100 | 100 | 98 | 95 | 100 | 98 | 100 | 100 | 95 | 100 | |
| Groundsel | 0 | 0 | 0 | - | - | - | 0 | - | - | - | - | 0 | 0 | |
| Guineagrass | 98 | 100 | 100 | 100 | 100 | 100 | 100 | 100 | 100 | 100 | 100 | 100 | 100 | |
| Itchgrass | 80 | 80 | 80 | 75 | 75 | 80 | 85 | 75 | 75 | 75 | 75 | 50 | 85 | |
| Johnsongrass | 95 | 95 | 98 | 90 | 80 | 85 | 90 | 80 | 85 | 80 | 75 | 50 | 90 | |
| Kochia | 0 | 0 | 0 | - | - | - | 0 | - | - | - | - | 0 | 0 | |
| Mallow | - | 0 | 0 | - | 0 | 0 | 0 | - | 0 | - | - | 0 | - | |
| Morningglory | 0 | 0 | 0 | 0 | 0 | 0 | 0 | 0 | 0 | 0 | 0 | 20 | 0 | |
| Prickly Sida | 0 | 0 | 0 | - | - | - | 0 | - | - | - | - | 0 | 0 | |
| Nutsedge, Purple | 0 | 0 | - | 0 | 0 | 0 | 20 | - | 0 | 0 | 0 | 0 | 35 | |
| Purslane | 0 | 0 | 0 | 0 | 0 | 0 | 0 | 0 | 0 | 0 | 0 | 0 | 0 | |
| Quackgrass | 75 | 80 | 80 | 90 | 20 | 20 | 50 | 40 | 50 | 95 | 80 | 80 | 65 | |
| Ragweed | 0 | 0 | 0 | - | - | - | - | - | - | - | - | - | - | |
| Thistle, Russian | 0 | - | 0 | - | - | - | 0 | - | - | - | - | 0 | 0 | |
| Ryegrass, Italian | 100 | 98 | 100 | 100 | 90 | 100 | 100 | 100 | 100 | 100 | 100 | 90 | 100 | |
| Sandbur, S. | 95 | 95 | 95 | 100 | 20 | 90 | 90 | 90 | 100 | 100 | 100 | 75 | 100 | |
| Signalgrass | 100 | 95 | 80 | 100 | 65 | 98 | 90 | 100 | 100 | 95 | 98 | 100 | 98 | |
| Sowthistle | - | - | - | - | - | - | 0 | - | - | - | - | 0 | 0 | |
| Spanishneedles | 0 | 0 | 0 | - | - | - | - | - | - | - | - | - | - | |
| Spiderwort, T. | - | - | - | 0 | 0 | 0 | - | 0 | 0 | 0 | 0 | - | - | |
| Surinam Grass | 95 | 95 | 100 | 100 | 95 | 98 | 100 | 100 | 100 | 100 | 100 | 70 | 100 | |

| Table E | Compounds | | | | | | | | | | | | | |
|---|---|---|---|---|---|---|---|---|---|---|---|---|---|---|
| 16 g ai/ha | 11 | 19 | 21 | 31 | 33 | 34 | 35 | 36 | 39 | 40 | 43 | 53 | | |
| Preemergence | | | | | | | | | | | | | | |
| Barnyardgrass | 90 | 95 | 100 | 15 | 80 | 80 | 80 | 95 | 90 | 95 | 50 | 95 | | |
| Bermudagrass | 40 | 95 | 90 | 75 | 90 | 40 | 80 | 100 | 75 | 80 | 30 | 80 | | |
| Mustard, Black | 0 | 0 | 0 | 0 | 0 | 0 | 0 | 0 | 0 | 0 | 0 | 0 | | |
| Bluegrass | 0 | 0 | 0 | 0 | 0 | 0 | 0 | 0 | 0 | 90 | 0 | 0 | | |
| Chickweed | - | - | - | - | - | 0 | - | - | - | - | 0 | - | | |
| Crabgrass, Large | 30 | 95 | 100 | 40 | 75 | - | 20 | 95 | 60 | 70 | 0 | 40 | | |
| Crabgrass, Naked | 95 | - | 95 | 50 | 95 | 100 | 98 | 100 | 98 | 90 | 35 | 80 | | |
| Dallisgrass | 35 | 90 | 100 | 15 | 50 | 50 | 40 | 60 | 98 | 95 | 40 | 65 | | |
| Dayflower, V. | 0 | 0 | - | - | - | 0 | - | - | - | - | 0 | 0 | | |
| Field Bindweed | 0 | 0 | - | - | - | - | - | - | - | - | - | - | | |
| Foxtail, Green | 98 | 65 | 100 | 5 | 65 | 65 | 35 | 60 | 90 | 75 | 25 | 20 | | |
| Goosegrass | 50 | 70 | 100 | 35 | 98 | 65 | 75 | 98 | 80 | 98 | 65 | 80 | | |
| Groundsel | 0 | 0 | - | - | - | 0 | - | - | - | - | 0 | 0 | | |
| Guineagrass | 98 | 100 | 100 | 90 | 100 | 100 | 100 | 100 | 100 | 100 | 95 | 98 | | |
| Itchgrass | 65 | 65 | 65 | 0 | 60 | 80 | 75 | 65 | 60 | 60 | 35 | 75 | | |
| Johnsongrass | 90 | 95 | 75 | 25 | 75 | 50 | 75 | 75 | 75 | 75 | 0 | 70 | | |
| Kochia | 0 | 0 | - | - | - | 0 | - | - | - | - | 0 | 0 | | |
| Mallow | - | 0 | - | - | 0 | 0 | - | 0 | - | - | 0 | - | | |
| Morningglory | 0 | 0 | 0 | 0 | 0 | 0 | 0 | 0 | 0 | 0 | 0 | 0 | | |
| Prickly Sida | 0 | 0 | - | - | - | 0 | - | - | - | - | 0 | 0 | | |
| Nutsedge, Purple | 0 | - | 0 | 0 | 0 | 0 | 0 | 0 | 0 | 0 | 0 | 0 | | |
| Purslane | 0 | 0 | 0 | 0 | 0 | 0 | 0 | 0 | 0 | 0 | 0 | 0 | | |
| Quackgrass | 60 | 65 | 50 | 0 | 20 | 20 | 20 | 20 | 60 | 60 | 70 | 35 | | |
| Ragweed | 0 | 0 | - | - | - | - | - | - | - | - | - | - | | |
| Thistle, Russian | - | - | - | - | - | 0 | - | - | - | - | 0 | 0 | | |
| Ryegrass, Italian | 80 | 100 | 100 | 65 | 98 | 80 | 98 | 100 | 100 | 98 | 90 | 90 | | |
| Sandbur, S. | 90 | 80 | 98 | 15 | 90 | 75 | 75 | 90 | 90 | 98 | 60 | 30 | | |
| Signalgrass | 90 | 80 | 98 | 15 | 75 | 65 | 80 | 70 | 85 | 95 | 60 | 40 | | |
| Sowthistle | 0 | - | - | - | - | 0 | - | - | - | - | 0 | 0 | | |
| Spanishneedles | 0 | 0 | - | - | - | - | - | - | - | - | - | - | | |
| Spiderwort, T. | - | - | 0 | 0 | 0 | - | 0 | 0 | 0 | 0 | - | - | | |
| Surinam Grass | 95 | 100 | 100 | 20 | 98 | 98 | 95 | 95 | 100 | 100 | 50 | 100 | | |

| Table E | Compounds | | | | | | | | | | | | | |
|---|---|---|---|---|---|---|---|---|---|---|---|---|---|---|
| 8 g ai/ha | 11 | 12 | 19 | 21 | 31 | 33 | 35 | 36 | 39 | 40 | | | | |
| Preemergence | | | | | | | | | | | | | | |
| Barnyardgrass | 75 | 70 | 75 | 95 | 0 | 65 | 40 | 80 | 90 | 75 | | | | |
| Bermudagrass | 25 | 75 | 60 | 40 | 0 | 90 | 60 | 75 | 20 | 40 | | | | |
| Mustard, Black | 0 | 0 | 0 | 0 | 0 | 0 | 0 | 0 | 0 | 0 | | | | |
| Bluegrass | 0 | 0 | 0 | 0 | 0 | 0 | 0 | 0 | 0 | 60 | | | | |
| Crabgrass, Large | 0 | 35 | 15 | 65 | 0 | 40 | 20 | 35 | 30 | 50 | | | | |
| Crabgrass, Naked | 40 | 95 | 98 | - | 0 | 75 | 75 | 90 | 75 | 90 | | | | |
| Dallisgrass | 20 | 40 | 40 | 80 | 0 | 25 | 0 | 35 | 35 | 80 | | | | |
| Dayflower, V. | 0 | 0 | 0 | - | - | - | - | - | - | - | | | | |
| Field Bindweed | 0 | 0 | 0 | - | - | - | - | - | - | - | | | | |
| Foxtail, Green | 50 | 60 | 40 | 75 | 0 | 40 | 15 | 15 | 75 | 40 | | | | |
| Goosegrass | 25 | 65 | 40 | 95 | 20 | 75 | 20 | 30 | 75 | 75 | | | | |
| Groundsel | 0 | 0 | 0 | - | - | - | - | - | - | - | | | | |
| Guineagrass | 0 | 95 | 75 | 100 | 35 | 100 | 90 | 100 | 100 | 100 | | | | |
| Itchgrass | 35 | 35 | 65 | 40 | 0 | 50 | 0 | 15 | 50 | 35 | | | | |
| Johnsongrass | 75 | 90 | 90 | 60 | 15 | 35 | 30 | 60 | 60 | 40 | | | | |
| Kochia | 0 | 0 | 0 | - | - | - | - | - | - | - | | | | |
| Mallow | 0 | 0 | - | - | - | 0 | 0 | 0 | 0 | - | | | | |
| Morningglory | 0 | 0 | 0 | 0 | 0 | 0 | 0 | 0 | 0 | 0 | | | | |
| Prickly Sida | 0 | 0 | 0 | - | - | - | - | - | - | - | | | | |
| Nutsedge, Purple | - | - | - | 0 | - | 0 | 0 | 0 | 0 | 0 | | | | |
| Purslane | 0 | 0 | 0 | 0 | 0 | 0 | 0 | 0 | 0 | 0 | | | | |
| Quackgrass | 10 | 20 | 30 | 40 | 0 | 0 | 0 | 0 | 50 | 40 | | | | |
| Ragweed | 0 | 0 | 0 | - | - | - | - | - | - | - | | | | |
| Thistle, Russian | 0 | - | - | - | - | - | - | - | - | - | | | | |
| Ryegrass, Italian | 80 | 80 | 75 | 100 | 0 | 90 | 75 | 80 | 100 | 90 | | | | |
| Sandbur, S. | 35 | 40 | 60 | 90 | 0 | 50 | 30 | 40 | 90 | 50 | | | | |
| Signalgrass | 60 | 15 | 80 | 80 | 0 | 75 | 60 | 50 | 80 | 60 | | | | |
| Spanishneedles | 0 | 0 | 0 | - | - | - | - | - | - | - | | | | |
| Spiderwort, T. | - | - | - | 0 | 0 | 0 | 0 | 0 | 0 | 0 | | | | |
| Surinam Grass | 70 | 80 | 100 | 98 | 10 | 90 | 75 | 95 | 100 | 98 | | | | |

| Table E | Compounds | | | | | | Table E | | | | | Compounds | | |
|---|---|---|---|---|---|---|---|---|---|---|---|---|---|---|
| 4 g ai/ha | 11 | 12 | 19 | | | | 4 g ai/ha | | | | | 11 | 12 | 19 |
| Preemergence | | | | | | | Preemergence | | | | | | | |
| Barnyardgrass | 0 | 20 | 0 | | | | Kochia | | | | | 0 | 0 | 0 |
| Bermudagrass | 15 | 35 | 25 | | | | Mallow | | | | | - | 0 | - |
| Mustard, Black | 0 | 0 | 0 | | | | Morningglory | | | | | 0 | 0 | 0 |
| Bluegrass | 0 | 0 | 0 | | | | Prickly Sida | | | | | 0 | 0 | 0 |
| Crabgrass, Large | 0 | 0 | 0 | | | | Nutsedge, Purple | | | | | - | - | 0 |
| Crabgrass, Naked | - | 15 | 35 | | | | Purslane | | | | | 0 | 0 | 0 |
| Dallisgrass | 10 | 0 | 20 | | | | Quackgrass | | | | | 0 | 0 | 0 |
| Dayflower, V. | 0 | 0 | 0 | | | | Ragweed | | | | | 0 | 0 | 0 |
| Field Bindweed | 0 | 0 | 0 | | | | Thistle, Russian | | | | | - | 0 | - |
| Foxtail, Green | 0 | 0 | 0 | | | | Ryegrass, Italian | | | | | 20 | 40 | 65 |
| Goosegrass | 25 | 25 | 15 | | | | Sandbur, S. | | | | | 0 | 0 | 0 |
| Groundsel | 0 | 0 | 0 | | | | Signalgrass | | | | | 0 | 0 | 40 |
| Guineagrass | 0 | 0 | 60 | | | | Sowthistle | | | | | - | 0 | - |
| Itchgrass | 0 | 20 | 10 | | | | Spanishneedles | | | | | 0 | - | 0 |
| Johnsongrass | 75 | 75 | 75 | | | | Surinam Grass | | | | | 0 | 0 | 50 |

### TEST F

This test evaluated the effect of mixtures of Compound 3, Compound 11, or Compound 12 with the commercial crop safener cloquintocet-mexyl on three plant species. Seeds of test plants consisting of winter wheat (TRZAW, *Triticum aestivum*), winter barley (HORVX, *Hordeum vulgare*), and wild oat (AVEFA, *Avena fatua*) (a weed) were planted into a blend of loam soil and sand and treated preemergence with a directed soil spray using test chemicals formulated in a non-phytotoxic solvent mixture that included a surfactant. At the same time, plants selected from these crop and weed species were treated with postemergence applications of the test chemicals formulated in the same manner. Plants ranged in height from 2 to 18 cm (1- to 4-leaf stage) for postemergence treatments.

Plants were grown in a greenhouse using supplemental lighting to maintain a photoperiod of about 16 hours; daytime and nighttime temperatures were about 24-30 °C and 19-21 °C, respectively. Balanced fertilizer was applied through the watering system. Treatments consisted of Compound 3, Compound 11, or Compound 12 and the above mentioned safener alone and in combination using a spray volume of 457 L/ha. Each treatment was replicated three times. Treated plants and controls were maintained in a greenhouse for 14 to 21 days, after which time all treated plants were compared to controls and visually evaluated. Plant response ratings were calculated as the means of the three replicates and are summarized in Tables F1 to F6, and are based on a scale of 0 to 100 where 0 is no effect and 100 is complete control. Because cloquintocet-mexyl alone caused no injury at the rates tested, the expected effect of a mixture of Compound 3 with cloquintocet-mexyl is the same as the observed effect of Compound 3 applied alone. The observed and expected additive effects are listed in Tables F1 to F6.

**Table F1 - Observed and Expected Results from Compound 3 Alone and in Combination with cloquintocet-mexyl***

| Preemergence Application Rate (g a.i./ha) | | TRZAW | | HORVX | | AVEFA | |
|---|---|---|---|---|---|---|---|
| Compound 3 | cloquintocet-mexyl | Obsd. | Exp. | Obsd. | Exp. | Obsd. | Exp. |
| 4 | - | 0 | - | 10 | - | 0 | - |
| 8 | - | 33 | - | 32 | - | 35 | - |
| 16 | - | 77 | - | 68 | - | 60 | - |
| - | 4 | 0 | - | 0 | - | 0 | - |
| - | 8 | 0 | - | 0 | - | 0 | - |
| 4 | 4 | 0 | 0 | 0 | 10 | 0 | 0 |
| 4 | 8 | 5 | 0 | 3 | 10 | 0 | 0 |
| 8 | 4 | 17 | 33 | 20 | 32 | 53 | 35 |
| 8 | 8 | 33 | 33 | 17 | 32 | 40 | 35 |
| 16 | 4 | 58 | 77 | 75 | 68 | 67 | 60 |
| 16 | 8 | 75 | 77 | 65 | 68 | 78 | 60 |

| | | | | | | | |
|---|---|---|---|---|---|---|---|
| * Application rates are grams of active ingredient per hectare (g a.iha). "Obsd." is observed effect. "Exp." is expected effect. | | | | | | | |

As can be seen from the results listed in Table F1, the observed results for TRZAW at three of the six combination treatments and for HORVX at five of the six combination treatments were less than expected, thereby indicating safening at these mixture application rates of Compound 3 and cloquintocet-mexyl.

**Table F2 - Observed and Expected Results from Compound 3 Alone and in Combination with cloquintocet-mexyl***

| Postemergence Application Rate (g a.i./ha) | | TRZAW | | HORVX | | AVEFA | |
|---|---|---|---|---|---|---|---|
| Compound 3 | cloquintocet-mexyl | Obsd. | Exp. | Obsd. | Exp. | Obsd. | Exp. |
| 4 | - | 0 | - | 0 | - | 27 | - |
| 8 | - | 0 | - | 0 | - | 67 | - |
| 16 | - | 22 | - | 15 | - | 83 | - |
| - | 4 | 0 | - | 0 | - | 0 | - |
| - | 8 | 0 | - | 0 | - | 0 | - |
| 4 | 4 | 0 | 0 | 0 | 0 | 30 | 27 |
| 4 | 8 | 0 | 0 | 0 | 0 | 28 | 27 |
| 8 | 4 | 0 | 0 | 3 | 0 | 55 | 67 |
| 8 | 8 | 0 | 0 | 0 | 0 | 67 | 67 |
| 16 | 4 | 0 | 22 | 0 | 15 | 85 | 83 |
| 16 | 8 | 5 | 22 | 0 | 15 | 85 | 83 |

| | | | | | | | |
|---|---|---|---|---|---|---|---|
| * Application rates are grams of active ingredient per hectare (g a.i./ha). "Obsd." is observed effect. "Exp." is expected effect. | | | | | | | |

As can be seen from the results listed in Table F2, the observed results for TRZAW and HORVX at 16 g ai/ha of compound 3 and 4 or 8 g ai/ha of cloquintocet-mexyl were less than expected, thereby indicating safening at these mixture application rates of Compound 3 and cloquintocet-mexyl.

**Table F3 - Observed and Expected Results from Compound 11 Alone and in Combination with cloquintocet-mexyl***

| Preemergence Application Rate (g a.i./ha) | | TRZAW | | HORVX | | AVEFA | |
|---|---|---|---|---|---|---|---|
| Compound 11 | cloquintocel-mexyl | Obsd. | Exp. | Obsd. | Exp. | Obsd. | Exp. |
| 4 | - | 32 | - | 22 | - | 58 | - |
| 8 | - | 72 | - | 70 | - | 77 | - |
| 16 | - | 92 | - | 92 | - | 93 | - |
| - | 4 | 0 | - | 0 | - | 0 | - |
| - | 8 | 0 | - | 0 | - | 0 | - |
| 4 | 4 | 23 | 32 | 38 | 22 | 73 | 58 |
| 4 | 8 | 60 | 32 | 63 | 22 | 77 | 58 |
| 8 | 4 | 73 | 72 | 82 | 70 | 90 | 77 |
| 8 | 8 | 92 | 72 | 85 | 70 | 92 | 77 |
| 16 | 4 | 95 | 92 | 93 | 92 | 96 | 93 |
| 16 | 8 | 98 | 92 | 95 | 92 | 96 | 93 |

| | | | | | | | |
|---|---|---|---|---|---|---|---|
| * Application rates are grams of active ingredient per hectare (g a.i./ha). "Obsd." is observed effect. "Exp." is expected effect. | | | | | | | |

As can be seen from the results listed in Table F3, the observed results for TRZAW at one of the six combination treatments were less than expected, thereby indicating safening at this mixture application rate of Compound 11 and cloquintocet-mexyl.

**Table F4 - Observed and Expected Results from Compound 11 Alone and in Combination with cloquintocet-mexyl***

| Postemergence Application Rate (g a.i./ha) | | TRZAW | | HORVX | | AVEFA | |
|---|---|---|---|---|---|---|---|
| Compound 11 | cloquintocet-mexyl | Obsd. | Exp. | Obsd. | Exp. | Obsd. | Exp. |
| 4 | - | 18 | - | 5 | - | 75 | - |
| 8 | - | 3 | - | 55 | - | 97 | - |
| 16 | - | 32 | - | 90 | - | 98 | - |
| - | 4 | 0 | - | 0 | - | 0 | - |
| - | 8 | 0 | - | 0 | - | 0 | - |
| 4 | 4 | 18 | 18 | 3 | 5 | 94 | 75 |
| 4 | 8 | 0 | 18 | 7 | 5 | 91 | 75 |
| 8 | 4 | 32 | 3 | 32 | 55 | 96 | 97 |
| 8 | 8 | 28 | 3 | 18 | 55 | 98 | 97 |
| 16 | 4 | 98 | 32 | 87 | 90 | 99 | 98 |
| 16 | 8 | 67 | 32 | 90 | 90 | 100 | 98 |

| | | | | | | | |
|---|---|---|---|---|---|---|---|
| * Application rates are grams of active ingredient per hectare (g a.i./ha). "Obsd." is observed effect. "Exp." is expected effect. | | | | | | | |

As can be seen from the results listed in Table F4, the observed results for HORVX at four of the six combination treatments and TRZAW at 4 g ai/ha of compound 11 and 8 g ai/ha of cloquintocet-mexyl were less than expected, thereby indicating safening at these mixture application rates of Compound 11 and cloquintocet-mexyl.

**Table F5 - Observed and Expected Results from Compound 12 Alone and in Combination with cloquintocet-mexyl***

| Preemergence Application Rate (g a.i./ha) | | TRZAW | | HORVX | | AVEFA | |
|---|---|---|---|---|---|---|---|
| Compound 12 | cloquintocet-mexyl | Obsd. | Exp. | Obsd. | Exp. | Obsd. | Exp. |
| 4 | - | 0 | - | 10 | - | 7 | - |
| 8 | - | 75 | - | 73 | - | 92 | - |
| 16 | - | 97 | - | 80 | - | 96 | - |
| - | 4 | 0 | - | 0 | - | 0 | - |
| - | 8 | 0 | - | 0 | - | 0 | - |
| 4 | 4 | 23 | 0 | 35 | 10 | 70 | 7 |
| 4 | 8 | 30 | 0 | 37 | 10 | 67 | 7 |
| 8 | 4 | 73 | 75 | 75 | 73 | 85 | 92 |
| 8 | 8 | 83 | 75 | 80 | 73 | 90 | 92 |
| 16 | 4 | 93 | 97 | 93 | 80 | 96 | 96 |
| 16 | 8 | 97 | 97 | 92 | 80 | 93 | 96 |

| | | | | | | | |
|---|---|---|---|---|---|---|---|
| * Application rates are grams of active ingredient per hectare (g a.i./ha). "Obsd." is observed effect. "Exp." is expected effect. | | | | | | | |

As can be seen from the results listed in Table F5, the observed results for TRZAW show minimal safening at these mixture application rates of Compound 12 and cloquintocet-mexyl.

**Table F6 - Observed and Expected Results from Compound 12 Alone and in Combination with cloquintocet-mexyl***

| Postemergence Application Rate (g a.i./ha) | | TRZAW | | HORVX | | AVEFA | |
|---|---|---|---|---|---|---|---|
| Compound 12 | cloquintocet-mexyl | Obsd. | Exp. | Obsd. | Exp. | Obsd. | Exp. |
| 4 | - | 0 | - | 0 | - | 13 | - |
| 8 | - | 18 | - | 32 | - | 98 | - |
| 16 | - | 77 | - | 65 | - | 100 | - |
| - | 4 | 0 | - | 0 | - | 0 | - |
| - | 8 | 0 | - | 0 | - | 0 | - |
| 4 | 4 | 7 | 0 | 0 | 0 | 75 | 13 |
| 4 | 8 | 0 | 0 | 0 | 0 | 93 | 13 |
| 8 | 4 | 37 | 18 | 0 | 32 | 98 | 98 |
| 8 | 8 | 0 | 18 | 3 | 32 | 96 | 98 |
| 16 | 4 | 47 | 77 | 67 | 65 | 99 | 100 |
| 16 | 8 | 7 | 77 | 57 | 65 | 99 | 100 |

| | | | | | | | |
|---|---|---|---|---|---|---|---|
| * Application rates are grams of active ingredient per hectare (g a.i./ha). "Obsd." is observed effect. "Exp." is expected effect. | | | | | | | |

As can be seen from the results listed in Table F6, the observed results for TRZAW and HORVX at three of the six combination treatments were less than expected, thereby indicating safening at these mixture application rates of Compound 12 and cloquintocet-mexyl.

### TEST G

This test evaluated the effect of mixtures of Compound 26 with commercial crop safeners cloquintocet-mexyl, mefenpyr-diethyl, and fenchlorazole-ethyl on three plant species. Seeds of winter wheat (TRZAW, *Triticum aestivum*), winter barley (HORVX, *Hordeum vulgare*), and wild oat (AVEFA, *Avena fatua*) (a weed) were planted into a silt loam soil. Plants from these seeds were treated postemergence using test chemicals formulated in a non-phytotoxic solvent mixture that included a surfactant. Plants ranged in height from 6 to 10 cm (2- to 3-leaf stage).

Plants were grown in a greenhouse using supplemental lighting to maintain a photoperiod of about 14 hours; daytime and nighttime temperatures were about 22-26 °C and 15-19 °C, respectively. Balanced fertilizer was applied through the watering system. Treatments consisted of Compound 26 and the above mentioned safeners alone and in combination using a spray volume of 281 L/ha. Each treatment was replicated four times. Treated plants and controls were maintained in a greenhouse for 15 days, after which time all treated plants were compared to controls and visually evaluated. Plant response ratings were calculated as the means of the four replicates based on a scale of 0 to 100 where 0 is no effect and 100 is complete control and are summarized in Tables G1 to G3. Because the safeners alone caused no injury at the rates tested, the expected effect of a mixture of Compound 26 with a safener is the same as the observed effect of Compound 26 alone. The observed and expected effects are listed in Tables G1 to G3.

The highest application rate of each safener was tested alone to confirm the absence of herbicidal activity on the test species when appled postemergence at 125 g ai/ha. Therefore, an extrapolated result of zero for each safener alone at 16, 31, or 62 g ai/ha was used to calculate the mixture response of Compound 26 and these safener application rates.

**Table G1 - Observed and Expected Results from Compound 26 Alone and in Combination with cloquintocet-mexyl***

| Postemergence Application Rate (g a.i./ha) | | TRZAW | | HORVX | | AVEFA | |
|---|---|---|---|---|---|---|---|
| Cmpd 26 | cloquintocet-mexyl | Obsd. | Exp. | Obsd. | Exp. | Obsd. | Exp. |
| 62 | - | 10 | - | 19 | - | 89 | - |
| 125 | - | 9 | - | 34 | - | 97 | - |
| 250 | - | 36 | - | 45 | - | 99 | - |
| - | 125 | 0 | - | 0 | - | 0 | - |
| 62 | 16 | 3 | 10 | 4 | 19 | 97 | 89 |
| 62 | 31 | 3 | 10 | 15 | 19 | 96 | 89 |
| 125 | 31 | 8 | 9 | 20 | 34 | 96 | 97 |
| 125 | 62 | 8 | 9 | 21 | 34 | 99 | 97 |
| 250 | 62 | 21 | 36 | 25 | 45 | 99 | 99 |
| 250 | 125 | 18 | 36 | 20 | 45 | 100 | 99 |

| | | | | | | | |
|---|---|---|---|---|---|---|---|
| * Application rates are grams of active ingredient per hectare (g a.i./ha). "Obsd." is observed effect. "Exp." is expected effect. Cmpd means Compound. | | | | | | | |

As can be seen from the results listed in Table G1, the observed results for TRZAW and HORVX were less than expected, thereby indicating safening at these application mixture rates of Compound 26 and cloquintocet-mexyl.

**Table G2 - Observed and Expected Results from Compound 26 Alone and in Combination with mefenpyr-diethyl***

| Postemergence Application Rate (g a.i./ha) | | TRZAW | | HORVX | | AVEFA | |
|---|---|---|---|---|---|---|---|
| Cmpd 26 | mefenpyr-diethyl | Obsd. | Exp. | Obsd. | Exp. | Obsd. | Exp. |
| 62 | - | 10 | - | 19 | - | 89 | - |
| 125 | - | 9 | - | 34 | - | 97 | - |
| 250 | - | 36 | - | 45 | - | 99 | - |
| - | 125 | 0 | - | 0 | - | 0 | - |
| 62 | 16 | 0 | 10 | 8 | 19 | 95 | 89 |
| 62 | 31 | 0 | 10 | 3 | 19 | 95 | 89 |
| 125 | 31 | 0 | 9 | 16 | 34 | 100 | 97 |
| 125 | 62 | 3 | 9 | 14 | 34 | 96 | 97 |
| 250 | 62 | 14 | 36 | 16 | 45 | 100 | 99 |
| 250 | 125 | 8 | 36 | 30 | 45 | 99 | 99 |

| | | | | | | | |
|---|---|---|---|---|---|---|---|
| * Application rates are grams of active ingredient per hectare (g a.i./ha). "Obsd." is observed effect. "Exp." is expected effect. Cmpd means Compound. | | | | | | | |

As can be seen from the results listed in Table G2, the observed results for TRZAW and HORVX were less than expected, thereby indicating safening at these application mixture rates of Compound 26 and mefenpyr-diethyl.

**Table G3 - Observed and Expected Results from Compound 26 Alone and in Combination with fenchlorazole-ethyl***

| Postemergence Application Rate (g a.i./ha) | | TRZAW | | HORVX | | AVEFA | |
|---|---|---|---|---|---|---|---|
| Cmpd 26 | fenchlorazole-ethyl | Obsd. | Exp. | Obsd. | Exp. | Obsd. | Exp. |
| 62 | - | 10 | - | 19 | - | 89 | - |
| 125 | - | 9 | - | 34 | - | 97 | - |
| 250 | - | 36 | - | 45 | - | 99 | - |
| - | 125 | 0 | - | 0 | - | 0 | - |
| 62 | 16 | 0 | 10 | 5 | 19 | 93 | 89 |
| 62 | 31 | 0 | 10 | 13 | 19 | 95 | 89 |
| 125 | 31 | 0 | 9 | 14 | 34 | 96 | 97 |
| 125 | 62 | 0 | 9 | 20 | 34 | 98 | 97 |
| 250 | 62 | 19 | 36 | 46 | 45 | 100 | 99 |
| 250 | 125 | 16 | 36 | 44 | 45 | 99 | 99 |

| | | | | | | | |
|---|---|---|---|---|---|---|---|
| * Application rates are grams of active ingredient per hectare (g a.i./ha). "Obsd." is observed effect. "Exp." is expected effect. Cmpd means Compound. | | | | | | | |

As can be seen from the results listed in Table G3, the observed results for TRZAW and HORVX at eleven of the twelve combination treatments were less than expected, thereby indicating safening at these application mixture rates of Compound 26 and fenchlorazole-ethyl.

## Claims

1. A compound selected from Formula 1, *N*-oxides and salts thereof, 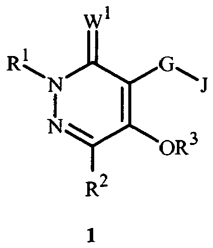 wherein
R¹ is H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, tetrahydropyranyl, -C(=W⁶)R⁴, -C(=W²)W³R⁵, -S(=O)₂R⁶, -P(=W⁴)R⁷R⁸ or -C(=W⁵)NR⁹R¹⁰;
R² is H, halogen, cyano, -C(=O)OH, -C(=O)NH₂, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₃-C₈ cycloalkenyl, C₃-C₈ halocycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ allcylsulfinylalkyl, C₂-C₈ alkylsulfonylalkyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ haloalkoxy, C₁-C₆ alkylthio, C₁-C₆ haloalkylthio, nitro, C₃-C₆ cycloalkoxy or C₄-C₈ cycloalkylalkoxy;
R³ is H, -C(=W⁶)R⁴, -C(=W²)W³R⁵, -S(=O)₂R⁶, -P(=W⁴)R⁷R⁸ or -C(=W⁵)NR⁹R¹⁰;
G is a phenyl ring or a 5- or 6-membered heteroaromatic ring, each ring optionally substituted with R^{x} on nitrogen ring members and optionally substituted with up to 4 substituents selected from R^{w} on carbon ring members;
J is a phenyl ring or a 5- or 6-membered heteroaromatic ring, each ring optionally substituted with up to 5 substituents independently selected from R^{u};
each R⁴ is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ allcylcycloalkyl, C₄-C₁₀ cycloalkylalkyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyle, C₂-C₈ alkylsulfinylalkyl, C₂-C₈ alkylsulfonylalkyl, C₂-C₈ alkylaminoalkyl, C₃-C₁₀ dialkylaminoalkyl, C₂-C₈ haloalkylaminoalkyl or C₄-C₁₀ cycloalkylaminoalkyl, naphthalenyl or -(CR¹¹R¹²)ₙG^{A};
each R⁵ is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₅-C₁₂ alkylcycloalkylalkyl, C₃-C₁₀ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₃-C₁₀ dialkylaminoalkyl, C₂-C₈ haloalkylaminoalkyl, C₄-C₁₀ cycloalkylaminoalkyl, naphthalenyl or -(CR¹¹R¹²)ₙG^{A};
each R⁶ is independently C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₄-C₁₀ halocycloalkylalkyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₃-C₁₀ dialkylaminoalkyl, C₂-C₈ haloalkylaminoalkyl, C₄-C₁₀ cycloalkylaminoalkyl, C₁-C₆ alkylamino, C₂-C₈ dialkylamino, C₂-C₆ haloalkylamino, C₂-C₈ halodialkylamino, C₃-C₈ cycloalkylamino, C₂-C₈ alkylcarbonylamino, C₂-C₈ haloalkylcarbonylamino, C₁-C₆ alkylsulfonylamino, C₁-C₆ haloalkylsulfonylamino, naphthalenyl or -(CR¹¹R¹²)ₙG^{A};
each R⁷ and R⁸ is independently C₁-C₆ alkyl, C₁-C₆ alkoxy, C₁-C₆ alkylamino, C₂-C₈ dialkylamino, C₂-C₆ haloalkylamino, C₂-C₈ halodialkylamino, C₃-C₈ cycloalkylamino, naphthalenyl or -(CR¹¹R¹²)ₙG^{A};
each R⁹ is independently H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₅-C₁₂ alkylcycloalkylalkyl, C₃-C₈ cycloalkenyl, C₂-C₈ alkoxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₄-C₁₀ dialkylaminoalkyl, naphthalenyl or -(CR¹¹R¹²)ₙG^{A};
each R⁹ and R¹⁰ is independently H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl or C₄-C₁₀ cycloalkylalkyl; or R¹⁰ are taken together with the nitrogen to which they are attached to form a 3-to 7-membered heterocyclic ring containing, in addition to the linking nitrogen, ring members selected from carbon and optionally O, S and NR¹³, the carbon ring members optionally in the form of C(=O), and the ring optionally substituted on carbon ring members with up to 4 substituents independently selected from the group consisting of halogen, -CN, C₁-C₃ alkyl and C₁-C₃ alkoxy;
each R¹¹ and R¹² is independently H or C₁-C₃ alkyl;
each R¹³ is independently H or C₁-C₃ alkyl;
each G^{A} is independently a phenyl ring or a 5- or 6-membered heteroaromatic ring, each ring optionally substituted with up to 5 substituents independently selected from R^{u}; or a naphthalenyl ring system optionally substituted with up to 5 substituents independently selected from R^{u};
each R^{u} is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₄-C₁₀ alkylcycloalkyl, C₄-C₁₀ cycloalkylalkyl, C₄-C₁₀ halocycloalkylalkyl, C₅-C₁₂ alkylcycloalkylalkyl, C₂-C₈ alkylcarbonyl, C₂-C₈ haloalkylcarbonyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₁-C₆ alkylaminosulfonyl, C₂-C₈ dialkylaminosulfonyl, C₃-C₁₀ trialkylsilyl, C₁-C₆ alkylamino, C₂-C₈ dialkylamino, C₂-C₈ alkylcarbonylamino, C₁-C₆ alkylsulfonylamino, phenyl, pyridinyl or thienyl;
each R^{w} is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₄-C₁₀ alkylcycloalkyl, C₄-C₁₀ cycloalkylalkyl, C₄-C₁₀ halocycloalkylalkyl, C₅-C₁₂ alkylcycloalkylalkyl, C₂-C₈ alkylcarbonyl, C₂-C₈ haloalkylcarbonyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₁-C₆ alkylaminosulfonyl, C₂-C₈ dialkylaminosulfonyl, C₃-C₁₀ trialkylsilyl, C₁-C₆ alkylamino, C₂-C₈ dialkylamino, C₂-C₈ allcylcarbonylamino, C₁-C₆ alkylsulfonylamino, naphthalenyl, -O(CR¹¹R¹²)ₙG^{A} or -(CR¹¹R¹²)ₙG^{A};
each R^{x} is independently H, C₁-C₃ alkyl or C₃-C₇ cycloalkyl;
each W¹, W², W³, W⁴, W⁵ and W⁶ is independently O or S; and
each n is independently an integer selected from 0 through 3.

2. A compound of Claim 1 wherein
R¹ is H, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₈ cycloalkyl, C₃-C₈ halocycloalkyl, C₄-C₁₀ alkylcycloalkyl, C₄-C₁₀ cycloalkylalkyl, C₆-C₁₄ cycloalkylcycloalkyl, C₄-C₁₀ halocycloalkylalkyl, C₅-C₁₂ alkylcycloalkylalkyl, C₂-C₈ alkoxyalkyl, C₄-C₁₀ cycloalkoxyalkyl or C₃-C₁₀ alkoxyalkoxyalkyl;
R² is H, halogen, C₁-C₆ alkyl, C₁-C₆ haloalkyl, C₃-C₈ cycloalkyl, C₂-C₈ alkoxyalkyl or C₁-C₄ alkoxy;
R³ is H, -C(=W⁶)R⁴, -C(=W²)W³R⁵, -S(=O)₂R⁶ or -C(=W⁵)NR⁹R¹⁰;
G is a phenyl ring or a 5- or 6-membered heteroaromatic ring, each ring optionally substituted with R^{x} on nitrogen ring members and optionally substituted with up to 2 substituents selected from R^{w} on carbon ring members;
each R^{w} is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₄-C₁₀ alkylcycloalkyl, C₄-C₁₀ cycloalkylalkyl, C₂-C₈ alkylcarbonyl, C₂-C₈ haloalkylcarbonyl, C₂-C₈ alkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfonyl, C₁-C₆ haloalkylsulfonyl, C₁-C₆ alkylamino, C₂-C₈ dialkylamino, -O(CR¹¹R¹²)ₙG^{A} or-(CR¹¹R¹²)ₙG^{A};
each R^{x} is independently H or C₁-C₃ alkyl;
J is a phenyl or a 5- or 6-membered heteroaromatic ring, each ring substituted with up to 3 substituents independently selected from R^{u}; and
each R^{u} is independently halogen, cyano, hydroxy, amino, nitro, -CHO, -C(=O)OH, -C(=O)NH₂, -SO₂NH₂, SF₅, C₁-C₆ alkyl, C₂-C₆ alkenyl, C₂-C₆ alkynyl, C₁-C₆ haloalkyl, C₃-C₈ cycloalkyl, C₃-C₈ halocycloalkyl, C₂-C₈ alkylcarbonyl, C₂-C₈ haloalkylcarbonyl, C₂-C₈ alkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfonyl, C₁-C₆ haloalkylsulfonyl, C₁-C₆ allcylaminosulfonyl, C₂-C₈ dialkylaminosulfonyl, C₁-C₆ alkylamino, C₂-C₈ dialkylamino, C₂-C₈ alkylcarbonylamino, C₁-C₆ alkylsulfonylamino, phenyl, pyridinyl or thienyl.

3. A compound of Claim 2 wherein
R¹ is H, C₁-C₆ alkyl, C₁-C₆ haloalkyl or C₃-C₈ cycloalkyl;
R² is H, halogen, C₁-C₆ alkyl or C₁-C₄ alkoxy;
G is selected from 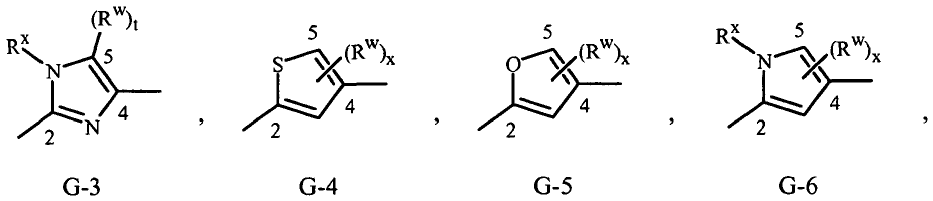 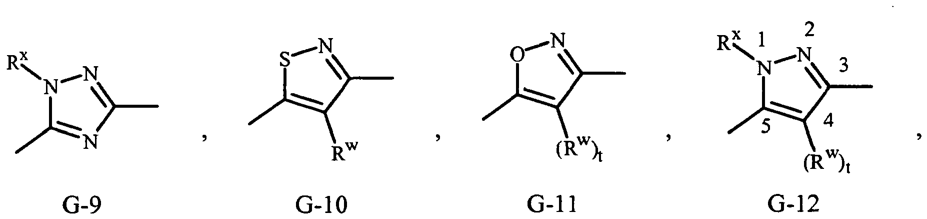 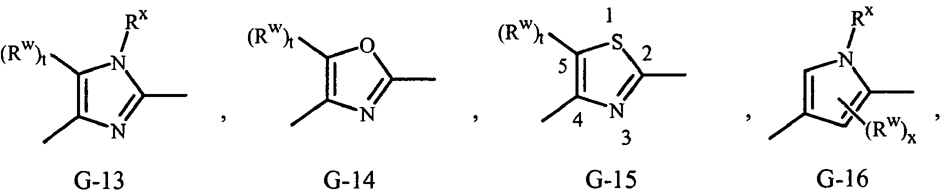 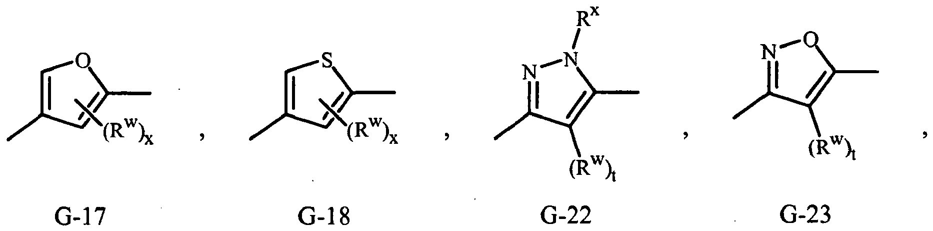 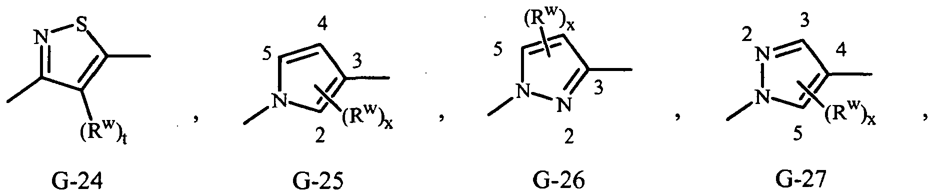 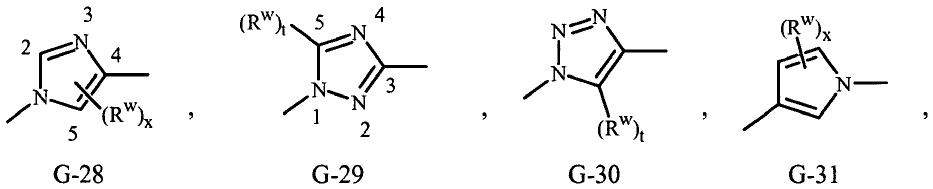 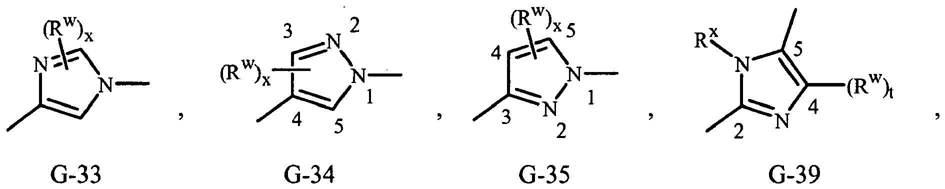 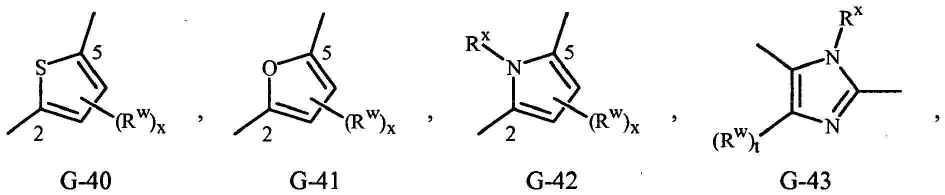 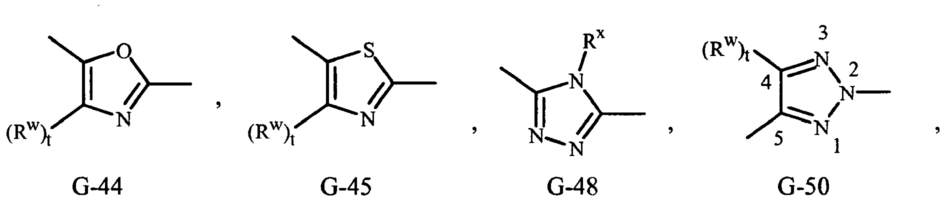 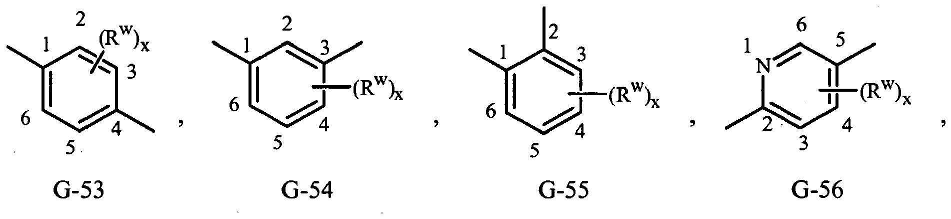 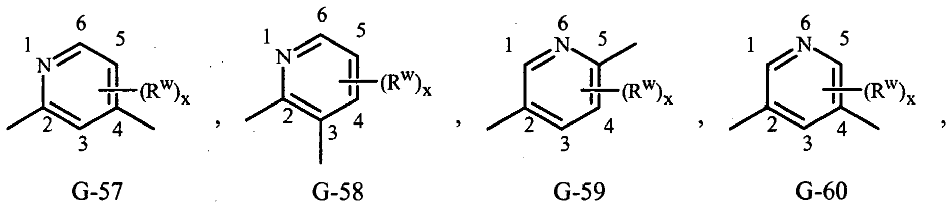 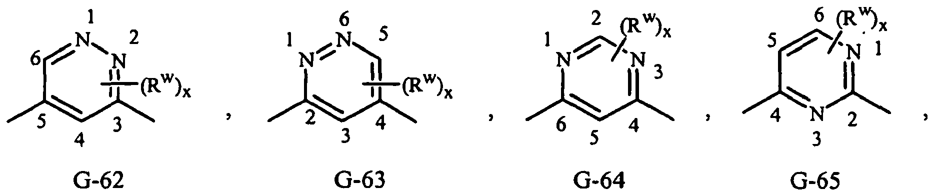 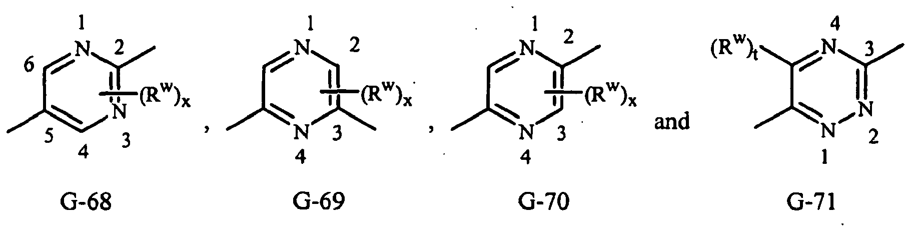 wherein the bond projecting to the left is bonded to the pyridazinone ring of Formula 1, and the bond projecting to the right is bonded to J; x is an integer selected from 0 through 2; and t is an integer selected from 0 or 1;
each R^{w} is independently halogen, hydroxy, C₁-C₆ alkyl, C₁-C₆ haloalkyl, C₃-C₈ cycloalkyl, C₁-C₆ alkoxy, C₁-C₆ haloalkoxy, -O(CR¹¹R¹²)ₙG^{A} or -(CR¹¹R¹²)ₙG^{A};
J is a phenyl ring optionally substituted with up to 3 substituents independently selected from R^{u}; each R^{u} is independently halogen, cyano, nitro, -CHO, -C(=O)OH, C₁-C₆ alkyl, C₁-C₆ haloalkyl, C₃-C₈ cycloalkyl, C₃-C₈ halocycloalkyl, C₁-C₆ alkoxy, C₁-C₆ haloalkoxy, C₁-C₆ alkylamino, C₂-C₈ dialkylamino or phenyl; and
W¹ is O.

4. A compound of Claim 3 wherein
R¹ is H or C₁-C₆ alkyl;
R² is H, Cl, CH₃, Et or OMe;
R³ is H, CO₂-*i*-Pr, or CO-*t*-Bu;
G is selected from G-12 through G-15, G-26 through G-29, G-34, G-35, G-54 and G-65;
each R^{w} is independently halogen, C₁-C₆ alkyl or -O(CR¹¹R¹²)ₙG^{A};
J is a phenyl substituted with a substituent selected from R^{u}; and
each R^{u} is independently halogen, C₁-C₆ alkyl or C₁-C₆ haloalkyl.

5. A compound of Claim 4 wherein
R¹ is CH₃;
R² is H;
G is selected from G-12, G-15, G-26, G-28, G-29, G-34, G-35, G-54 and G-65;
each R^{w} is independently CH₃ or Et;
J is a phenyl substituted at the para position with a substituent selected from R^{u}; and
each R^{u} is independently halogen, C₁-C₃ alkyl or C₁-C₃ haloalkyl.

6. A compound of Claim 5 wherein
G is G-26;
x is 1;
R^{w} is positioned at the 5-position of G-26; and
each R^{u} is independently Cl, Br or CF₃.

7. A compound of Claim 1 which is selected from the group consisting of
5-hydroxy-2-methyl-4-[5-methyl-3-[4-(trifluoromethyl)phenyl]-1*H*-pyrazol-1-yl]-3(2*H*)-pyridazinone,
4-[3-(4-chlorophenyl)-5-methyl-1*H*-pyrazol-1-yl]-5-hydroxy-2-methyl-3(2H)-pyridazinone,
5-[3-(4-chlorophenyl)-5-methyl-1*H*-pyrazol-1-yl]-1,6-dihydro-1-methyl-6-oxo-4-pyridazinyl-1-methylethyl carbonate,
-[3-(4-bromophenyl)-5-methyl-1*H*-pyrazol-1-yl]-5-hydroxy-3(2*H*)-pyridazinone,
4-[5-ethyl-3-[4-(trifluoromethyl)phenyl]-1*H*-pyrazol-1-yl]-5-hydroxy-2-methyl-3(2*H*)-pyridazinone and
4-[3-(4-chlorophenyl)-5-ethyl-4-methyl-1*H*-pyrazol-1-yl]-5-hydroxy-2-methyl-3(2*H*)-pyridazinone.

8. A herbicidal composition comprising a herbicidally effective amount of a compound of Claim 1 and at least one component selected from the group consisting of surfactants, solid diluents and liquid diluents.

9. A herbicidal composition comprising a herbicidally effective amount of a compound of Claim 1, an effective amount of at least one additional active ingredient selected from the group consisting of other herbicides and herbicide safeners, and at least one component selected from the group consisting of surfactants, solid diluents and liquid diluents.

10. A method for controlling the growth of undesired vegetation comprising contacting the vegetation or its environment with a herbicidally effective amount of a compound of Claim 1.

## Patentansprüche

1. Verbindung, ausgewählt aus Formel 1, *N*-Oxiden und Salzen davon, 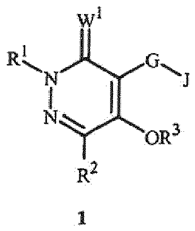 wobei
R¹ H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl, C₄-C₁₀-Cycloalkylalkyl, C₆-C₁₄-Cycloalkylcycloalkyl, C₄-C₁₀-Halocycloalkylalkyl, C₅-C₁₂-Alkylcycloalkylalkyl, C₃-C₈-Cycloalkenyl, C₃-C₈-Halocycloalkenyl, C₂-C₈-Alkoxyalkyl, C₄-C₁₀-Cycloalkoxyalkyl, C₃-C₁₀-Alkoxyalkoxyalkyl, C₂-C₈-Alkylthioalkyl, C₂-C₈-Alkylsulfinylalkyl, C₂-C₈-Alkylsulfonylalkyl, Tetrahydropyranyl, -C(=W⁶)R⁴, -C(=W²)W³R⁵, -S(=O)₂R⁶,-P(=W⁴)R⁷R⁸ oder -C(=W⁵)NR⁹R¹⁰ ist;
R² H, Halogen, Cyano, -C(=O)OH, -C(=O)NH₂, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl, C₄-C₁₀-Cycloalkylalkyl, C₆-C₁₄-Cycloalkylcycloalkyl, C₄-C₁₀-Halocycloalkylalkyl, C₅-C₁₂-Alkylcycloalkylalkyl, C₃-C₈-Cycloalkenyl, C₃-C₈-Halocycloalkenyl, C₂-C₈-Alkoxyalkyl, C₄-C₁₀-Cycloalkoxyalkyl, C₃-C₁₀-Alkoxyalkoxyalkyl, C₂-C₈-Alkylthioalkyl, C₂-C₈-Alkylsulfinylalkyl, C₂-C₈-Alkylsulfonylalkyl, C₂-C₈-Alkoxycarbonyl, C₄-C₁₀-Cycloalkoxycarbonyl, C₅-C₁₂-Cycloalkylalkoxycarbonyl, C₂-C₈-Alkylaminocarbonyl, C₃-C₁₀-Dialkylaminocarbonyl, C₄-C₁₀-Cycloalkylaminocarbonyl, C₁-C₆-Alkoxy, C₁-C₆-Haloalkoxy, C₁-C₆-Alkylthio, C₁-C₆-Haloalkylthio, Nitro, C₃-C₆-Cycloalkoxy oder C₄-C₈-Cycloalkylalkoxy ist;
R³ H, -C(=W⁶)R⁴, -C(=W²)W³R⁵, -S(=O)₂R⁶, -P(=W⁴)R⁷R⁸ oder -C(=W⁵)NR⁹R¹⁰ ist;
G ein Phenylring oder ein 5- oder 6-gliedriger heteroaromatischer Ring ist, jeder Ring wahlweise substituiert mit R^{x} an Stickstoff-Ringliedern und wahlweise substituiert mit bis zu 4 Substituenten, ausgewählt aus R^{w}, an Kohlenstoff-Ringliedern;
J ein Phenylring oder ein 5- oder 6-gliedriger heteroaromatischer Ring ist, jeder Ring wahlweise substituiert mit bis zu 5 Substituenten, unabhängig ausgewählt aus R^{u};
jedes R⁴ unabhängig C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl, C₄-C₁₀-Cycloalkylalkyl, C₅-C₁₂-Alkylcycloalkylalkyl, C₃-C₈-Cycloalkenyl, C₂-C₈-Alkoxyalkyl, C₄-C₁₀-Cycloalkoxyalkyl, C₃-C₁₀-Alkoxyalkoxyalkyl, C₂-C₈-Alkylthioalkyl, C₂-C₈-Alkylsulfinylalkyl, C₂-C₈-Alkylsulfonylalkyl, C₂-C₈-Alkylaminoalkyl, C₃-C₁₀-Dialkylaminoalkyl, C₂-C₈-Haloalkylaminoalkyl oder C₄-C₁₀-Cycloalkylaminoalkyl, Naphthalenyl oder - (CR¹¹R¹²)ₙG^{A} ist;
jedes R⁵ unabhängig C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl, C₄-C₁₀-Cycloalkylalkyl, C₅-C₁₂-Alkylcycloalkylalkyl, C₃-C₁₀-Cycloalkenyl, C₂-C₈-Alkoxyalkyl, C₄-C₁₀-Cycloalkoxyalkyl, C₃-C₁₀-Alkoxyalkoxyalkyl, C₂-C₈-Alkylthioalkyl, C₂-C₈-Alkylsulfinylalkyl, C₂-C₈-Alkylsulfonylalkyl, C₂-C₈-Alkylaminoalkyl, C₃-C₁₀-Dialkylaminoalkyl, C₂-C₈-Haloalkylaminoalkyl, C₄-C₁₀-Cycloalkylaminoalkyl, Naphthalenyl oder -(CR¹¹R¹¹)ₙG^{A} ist;
jedes R⁶ unabhängig C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl, C₄-C₁₀-Cycloalkylalkyl, C₄-C₁₀-Halocycloalkylalkyl, C₅-C₁₂-Alkylcycloalkylalkyl, C₃-C₈-Cycloalkenyl, C₂-C₈-Alkoxyalkyl, C₄-C₁₀-Cycloalkoxyalkyl, C₃-C₁₀-Alkoxyalkoxyalkyl, C₂-C₈-Alkylthioalkyl, C₂-C₈-Alkylsulfinylalkyl, C₂-C₈-Alkylsulfonylalkyl, C₂-C₈-Alkylaminoalkyl, C₃-C₁₀-Dialkylaminoalkyl, C₂-C₈-Haloalkylaminoalkyl, C₄-C₁₀-Cycloalkylaminoalkyl, C₁-C₆-Alkylamino, C₂-C₈-Dialkylamino, C₂-C₆-Haloalkylamino, C₂-C₈-Halodialkylamino, C₃-C₈-Cycloalkylamino, C₂-C₈-Alkylcarbonylamino, C₂-C₈-Haloalkylcarbonylamino, C₁-C₆-Alkylsulfonylamino, C₁-C₆-Haloalkylsulfonylamino, Naphthalenyl oder -(CR¹¹R¹²)ₙG^{A} ist;
jedes R⁷ und R⁸ unabhängig C₁-C₆-Alkyl, C₁-C₆-Alkoxy, C₁-C₆-Alkylamino, C₂-C₈-Dialkylamino, C₂-C₆-Haloalkylamino, C₂-C₈-Halodialkylamino, C₃-C₈-Cycloalkylamino, Naphthalenyl oder -(CR¹¹R¹²)ₙG^{A} ist;
jedes R⁹ unabhängig H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl, C₄-C₁₀-Cycloalkylalkyl, C₅-C₁₂-Alkylcycloalkylalkyl, C₃-C₈-Cycloalkenyl, C₂-C₈-Alkoxyalkyl, C₄-C₁₀-Cycloalkoxyalkyl, C₃-C₁₀-Alkoxyalkoxyalkyl, C₂-C₈-Alkylthioalkyl, C₂-C₈-Alkylsulfinylalkyl, C₂-C₈-Alkylsulfonylalkyl, C₂-C₈-Alkylaminoalkyl, C₄-C₁₀-Dialkylaminoalkyl, Naphthalenyl oder -(CR¹¹R¹²)ₙG^{A} ist;
jedes R¹⁰ unabhängig H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl oder C₄-C₁₀-Cycloalkylalkyl ist; oder
R⁹ und R¹⁰ zusammengenommen werden mit dem Stickstoff, an das sie angefügt sind, um einen 3- bis 7-gliedrigen heterocyclischen Ring zu bilden, enthaltend, zusätzlich zu dem verbindenden Stickstoff, Ringglieder, ausgewählt aus Kohlenstoff und wahlweise O, S und NR¹³, die Kohlenstoff-Ringglieder wahlweise in der Form C(=O) und der Ring wahlweise an Kohlenstoff-Ringliedem mit bis zu 4 Substituenten substituiert, unabhängig ausgewählt aus der Gruppe, bestehend aus Halogen, -CN, C₁-C₃-Alkyl und C₁-C₃-Alkoxy;
jedes R¹¹ und R¹² unabhängig H oder C₁-C₃-Alkyl ist;
jedes R¹³ unabhängig H oder C₁-C₃-Alkyl ist;
jedes G^{A} unabhängig ein Phenylring oder ein 5- oder 6-gliedriger heteroaromatischer Ring ist, jeder Ring wahlweise substituiert mit bis zu 5 Substituenten, unabhängig ausgewählt aus R^{u}; oder ein Naphthalenyl-Ringsystem, wahlweise substituiert mit bis zu 5 Substituenten, unabhängig ausgewählt aus R^{u};
jedes R^{u} unabhängig Halogen, Cyano, Hydroxy, Amino, Nitro, -CHO, -C(=O)OH, -C(=O)NH₂, -SO₂NH₂, SF₅, C₁-C₆-Alkyl, C₂-C₆-Alkenyl, C₂-C₆-Alkynyl, C₁-C₆-Haloalkyl, C₃-C₈-Cycloalkyl, C₃-C₈-Halocycloalkyl, C₄-C₁₀-Alkylcycloalkyl, C₄-C₁₀-Cycloalkylalkyl, C₄-C₁₀-Halocycloalkylalkyl, C₅-C₁₂-Alkylcycloalkylalkyl, C₂-C₈-Alkylcarbonyl, C₂-C₈-Haloalkylcarbonyl, C₂-C₈-Alkoxycarbonyl, C₄-C₁₀-Cycloalkoxycarbonyl, C₅-C₁₂-Cycloalkylalkoxycarbonyl, C₂-C₈-Alkylaminocarbonyl, C₃-C₁₀-Dialkylaminocarbonyl, C₁-C₆-Alkoxy, C₁-C₆-Haloalkoxy, C₂-C₈-Alkylcarbonyloxy, C₁-C₆-Alkylthio, C₁-C₆-Haloalkylthio, C₁-C₆-Alkylsulfinyl, C₁-C₆-Haloalkylsulfinyl, C₁-C₆-Alkylsulfonyl, C₁-C₆-Haloalkylsulfonyl, C₁-C₆-Alkylaminosulfonyl, C₂-C₈-Dialkylaminosulfonyl, C₃-C₁₀-Trialkylsilyl, C₁-C₆-Alkylamino, C₂-C₈-Dialkylamino, C₂-C₈-Alkylcarbonylamino, C₁-C₆-Alkylsulfonylamino, Phenyl, Pyridinyl oder Thienyl ist;
jedes R^{w} unabhängig Halogen, Cyano, Hydroxy, Amino, Nitro, -CHO, - C(=O)OH, -C(=O)NH₂, -SO₂NH₂, SF₅, C₁-C₆-Alkyl, C₂-C₆-Alkenyl, C₂-C₆-Alkynyl, C₁-C₆-Haloalkyl, C₃-C₈-Cycloalkyl, C₃-C₈-Halocycloalkyl, C₄-C₁₀-Alkylcycloalkyl, C₄-C₁₀-Cycloalkylalkyl, C₄-C₁₀-Halocycloalkylalkyl, C₅-C₁₂-Alkylcycloalkylalkyl, C₂-C₈-Alkylcarbonyl, C₂-C₈-Haloalkylcarbonyl, C₂-C₈-Alkoxycarbonyl, C₄-C₁₀-Cycloalkoxycarbonyl, C₅-C₁₂-Cycloalkylalkoxycarbonyl, C₂-C₈-Alkylaminocarbonyl, C₃-C₁₀-Dialkylaminocarbonyl, C₁-C₆-Alkoxy, C₁-C₆-Haloalkoxy, C₂-C₈-Alkylcarbonyloxy, C₁-C₆-Alkylthio, C₁-C₆-Haloalkylthio, C₁-C₆-Alkylsulfinyl, C₁-C₆-Haloalkylsulfinyl, C₁-C₆-Alkylsulfonyl, C₁-C₆-Haloalkylsulfonyl, C₁-C₆-Alkylaminosulfonyl, C₂-C₈-Dialkylaminosulfonyl, C₃-C₁₀-Trialkylsilyl, C₁-C₆-Alkylamino, C₂-C₈-Dialkylamino, C₂-C₈-Alkylcarbonylamino, C₁-C₆-Alkylsulfonylamino, Naphthalenyl, -O(CR¹¹R¹²)ₙG^{A} oder -(CR¹¹R¹²)ₙG^{A} ist;
jedes R^{x} unabhängig H, C₁-C₃-Alkyl oder C₃-C₇-Cycloalkyl ist;
jedes W¹, W², W³, W⁴, W⁵ und W⁶ unabhängig O oder S ist; und
jedes n unabhängig eine ganze Zahl ist, ausgewählt aus 0 bis 3.

2. Verbindung nach Anspruch 1, wobei
R¹ H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₃-C₈-Cycloalkyl, C₃-C₈-Halocycloalkyl, C₄-C₁₀-Alkylcycloalkyl, C₄-C₁₀-Cycloalkylalkyl, C₆-C₁₄-Cycloalkylcycloalkyl, C₄-C₁₀-Halocycloalkylalkyl, C₅-C₁₂-Alkylcycloalkylalkyl, C₂-C₈-Alkoxyalkyl, C₄-C₁₀-Cycloalkoxyalkyl oder C₃-C₁₀-Alkoxyalkoxyalkyl ist;
R² H, Halogen, C₁-C₆-Alkyl, C₁-C₆-Haloalkyl, C₃-C₈-Cycloalkyl, C₂-C₈-Alkoxyalkyl oder C₁-C₄-Alkoxy ist;
R³ H, -C(=W⁶)R⁴, -C(=W²)W³R⁵, -S(=O)₂R⁶ oder -C(=W⁵)NR⁹R¹⁰ ist;
G ein Phenylring oder ein 5- oder 6-gliedriger heteroaromatischer Ring ist, jeder Ring wahlweise substituiert mit R^{x} an Stickstoff-Ringliedern und wahlweise substituiert mit bis zu 2 Substituenten, ausgewählt aus R^{w}, an Kohlenstoff-Ringliedern;
jedes R^{w} unabhängig Halogen, Cyano, Hydroxy, Amino, Nitro, -CHO, - C(=O)OH, -C(=O)NH₂, -SO₂NH₂, SF₅, C₁-C₆-Alkyl, C₂-C₆-Alkenyl, C₂-C₆-Alkynyl, C₁-C₆-Haloalkyl, C₃-C₈-Cycloalkyl, C₃-C₈-Halocycloalkyl, C₄-C₁₀-Alkylcycloalkyl, C₄-C₁₀-Cycloalkylalkyl, C₂-C₈-Alkylcarbonyl, C₂-C₈-Haloalkylcarbonyl, C₂-C₈-Alkoxycarbonyl, C₂-C₈-Alkylaminocarbonyl, C₃-C₁₀-Dialkylaminocarbonyl, C₁-C₆-Alkoxy, C₁-C₆-Haloalkoxy, C₂-C₈-Alkylcarbonyloxy, C₁-C₆-Alkylthio, C₁-C₆-Haloalkylthio, C₁-C₆-Alkylsulfonyl, C₁-C₆-Haloalkylsulfonyl, C₁-C₆-Alkylamino, C₂-C₈-Dialkylamino, - O(CR¹¹R¹²)ₙG^{A} oder -(CR¹¹R¹²)ₙG^{A} ist;
jedes R^{x} unabhängig H oder C₁-C₃-Alkyl ist;
J ein Phenyl oder ein 5- oder 6-gliedriger heteroaromatischer Ring ist, jeder Ring substituiert mit bis zu 3 Substituenten, unabhängig ausgewählt aus R^{u}; und
jedes R^{u} unabhängig Halogen, Cyano, Hydroxy, Amino, Nitro, -CHO, -C(=O)OH, -C(=O)NH₂, -SO₂NH₂, SF₅, C₁-C₆-Alkyl, C₂-C₆-Alkenyl, C₂-C₆-Alkynyl, C₁-C₆-Haloalkyl, C₃-C₈-Cycloalkyl, C₃-C₈-Halocycloalkyl, C₂-C₈-Alkylcarbonyl, C₂-C₈-Haloalkylcarbonyl, C₂-C₈-Alkoxycarbonyl, C₂-C₈-Alkylaminocarbonyl, C₃-C₁₀-Dialkylaminocarbonyl, C₁-C₆-Alkoxy, C₁-C₆-Haloalkoxy, C₂-C₈-Alkylcarbonyloxy, C₁-C₆-Alkylthio, C₁-C₆-Haloalkylthio, C₁-C₆-Alkylsulfonyl, C₁-C₆-Haloalkylsulfonyl, C₁-C₆-Alkylaminosulfonyl, C₂-C₈-Dialkylaminosulfonyl, C₁-C₆-Alkylamino, C₂-C₈-Dialkylamino, C₂-C₈-Alkylcarbonylamino, C₁-C₆-Alkylsulfonylamino, Phenyl, Pyridinyl oder Thienyl ist.

3. Verbindung nach Anspruch 2, wobei
R¹ H, C₁-C₆-Alkyl, C₁-C₆-Haloalkyl oder C₃-C₈-Cycloalkyl ist;
R² H, Halogen, C₁-C₆-Alkyl oder C₁-C₄-Alkoxy ist;
G ausgewählt ist aus 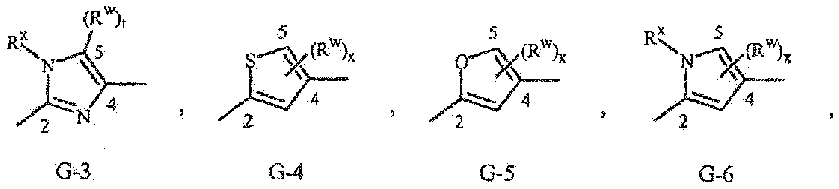 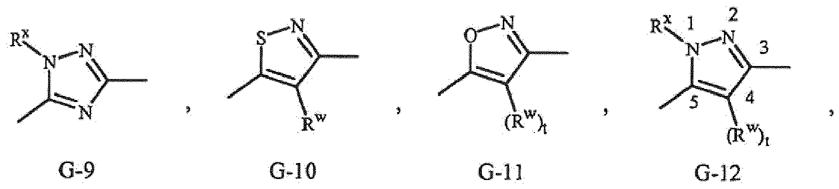 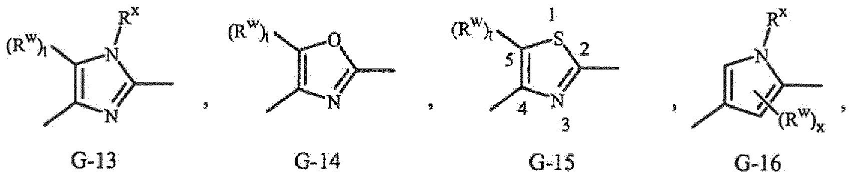 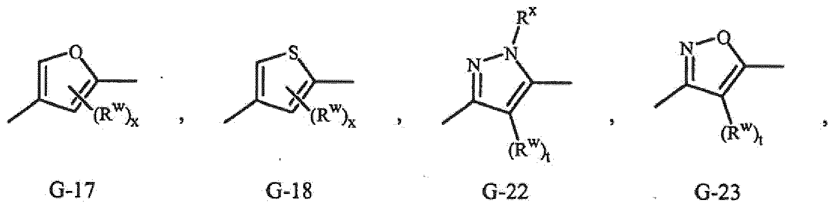 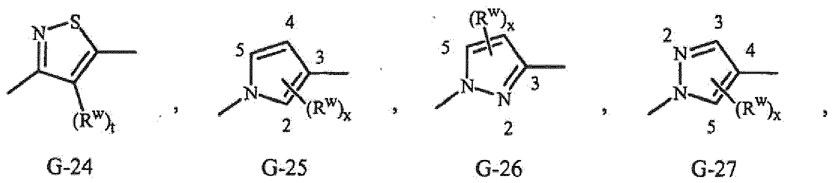 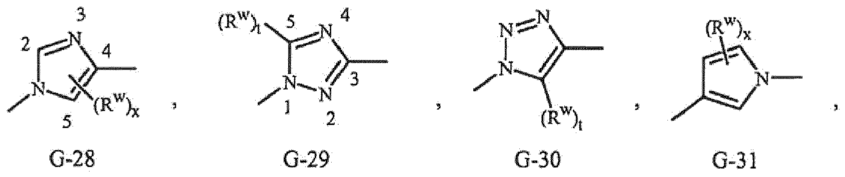 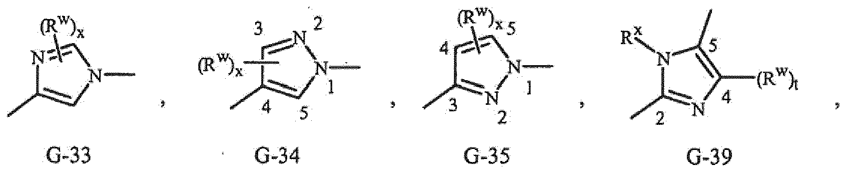 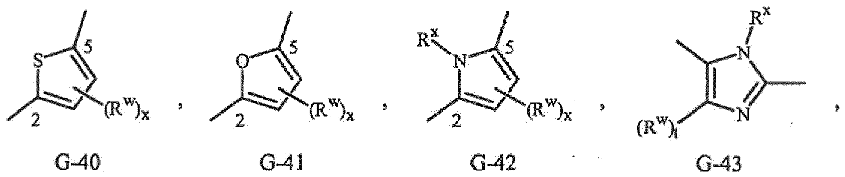 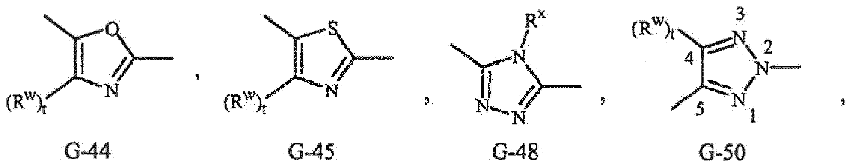 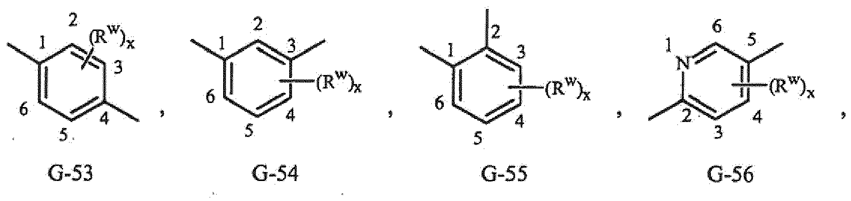 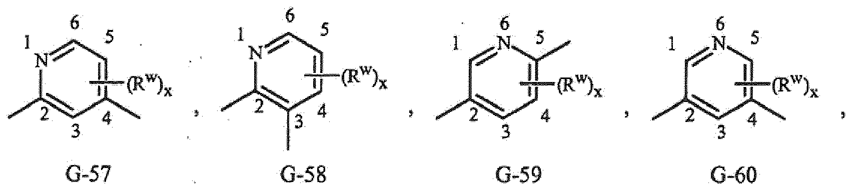 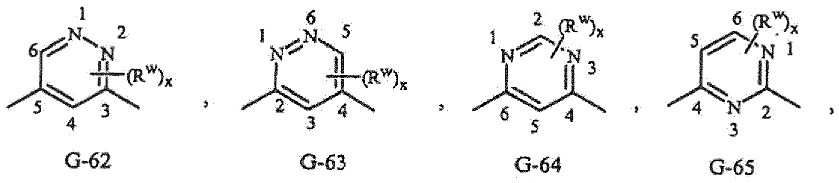 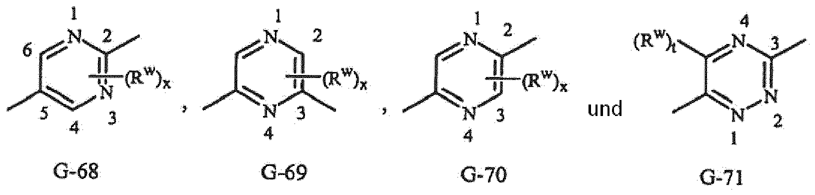 wobei die Bindung, die nach links herausragt, an den Pyridazinonring von Formel 1 gebunden ist, und die Bindung, die nach rechts herausragt, an J gebunden ist; x eine ganze Zahl ist, ausgewählt aus 0 bis 2; und t eine ganze Zahl ist, ausgewählt aus 0 oder 1;
jedes R^{w} unabhängig Halogen, Hydroxy, C₁-C₆-Alkyl, C₁-C₆-Haloalkyl, C₃-C₈-Cycloalkyl, C₁-C₆-Alkoxy, C₁-C₆-Haloalkoxy, -O(CR¹¹R¹²)ₙG^{A} oder -(CR¹¹R¹²)ₙG^{A} ist;
J ein Phenylring ist, wahlweise substituiert mit bis zu 3 Substituenten, unabhängig ausgewählt aus R^{u};
jedes R^{u} unabhängig Halogen, Cyano, Nitro, -CHO, -C(=O)OH, C₁-C₆-Alkyl, C₁-C₆-Haloalkyl, C₃-C₈-Cycloalkyl, C₃-C₈-Halocycloalkyl, C₁-C₆-Alkoxy, C₁-C₆-Haloalkoxy, C₁-C₆-Alkylamino, C₂-C₈-Dialkylamino oder Phenyl ist; und
W¹ O ist.

4. Verbindung nach Anspruch 3, wobei
R¹ H oder C₁-C₆-Alkyl ist;
R² H, Cl, CH₃, Et oder OMe ist;
R³ H, CO₂-*i*-Pr oder CO-*t*-Bu ist;
G aus G-12 bis G-15, G-26 bis G-29, G-34, G-35, G-54 und G-65 ausgewählt ist; jedes R^{w} unabhängig Halogen, C₁-C₆-Alkyl oder -O(CR¹¹R¹²)ₙG^{A} ist;
J ein Phenyl ist, substituiert mit einem Substituenten, ausgewählt aus R^{u}; und jedes R^{u} unabhängig Halogen, C₁-C₆-Alkyl oder C₁-C₆-Haloalkyl ist.

5. Verbindung nach Anspruch 4, wobei
R¹ CH₃ ist;
R² H ist;
G aus G-12, G-15, G-26, G-28, G-29, G-34, G-35, G-54 und G-65 ausgewählt ist; jedes R^{w} unabhängig CH₃ oder Et ist;
J ein Phenyl ist, substituiert an der para-Stellung mit einem Substituenten, ausgewählt aus R^{u}; und
jedes R^{u} unabhängig Halogen, C₁-C₃-Alkyl oder C₁-C₃-Haloalkyl ist.

6. Verbindung nach Anspruch 5, wobei
G G-26 ist;
x 1 ist;
R^{w} an der 5-Stellung von G-26 positioniert ist; und
jedes R^{u} unabhängig Cl, Br oder CF₃ ist.

7. Verbindung nach Anspruch 1, die aus der Gruppe ausgewählt ist, bestehend aus
5-Hydroxy-2-methyl-4-[5-methyl-3-[4-(trifluormethyl)phenyl]-1*H*-pyrazol-1-yl]-3(2*H*)-pyridazinon,
4-[3-(4-Chlorphenyl)-5-methyl-1*H*-pyrazol-1-yl]-5-hydroxy-2-methyl-3(2*H*)-pyridazinon,
5-[3-(4-Chlorphenyl)-5-methyl-1*H*-pyrazol-1-yl]-1,6-dihydro-1-methyl-6-oxo-4-pyridazinyl-1-methylethylcarbonat,
4-[3-(4-Bromphenyl)-5-methyl-1*H*-pyrazol-1-yl]-5-hydroxy-3(2*H*)-pyridazinon,
4-[5-Ethyl-3-[4-(trifluormethyl)phenyl]-1*H*-pyrazol-1-yl]-5-hydroxy-2-methyl-3(2*H*)-pyridazinon und
4-[3-(4-Chlorphenyl)-5-ethyl-4-methyl-1*H*-pyrazol-1-yl]-5-hydroxy-2-methyl-3(2*H*)-pyridazinon.

8. Herbizide Zusammensetzung, umfassend eine herbizid wirksame Menge einer Verbindung nach Anspruch 1 und mindestens eine Komponente, ausgewählt aus der Gruppe, bestehend aus Tensiden, feststofflichen Verdünnungsmitteln und flüssigen Verdünnungsmitteln.

9. Herbizide Zusammensetzung, umfassend eine herbizid wirksame Menge einer Verbindung nach Anspruch 1, eine wirksame Menge mindestens eines weiteren Wirkstoffs, ausgewählt aus der Gruppe, bestehend aus anderen Herbiziden und herbiziden Safenem und mindestens einer Komponente, ausgewählt aus der Gruppe, bestehend aus Tensiden, feststofflichen Verdünnungsmitteln und flüssigen Verdünnungsmitteln.

10. Verfahren zum Kontrollieren des Wachstums unerwünschter Vegetation, umfassend, die Vegetation oder ihre Umgebung mit einer herbizid wirksamen Menge einer Verbindung nach Anspruch 1 in Kontakt zu bringen.

## Revendications

1. Composé choisi parmi la Formule I, des N-oxydes et des sels de celui-ci: 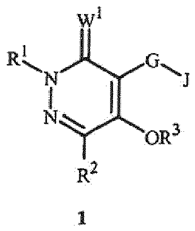 dans lequel:
R¹ est H, un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀, un cycloalkylalkyle C₄-C₁₀, un cycloalkylcycloalkyle C₆-C₁₄, un halocycloalkylalkyle C₄-C₁₀, un alkylcycloalkylalkyle C₅-C₁₂, un cycloalcényle C₃-C₈, un halocycloalcényle C₃-C₈, un alcoxyalkyle C₂-C₈, un cycloalcoxyalkyle C₄-C₁₀, un alcoxyalcoxyalkyle C₃-C₁₀, un alkylthioalkyle C₂-C₈, un alkylsulfinylalkyle C₂-C₈, un alkylsulfonylalkyle C₂-C₈, un tétrahydropyranyle, - C(=W⁶)R⁴, -C(=W²)W³R⁵, -S(=O)₂R⁶, -P(=W⁴)R⁷R⁸ ou -C(=W⁵)NR⁹R¹⁰;
R² est H, un halogène, un cyano, -C(=O)OH, -C(=O)NH₂, un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀, un cycloalkylalkyle C₄-C₁₀, un cycloalkylcycloalkyle C₆-C₁₄, un halocycloalkylalkyle C₄-C₁₀, un alkylcycloalkylalkyle C₅-C₁₂, un cycloalcényle C₃-C₈, un halocycloalcényle C₃-C₈, un alcoxyalkyle C₂-C₈, un cycloalcoxyalkyle C₄-C₁₀, un alcoxyalcoxyalkyle C₃-C₁₀, un alkylthioalkyle C₂-C₈, un alkylsulfinylalkyle C₂-C₈, un alkylsulfonylalkyle C₂-C₈, un alcoxycarbonyle C₂-C₈, un cycloalcoxycarbonyle C₄-C₁₀, un cycloalkylalcoxycarbonyle C₅-C₁₂, un alkylaminocarbonyle C₂-C₈, un dialkylaminocarbonyle C₃-C₁₀, un cycloalkylaminocarbonyle C₄-C₁₀, un alcoxy C₁-C₆, un haloalcoxy C₁-C₆, un alkylthio C₁-C₆, un haloalkylthio C₁-C₆, un nitro, un cycloalcoxy C₃-C₆ ou un cycloalkylalcoxy C₄-C₈;
R³ est H, -C(=W⁶)R⁴, -C(=W²)W³R⁵, -S(=O)₂R⁶, -P(=W⁴)R⁷R⁸ ou - C(=W⁵)NR⁹R¹⁰;
G est un cycle de phényle ou un cycle hétéroaromatique de 5 ou 6 membres, chaque cycle étant optionnellement substitué avec R^{x} sur des membres de cycle d'azote et optionnellement substitué avec jusqu'à 4 substituants choisis parmi R^{w} sur des membres de cycle de carbone;
J est un cycle de phényle ou un cycle hétéroaromatique de 5 ou 6 membres, chaque cycle étant optionnellement substitué avec jusqu'à 5 substituants indépendamment choisis parmi R^{u};
chaque R⁴ est indépendamment un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀, un cycloalkylalkyle C₄-C₁₀, un alkylcycloalkylalkyle C₅-C₁₂, un cycloalcényle C₃-C₈, un alcoxyalkyle C₂-C₈, un cycloalcoxyalkyle C₄-C₁₀, un alcoxyalcoxyalkyle C₃-C₁₀, un alkylthioalkyle C₂-C₈, un alkylsulfinylalkyle C₂-C₈, un alkylsulfonylalkyle C₂-C₈, un alkylaminoalkyle C₂-C₈, un dialkylaminoalkyle C₃-C₁₀, un haloalkylaminoalkyle C₂-C₈ ou un cycloalkylaminoalkyle C₄-C₁₀, un naphtalényle ou -C(R¹¹R¹²)ₙG^{A};
chaque R⁵ est indépendamment un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀, un cycloalkylalkyle C₄-C₁₀, un alkylcycloalkylalkyle C₅-C₁₂, un cycloalcényle C₃-C₁₀, un alcoxyalkyle C₂-C₈, un cycloalcoxyalkyle C₄-C₁₀, un alcoxyalcoxyalkyle C₃-C₁₀, un alkylthioalkyle C₂-C₈, un alkylsulfinylalkyle C₂-C₈, un alkylsulfonylalkyle C₂-C₈, un alkylaminoalkyle C₂-C₈, un dialkylaminoalkyle C₃-C₁₀, un haloalkylaminoalkyle C₂-C₈, un cycloalkylaminoalkyle C₄-C₁₀, un naphtalényle ou -C(R¹¹R¹²)ₙG^{A};
chaque R⁶ est indépendamment un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀, un cycloalkylalkyle C₄-C₁₀, un halocycloalkylalkyle C₄-C₁₀, un alkylcycloalkylalkyle C₅-C₁₂, un cycloalcényle C₃-C₈, un alcoxyalkyle C₂-C₈, un cycloalcoxyalkyle C₄-C₁₀, un alcoxyalcoxyalkyle C₃-C₁₀, un alkylthioalkyle C₂-C₈, un alkylsulfinylalkyle C₂-C₈, un alkylsulfonylalkyle C₂-C₈, un alkylaminoalkyle C₂-C₈, un dialkylaminoalkyle C₃-C₁₀, un haloalkylaminoalkyle C₂-C₈, un cycloalkylaminoalkyle C₄-C₁₀, un alkylamino C₁-C₆, un dialkylamino C₂-C₈, un haloalkylamino C₂-C₆, un halodialkylamino C₂-C₈, un cycloalkylamino C₃-C₈, un alkylcarbonylamino C₂-C₈, un haloalkylcarbonylamino C₂-C₈, un alkylsulfonylamino C₁-C₆, un haloalkylsulfonylamino C₁-C₆, un naphtalényle ou -C(R¹¹R¹²)ₙG^{A};
chaque R⁷ et R⁸ est indépendamment un alkyle C₁-C₆, un alcoxy C₁-C₆, un alkylamino C₁-C₆, un dialkylamino C₂-C₈, un haloalkylamino C₂-C₆, un halodialkylamino C₂-C₈, un cycloalkylamino C₃-C₈, un naphtalényle ou -C(R¹¹R¹²)ₙG^{A};
chaque R⁹ est indépendamment H, un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀, un cycloalkylalkyle C₄-C₁₀, un alkylcycloalkylalkyle C₅-C₁₂, un cycloalcényle C₃-C₈, un alcoxyalkyle C₂-C₈, un cycloalcoxyalkyle C₄-C₁₀, un alcoxyalcoxyalkyle C₃-C₁₀, un alkylthioalkyle C₂-C₈, un alkylsulfinylalkyle C₂-C₈, un alkylsulfonylalkyle C₂-C₈, un alkylaminoalkyle C₂-C₈, un dialkylaminoalkyle C₄-C₁₀, un naphtalényle ou -C(R¹¹R¹²)ₙG^{A};
chaque R¹⁰ est indépendamment H, un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀ ou un cycloalkylalkyle C₄-C₁₀; ou
R⁹ et R¹⁰ sont pris ensemble avec l'azote auquel ils sont attachés pour former un cycle hétérocyclique de 3 à 7 membres contenant, en plus de l'azote de liaison, des membres de cycle choisis parmi le carbone et optionnellement O, S et NR¹³, les membres de cycle de carbone étant optionnellement sous la forme de C(=O) et le cycle étant optionnellement substitué sur les membres de cycle de carbone avec jusqu'à 4 substituants indépendamment choisis dans le groupe constitué de: un halogène, -CN, un alkyle C₁-C₃ et un alcoxy C₁-C₃;
chaque R¹¹ et R¹² est indépendamment H ou un alkyle C₁-C₃;
chaque R¹³ est indépendamment H ou un alkyle C₁-C₃;
chaque G^{A} est indépendamment un cycle de phényle ou un cycle hétéroaromatique de 5 ou 6 membres, chaque cycle étant optionnellement substitué avec jusqu'à 5 substituants indépendamment choisis parmi R^{u}; ou un système cyclique de naphtalényle optionnellement substitué avec jusqu'à 5 substituants indépendamment choisis parmi R^{u};
chaque R^{u} est indépendamment un halogène, un cyano, un hydroxy, un amino, un nitro, -CHO, -C(=O)OH, -C(=O)NH₂, -SO₂NH₂, SF₅, un alkyle C₁-C₆, un alcényle C₂-C₆, un alcynyle C₂-C₆, un haloalkyle C₁-C₆, un cycloalkyle C₃-C₈, un halocycloalkyle C₃-C₈, un alkylcycloalkyle C₄-C₁₀, un cycloalkylalkyle C₄-C₁₀, un halocycloalkylalkyle C₄-C₁₀, un alkylcycloalkylalkyle C₅-C₁₂, un alkylcarbonyle C₂-C₈, un haloalkylcarbonyle C₂-C₈, un alcoxycarbonyle C₂-C₈, un cycloalcoxycarbonyle C₄-C₁₀, un cycloalkylalcoxycarbonyle C₅-C₁₂, un alkylaminocarbonyle C₂-C₈, un dialkylaminocarbonyle C₃-C₁₀, un alcoxy C₁-C₆, un haloalcoxy C₁-C₆, un alkylcarbonyloxy C₂-C₈, un alkylthio C₁-C₆, un haloalkylthio C₁-C₆, un alkylsulfinyle C₁-C₆, un haloalkylsulfinyle C₁-C₆, un alkylsulfonyle C₁-C₆, un haloalkylsulfonyle C₁-C₆, un alkylaminosulfonyle C₁-C₆, un dialkylaminosulfonyle C₂-C₈, un trialkylsilyle C₃-C₁₀, un alkylamino C₁-C₆, un dialkylamino C₂-C₈, un alkylcarbonylamino C₂-C₈, un alkylsulfonylamino C₁-C₆, un phényle, un pyridinyle ou un thiényle;
chaque R^{w} est indépendamment un halogène, un cyano, un hydroxy, un amino, un nitro, -CHO, -C(=O)OH, -C(=O)NH₂, -SO₂NH₂, SF₅, un alkyle C₁-C₆, un alcényle C₂-C₆, un alcynyle C₂-C₆, un haloalkyle C₁-C₆, un cycloalkyle C₃-C₈, un halocycloalkyle C₃-C₈, un alkylcycloalkyle C₄-C₁₀, un cycloalkylalkyle C₄-C₁₀, un halocycloalkylalkyle C₄-C₁₀, un alkylcycloalkylalkyle C₅-C₁₂, un alkylcarbonyle C₂-C₈, un haloalkylcarbonyle C₂-C₈, un alcoxycarbonyle C₂-C₈, un cycloalcoxycarbonyle C₄-C₁₀, un cycloalkylalcoxycarbonyle C₅-C₁₂, un alkylaminocarbonyle C₂-C₈, un dialkylaminocarbonyle C₃-C₁₀, un alcoxy C₁-C₆, un haloalcoxy C₁-C₆, un alkylcarbonyloxy C₂-C₈, un alkylthio C₁-C₆, un haloalkylthio C₁-C₆, un alkylsulfinyle C₁-C₆, un haloalkylsulfinyle C₁-C₆, un alkylsulfonyle C₁-C₆, un haloalkylsulfonyle C₁-C₆, un alkylaminosulfonyle C₁-C₆, un dialkylaminosulfonyle C₂-C₈, un trialkylsilyle C₃-C₁₀, un alkylamino C₁-C₆, un dialkylamino C₂-C₈, un alkylcarbonylamino C₂-C₈, un alkylsulfonylamino C₁-C₆, un naphtalényle, -O(CR¹¹R¹²)ₙG^{A} ou -(CR¹¹R¹²)ₙG^{A};
chaque R^{X} est indépendamment H, un alkyle C₁-C₃ ou un cycloalkyle C₃-C₇;
chaque W¹ W², W³, W⁴, W⁵ et W⁶ est indépendamment O ou S; et
chaque n est indépendamment un nombre entier choisi parmi 0 à 3.

2. Composé selon la revendication 1, dans lequel:
R¹ est H, un alkyle C₁-C₆, un alcényle C₂-C₆, un alcynyle C₂-C₆, un haloalkyle C₁-C₆, un haloalcényle C₂-C₆, un haloalcynyle C₂-C₆, un cycloalkyle C₃-C₈, un halocycloalkyle C₃-C₈, un alkylcycloalkyle C₄-C₁₀, un cycloalkylalkyle C₄-C₁₀, un cycloalkylcycloalkyle C₆-C₁₄, un halocycloalkylalkyle C₄-C₁₀, un alkylcycloalkylalkyle C₅-C₁₂, un alcoxyalkyle C₂-C₈, un cycloalcoxyalkyle C₄-C₁₀ ou un alcoxyalcoxyalkyle C₃-C₁₀;
R² est H, un halogène, un alkyle C₁-C₆, un haloalkyle C₁-C₆, un cycloalkyle C₃-C₈, un alcoxyalkyle C₂-C₈ ou un alcoxy C₁-C₄;
R³ est H, -C(=W⁶)R⁴, -C(=W²)W³R⁵, -S(=O)₂R⁶ ou -C(=W⁵)NR⁹R¹⁰;
G est un cycle de phényle ou un cycle hétéroaromatique de 5 ou 6 membres, chaque cycle étant optionnellement substitué avec R^{X} sur des membres de cycle d'azote et optionnellement substitué avec jusqu'à 2 substituants choisis parmi R^{W} sur des membres de cycle de carbone;
chaque R^{w} est indépendamment un halogène, un cyano, un hydroxy, un amino, un nitro, -CHO, -C(=O)OH, -C(=O)NH₂, -SO₂NH₂, SF₅, un alkyle C₁-C₆, un alcényle C₂-C₆, un alcynyle C₂-C₆, un haloalkyle C₁-C₆, un cycloalkyle C₃-C₈, un halocycloalkyle C₃-C₈, un alkylcycloalkyle C₄-C₁₀, un cycloalkylalkyle C₄-C₁₀, un alkylcarbonyle C₂-C₈, un haloalkylcarbonyle C₂-C₈, un alcoxycarbonyle C₂-C₈, un alkylaminocarbonyle C₂-C₈, un dialkylaminocarbonyle C₃-C₁₀, un alcoxy C₁-C₆, un haloalcoxy C₁-C₆, un alkylcarbonyloxy C₂-C₈, un alkylthio C₁-C₆, un haloalkylthio C₁-C₆, un alkylsulfonyle C₁-C₆, un haloalkylsulfonyle C₁-C₆, un alkylamino C₁-C₆, un dialkylamino C₂-C₈, - O(CR¹¹R¹²)ₙG^{A} ou -(CR¹¹R¹²)ₙG^{A};
chaque R^{X} est indépendamment H ou un alkyle C₁-C₃;
J est un cycle de phényle ou un cycle hétéroaromatique de 5 ou 6 membres, chaque cycle étant substitué avec jusqu'à 3 substituants indépendamment choisis parmi R^{u}; et
chaque R^{u} est indépendamment un halogène, un cyano, un hydroxy, un amino, un nitro, -CHO, -C(=O)OH, -C(=O)NH₂, -SO₂NH₂, SF₅, un alkyle C₁-C₆, un alcényle C₂-C₆, un alcynyle C₂-C₆, un haloalkyle C₁-C₆, un cycloalkyle C₃-C₈, un halocycloalkyle C₃-C₈, un alkylcarbonyle C₂-C₈, un haloalkylcarbonyle C₂-C₈, un alcoxycarbonyle C₂-C₈, un alkylaminocarbonyle C₂-C₈, un dialkylaminocarbonyle C₃-C₁₀, un alcoxy C₁-C₆, un haloalcoxy C₁-C₆, un alkylcarbonyloxy C₂-C₈, un alkylthio C₁-C₆, un haloalkylthio C₁-C₆, un alkylsulfonyle C₁-C₆, un haloalkylsulfonyle C₁-C₆, un alkylaminosulfonyle C₁-C₆, un dialkylaminosulfonyle C₂-C₈, un alkylamino C₁-C₆, un dialkylamino C₂-C₈, un alkylcarbonylamino C₂-C₈, un alkylsulfonylamino C₁-C₆, un phényle, un pyridinyle ou un thiényle.

3. Composé selon la revendication 2, dans lequel:
R¹ est H, un alkyle C₁-C₆, un haloalkyle C₁-C₆ ou un cycloalkyle C₃-C₈;
R² est H, un halogène, un alkyle C₁-C₆ ou un alcoxy C₁-C₄;
G est choisi parmi: 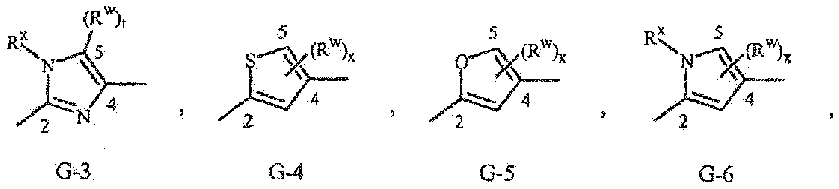 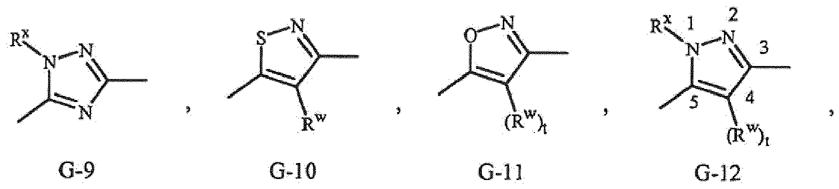 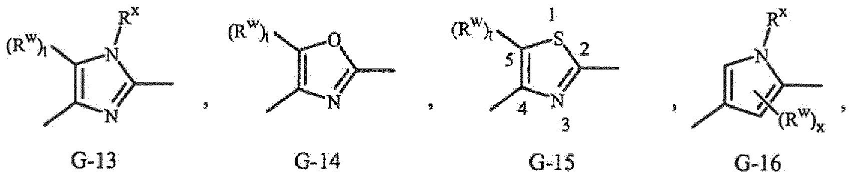 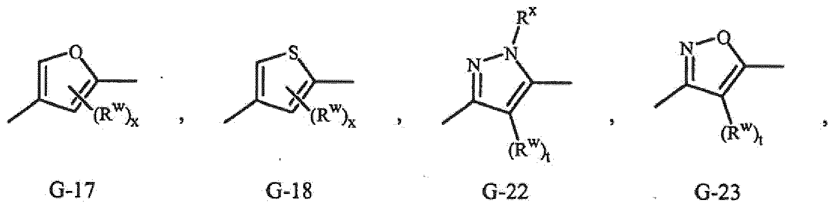 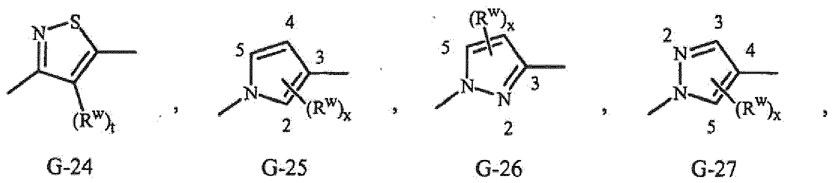 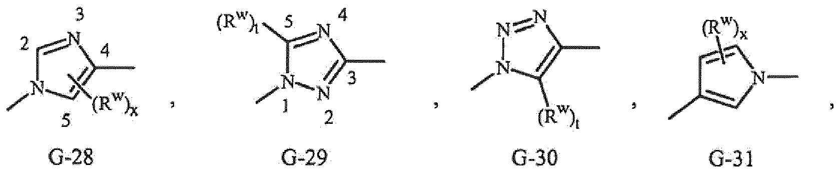 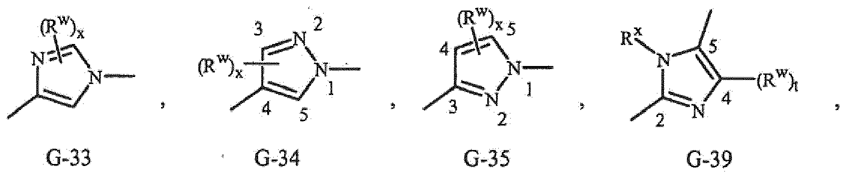 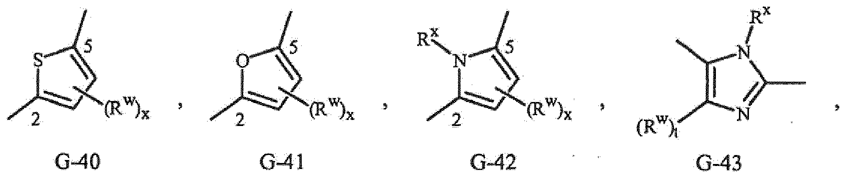 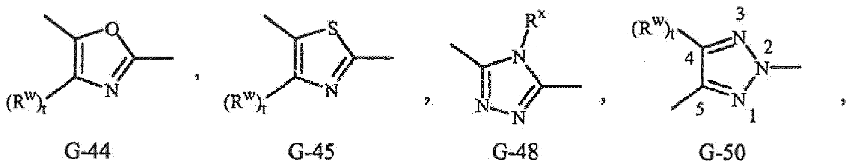 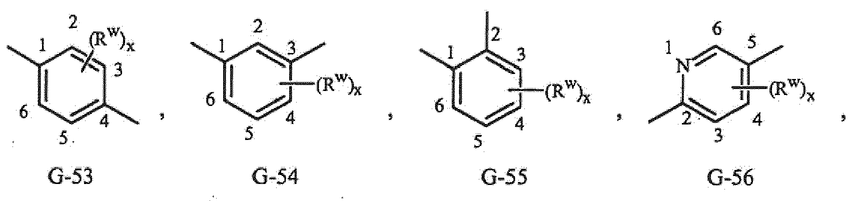 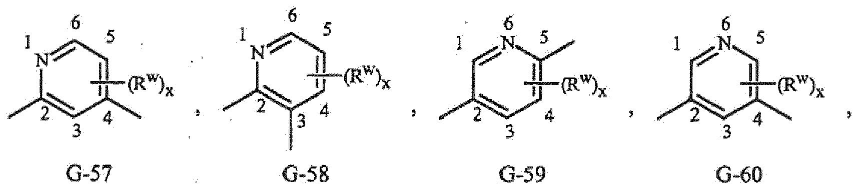 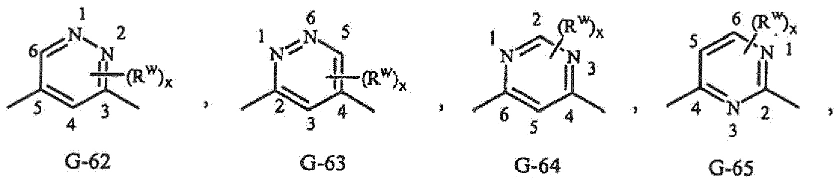 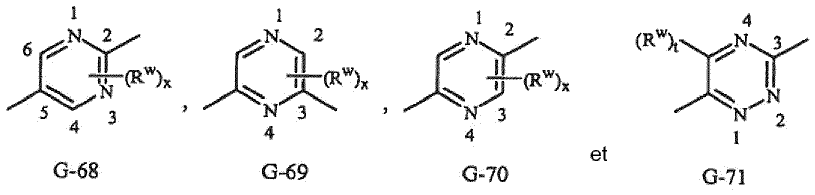 où la liaison se projetant vers la gauche est liée au cycle de pyridazinone de la Formule 1 et la liaison se projetant vers la droite est liée à J; x est un nombre entier choisi parmi 0 à 2; et t est un nombre entier choisi parmi 0 ou 1;
chaque R^{w} est indépendamment un halogène, un hydroxy, un alkyle C₁-C₆, un haloalkyle C₁-C₆, un cycloalkyle C₃-C₈, un alcoxy C₁-C₆, un haloalcoxy C₁-C₆, - O(CR¹¹R¹²)ₙG^{A} ou -(CR¹¹R¹²)ₙG^{A};
J est un cycle de phényle optionnellement substitué avec jusqu'à 3 substituants indépendamment choisis parmi R^{u};
chaque R^{u} est indépendamment un halogène, un cyano, un nitro, -CHO, - C(=O)OH, un alkyle C₁-C₆, un haloalkyle C₁-C₆, un cycloalkyle C₃-C₈, un halocycloalkyle C₃-C₈, un alcoxy C₁-C₆, un haloalcoxy C₁-C₆, un alkylamino C₁-C₆, un dialkylamino C₂-C₈ ou un phényle; et
W¹ est O.

4. Composé selon la revendication 3, dans lequel:
R¹ est H ou un alkyle C₁-C₆;
R² est H, Cl, CH₃, Et ou OMe;
R³ est H, CO₂-i-Pr ou CO-t-Bu;
G est choisi parmi G-12 à G-15, G-26 à G-29, G-34, G-35, G-54 et G-65;
chaque R^{w} est indépendamment un halogène, un alkyle C₁-C₆ ou -O(CR¹¹R¹²)ₙG^{A};
J est un phényle substitué avec un substituant choisi parmi R^{u}; et
chaque R^{u} est indépendamment un halogène, un alkyle C₁-C₆ ou un haloalkyle C₁-C₆.

5. Composé selon la revendication 4, dans lequel:
R¹ est CH₃;
R² est H;
G est choisi parmi G-12, G-15, G-26, G-28, G-29, G-34, G-35, G-54 et G-65;
chaque R^{w} est indépendamment CH₃ ou Et;
J est un phényle substitué à la position para avec un substituant choisi parmi R^{u}; et
chaque R^{u} est indépendamment un halogène, un alkyle C₁-C₃ ou un haloalkyle C₁-C₃.

6. Composé selon la revendication 5, dans lequel:
G est G-26;
x est 1;
R^{w} est positionné à la position 5 de G-26; et
chaque R^{u} est indépendamment Cl, Br ou CF₃.

7. Composé selon la revendication 1, qui est choisi dans le groupe constitué de:
5-hydroxy-2-méthyl-4-[5-méthyl-3-[4-(trifluorométhyl)phényl]-1H-pyrazol-1-yl]-3(2H)-pyridazinone,
4-[3-(4-chlorophényl)-5-méthyl-1H-pyrazol-1-yl]-5-hydroxy-2-méthyl-3(2H)-pyridazinone,
carbonate de 5-[3-(4-chlorophényl)-5-méthyl-1H-pyrazol-1-yl]-1,6-dihydro-1-méthyl-6-oxo-4-pyridazinyl-1-méthyléthyle,
4-[3-(4-bromophényl)-5-méthyl-1H-pyrazol-1-yl]-5-hydroxy-3(2H)-pyridazinone,
4-[5-éthyl-3-[4-(trifluorométhyl)phényl]-1H-pyrazol-1-yl]-5-hydroxy-2-méthyl-3(2H)-pyridazinone, et
4-[3-(4-chlorophényl)-5-éthyl-4-méthyl-1H-pyrazol-1-yl]-5-hydroxy-2-méthyl-3(2H)-pyridazinone.

8. Composition herbicide comprenant une quantité à effet herbicide d'un composé selon la revendication 1 et au moins un composant choisi dans le groupe constitué de tensioactifs, de diluants solides et de diluants liquides.

9. Composition herbicide comprenant une quantité à effet herbicide d'un composé selon la revendication 1, une quantité efficace d'au moins un ingrédient actif supplémentaire choisi dans le groupe constitué d'autres herbicides et d'antidotes d'herbicides, et au moins un composant choisi dans le groupe constitué de tensioactifs, de diluants solides et de diluants liquides.

10. Procédé pour lutter contre la croissance d'une végétation non désirée comprenant la mise en contact de la végétation ou de son environnement avec une quantité à effet herbicide d'un composé selon la revendication 1.
